# EUROPEAN PATENT APPLICATION

(11) **EP 2 336 166 A1**
(43) Date of publication of application: **22.06.2011**
(21) Application number: 10012959.2
(22) Date of filing: 12.10.2001
(51) Int. Cl.: C07K 14/705, A61K 38/17, A61K 31/7088, A61K 38/39, G01N 33/566, G01N 33/50, C12Q 1/68

(54) **Compositions that inhibit proliferation of cancer cells**

(30) Priority: 12.10.2000 US 239705 P; 24.10.2000 US 242812 P
(62) Divisional of application: 01977754.9
(71) Applicant: UNIVERSITY OF ROCHESTER, Rochester, NY 14642 (US)
(72) Inventor: Land, Hartmut, Rochester NY 14618 (US); Deleu, Laurent, Rochester NY 14618 (US)
(74) Representative: Leissler-Gerstl, Gabriele

(57) **Abstract**

The present invention relates to compositions and methods for reducing the proliferation of cancer cells through targeted interactions with integrin.

## Description

This application claims priority to United States Provisional Application No. 60/239,705 filed on October 12, 2000, entitled "Agents promoting apoptosis in cancer cells via interruption of oncogene-induced integrin signaling," which application is herein incorporated by reference in its entirety and to United States Provisional Application No. 60/242,812 filed on October 24, 2000 entitled, "Agents promoting apoptosis in cancer cells via interruption of oncogene-induced integrin signaling", and which application is herein incorporated by reference in its entirety.

### I. BACKGROUND OF THE INVENTION

In cancer cells multiple oncogenic lesions cooperate in malignant transformation. Such cooperation permits survival and proliferation of tumor cells in absence of contact with extra-oellular matrix (ECM), suggesting that tumor cell survival and proliferation have become independent of the engagement of integrin signaling by ECM.

Carcinogenesis is caused by multiple cooperating genetic lesions leading to a progressive deregulation of cellular signaling and cell cycle restriction point control. The mutations involved result in oncogene activation or loss of tumor-suppressor gene function. Typically, single oncogenes are insufficient to cause malignant transformation because they simultaneously induce signals stimulating and inhibiting cell growth. As a result cell proliferation remains restricted. In contrast, cooperating oncogenic lesions act in concert to disable such inhibitory signals while reinforcing the growth-promoting stimuli. The co-operation of oncogenic lesions involves integration of multiple signals converging on the regulation of cell cycle-dependent kinase complexes (Lloyd *et al.,* 1997; Perez-Roger *et al.,* 1999; Roper *et a*/*.,* 2001; Sewing *et al.,* 1997).

Disclosed herein are compositions and methods that show survival of various transformed cell types requires cell-autonomous (autocrine) integrin signaling activity. This activity is induced by cooperating oncogenic lesions and involves induction of integrin receptor and ligand components such as integrin alpha6 and integrin beta4, and laminin5-gamma2 chains. Blocking of integrin or the laminin ligand function induces rapid apoptosis of the transformed cells, even when growing in presence of ECM. In contrast, normal cells remain viable when exposed to the same treatment.

The disclosed compositions and methods are related to the cooperation of oncogenic lesions controlling the ability of transformed cells to proliferate in the absence of contact with the extra-cellular matrix (ECM). As taught herein, oncogenes cooperate to promote a cell-autonomous (autocrine) integrin signaling loop that proves essential for the survival of various transformed cell types. As this signaling loop is not established in corresponding normal cells, the signaling components of this loop constitute attractive targets for cancer therapy.

### II. SUMMARY OF THE INVENTION

In accordance with the purposes of this invention, as embodied and broadly described herein, this invention, in one aspect, relates to compositions and methods related to integrin mediated cancer cell growth.

Additional advantages of the invention will be set forth in part in the description which follows, and in part will be obvious from the description, or may be learned by practice of the invention. The advantages of the invention will be realized and attained by means of the elements and combinations particularly pointed out in the appended claims. It is to be understood that both the foregoing general description and the following detailed description are exemplary and explanatory only and are not restrictive of the invention, as claimed.

### III. BRIEF DESCRIPTION OF THE DRAWINGS

The accompanying drawings, which are incorporated in and constitute a part of this specification, illustrate several embodiments of the invention and together with the description, serve to explain the principles of the invention.

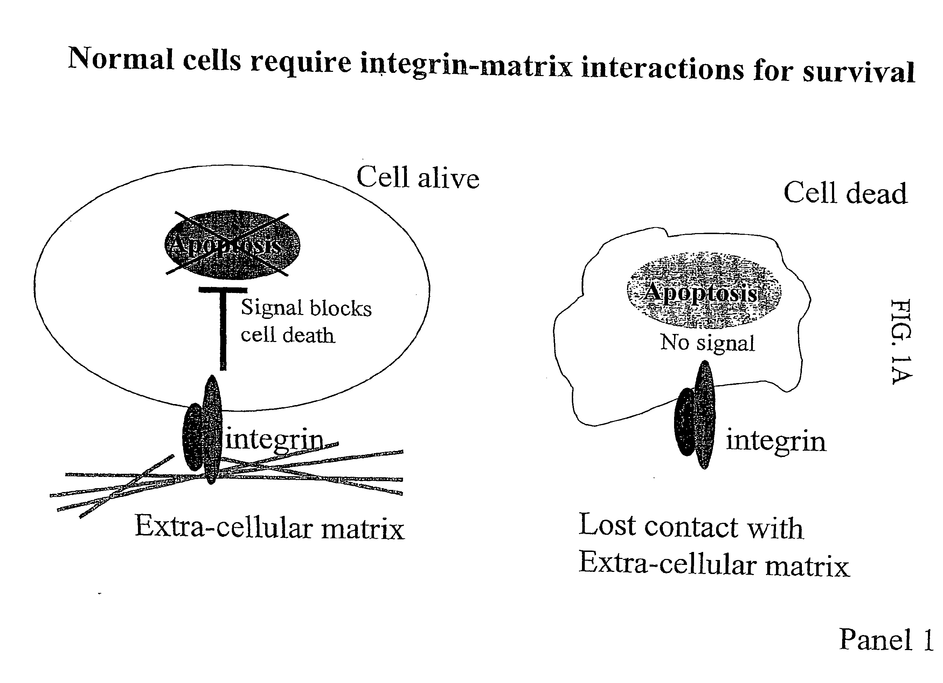

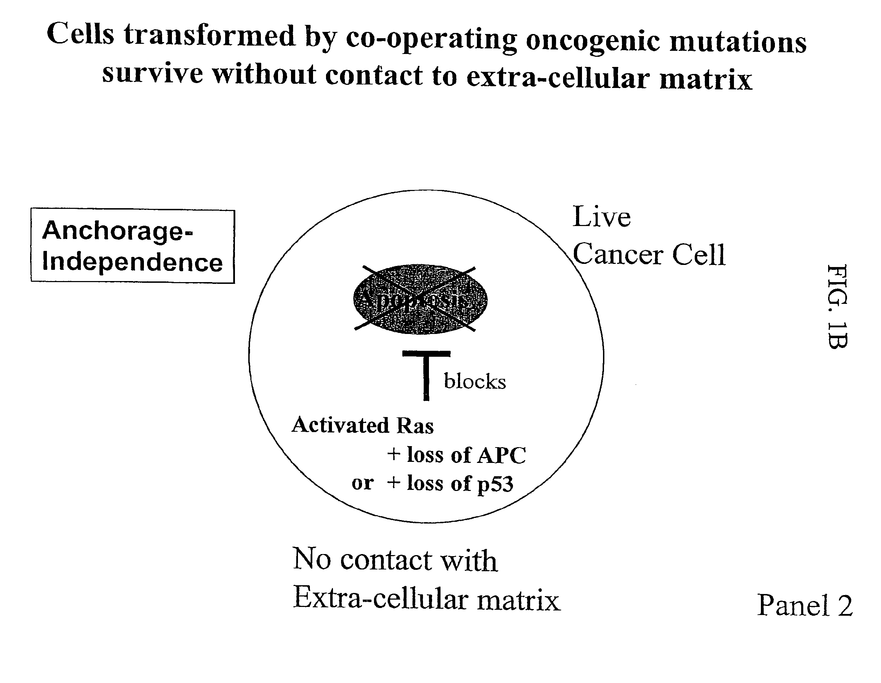

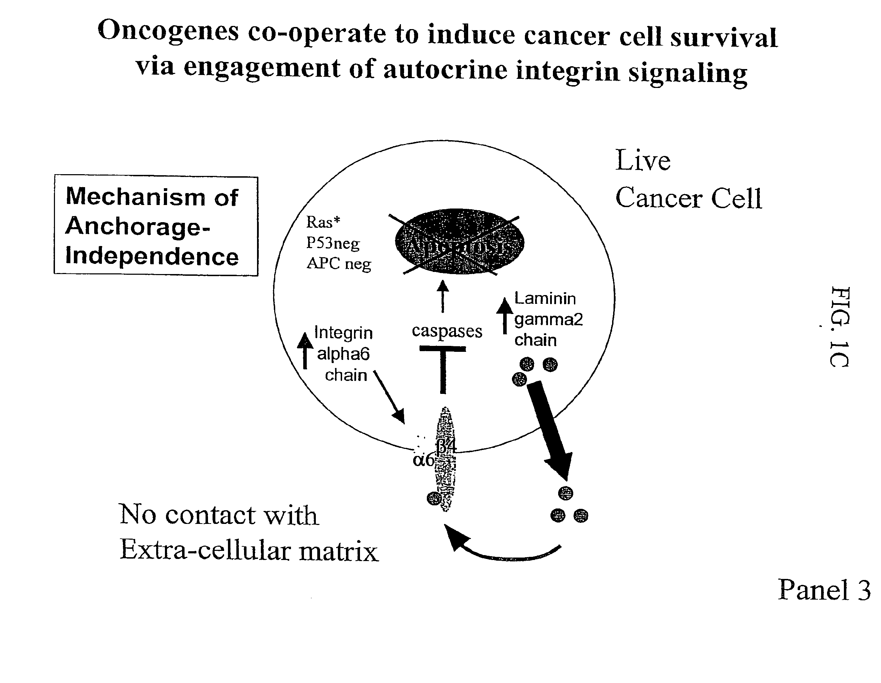

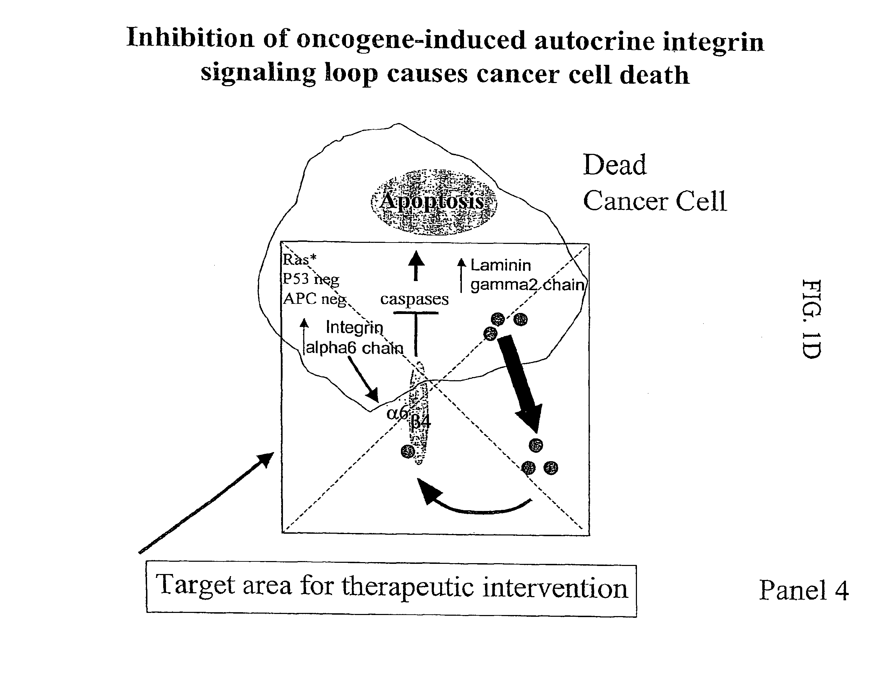

Figure 1 shows a series of schematics representative of the disclosed relationships and compositions. In normal cells integrin receptors signal to suppress programmed cell death (apoptosis) when engaged by appropriate extra-cellular matrix (ECM) ligands. When receptor-ligand interaction is lost, the cells undergo apoptosis due to the lack of survival signals (Figure 1-. Panel 1). In cancer cells multiple oncogenic lesions cooperate to cause malignant transformation. Such cooperation permits survival and proliferation of tumor cells independent of integrin receptor-ECM interactions. This property has been termed anchorage-independence (Panel 2). We have discovered the mechanistic basis of anchorage-independence. The transformed cells replace the requirement for ECM-dependent signaling with a surrogate integrin signaling loop on which they rely for survival. In colonic epithelial cells, activation of Ras in combination with APC (adenomatous polyposis coli) or p53 mutations leads to induction of integrin receptor and ligand components such as integrin alpha6 and laminin gamma2 chains. As a consequence, laminin-dependent activation of alpha6/beta4 integrin receptors signals to inhibit caspase activity and thus to suppress apoptosis (Panel 3). Ablation of integrin alpha6, laminin ligand function or alpha6/beta4 integrin receptor function induces apoptosis of the transformed cells, even when growing in the presence of ECM, In contrast, non-transformed control cells remain viable when exposed to the same treatment, indicating that the dependence of the transformed cells on autocrine integrin signaling may be a particular feature of the cancer cell phenotype. The essential role of alpha6 integrin extends to various transformed cell types including mesenchymal and SW480 human colon carcinoma cells. Thus inhibition of laminin/alpha6 integrin-mediated signaling is an important method to induce cancer cell-specific death in a variety of cell types (Panel 4).

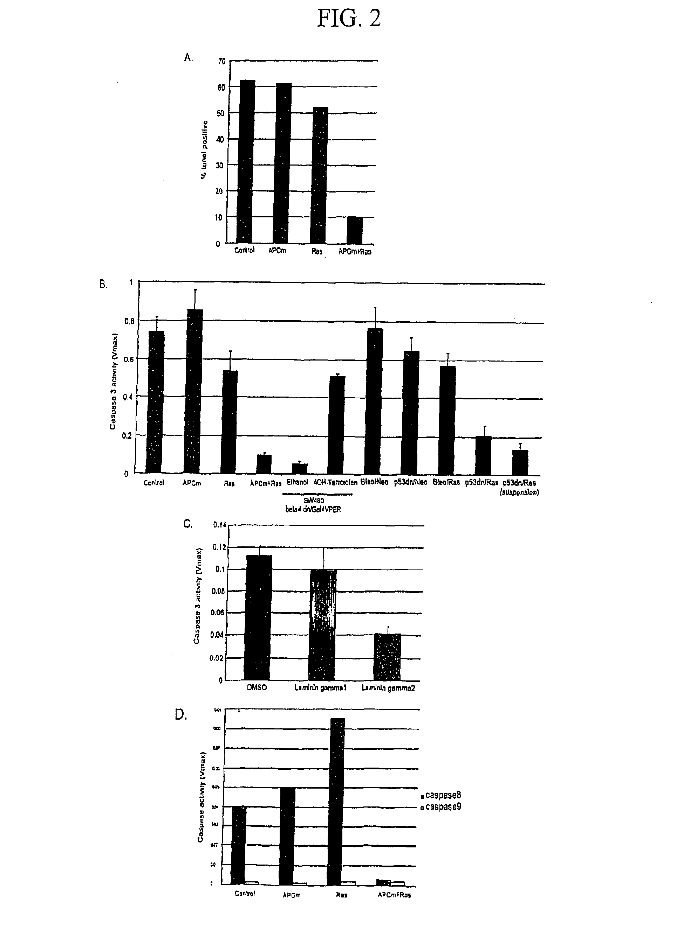

Figure 2 shows that oncogene cooperation protects cells from apoptosis. Figure 2A shows control, APCm, Ras, APCm+Ras cells that were detached from collagen IV substrates with Trypsin/EDTA and kept in suspension at 2x10⁵ cells/ml in RPMI 10%FCS for 12 h at 39°C. Subsequently TUNEL analysis was performed on poly-lysine treated slides. The percentage of tunel-positive cells was determined by immunofluorescence microscopy. Figure 2B shows control, APCm, Ras, APCm Ras, SW480 cells were detached and maintained in suspension as described in (A). SW480 beta4 dn/Gal4VPER cells, express a 4OH-tamoxifen-inducible dominant-negative form of the beta4 integrin. Cells were pelleted and protein extracts were prepared in 300 µl of 50 mM Tris-HCl, pH 7.4; 1% NP40 ; 0.25% sodium deoxycholate; 150 mM NaCl ; 1 mM EGTA. 200 µl of extracts were incubated for 10 min with 2 µl of Caspase 3 fluorometric Substrate (Upstate Biotechnology). Vmax of caspase activity was determined by measuring the fluorescence at 460 nm after excitation at 380 nm for 1 h. Figure 2C shows laminin gamma1 and gamma2-specific peptides were added to APCmin+Ras cells at the concentration of 100µg/ml. Caspase activity was measured as in (B). Figure 2D shows caspase 8 and caspase 9 activities were measured as in (B) using caspase 8 and caspase 9-specific fluorimetric substrates (Upstate Biotechnologies).

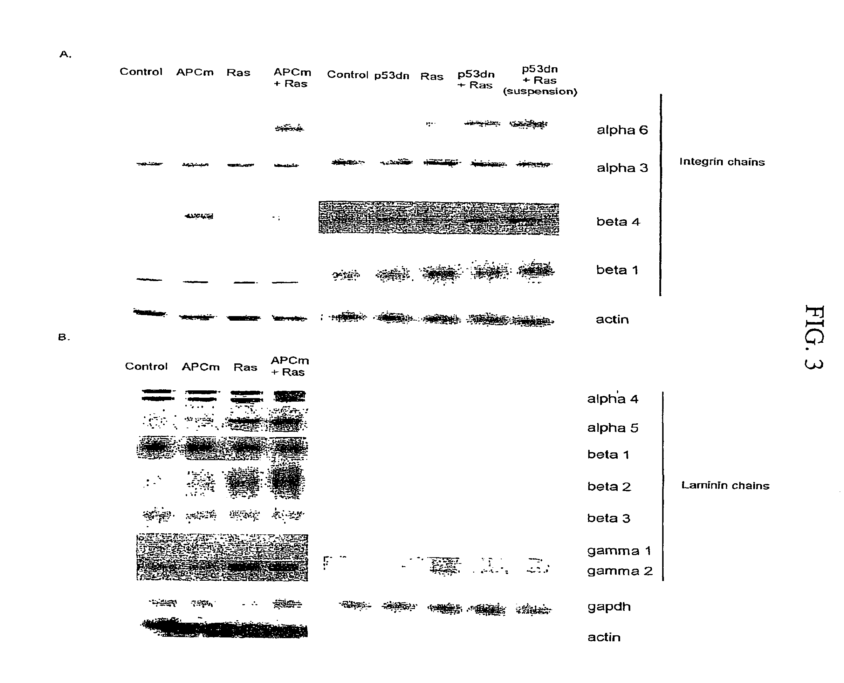

Figure 3 shows alterations of integrin and laminin expression profiles in malignant cell transformation. Figure 3A shows integrin expression and figure 3B shows laminin expression. The indicated cell populations were cultured on collagen IV-coated dishes at 39°C in RPMI 10% FCS. Total RNA was extracted from 10⁶ cells for each sample and used for RT-PCR (laminins, alpha4, gamma2 and GAPDH) or RNase protection. For RT-PCR, cDNA was subjected to 28 cycles (linear range) of PCR amplification. PCR products were analyzed on a 2% agarose gel. For RNase protection, 10 µg of total RNA was used per reaction. Products were resolved on a 4.5% polyacrylamide/10M urea gel.

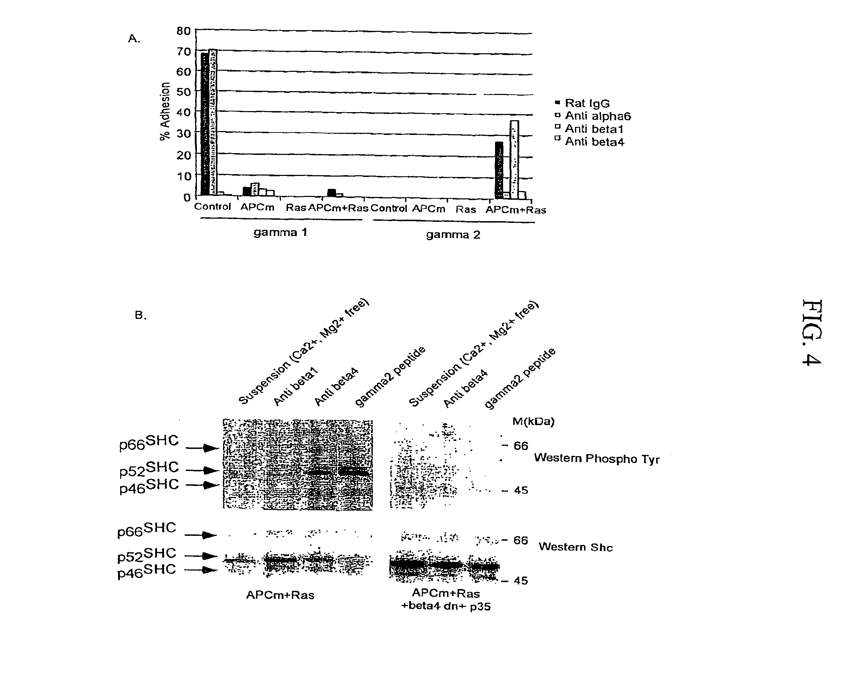

Figure 4 shows alpha6/beta4 integrin is engaged by the laminin gamma2 chain to activate Shc (a src homology domain containing protein). Figure 4A shows cells detached from collagen IV coated dishes with 3mM EDTA (in PBS) were incubated on ice with the indicated antibodies for 1 h. Cells were then plated on 96well dishes coated with gamma1 or gamma2-specific peptides and were permitted to attach for 30 min in RPMI medium at 39°C. After incubation, wells were washed with RPMI. The percentage of attached cells per well was measured by hexoaminidase activity after lysis of the cells in the well and incubation with the substrate p-nitrophenol-N-acetyl-β-D-glucosaminide for 5 h at 37°C. Figure 4B shows cells indicated were detached as in (A) and resuspended at 10⁷ cells/ml in PBS. After 1 h of incubation on ice, cells were pelleted by centrifugation 5 min at 900 rpm and resuspended in RPMI containing the phosphatase inhibitor pervanadate at 10µM. The cells were incubated with beta 1 and beta4 integrin antibodies as well as the laminin gamma2-specific peptide for 40 min. Protein extracts were prepared and subjected to an immuno-precipitation with an anti-She antibody. Phospho-tyrosine was detected with a phopho-tyrosine-specific antibody in IP-westem-blots (upper panels). The levels of Shc protein were monitored with a Shc-specific antibody reusing the same membrane (lower panels).

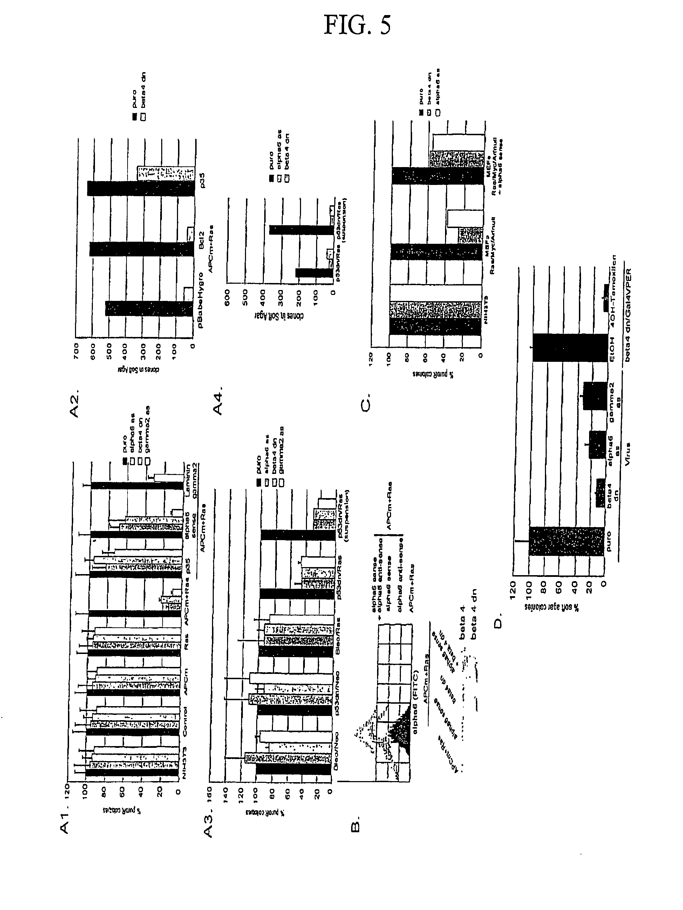

Figure 5 shows integrin alpha6/beta4 and Laminin gamma2 chain expression is essential for survival of transformed cells. Figure 5A1 shows the indicated cell populations were infected at an MOI of 2 with recombinant retroviruses expressing anti-sense RNA for alpha6 integrin, the gamma2 laminin chain or a beta4 integrin dominant-negative mutant together with a puromycin resistance gene. After 2 weeks of selection with puromycin, colonies were stained with Giemsa and counted. APCm+Ras alpha6 and APCm+Ras p35 cells express the alpha6 integrin chain and the anti-apoptotic baculovirus p35, respectively. APCm+Ras cells were also plated on dishes precoated with a laminin gamma2-specific peptide. Figure 5A2 shows APCm+Ras hygro, APCm+Ras Bcl2 and APCm+Ras p35 cells express the hygromycin resistance marker, and the anti-apoptotic proteins Bcl2 or p35, respectively. The cells were infected at an MOI of 2 with retroviruses carrying the beta4 integrin dominant-negative mutant and a puromycin resistance gene, or the puromycin resistance gene alone. Cells (10⁵) were maintained in soft agar at 39°C for two weeks after which macroscopically visible colonies were counted. Figure 5A3 shows the indicated cell populations were infected at an MOI of 2 with recombinant retroviruses, drug-selected and maintained as described in A1. Figure 5A4 shows the indicated cell populations were infected at an MOI of 2 with recombinant retroviruses expressing anti-sense RNA for alpha6 integrin, the beta4 integrin dominant-negative mutant or the puromycin resistance marker. Cells (10⁵) were maintained in soft agar and evaluated as described in A2. Figure 5B shows the effect of the alpha6 anti-sense RNA expression on the cell surface expression of alpha6 integrin was monitored by FACS analysis using an anti-alpha6 rat monoclonal antibody and a FITC conjugated goat anti-rat antiserum as the secondary antibody (upper panel). Expression of the beta4 dominant-negative mutant was confirmed by RNAse protection. A probe overlapping the c-terminal end of the beta4 dominant negative mRNA was used to measure beta4 integrin and beta4 dn expression in the same sample (lower panel). Figure 5C shows arf null mouse embryo fibroblasts (MEFs) that were infected with retroviruses expressing the oncogenes Myc and Ras. Cells were then additionally infected with a retroviruses expressing anti-sense RNA for alpha6 integrin or the beta4 integrin dominant-negative mutant as shown in (A). MEFS Ras/Myc/Arfnull+alpha6 sense express the alpha6 integrin chain. Figure 4D shows SW480 human colon carcinoma cells that were infected as described in (A) using VSV pseudo-typed viruses. Infected cells were selected with puromycin in soft agar. Clones were counted 2 weeks after selection, SW480 beta4 dn/Gal4VPER cells, express a 40H-tamoxifcn-inducible dominant-negative form of the beta4 integrin. The cells (10⁵) were maintained in soft agar for 2 weeks in presence or absence of 40H-tamoxifen.

### IV. DETAILED DESCRIPTION

The present invention may be understood more readily by reference to the following detailed description of preferred embodiments of the invention and the Examples included therein and to the Figures and their previous and following description.

Before the present compounds, compositions, articles, devices, and/or methods are disclosed and described, it is to be understood that this invention is not limited to specific synthetic methods, specific recombinant biotechnology methods unless otherwise specified, or to particular reagents unless otherwise specified, as such may, of course, vary. It is also to be understood that the terminology used herein is for the purpose of describing particular embodiments only and is not intended to be limiting.

### A. Definitions

As used in the specification and the appended claims, the singular forms "a," "an" and "the" include plural referents unless the context clearly dictates otherwise. Thus, for example, reference to "a pharmaceutical carrier" includes mixtures of two or more such carriers, and the like.

Ranges may be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint.

In this specification and in the claims which follow, reference will be made to a number of terms which shall be defined to have the following meanings:
"Optional" or "optionally" means that the subsequently described event or circumstance may or may not occur, and that the description includes instances where said event or circumstance occurs and instances where it does not.
"Primers" are a subset of probes which are capable of supporting some type of enzymatic manipulation and which can hybridize with a target nucleic acid such that the enzymatic manipulation can occur. A primer can be made from any combination ofnucleotides or nucleotide derivatives or analogs available in the art which do not interfere with the enzymatic manipulation.
"Probes" are molecules capable of interacting with a target nucleic acid, typically in a sequence specific manner, for example through hybridization. The hybridization of nucleic acids is well understood in the art and discussed herein. Typically a probe can be made from any combination of nucleotides or nucleotide derivatives or analogs available in the art.

### B. Compositions and methods

Disclosed are compositions and methods related to integrins and integrin signaling. It is shown herein that integrin alpha6, integrin beta4, and laminin5, through at least up regulation of the beta and gamma chains of Iaminin5, are upregulated in cancer cells. Integrin alpha6 and integrin beta4 interact to form the integrin receptor, A6B4. The integrin receptor A6B4 specifically interacts with laminin5 and laminin5 specifically interacts with A6B4. Furthermore, it is shown that interference with the production or function of alpha6, beta4, or the laminin gamma2 chain not only prevents proliferation of the cancer cells, dependent on the upregulation of alpha6, beta4, and the laminin gamma2 chain, but that this kills the cancer cells as well. Alpha6 and beta4 signaling occur through the integrin receptor A6B4. Thus, interference with the formation of A6B4, will interfere with the function of A6B4, for example, the signaling of A6B4. Thus, disclosed are compositions and methods that interfere with the function of alpha6, beta4, laminin5, the laminin gamma2 chain, or A6B4. Also disclosed are compositions and methods that interfere with the function of molecules involved with the signal transduction that is connected to either alpha6, beta4, laminin5, the laminin gamma2 chain, or A6B4. Also disclosed are methods for reducing the proliferation of cancer cells, as well as methods of killing cancer cells that involve using the compositions disclosed herein that interfere, reduce, or eliminate the function or the alpha6, beta4, laminin5, the laminin gamma2 chain, or A6B4 function.

Disclosed herein is a relationship between two types of molecules in a cancer cell. The first type of molecule is an integrin receptor, composed of integrins, and the second type of molecule is a ligand that interacts with the integrin receptor, through the integrins. There are specificities that exist between the integrin receptors and their ligands. One aspect, disclosed herein is that when a cell goes from a non-cancerous state to a cancerous state, there is a co-uprcgulation of both the ligand (or parts of the ligand, such as subunits) and the cognate integrin receptor. The co-upregulation of both types of molecules creates an autocrine loop situation, wherein the signaling pathways controlled by the integrin receptor become autonomously activated, rather than exogenously activated, as would normally occur. The upregulation of both types of molecules creates a more fully transformed cellular phenotype in which cancer cell survival depends on the autocrine loop. Now therapeutic activities can target both points in the autocrine loop. Specific examples, of this co-upregulation in cancer cells are disclosed herein. For example, laminin 5 (both the beta and gamma2 chains are upregulated) and integrin receptor alpha6beta4; laminin 10/1 and the integrin receptors alpha6beta1 and alpha3beta1.

As the Examples herein indicate which integrins, which integrin receptor, and which integrin ligand are involved in conferring cancer cells ability to grow in the absence of extra cellular matrix (ECM), also disclosed are methods using these integrins, integrin receptors and ligands to identify molecules that interact with them and/or interfere with their function.

### 1. Compositions

Disclosed are the components to be used to prepare the disclosed compositions as well as the compositions themselves to be used within the methods disclosed herein. These and other materials are disclosed herein, and it is understood that when combinations, subsets, interactions, groups, etc. of these materials are disclosed, while specific reference of each various individual and collective permutation of these compounds may not be explicitly disclosed, each is specifically contemplated and described herein. For example, if a particular beta4 or alpha6 is disclosed and discussed and a number of modifications that can be made to a number of molecules including the modifications to beta4 or alpha6 are discussed, specifically contemplated is each and every combination and permutation of these modifications and the modifications that are possible unless specifically indicated to the contrary. Thus, if a class of molecules A, B, and C are disclosed as well as a class of molecules D, E, and F and an example of a combination molecule, A-D is disclosed, then even if each is not individually recited, each is individually and collectively contemplated. Thus, combinations, A-E, A-F, B-D, B-E, B-F, C-D, C-E, and C-F are considered disclosed. Likewise, any subset or combination of these is also disclosed. Thus, for example, the sub-group of A-E, B-F, and C-E would be considered disclosed. This concept applies to all aspects of this application including, but not limited to, steps in methods of making and using the disclosed compositions. Thus, if there are a variety of additional steps that can be performed it is understood that each of these additional steps can be performed with any specific embodiment or combination of embodiments of the disclosed methods.

### a) Integrins and their ligands

For proper embryonic development, tissue homeostasis, and wound healing, cell proliferation must be tightly regulated, both in space and over time. In particular, a cell must be able to sense its relationship to other cells and the extra-cellular matrix (ECM) and convert these positional cues into biochemical signals affecting the regulation of proliferation. Because of their ability to couple the recognition of positional cues to the activation of intracellular signaling pathways, adhesion receptors, such as integrins and cadherins, are likely to be necessary to achieve this goal.

The integrins mediate cell adhesion primarily by binding to distinct, although overlapping, subsets of ECM proteins. Normal cells require contact with serum-derived ECM components for proliferation, differentiation and survival (e.g. Clark and Brugge, 1995; Lin and Bissell, 1993; Parise *el al.,* 2000), a phenomenon called anchorage-dependence. This involves signaling through integrin receptors (Hynes, 1992). Fibronectin and laminin as well as other ECM proteins are known to act as ligands for integrin receptors (Akiyama *et al.,* 1990). Integrins are transmembrane proteins forming alpha-beta chain heterodimers. Alpha and beta chain integrins are members of distinct gene families. The ligand binding specificity of the hetero dimers is determined by specific combinations of alpha and beta chain gene family members (Hynes, 1992). Ligand binding triggers signaling of integrin receptors through the cytoplasmic tail of the beta chain via interaction with various signaling components.

Integrins activate common as well as subgroup-specific signaling pathways (Clark and Brugge, 1995; Giancotti and Ruoslahti, 1999). In particular, while most integrins activate focal adhesion kinase (FAK), the all, α5β1, avβ3 and α6β4 integrins are coupled to the Ras-extracellular signal-regulated kinase (ERK) signaling pathway by Sho (Mainiero *et al.,* 1997; Mainiero *et al.,* 1995; Wary *et al.,* 1996). She is an SH2-PTB domain adapter protein expressed in three forms, p46, p52 and p66, two of which (p46 and p52) link various tyrosine kinases to Ras by recruiting the Grb2/SOS complex to the plasma membrane (Pawson and Scott, 1997). Upon activation by SOS, Ras stimulates a kinase cascade culminating in the activation of the mitogen-activated protein kinase (MAPK) ERK (Marshall, 1995). ERK phosphorylates ternary complex transcription factors, such as Elk-1 and Sap-1/2, and promotes transcription of the immediate-early gene Fos (Treisman, 1996). In primary endothelial cells and keratinocytes, mitogens and Shc-linked integrins cooperate, in a synergic fashion, to promote transcription from the Fos promoter. Accordingly, ligation of integrins linked to Shc enables these cells to progress through G1 in response to mitogens, whereas ligation of other integrins results in growth arrest, even in the presence of mitogens (Mainiero *el al.,* 1997; Wary *et al.,* 1996). Sho is like a binary switch controlling cell cycle progression in response to the ECM. Moreover, integrin receptors have been shown to induce intracellular signaling leading to AKT activation supporting cell survival (Lee and Juliano, 2000).

In contrast with normal cells, cancer cells generally are able to survive and proliferate in the absence of anchorage to ECM. (Giancotti and Mainiero, 1994), suggesting that tumor cell survival and proliferation have become independent of the engagement of integrin signaling through ECM.

Proliferation in the absence of anchorage to ECM of secondary rat embryo fibroblasts requires the cooperation of Ras and Myc or Ras and adenovirus Ela oncogenes (Land et al., 1983; Ruley, 1983). Similarly, murine colonic epithelial cells require both activated Ras and mutation of the adenomous polyposis coli gene (APCmin) (D'Abaco et al., 1996) in order to proliferate in suspension.

Integrins are a large family of cell surface receptor molecules that function to mediate interactions between cells and between cells and the extracellular matrix. Integrin receptors are heterodimers composed of two subunits, an alpha integrin and a beta integrin. The heterodimer forms, is expressed on the cell surface, and acts to transmit signals obtained from interactions with the extracellular matrix or other cells, through the cellular membrane and into the cytosol of the cell. The signal transduction that takes place occurs because of ligand interactions with the receptor. Integrin receptors can have a number of ligands, including collagens, fibronectins, and laminins.

There are currently at least 18 different alpha integrins and at least 8 different beta integrins that have been shown to form at least 24 different alphabeta heterodimers. Certain integrins, such as beta 1, interact in a number of different heterodimers, but many subunits only form a single heterodimer, either because of structural constraints on their interactions, or cellular expression patterns that provide only a limited number of potential dimer partners. Disclosed herein are specific relationships that occur between a subset of integrins, integrin receptors, and their ligands. The disclosed relationships, revolve around the alpha6beta4 receptor, formed by the alpha6 and beta4 integrins. Of particular interest is the relationship between the ligand for the alpha6beta4 receptor, laminin5. The laminins are made up of 3 chains, an alpha chain, a beta chain, and a gamma chain. The specificity of the interaction between laminin5 and alpha6beta4 receptor is controlled by the gamma chain. Laminin5 contains a gamma2 chain which only interacts with the alpha6beta4 integrin receptor.

Integrin alpha6 has seven amino-terminal repeating segments that may fold into a seven unit beta-propeller, five n-terminal FG-GAP domains and three divalent cation sites. The transmembrane domain is followed by a short cytoplasmic tail, that is alternatively spliced in A and B forms. The alpha6 integrin chain also shows alternative splicing between repeat units III and IV, resulting in the presence or absence of Exon X2. Integrin alpha6 is processed into a heavy and a light chain that are disulphide linked. A representative allele of the human alpha6 cDNA is set forth in SEQ ID NO:1. It is understood that the disclosed functional domains as well as the others contained within alpha6 are considered separately disclosed as discreet fragments of the alpha6 protein as well as the nucleic acid that encodes them.

Integrin beta4 contains a MIDAS-like motif and four oysteine-rich repeats, three EGF-like domains in the N-terminal extracellular domain, a trans-membrane region and a long cytoplasmic tail containing two pairs of fibronectin Type III repeats. The latter are connected by a variable segment that may undergo alternative splicing. Integrin beta4 also undergoes proteolytic processing in its cytoplasmic tail, causing the 200kD mature form to be converted to 165 and 130kD fragments. A representative allele of the human beta4 cDNA is set forth in SEQ ID NO:5. It is understood that the disclosed functional domains as well as the others contained within beta4 are considered separately disclosed as discreet fragments of the beta4 protein as well as the nucleic acid that encodes them.

Laminin5 is composed of the laminin chains alpha3, beta3 and gamma2. Laminin5 can may contain either the shorter laminin alpha3A chain or the longer alpha3B chain. Laminin5 can also be trimmed by proteolytic processing of the N-terminal portion of its alpha3A chain and the N-terminal portion of the gamma2 chain. The Laminin gamma2 chain contains at least six laminin EGF-like domains (Domains III and V) with an embedded laminin B domain (Domain IV) within the N-terminal half. The c-terminal tail contains a coiled-coil domain. The N-terminal processed portion of the gamma2 chain is sufficient to bind to and activate the integrin alpha6/beta4 receptor. A representative allele of the human laminin5-gamma2 cDNA is set forth in SEQ ID NO: 13. It is understood that the disclosed functional domains as well as the others contained within the laminin5 protein and laminin5-gamma2 protein are considered separately disclosed as discreet fragments of the laminin5 protein and laminin5-gamma2 protein as well as and the nucleic acid that encodes them.

Disclosed are compositions and methods for inhibiting integrin signaling, for example, integrin signaling dependent on alpha6 and beta4 integrins. For example, compositions and methods that inhibit integrin receptor signaling from, for example, the alpha6beta4 integrin receptor are disclosed. It is also understood that the integrin receptor signaling can be affected by, for example, interfering with a molecule, such as a ligand for the integrin receptor, or a downstream signaling molecule of the integrin receptor in a way that prevents the integrin receptor signal from being fully propagated. It is understood that the compositions and methods for inhibition of integrin signaling and function can be any composition or method that ultimately inhibits the cell proliferation in which the integrin is expressed, by for example killing the cell. It is understood that the compositions and methods typically can fall into three basic non-limiting classes of function regulators, which are discussed herein.

### (1) Classes

### (a) Production regulators

Production regulators is a broad class of integrin function regulators that are directed at the production of the target integrin, by for example, preventing mRNA synthesis or expression of the target integrin, or by causing mRNA degradation of the target integrin which inhibits the translation of the target intcgrin. While production regulators, can be any type of molecule targeting any point in the integrin production pathway, typically these types of compositions will target either the mRNA expression or the protein expression of the integrin. For example, if beta4 integrin, alpha6 integrin, or the gamma2 subunit of laminin5, which has been shown herein to be upregulated in cancer cells and which causes the cancer cell to be able to live in the absence of the ECM, was the target integrin, a typical production regulator of beta4 integrin, alpha6 integrin, or the gamma2 subunit of laminin5 would be for example, an antisense molecule that targeted the mRNA of beta4 integrin, alpha6 integrin, or the gamma2 subunit of laminin5. It is also understood that a production regulator could also target any molecule that is disclosed herein, or is within the signaling chain associated with a target integrin or integrin receptor. For example, the inhibition of the production of the ligand for a target integrin receptor is one way of inhibiting integrin function of that receptor. Thus, production regulators can either inhibit or enhance integrin production.

### (b) Integrin to integrin regulators

Another type of integrin function regulator is an integrin-integrin regulator. This type of function regulator, typically prevents integrins from interacting to form a functional integrin receptor. For example, an integrin to integrin regulator could be a composition that would interact with beta4 in a way that would prevent beta4 from interacting with alpha6 to form the alpha6beta4 integrin receptor or it could be a composition that would interact with alpha6 in a way that would prevent alpha6 from interacting with beta4 to form the alpha6beta4 integrin receptor. It is also contemplated that the function regulators of the integrin to integrin interaction can affect the signaling pathways dependent on integrin receptors containing alph6 or beta4 integrins. It is not required that an integrin to integrin regulator prevent an integrin from interacting with all of the possible integrin partners it could interact with, just that it prevent the interaction of the target integrin with another specific integrin. For example, the integrin alpha6 interacts with beta1 integrin and beta4 integrin. In certain embodiments. the compositions interfere with alpha6-beta4 interactions but do not interfere with alpha6-beta interactions.

### (c) Integrin to other molecule regulators

The third class of function regulators are the integrin to other molecule regulators. These compositions are designed to specifically interfere with molecules such as small molecule ligands or other proteins that interact with the integrin or integrin receptor. For example, an integrin to other molecule regulator might target a ligand for a particular integrin receptor, such as the alpha6beta4 receptor. The ligand for the alpha6beta4 receptor is laninin5 or the laminin gamma2 chain. Compositions that interact with laminin5 such that laminin5 or the laminin gamma2 chain interactions with alpha6beta4 are inhibited or reduced are specifically contemplated herein. Likewise, there are other molecules, such as She molecules, that also interact with integrins, such as the alpha6beta4 receptor. Compositions that specifically interact with the She molecules such that they prevent the appropriate interactions between the She molecule and the alpha6beta4 receptor are disclosed.

### (2) Types

Just as there are different general classes of molecules that can regulate (such as by inhibition) the function of the disclosed integrins and integrin receptors, so to there are many different types of molecules that perform that regulation. For example, any molecule that can perform the regulation of for example, the disclosed integrins, integrin receptors, or signaling pathways produced by the disclosed integrins and integrin receptors are contemplated. For example, antibodies or small molecules which inhibit the disclosed compositions are herein disclosed. Also disclosed are, for example, functional nucleic acids, such as ribozymes or antisense molecules that can inhibit the disclosed integrin function in a variety of ways. A non-limiting list of exemplary molecules is discussed herein.

### (a) Antibodies

Antibodies can be used to regulate, for example, the function of the disclosed integrins and integrin receptors, molecules that interact with the disclosed integrin receptors, and molecules in the signaling pathways of the disclosed integrin receptors.

As used herein, the term "antibody" encompasses, but is not limited to, whole immunoglobulin (i.e., an intact antibody) of any class. Native antibodies are usually heterotetrameric glycoproteins, composed of two identical light (L) chains and two identical heavy (H) chains. Typically, each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has at one end a variable domain (V(H)) followed by a number of constant domains. Each light chain has a variable domain at one end (V(L)) and a constant domain at its other end; the constant domain of the light chain is aligned with the first constant domain of the heavy chain, and the light chain variable domain is aligned with the variable domain of the heavy chain. Particular amino acid residues are believed to form an interface between the light and heavy chain variable domains. The light chains of antibodies from any vertebrate species can be assigned to one of two clearly distinct types, called kappa (k) and lambda (I), based on the amino acid sequences of their constant domains. Depending on the amino acid sequence of the constant domain of their heavy chains, immunoglobulins can be assigned to different classes. There are five major classes of human immunoglobulins: IgA, IgD, IgE, IgG and IgM, and several of these may be further divided into subclasses (isotypes), e.g., IgG-1, IgG-2. IgG-3, and IgG-4; IgA-1 and IgA-2. One skilled in the art would recognize the comparable classes for mouse. The heavy chain constant domains that correspond to the different classes of immunoglobulins are called alpha, delta, epsilon, gamma, and mu, respectively.

Antibodies can be either polyclonal or monoclonal. Polyclonal antibodies, typically are derived from the serum of an animal that has been immunogenically challenged, and monoclonal antibodies are derived as discussed herein.

The term "variable" is used herein to describe certain portions of the variable domains that differ in sequence among antibodies and are used in the binding and specificity of each particular antibody for its particular antigen. However, the variability is not usually evenly distributed through the variable domains of antibodies. It is typically concentrated in three segments called complementarity determining regions (CDRs) or hypervariable regions both in the light chain and the heavy chain variable domains. The more highly conserved portions of the variable domains are called the framework (FR). The variable domains of native heavy and light chains each comprise four FR regions, largely adopting a b-sheet configuration, connected by three CDRs, which form loops connecting, and in some cases forming part of, the b-sheet structure. The CDRs in each chain are held together in close proximity by the FR regions and, with the CDRs from the other chain, contribute to the formation of the antigen binding site of antibodies (see Kabat E. A. et al., "Sequences of Proteins of Immunological Interest," National Institutes of Health, Bethesda, Md. (1987)). The constant domains are not involved directly in binding an antibody to an antigen, but exhibit various effector functions, such as participation of the antibody in antibody-dependent cellular toxicity.

As used herein, the term "antibody or fragments thereof' encompasses chimeric antibodies and hybrid antibodies, with dual or multiple antigen or epitope specificities, and fragments, such as F(ab')2, Fab', Fab and the like, including hybrid fragments. Thus, fragments of the antibodies that retain the ability to bind their specific antigens are provided. For example, fragments of antibodies which maintain EphA2 binding activity are included within the meaning of the term "antibody or fragment thereof." Such antibodies and fragments can be made by techniques known in the art and can be screened for specificity and activity according to the methods set forth in the Examples and in general methods for producing antibodies and screening antibodies for specificity and activity (See Harlow and Lane. Antibodies, A Laboratory Manual. Cold Spring Harbor Publications, New York, (1988)).

Also included within the meaning of "antibody or fragments thereof' are conjugates of antibody fragments and antigen binding proteins (single chain antibodies) as described, for example, in U.S. Pat. No. 4,704,692, the contents of which are hereby incorporated by reference. Single chain divalent antibodies are also provided.

Optionally, the antibodies are generated in other species and "humanized" for administration in humans. Humanized forms of non-human (e.g., murine) antibodies are chimeric immunoglobulins, immunoglobulin chains or fragments thereof (such as Fv, Fab, Fab', F(ab')2, or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues that are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin (Jones et al., Nature, 321:522-525 (1986); Riechmann et al., Nature, 332:323-327 (1988); and Presta, Curr. Op. Struct. Biol., 2:593-596 (1992)).

Methods for humanizing non-human antibodies are well known in the art. Generally, a humanized antibody has one or more amino acid residues introduced into it from a source that is non-human. These non-human amino acid residues are often referred to as "import" residues, which are typically taken from an "import" variable domain. Humanization can be essentially performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody. Accordingly, such "humanized" antibodies are chimeric antibodies (U.S. Pat. No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

The choice of human variable domains, both light and heavy, to be used in making the humanized antibodies is very important in order to reduce antigenicity. According to the "best-fit" method, the sequence of the variable domain of a rodent antibody is screened against the entire library of known human variable domain sequences. The human sequence which is closest to that of the rodent is then accepted as the human framework (FR) for the humanized antibody (Sims et al., J. Immunol., 151:2296(1993) and Chothia et al., J. Mol. Biol., 196:901 (1987)). Another method uses a particular framework derived from the consensus sequence of all human antibodies of a particular subgroup of light or heavy chains. The same framework may be used for several different humanized antibodies (Carter et al., Proc. Natl. Acad. Sci. USA, 89:4285 (1992); Presta et al., J. Immunol., 151:2623 (1993)).

It is further important that antibodies be humanized with retention of high affinity for the antigen and other favorable biological properties. To achieve this goal, according to a preferred method, humanized antibodies are prepared by a process of analysis of the parental sequences and various conceptual humanized products using three dimensional models of the parental and humanized sequences. Three dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the consensus and import sequence so that the desired antibody characteristic, such as increased affinity for the target antigen(s), is achieved. In general, the CDR residues are directly and most substantially involved in influencing antigen binding (see, WO 94/04679, published 3 March 1994).

Transgenic animals (e.g., mice) that are capable, upon immunization, of producing a full repertoire of human antibodies in the absence of endogenous immunoglobulin production can be employed. For example, it has been described that the homozygous deletion of the antibody heavy chain joining region (J(H)) gene in chimeric and germ-line mutant mice results in complete inhibition of endogenous antibody production. Transfer of the human germ-line immunoglobulin gene array in such germ-line mutant mice will result in the production of human antibodies upon antigen challenge (see, e.g., Jakobovits et al Proc. Natl. Acad. Sci. USA, 90:2551-255 (1993); Jakobovits et al., Nature, 362:255-258 (1993); Bruggemann et al., Year in Immuno., 7:33 (1993)). Human antibodies can also be produced in phage display libraries (Hoogenboom et al., J. Mol. Biol., 227:381 (1991); Marks et al., .1. Mol. Biol., 222:581 (1991)). The techniques of Cote et al. and Boerner et al. are also available for the preparation of human monoclonal antibodies (Cole et al., Monoclonal Antibodies and Cancer Therapy, Alan R. Liss, p. 77 (1985); Boerner et at., J. Immunol., 147(1):86-95 (1991)).

The present invention further provides a hybridoma cell that produces the monoclonal antibody of the invention. The term "monoclonal antibody" as used herein refers to an antibody obtained from a substantially homogeneous population of antibodies, i.e., the individual antibodies comprising the population are identical except for possible naturally occurring mutations that may be present in minor amounts. The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired activity (See, U.S. Pat. No. 4,816,567 and Morrison et al., Proc. Natl. Acad. Sci. USA, 81:6851-6855 (1984)).

Monoclonal antibodies of the invention may be prepared using hybridoma methods, such as those described by Kohler and Milstein, Nature, 256:495 (1975) or Harlow and Lane. Antibodies, A Laboratory Manual. Cold Spring Harbor Publications, New York, (1988). In a hybridoma method, a mouse or other appropriate host animal, is typically immunized with an immunizing agent to elicit lymphocytes that produce or are capable of producing antibodies that will specifically bind to the immunizing agent. Alternatively, the lymphocytes may be immunized in vitro. Preferably, the immunizing agent comprises EphA2. Traditionally, the generation of monoclonal antibodies has depended on the availability of purified protein or peptides for use as the immunogen. More recently DNA based immunizations have shown promise as a way to elicit strong immune responses and generate monoclonal antibodies. In this approach, DNA-based immunization can be used, wherein DNA encoding a portion of EphA2 expressed as a fusion protein with human IgG1 is injected into the host animal according to methods known in the art (e.g., Kilpatrick KE, et al. Gene gun delivered DNA-based immunizations mediate rapid production of murine monoclonal antibodies to the Flt-3 receptor. Hybridoma. 1998 Dec;17(6):569-76; Kilpatrick KE et al. High-affinity monoclonal antibodies to PED/PEA-15 generated using 5 microg of DNA. Hybridoma. 2000 Aug;19(4):297-302, which are incorporated herein by referenced in full for the methods of antibody production).

An alternate approach to immunizations with either purified protein or DNA is to use antigen expressed in baculovirus. The advantages to this system include ease of generation, high levels of expression, and post-translational modifications that are highly similar to those seen in mammalian systems. Use of this system involves expressing domains of EphA2 antibody as fusion proteins. The antigen is produced by inserting a gene fragment in-frame between the signal sequence and the mature protein domain of the EphA2 antibody nucleotide sequence. This results in the display of the foreign proteins on the surface of the virion. This method allows immunization with whole virus, eliminating the need for purification of target antigens.

Generally, peripheral blood lymphocytes ("PBLs") are used in methods of producing monoclonal antibodies if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell (Goding, "Monoclonal Antibodies: Principles and Practice" Academic Press, (1986) pp. 59-103). Immortalized cell lines are usually transformed mammalian cells, including myeloma cells of rodent, bovine, equine, and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells. Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, Calif, and the American Type Culture Collection, Rockville, Md. Human myeloma and mouse-human heteromyeloma cell lines also have been described for the production of human monoclonal antibodies (Kozbor, J. Immunol., 133:3001 (1984); Brodcur et al., "Monoclonal Antibody Production Techniques and Applications" Marcel Dekker, Inc., New York, (1987) pp. 51-63).

The culture medium in which the hybridoma cells are cultured can then be assayed for the presence of monoclonal antibodies directed against EphA2. Preferably, the binding specificity of monoclonal antibodies produced by the hybridoma cells is determined by immunoprecipitation or by an in vitro binding assay, such as radioimmunoassay (RIA) or enzyme-linked immunoabsorbent assay (ELISA). Such techniques and assays are known in the art, and are described further in the Examples below or in Harlow and Lane "Antibodies, A Laboratory Manual" Cold Spring Harbor Publications, New York, (1988).

After the desired hybridoma cells are identified, the clones may be subcloned by limiting dilution or FACS sorting procedures and grown by standard methods. Suitable culture media for this purpose include, for example, Dulbecco's Modified Eagle's Medium and RPMI-1640 medium. Alternatively, the hybridoma cells may be grown in vivo as ascites in a mammal.

The monoclonal antibodies secreted by the subclones may be isolated or purified from the culture medium or ascites fluid by conventional immunoglobulin purification procedures such as, for example, protein A-Sepharose, protein G, hydroxylapatite chromatography, gel electrophoresis, dialysis, or affinity chromatography.

The monoclonal antibodies may also be made by recombinant DNA methods, such as those described in U.S. Pat. No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, plasmacytoma cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences (U.S. Pat. No. 4,816,567) or by covalently joining to the immunoglobulin coding sequence all or part of the coding sequence for a non-immunoglobulin polypeptide. Optionally, such a non-immunoglobulin polypeptide is substituted for the constant domains of an antibody of the invention or substituted for the variable domains of one antigen-combining site of an antibody of the invention to create a chimeric bivalent antibody comprising one antigen-combining site having specificity for EphA2 and another antigen-combining site having specificity for a different antigen.

*In vitro* methods are also suitable for preparing monovalent antibodies. Digestion of antibodies to produce fragments thereof, particularly, Fab fragments, can be accomplished using routine techniques known in the art. For instance, digestion can be performed using papain. Examples of papain digestion are described in WO 94/29348 published Dec. 22, 1994, U.S. Pat. No. 4,342,566, and Harlow and Lane, Antibodies, A. Laboratory Manual, Cold Spring Harbor Publications, New York, (1988). Papain digestion of antibodies typically produces two identical antigen binding fragments, called Fab fragments, each with a single antigen binding site, and a residual Fc fragment. Pepsin treatment yields a fragment, called the F(ab')2 fragment, that has two antigen combining sites and is still capable of cross-linking antigen.

The Fab fragments produced in the antibody digestion also contain the constant domains of the light chain and the first constant domain of the heavy chain. Fab' fragments differ from Fab fragments by the addition of a few residues at the carboxy terminus of the heavy chain domain including one or more cysteines from the antibody hinge region. The F(ab')2 fragment is a bivalent fragment comprising two Fab' fragments linked by a disulfide bridge at the hinge region. Fab'-SH is the designation herein for Fab' in which the cysteine residue(s) of the constant domains bear a free thiol group. Antibody fragments originally were produced as pairs of Fab' fragments which have hinge cysteines between them. Other chemical couplings of antibody fragments are also known.

An isolated immunogenically specific paratopc or fragment of the antibody is also provided. A specific immunogenic epitope of the antibody can be isolated from the whole antibody by chemical or mechanical disruption of the molecule. The purified fragments thus obtained are tested to determine their immunogcnicity and specificity by the methods taught herein. Immunoreactive paratopes of the antibody, optionally, are synthesized directly. An immunoreactive fragment is defined as an amino acid sequence of at least about two to five consecutive amino acids derived from the antibody amino acid sequence.

One method of producing proteins comprising the antibodies of the present invention is to link two or more peptides or polypeptides together by protein chemistry techniques. For example, peptides or polypeptides can be chemically synthesized using currently available laboratory equipment using either Fmoc (9-fluorenylmethyloxycarbonyl) or Boc (tert-butyloxycarbonoyl) chemistry. (Applied Biosystems, Inc., Foster City, CA). One skilled in the art can readily appreciate that a peptide or polypeptide corresponding to the antibody of the present invention, for example, can be synthesized by standard chemical reactions. For example, a peptide or polypeptide can be synthesized and not cleaved from its synthesis resin whereas the other fragment of an antibody can be synthesized and subsequently cleaved from the resin, thereby exposing a terminal group which is functionally blocked on the other fragment. By peptide condensation reactions, these two fragments can be covalently joined via a peptide bond at their carboxyl and amino termini, respectively, to form an antibody, or fragment thereof. (Grant GA (1992) Synthetic Peptides: A User Guide. W.H. Freeman and Co., N.Y. (1992); Bodansky M and Trost B., Ed. (1993) Principles of Peptide Synthesis. Springer-Verlag Inc., NY. Alternatively, the peptide or polypeptide is independently synthesized in vivo as described above. Once isolated, these independent peptides or polypeptides may be linked to form an antibody or fragment thereof via similar peptide condensation reactions.

For example, enzymatic ligation of cloned or synthetic peptide segments allow relatively short peptide fragments to be joined to produce larger peptide fragments, polypeptides or whole protein domains (Abrahmsen L et al., Biochemistry, 30:4151 (1991)). Alternatively, native chemical ligation of synthetic peptides can be utilized to synthetically construct large peptides or polypeptides from shorter peptide fragments. This method consists of a two step chemical reaction (Dawson et al, Synthesis of Proteins by Native Chemical Ligation. Science, 266:776-779 (1994)). The first step is the chemoselective reaction of an unprotected synthetic peptide-alpha-thioester with another unprotected peptide segment containing an amino-terminal Cys residue to give a thioester-linked intermediate as the initial covalent product. Without a change in the reaction conditions, this intermediate undergoes spontaneous, rapid intramolecular reaction to form a native peptide bond at the ligation site. Application of this native chemical ligation method to the total synthesis of a protein molecule is illustrated by the preparation of human interleukin 8 (IL-8) (Baggiolini M et al. (1992) FEBS Lett. 307:97-101; Clark-Lewis I et al., J.Biol.Chem., 269:16075 (1994); Clark-Lewis I et al., Biochemistry, 30:3128 (1991); Rajarathnam K et al., Biochemistry 33:6623-30 (1994)).

Alternatively, unprotected peptide segments are chemically linked where the bond formed between the peptide segments as a result of the chemical ligation is an unnatural (non-peptide) bond (Schnolzer, M et al. Science, 256:221 (1992)). This technique has been used to synthesize analogs of protein domains as well as large amounts of relatively pure proteins with full biological activity (deLisle Milton RC et al., Techniques in Protein Chemistry IV. Academic Press, New York, pp. 257-267 (1992)).

The invention also provides fragments of antibodies which have bioactivity. The polypeptide fragments of the present invention can be recombinant proteins obtained by cloning nucleic acids encoding the polypeptide in an expression system capable of producing the polypeptide fragments thereof, such as an adenovirus or baculovirus expression system. For example, one can determine the active domain of an antibody from a specific hybridoma that can cause a biological effect associated with the interaction of the antibody with EphA2. For example, amino acids found to not contribute to either the activity or the binding specificity or affinity of the antibody can be deleted without a loss in the respective activity. For example, in various embodiments, amino or carboxy-terminal amino acids are sequentially removed from either the native or the modified non-immunoglobulin molecule or the immunoglobulin molecule and the respective activity assayed in one of many available assays. In another example, a fragment of an antibody comprises a modified antibody wherein at least one amino acid has been substituted for the naturally occurring amino acid at a specific position, and a portion of either amino terminal or carboxy terminal amino acids, or even an internal region of the antibody, has been replaced with a polypeptide fragment or other moiety, such as biotin, which can facilitate in the purification of the modified antibody. For example, a modified antibody can be fused to a maltose binding protein, through either peptide chemistry or cloning the respective nucleic acids encoding the two polypeptide fragments into an expression vector such that the expression of the coding region results in a hybrid polypeptide. The hybrid polypeptide can be affinity purified by passing it over an amylose affinity solid support, and the modified antibody receptor can then be separated from the maltose binding region by cleaving the hybrid polypeptide with the specific protease factor Xa. (See, for example, New England Biolabs Product Catalog, 1996, pg. 164.). Similar purification procedures are available for isolating hybrid proteins from eukaryotic cells as well.

The fragments, whether attached to other sequences or not, include insertions, deletions, substitutions, or other selected modifications of particular regions or specific amino acids residues, provided the activity of the fragment is not significantly altered or impaired compared to the nonmodified antibody or antibody fragment. These modifications can provide for some additional property, such as to remove or add amino acids capable of disulfide bonding, to increase its bio-longevity, to alter its secretory characteristics, etc. In any case, the fragment must possess a bioactive property, such as binding activity, regulation of binding at the binding domain, etc. Functional or active regions of the antibody may be identified by mutagenesis of a specific region of the protein, followed by expression and testing of the expressed polypeptide. Such methods are readily apparent to a skilled practitioner in the art and can include site-specific mutagenesis of the nucleic acid encoding the antigen. (Zoller MJ et al. Nucl. Acids Res. 10:6487-500 (1982).

A variety of immunoassay formats may be used to select antibodies that selectively bind with a particular protein, variant, or fragment. For example, solid-phase ELISA immunoassays are routinely used to select antibodies selectively immunoreactive with a protein, protein variant, or fragment thereof. See Harlow and Lane. Antibodies, A Laboratory Manual. Cold Spring Harbor Publications, New York, (1988), for a description of immunoassay formats and conditions that could be used to determine selective binding. The binding affinity of a monoclonal antibody can, for example, be determined by the Scatchard analysis of Munson et al., Anal. Biochem., 107:220 (1980).

Also provided is an antibody reagent kit comprising containers of the monoclonal antibody or fragment thereof of the invention and one or more reagents for detecting binding of the antibody or fragment thereof to the EphA2 receptor molecule. The reagents can include, for example, fluorescent tags, enzymatic tags, or other tags. The reagents can also include secondary or tertiary antibodies or reagents for enzymatic reactions, wherein the enzymatic reactions produce a product that can be visualized.

### (b) Functional Nucleic Acids

Functional nucleic acids can also be used to regulate the , for example, the function of the disclosed integrins, integrin receptors, molecules that interact with the disclosed integrin receptors, and molecules in the signaling pathways of the disclosed integrin receptors.

Functional nucleic acids are nucleic acid molecules that have a specific function, such as binding a target molecule or catalyzing a specific reaction. Functional nucleic acid molecules can be divided into the following categories, which are not meant to be limiting. For example, functional nucleic acids include antisense molecules, aptamers, ribozymes, triplex forming molecules, and external guide sequences. The functional nucleic acid molecules can act as affeotors, inhibitors, modulators, and stimulators of a specific activity possessed by a target molecule, or the functional nucleic acid molecules can possess a de novo activity independent of any other molecules.

Functional nucleic acid molecules can interact with any macromolecule, such as DNA, RNA, polypeptides, or carbohydrate chains. Thus, functional nucleic acids can interact with the mRNA of beta4 integrin for example, or the genomic DNA of alpha6 integrin for example, or they can interact with the polypeptide laminin5, or the gamma2 subunit of laminin5. Often functional nucleic acids are designed to interact with other nucleic acids based on sequence homology between the target molecule and the functional nucleic acid molecule. In other situations, the specific recognition between the functional nucleic acid molecule and the target molecule is not based on sequence homology between the functional nucleic acid molecule and the target molecule, but rather is based on the formation of tertiary structure that allows specific recognition to take place, Antisense molecules are designed to interact with a target nucleic acid molecule through either canonical or non-canonical base pairing. The interaction of the antisense molecule and the target molecule is designed to promote the destruction of the target molecule through, for example, RNAseH mediated RNA-DNA hybrid degradation. Alternatively the antisense molecule is designed to interrupt a processing function that normally would take place on the target molecule, such as transcription or replication. Antisense molecules can be designed based on the sequence of the target molecule. Numerous methods for optimization of antisense efficiency by finding the most accessible regions of the target molecule exist. Exemplary methods would be in vitro selection experiments and DNA modification studies using DMS and DEPC. It is preferred that antisense molecules bind the target molecule with a dissociation constant (k_{d})less than 10⁻⁶. It is more preferred that antisense molecules bind with a k_{d} less than 10⁻⁸. It is also more preferred that the antisense molecules bind the target molecule with a k_{d} less than 10⁻¹⁰. It is also preferred that the antisense molecules bind the target molecule with a k_{d} less than 10⁻¹². A representative sample of methods and techniques which aid in the design and use of antisense molecules can be found in the following non-limiting list of United States patents: 5,135,917, 5,294,533, 5,627,158, 5,641,754, 5,691,317, 5,780,607, 5,786,138, 5,849,903, 5,856,103, 5,919,772, 5,955,590, 5,990,088, 5,994,320, 5,998,602, 6,005,095, 6,007,995, 6,013,522, 6,017,898, 6,018,042, 6,025,198, 6,033,910, 6,040,296, 6,046,004, 6,046,319, and 6,057,437.

Aptamers are molecules that interact with a target molecule, preferably in a specific way. Typically aptamers are small nucleic acids ranging from 15-50 bases in length that fold into defined secondary and tertiary structures, such as stem-loops or G-quartets. Aptamers can bind small molecules, such as ATP (United States patent 5,631,146) and theophiline (United States patent 5,580,737), as well as large molecules, such as reverse transcriptase (United States patent 5,786,462) and thrombin (United States patent 5,543,293). Aptamers can bind very tightly with k_{d}s from the target molecule of less than 10⁻¹² M. It is preferred that the aptamers bind the target molecule with a k_{d} less than 10⁻⁶. It is more preferred that the aptamers bind the target molecule with a k_{d} less than 10⁻⁸, It is also more preferred that the aptamers bind the target molecule with a k_{d} less than 10⁻¹⁰. It is also preferred that the aptamers bind the target molecule with a k_{d} less than 10⁻¹². Aptamers can bind the target molecule with a very high degree of specificity. For example, aptamers have been isolated that have greater than a 10000 fold difference in binding affinities between the target molecule and another molecule that differ at only a single position on the molecule (United States patent 5,543,293). It is preferred that the aptamer have a k_{d} with the target molecule at least 10 fold lower than the k_{d} with a background binding molecule. It is more preferred that the aptamer have a k_{d} with the target molecule at least 100 fold lower than the k_{d} with a background binding molecule. It is more preferred that the aptamer have a k_{d} with the target molecule at least 1000 fold lower than the k_{d} with a background binding molecule. It is preferred that the aptamer have a k_{d} with the target molecule at least 10000 fold lower than the k_{d} with a background binding molecule. It is preferred when doing the comparison for a polypeptide for example, that the background molecule be a di fferent polypeptide. For example, when determining the specificity of beta4 integrin aptamers, the background protein could be bovine serum albumin. Representative examples of how to make and use aptamers to bind a variety of different target molecules can be found in the following non-limiting list of United States patents: 5,476,766, 5,503,978, 5,631,146, 5,731,424 , 5,780,228, 5,792,613, 5,795,721, 5,846,713, 5,858,660, 5,861,254, 5,864,026, 5,869,641, 5,958,G91, 6,001,988, 6,011,020, 6,013,443, 6,020,1.30, 6,028,186, 6,030,776, and 6,051,698.

Ribozymes are nucleic acid molecules that are capable of catalyzing a chemical reaction, either intramolecularly or intermolecularly. Ribozymes are thus catalytic nucleic acid. It is preferred that the ribozymes catalyze intermolecular reactions. There are a number of different types of ribozymes that catalyze nuclease or nucleic acid polymerase type reactions which are based on ribozymes found in natural systems, such as hammerhead ribozymes, (for example, but not limited to the following United States patents: 5,334,711, 5,436,330, 5,616,466, 5,633,133, 5,64G,020, 5,652,094, 5,712,384, 5,770,715, 5,856,463, 5,861,288, 5,891,683, 5,891,684, 5,985,621, 5,989,908, 5,998,193, 5,998,203, WO 9858058 by Ludwig and Sproat, WO 9858057 by Ludwig and Sproat, and WO 9718312 by Ludwig and Sproat) hairpin ribozymes (for example, but not limited to the following United States patents: 5,631,115, 5,646,031, 5,683,902, 5,712,384, 5,856,188, 5,866,701, 5,869,339, and 6,022,962), and tetrahymena ribozymes (for example, but not limited to the following United States patents: 5,595,873 and 5,652,107). There are also a number of ribozymes that are not found in natural systems, but which have been engineered to catalyze specific reactions de novo (for example, but not limited to the following United States patents: 5,580,967, 5,688,670, 5,807,718, and 5,910,408). Preferred ribozymes cleave RNA or DNA substrates, and more preferably cleave RNA substrates. Ribozymes typically cleave nucleic acid substrates through recognition and binding of the target substrate with subsequent cleavage. This recognition is often based mostly on canonical or non-canonical base pair interactions. This property makes ribozymes particularly good candidates for target specific cleavage of nucleic acids because recognition of the target substrate is based on the target substrates sequence. Representative examples of how to make and use ribozymes to catalyze a variety of different reactions can be found in the following non-limiting list of United States patents: 5,646,042, 5,693,535, 5,731,295, 5,811,300, 5,837,855, 5,869,253, 5,877,021, 5,877,022, 5,972,699, 5,972,704, 5,989,906, and 6,017,756.

Triplex forming functional nucleic acid molecules are molecules that can interact with either double-stranded or single-stranded nucleic acid. When triplex molecules interact with a target region, a structure called a triplex is formed, in which there are three strands of DNA forming a complex dependant on both Watson-Crick and Hoogsteen base-pairing. Triplex molecules are preferred because they can bind target regions with high affinity and specificity. It is preferred that the triplex forming molecules bind the target molecule with a k_{d} less than 10⁻⁶ It is more preferred that the triplex forming molecules bind with a k_{d} less than 10⁻⁸. It is also more preferred that the triplex forming molecules bind the target moelcule with a k_{d} less than 10⁻¹⁰ It is also preferred that the triplex forming molecules bind the target molecule with a k_{d} less than 10⁻¹². Representative examples of how to make and use triplex forming molecules to bind a variety of different target molecules can be found in the following non-limiting list of United States patents: 5,176,996, 5,645,985, 5,650,316, 5,683,874, 5,693,773, 5,834,185, 5,869,246, 5,874,566, and 5,962,426.

External guide sequences (EGSs) are molecules that bind a target nucleic acid molecule forming a complex, and this complex is recognized by RNase P, which cleaves the target molecule. EGSs can be designed to specifically target a RNA molecule of choice. RNAse P aids in processing transfer RNA (tRNA) within a cell. Bacterial RNAse P can be recruited to cleave virtually any RNA sequence by using an EGS that causes the target RNA:EGS complex to mimic the natural tRNA substrate. (WO 92/03566 by Yale, and Forster and Altman, Science 238:407-409 (1990)).

Similarly, eukaryotic EGS/RNAse P-directed cleavage of RNA can be utilized to cleave desired targets within eukaryotic cells. (Yuan etal., Proc. Natl. Acad. Sci. USA 89:8006-8010 0 (1992); WO 93/22434 by Yale; WO 95/24489 by Yale; Yuan and Altman, EMBO J 14:159-168 (1995), and Carrara et al., Proc. Natl. Acad. Sci. (USA) 92:2627-2631 (1995)). Representative examples of how to make and use EGS molecules to facilitate cleavage of a variety of different target molecules be found in the following non-limiting list of United States patents: 5,168,053, 5,624,824, 5,683,873, 5,728,521, 5,869,248, and 5,877,162

### (c) Small molecules

Small molecules can also be used to regulate, for example, the function of the disclosed integrins, integrin receptors, molecules that interact with the disclosed integrin receptors, and molecules in the signaling pathways of the disclosed integrin receptors. Those of skill in the art understand how to generate small molecules of this type, and exemplary libraries and methods for isolating small molecule regulators are disclosed herein,

### b) Compositions identified by screening with disclosed compositions / combinatorial chemistry

### (1) Combinatorial chemistry

The disclosed compositions can be used as targets for any combinatorial technique to identify molecules or macromolecular molecules that interact with the disclosed compositions, such as beta4 integrin, alpha6 integrin, or the gamma2 subunit of laminin5, in a desired way. The nucleic acids, peptides, and related molecules disclosed herein can be used as targets for the combinatorial approaches. Also disclosed are the compositions that are identified through combinatorial techniques or screening techniques in which the herein disclosed compositions, for example set forth in SEQ ID NOS:1-19 or portions thereof, are used as the target or reagent in a combinatorial or screening protocol.

It is understood that when using the disclosed compositions in combinatorial techniques or screening methods, molecules, such as macromolecular molecules, will be identified that have particular desired properties such as inhibition or stimulation or the target molecule's function. The molecules identified and isolated when using the disclosed compositions, such as, beta4 integrin, alpa6 integrin, or the gamma2 subunit of laminin5, are also disclosed. Thus, the products produced using the combinatorial or screening approaches that involve the disclosed compositions, such as, beta4 integrin, alpha6 integrin, or the gamma2 subunit of laminin5, are also considered herein disclosed.

Combinatorial chemistry includes but is not limited to all methods for isolating small molecules or macromolecules that are capable of binding either a small molecule or another macromolecule, typically in an iterative process. Proteins, oligonucleotides, and sugars are examples of macromolecules. For example, oligonucleotide molecules with a given function, catalytic or ligand-binding, can be isolated from a complex mixture of random oligonucleotides in what has been referred to as "in vitro genetics" (Szostak, TIBS 19:89, 1992). One synthesizes a large pool of molecules bearing random and defined sequences and subjects that complex mixture, for example, approximately 10¹⁵ individual sequences in 100 µg of a 100 nucleotide RNA, to some selection and enrichment process. Through repeated cycles of affinity chromatography and PCR amplification of the molecules bound to the ligand on the solid support, Ellington and Szostak (1990) estimated that 1 in 10¹⁰ RNA molecules folded in such a way as to bind a small molecule dyes. DNA molecules with such ligand-binding behavior have been isolated as well (Ellington and Szostak, 1992; Bock et al, 1992). Techniques aimed at similar goals exist for small organic molecules, proteins, antibodies and other macromolecules known to those of skill in the art. Screening sets of molecules for a desired activity whether based on small organic libraries, oligonucleotides, or antibodies is broadly referred to as combinatorial chemistry. Combinatorial techniques are particularly suited for defining binding interactions between molecules and for isolating molecules that have a specific binding activity, often called aptamers when the macromolecules are nucleic acids.

There are a number of methods for isolating proteins which either have de novo activity or a modified activity. For example, phage display libraries have been used to isolate numerous peptides that interact with a specific target. (See for example, United States Patent No. 6,031,071; 5,824,520; 5,596,079; and 5,565,332 which are herein incorporated by reference at least for their material related to phage display and methods relate to combinatorial chemistry)

A preferred method for isolating proteins that have a given function is described by Roberts and Szostak (Roberts R.W. and Szostak J.W. Proc. Natl. Acad. Sci. USA, 94(23)12997-302 (1997). This combinatorial chemistry method couples the functional power of proteins and the genetic power of nucleic acids. An RNA molecule is generated in which a puromycin molecule is covalently attached to the 3'-end of the RNA molecule. An *in vitro* translation of this modified RNA molecule causes the correct protein, encoded by the RNA to be translated. In addition, because of the attachment of the puromycin, a peptdyl acceptor which cannot be extended, the growing peptide chain is attached to the puromycin which is attached to the RNA. Thus, the protein molecule is attached to the genetic material that encodes it. Normal *in vitro* selection procedures can now be done to isolate functional peptides. Once the selection procedure for peptide function is complete traditional nucleic acid manipulation procedures are performed to amplify the nucleic acid that codes for the selected functional peptides. After amplification of the genetic material, new RNA is transcribed with puromycin at the 3'-end, new peptide is translated and another functional round of selection is performed. Thus, protein selection can be performed in an iterative manner just like nucleic acid selection techniques. The peptide which is translated is controlled by the sequence of the RNA attached to the puromycin. This sequence can be anything from a random sequence engineered for optimum translation (i.e. no stop codons etc.) or it can be a degenerate sequence of a known RNA molecule to look for improved or altered function of a known peptide. The conditions for nucleic acid amplification and in vitro translation are well known to those of ordinary skill in the art and are preferably performed as in Roberts and Szostak (Roberts R.W. and Szostak J.W. Proc. Natl. Acad. Sci. USA, 94(23)12997-302 (1997)).

Another preferred method for combinatorial methods designed to isolate peptides is described in Cohen et al. (Cohen B.A.,et al., Proc. Natl. Acad. Sci. USA 95(24):14272-7 (1998)). This method utilizes and modifies two-hybrid technology. Yeast two-hybrid systems are useful for the detection and analysis ofprotein:protein interactions. The two-hybrid system, initially described in the yeast *Saccharomyces cerevisiae,* is a powerful molecular genetic technique for identifying new regulatory molecules, specific to the protein of interest (Fields and Song, Nature 340:245-6 (1989)). Cohen et al., modified this technology so that novel interactions between synthetic or engineered peptide sequences could be identified which bind a molecule of choice. The benefit of this type of technology is that the selection is done in an intracellular environment. The method utilizes a library of peptide molecules that attached to an acidic activation domain. A peptide of choice, for example a portion of beta4 or alpha6 or gamma2 or laminin5 is attached to a DNA binding domain of a transcriptional activation protein, such as Gal 4. By performing the Two-hybrid technique on this type of system, molecules that bind the portion of beta4 or alpha6 or gamma2 or laminin5 can be identified.

There are molecules that can act like antibodies, in that they can having varying binding specificities, that are based on a fibronectin motif. The fibronectin type III domain (FN3) is a small autonomous folding unit. This FN3 domain can be found in numeorus proteins that bind ligand, such as animal proteins. The beta-sandwich structure of FN3 closely resembles that of immunoglobulin domains. FN3 mutants can be isolated using combinatorial approaches disclosed herein, for example phage display, that bind desired targets. Typically the libraries of FN3 molecules have been randomized in the two surface loops. Thus, FN3 can be used at least as a scaffold for engineering novel binding proteins. (Koide A, Bailey CW, Huang X, Koide S., "The fibronectin type III domain as a scaffold for novel binding proteins."J Mol Biol 1998: 284,1141-1151 which is herein incorporated by reference at least for material related to the fibronectin based novel binding proteins).

Using methodology well known to those of skill in the art, in combination with various combinatorial libraries, one can isolate and characterize those small molecules or macromolecules, which bind to or interact with the desired target. The relative binding affinity of these compounds can be compared and optimum compounds identified using competitive binding studies, which are well known to those of skill in the art.

Techniques for making combinatorial libraries and screening combinatorial libraries to isolate molecules which bind a desired target are well known to those of skill in the art. Representative techniques and methods can be found in but are not limited to United States patents 5,084,824, 5,288,514, 5,449,754, 5,506,337, 5,539,083, 5,545,568, 5,556,762, 5,565,324, 5,565,332, 5,573,905, 5,618,825, 5,619,680, 5,627,210, 5,646,285, 5,663,046, 5,670,326, 5,677,195, 5,683,899, 5,688,696, 5,688,997, 5,698,685, 5,712,146, 5,721,099, 5,723,598, 5,741,713, 5,792,431, 5,807,683, 5,807,754, 5,821,130, 5,831,014, 5,834,195, 5,834,318, 5,834,588, 5,840,500, 5,847,150, 5,856,107, 5,856,496, 5,859,190, 5,864,010, 5,874,443, 5,877,214, 5,880,972, 5,886,126, 5,886,127, 5,891,737, 5,916,899, 5,919,955, 5,925,527, 5,939,268, 5,942,387, 5,945,070, 5,948,G9G, 5,958,702, 5,958,792, 5,962,337, 5,965,719, 5,972,719, 5,976,894, 5,980,704, 5,985,356, 5,999,086, 6,001,579, 6,004,617, 6,008,321, 6,017,768, 6,025,371, 6,030,917, 6,040,193, 6,045,671, 6,045,755, 6,060,596, and 6,061,636.

Combinatorial libraries can be made from a wide array of molecules using a number of different synthetic techniques. For example, libraries containing fused 2,4-pyrimidinediones (United States patent 6,025,371) dihydrobenzopyrans (United States Patent 6,017,768and 5,821,130), amide alcohols (United States Patent 5,976,894), hydroxy-amino acid amides (United States Patent 5,972,719) carbohydrates (United States patent 5,965,719), 1,4-benzodiazepin-2,5-diones (United States patent 5,962,337), cyclics (United States patent 5,958,792), biaryl amino acid amides (United States patent 5,948,696), thiophenes (United States patent 5,942,387), tricyclic Tetrahydroquinolines (United States patent 5,925,527), benzofurans (United States patent 5,919,955), isoquinolines (United States patent 5,916,899), hydantoin and thiohydantoin (United States patent 5,859,190), indoles (United States patent 5,856,496), imidazol-pyrido-indole and imidazol-pyrido-benzothiophenes (United States patent 5,856,107) substituted 2-methylene-2, 3-dihydrothiazoles (United States patent 5,847,150), quinolines (United States patent 5,840,500), PNA (United States patent 5,831,014), containing tags (United States patent 5,721,099), polyketides (United States patent 5,712,146), morpholino-subunits (United States patent 5,698,685 and 5,506,337), sulfamides (United States patent 5,618,825), and benzodiazepines (United States patent 5,288,5 14).

Screening molecules similar to alpha6 for inhibition of alpha6beta4 formation is a method of identifying and isolating desired compounds that can inhibit the formation of A6B4 receptor. For example, the disclosed compositions, such as alpha6 integrin or beta4 integrin can be used as targets in a selection scheme disclosed herein, and then the counter part integrin could be used as a competitive inhibitor to isolate the desired molecules. For example, a library of molecules could be incubated with beta4 integrin, which is bound to a solid support. The solid support can be washed to remove the unbound molecules and then the solid support can be incubated with, for example, alpha6 integrin at a concentration that will saturate all beta4 binding sites. The molecules which are collected in the flowthrough after washing the solid support will be enriched for molecules that interact with beta4 integrin in a way that is competitive to the alpha6-beta4 interaction. Likewise, the solid support, bound with a target integrin, or more preferably a target integrin receptor, such as alpha6beta4 receptor, could also be washed, with for example, laminin5 or the gamma2 subunit of laminin5 at a concentration that will saturate all of the gamma2 binding sites on the beta4 integrin, Collection of the wash under these conditions will yield a population of molecules enriched for molecules that competitively interact with beta4 integrin at the beta4-gamma2 site. Another example, is the following: bind target to solid support on microtiter plate. Incubate with ligand in presence of gridded subset of library members (or single compounds), wash, identify competitor by reduction of ligand binding It is understood that the exemplary discussions of alpha6beta4 and/or beta4 are equally applicable to alpha6, as well as other alpha6betax receptors, such alpha6beta1.

Also disclosed are methods of isolating molecules that bind with a target molecule selected from the group consisting, B4 integrin, alpha6 integrin, and the gamma2 subunit of laminin5 comprising I) contacting a library of molecules with the target molecule and 2) collecting molecules that bind the target molecule producing an enriched population of molecules.

Disclosed are methods, further comprising the step of repeating steps I and 2 with the enriched population of molecules, and/or wherein the library comprises a small molecule, peptide, peptide mimetic, or oligonucleotide.

As used herein combinatorial methods and libraries included traditional screening methods and libraries as well as methods and libraries used in iterative processes.

### (2) Computer assisted design

The disclosed compositions can be used as targets for any molecular modeling technique to identify either the structure of the disclosed compositions or to identify potential or actual molecules, such as small molecules, which interact in a desired way with the disclosed compositions. The nucleic acids, peptides, and related molecules disclosed herein can be used as targets in any molecular modeling program or approach.

It is understood that when using the disclosed compositions in modeling techniques, molecules, such as macromolecular molecules, will be identified that have particular desired properties such as inhibition or stimulation or the target molecule's function. The molecules identified and isolated when using the disclosed compositions, such as, beta4, are also disclosed. Thus, the products produced using the molecular modeling approaches that involve the disclosed compositions, such as, beta4 integrin, alpha6 integrin, or laminin5, are also considered herein disclosed.

Thus, one way to isolate molecules that bind a molecule of choice is through rational design. This is achieved through structural information and computer modeling. Computer modeling technology allows visualization of the three-dimensional atomic structure of a selected molecule and the rational design of new compounds that will interact with the molecule. The three-dimensional construct typically depends on data from x-ray crystallographic analyses or NMR imaging of the selected molecule. The molecular dynamics require force field data. The computer graphics systems enable prediction of how a new compound will link to the target molecule and allow experimental manipulation of the structures of the compound and target molecule to perfect binding specificity. Prediction of what the molecule-compound interaction will be when small changes are made in one or both requires molecular mechanics software and computationally intensive computers, usually coupled with user-friendly, menu-driven interfaces between the molecular design program and the user.

Examples of molecular modeling systems are the CHARMm and QUANTA programs, Polygen Corporation, Waltham, MA. CHARMm performs the energy minimization and molecular dynamics functions. QUANTA performs the construction, graphic modeling and analysis of molecular structure. QUANTA allows interactive construction, modification, visualization, and analysis of the behavior of molecules with each other.

A number of articles review computer modeling of drugs interactive with specific proteins, such as Rotivinen, et al., 1988 Acta Pharmaceutica Fennica 97, 159-166; Ripka, New Scientist 54-57 (June 16, 1988); McKinaly and Rossmann, 1989 Annu.Rev. Pharmacol. Toxiciol. 29, 111-122; Perry and Davies, OSAR: Quantitative Structure-Activity Relationships in Drug Design pp. 189-193 (Alan R. Liss, Inc. 1989); Lewis and Dean, 1989 Proc. R. Soc. Lond. 236, 125-140 and 141-162; and, with respect to a model enzyme for nucleic acid components, Askew, et al., 1989 J. Am. Chem. Soc. 111, 1082-1090. Other computer programs that screen and graphically depict chemicals are available from companies such as BioDesign, Inc., Pasadena, CA., Allelix, Inc, Mississauga, Ontario, Canada, and Hypercube, Inc., Cambridge, Ontario. Although these are primarily designed for application to drugs specific to particular proteins, they can be adapted to design of molecules specifically interacting with specific regions of DNA or RNA, once that region is identified.

Although described above with reference to design and generation of compounds which could alter binding, one could also screen libraries of known compounds, including natural products or synthetic chemicals, and biologically active materials, including proteins, for compounds which alter substrate binding or enzymatic activity.

### c) Nucleic acids

There are a variety of molecules disclosed herein that are nucleic acid based, including for example the nucleic acids that encode, for example beta4 and the gamma2 subunit of laminin5, as well as various functional nucleic acids. The disclosed nucleic acids are made up of for example, nucleotides, nucleotide analogs, or nucleotide substitutes. Non-limiting examples of these and other molecules are discussed herein. It is understood that for example, when a vector is expressed in a cell, that the expressed mRNA will typically be made up of A, C, G, and U. Likewise, it is understood that if, for example, an antisense molecule is introduced into a cell or cell environment through for example exogenous delivery, it is advantageous that the antisense molecule be made up of nucleotide analogs that reduce the degradation of the antisense molecule in the cellular environment.

### (1) Nucleotides and related molecules

A nucleotide is a molecule that contains a base moiety, a sugar moiety and a phosphate moiety. Nucleotides can be linked together through their phosphate moieties and sugar moieties creating an internucleoside linkage. The base moiety of a nucleotide can be adenin-9-yl (A), cytosin-1-yl (C), guanin-9-yl (G), uracil-1-yl (U), and thymin-1-yl (T). The sugar moiety of a nucleotide is a ribose or a deoxyribose. The phosphate moiety of a nucleotide is pentavalent phosphate. An non-limiting example of a nucleotide would be 3'-AMP (3'-adenosine monophosphate) or 5'-GMP (5'-guanosine monophosphate).

A nucleotide analog is a nucleotide which contains some type of modification to either the base, sugar, or phosphate moieties. Modifications to the base moiety would include natural and synthetic modifications of A, C, G, and T/U as well as different purine or pyrimidine bases, such as uracil-5-yl (.psi.), hypoxanthin-9-yl (I), and 2-aminoadenin-9-yl. A modified base includes but is not limited to 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, 7-deazaguanine and 7-deazaadenine and 3-deazaguanine and 3-deazaadenine. Additional base modifications can be found for example in U.S. Pat. No. 3,687,808, Englisch et al., Angewandte Chemie, International Edition, 1991, 30, 613, and Sanghvi, Y. S., Chapter 15, Antisense Research and Applications, pages 289-302, Crooke, S. T. and Lebleu, B. ed., CRC Press, 1993. Certain nucleotide analogs, such as 5-substituted pyrimidines, 6-azapyrimidines and N-2, N-6 and O-6 substituted purines, including 2-aminopropyladenine, 5-propynyluracil and 5-propynylcytosine. 5-methylcytosine can increase the stability of duplex formation. Base modifications can be combined, for example, with a sugar modification, such as 2'-O-methoxyethyl, to achieve unique properties such as increased duplex stability. There are numerous United States patents such as 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; and 5,681,941, which detail and describe a range ofbase modifications. Each of these patents is herein incorporated by reference.

Nucleotide analogs can also include modifications of the sugar moiety. Modifications to the sugar moiety would include natural modifications of the ribose and deoxy ribose as well as synthetic modifications. Sugar modifications include but are not limited to the following modifications at the 2' position: OH-; F-; O-, S-, or N-alkyl; O-, S-, or N-alkenyl; O-, S- or N-alkynyl; or O-alkyl-O-alkyl, wherein the alkyl, alkenyl and alkynyl may be substituted or unsubstituted C₁ to C₁₀, alkyl or C₂ to C₁₀ alkenyl and alkynyl, 2' sugar modifications also include but are not limited to -O[(CH₂)ₙ O]ₘ CH₃, -O(CH₂)ₙ OCH₃, -O(CH₂)ₙ NH₂, -O(CH₂)ₙ CH₃, - O(CH₂)ₙ -ONH₂, and -O(CH₂)ₙON[(CH₂)ₙ CH₃)]₂, where n and m are from 1 to about 10.

Other modi fications at the 2' position include but are not limited to: C₁ to C₁₀ lower alkyl, substituted lower alkyl, alkaryl, aralkyl, O-alkaryl or O-aralkyl, SH, SCH₃, OCN, Cl, Br, CN, CF₃, OCF₃, SOCH₃, SO₂ CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. Similar modifications may also be made at other positions on the sugar, particularly the 3' position of the sugar on the 3' terminal nucleotide or in 2'-5' linked oligonucleotides and the 5' position of 5' terminal nucleotide. Modified sugars would also include those that contain modifications at the bridging ring oxygen, such as CH₂ and S. Nucleotide sugar analogs may also have sugar mimetics such as cyclobutyl moieties in place of the pentofuranosyl sugar. There are numerous United States patents that teach the preparation of such modified sugar structures such as 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; and 5,700,920, each of which is herein incorporated by reference in its entirety.

Nucleotide analogs can also be modified at the phosphate moiety. Modified phosphate moieties include but are not limited to those that can be modified so that the linkage between two nucleotides contains a phosphorothioate, chiral phosphorothioate, phosphorodithioate, phosphotriester, aminoalkylphosphotriester, methyl and other alkyl phosphonates including 3'-alkylene phosphonate and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates. It is understood that these phosphate or modified phosphate linkage between two nucleotides can be through a 3'-5' linkage or a 2'-5' linkage, and the linkage can contain inverted polarity such as 3'-5' to 5'-3' or 2'-5' to 5'-2'. Various salts, mixed salts and free acid forms are also included. Numerous United States patents teach how to make and use nucleotides containing modified phosphates and include but are not limited to, 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; and 5,625,050, each of which is herein incorporated by reference.

It is understood that nucleotide analogs need only contain a single modification, but may also contain multiple modifications within one of the moieties or between different moieties.

Nucleotide substitutes are molecules having similar functional properties to nucleotides, but which do not contain a phosphate moiety, such as peptide nucleic acid (PNA). Nucleotide substitutes are molecules that will recognize nucleic acids in a Watson-Crick or Hoogsteen manner, but which are linked together through a moiety other than a phosphate moiety. Nucleotide substitutes are able to conform to a double helix type structure when interacting with the appropriate target nucleic acid.

Nucleotide substitutes are nucleotides or nucleotide analogs that have had the phosphate moiety and/or sugar moieties replaced. Nucleotide substitutes do not contain a standard phosphorus atom. Substitutes for the phosphate can be for example, short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones;formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH₂ component parts. Numerous United States patents disclose how to make and use these types of phosphate replacements and include but are not limited to 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; and 5,677,439, each of which is herein incorporated by reference.

It is also understood in a nucleotide substitute that both the sugar and the phosphate moieties of the nucleotide can be replaced, by for example an amide type linkage (aminoethylglycine) (PNA). United States patents 5,539,082; 5,714,331;and 5,719,262 teach how to make and use PNA molecules, each of which is herein incorporated by reference. (See also Nielsen et al., Science, 1991, 254, 1497-1500).

It is also possible to link other types of molecules (conjugates) to nucleotides or nucleotide analogs to enhance for example, cellular uptake. Conjugates can be chemically linked to the nucleotide or nucleotide analogs. Such conjugates include but are not limited to lipid moieties such as a cholesterol moiety (Letsinger et al., Proc. Natl. Acad. Sci. USA, 1989,

86, 6553-6556), cholic acid (Manoharan et al., Bioorg. Med. Chem. Let., 1994, 4, 1053-1060), a thioether, e.g., hexyl-S-tritylthiol (Manoharan et al., Ann. N.Y. Acad. Sci., 1992, 660, 306-309; Manoharan et al., Bioorg. Med. Chem. Let., 1993, 3, 2765-2770), a thiocholesterol (Oberhauscr et al., Nucl. Acids Res., 1992, 20. 533-538), an aliphatic chain, e.g., dodecandiol or undecyl residues (Saison-Behmoaras et al., EMBO J., 1991, 10, 1111-1118; Kabanov et al., FEBS Lett., 1990, 259, 327-330; Svinarchuk et al., Biochimie, 1993, 75, 49-54), a phospholipid, e.g., di-hexadecyl-rac-glyoerol or triethylammonium 1,2-di-O-hexadecyl-rac-glycero-3-H-phosphonate (Manoharan et al., Tetrahedron Lett., 1995, 36, 3651-3654; Shea et al., Nucl. Acids Res., 1990, 18, 3777-3783), a polyamine or a polyethylene glycol chain (Manoharan et al., Nucleosides & Nucleotides, 1995, 14, 969-973), or adamantane acetic acid (Manoharan et al., Tetrahedron Lett., 1995, 36, 3651-3654), a palmityl moiety (Mishra et al., Biochim. Biophys. Acta, 1995, 1264, 229-237), or an octadecylamine or hexylamino-carbonyl-oxycholesterol moiety (Crooke et al., J. Pharmacol. Exp. Ther., 1996, 277, 923-937. Numerous United States patents teach the preparation of such conjugates and include, but are not limited to U.S. Pat. Nos. 4,828,979; 4,948,882; 5,218,105; 5,525,465; 5,541,313; 5,545,730; 5,552,538; 5,578,717, 5,580,731; 5,580,731; 5,591,584; 5,109,124; 5,118,802; 5,138,045; 5,414,077; 5,486,603; 5,512,439; 5,578,718; 5,608,046; 4,587,044; 4,605,735; 4,667,025; 4,762,779; 4,789,737; 4,824,941; 4,835,263; 4,876,335; 4,904,582; 4,958,013; 5,082,830; 5,112,963; 5,214,136; 5,082,830; 5,112,963; 5,214,136; 5,245,022; 5,254,469; 5,258,506; 5,262,536; 5,272,250; 5,292,873; 5,317,098; 5,371,241, 5,391,723; 5,416,203, 5,451,463; 5,510,475; 5,512,667; 5,514,785; 5,565,552; 5,567,810; 5,574,142; 5,585,481; 5,587,371; 5,595,726; 5,597,696; 5,599,923; 5,599,928 and 5,688,941, each of which is herein incorporated by reference.

It is understood that an oligonucleotide can be made from any combination of nucleotides, nucleotide analogs, or nucleotide substitutes disclosed herein or related molecules not specifically recited herein.

A Watson-Crick interaction is at least one interaction with the Watson-Crick face of a nucleotide, nucleotide analog, or nucleotide substitute. The Watson-Crick face of a nucleotide, nucleotide analog, or nucleotide substitute includes the C2, N1, and C6 positions of a purine based nucleotide, nucleotide analog, or nucleotide substitute and the C2, N3, C4 positions of a pyrimidine based nucleotide, nucleotide analog, or nucleotide substitute.

A Hoogsteen interaction is the interaction that takes place on the Hoogsteen face of a nucleotide or nucleotide analog, which is exposed in the major groove of duplex DNA. The Hoogsteen face includes the N7 position and reactive groups (NH2 or O) at the C6 position of purine nucleotides.

### (2) Sequences

There are a variety of sequences related to the beta4 integrin or the laminin5-gamma2 gene or the alpha6 integrin gene having the following Genbank Accession Numbers 6453379,4557674, and AH006634, respectively. These sequences and others are herein incorporated by reference in their entireties as well as for individual subsequences contained therein.

One particular sequence set forth in SEQ ID NO:1 (beta4 integrin) and having Genbank accession number 6453379 is used herein, at various points, as an example, to exemplify the disclosed compositions and methods (or when another particular sequence is used as an example). It is understood that the description related to this sequence is applicable to any sequence related to beta4 integrin or any of the other molecules disclosed herein, such as alpha6 integrin, or the subunits of laminin5, such as gamma2, unless specifically indicated otherwise. Those of skill in the art understand how to resolve sequence discrepancies and differences and to adjust the compositions and methods relating to a particular sequence to other related sequences (i.e. sequences of beta4 integrin). Primers and/or probes can be designed for any beta4 integrin sequence given the information disclosed herein and known in the art.

### (3) Primers and probes

Disclosed are compositions including primers and probes, which are capable of interacting with the for example, the alpha6 gene or mRNA, beta4 gene or mRNA, or gamma2 subunit of the laminin5 ligand gene as disclosed herein or mRNA as wells as primers or probes for any of the sequences or fragments of the sequences, set forth in SEQ ID NOs:1,3,5,7,9,11, and 13. In certain embodiments the primers are used to support DNA amplification reactions. Typically the primers will be capable of being extended in a sequence specific manner. Extension of a primer in a sequence specific manner includes any methods wherein the sequence and/or composition of the nucleic acid molecule to which the primer is hybridized or otherwise associated directs or influences the composition or sequence of the product produced by the extension of the primer. Extension of the primer in a sequence specific manner therefore includes, but is not limited to, PCR, DNA sequencing, DNA extension, DNA polymerization, RNA transcription, or reverse transcription. Techniques and conditions that amplify the primer in a sequence specific manner are preferred. In certain embodiments the primers are used for the DNA amplification reactions, such as PCR or direct sequencing. It is understood that in certain embodiments the primers can also be extended using non-enzymatic techniques, where for example, the nucleotides or oligonucleotides used to extend the primer are modified such that they will chemically react to extend the primer in a sequence specific manner. Typically the disclosed primers hybridize with the beta4 gene, alpha6 gene, or laminin5-gamma2 subunit gene, for example, or region of the beta4 gene, alpha6 gene, or laminin5-gamma2 subunit gene, for example, or they hybridize with the complement of the beta4 gene, alpha6 gene, or laminin5-gamma2 subunit gene, for example, or complement of a region of the beta4 gene, alpha6 gene, or laminin5-gamma2 subunit gene, or any of the sequences or fragments of the sequences, set forth in SEQ ID NOs:1,3,5,7,9,11, and 13, for example.

The size of the primers or probes for interaction with the beta4 gene, alpha6 gene, or laminin5-gamma2 subunit gene, for example, in certain embodiments can be any size that supports the desired enzymatic manipulation of the primer, such as DNA amplification or the simple hybridization of the probe or primer. A typical primer or probe for beta4 gene, alpha6 gene, or laminin5-gamma2 subunit gene, for example, or primer or probe for any of the sequences or fragments of the sequences, set forth in SEQ ID NOs:1,3,5,7,9,11, and 13 would be at least about 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 125, 150, 175, 200, 225, 250, 275, 300, 325, 350, 375, 400, 425, 450, 475, 500, 550, 600, 650, 700, 750, 800, 850, 900, 950, 1000, 1250, 1500, 1750, 2000, 2250, 2500, 2750, 3000, 3500, or 4000 nucleotides long.

In other embodiments a primer or probe for beta4 gene, alpha6 gene, or laminin5-gamma2 subunit gene, for example, primer or probe or a primer or probe for any of the sequences or fragments of the sequences, set forth in SEQ ID NOs:1,3,5,7,9,11, and 13 can be less than or equal to about 6, 7, 8, 9, 10, 11, 12 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 4G, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 125, 150, 175, 200, 225, 250, 275, 300, 325, 350, 375, 400, 425, 450, 475, 500, 550, 600, 650, 700, 750, 800, 850, 900, 950, 1000, 1250, 1500, 1750, 2000, 2250, 2500, 2750, 3000, 3500, or 4000 nucleotides long.

The primers for the beta4 gene, alpha6 gene, or laminin5-gamma2 subunit gene, or any of the sequences or fragments of the sequences, set forth in SEQ ID NOs:1,3,5,7,9,11, and 13, for example, typically will be used to produce an amplified DNA product that contains a desired region. In general, typically the size of the product will be such that the size can be accurately determined to within 3, or 2 or 1 nucleotides.

In certain embodiments this product is at least about 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 8S, 8G, 87, 88, 89, 90, 91, 92, 93, 94, 95, 96, 97, 98, 99, 100, 125, 150, 175, 200, 225, 250, 275, 300, 325, 350, 375, 400, 425, 450, 475, 500, 550,600, 50, 700, 750, 800, 850, 900, 950, 1000, 1250, 1500, 1750, 2000, 2250, 2500, 2750, 3000, 3500, or 4000 nucleotides long.

In other embodiments the product is less than or equal to about 20, 21, 22, 23, 24, 25, 26, 27, 28, 29, 30, 31, 32, 33, 34, 35, 36, 37, 3 8, 39, 40, 41 , 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 125, 150, 175, 200, 225, 250, 275, 300, 325, 350, 375, 400, 425, 450, 475, 500, 550, 600, 650, 700, 750, 800, 850, 900, 950, 1000, 1250, 1500, 1750, 2000, 2250, 2500, 2750, 3000, 3500, or 4000 nucleotides long.

### d) Sequence similarities

It is understood that as discussed herein the use of the terms homology and identity mean the same thing as similarity. Thus, for example, if the word homology is used between two non-natural sequences it is understood that this is not necessarily indicating an evolutionary relationship between these two sequences, but rather is looking at the similarity or relatedness between their nucleic acid sequences. Many of the methods for determining homology between two evolutionarily related molecules are routinely applied to any two or more nucleic acids or proteins for the purpose of measuring sequence similarity regardless of whether or not they are evolutionarily related.

In general, it is understood that one way to define any known variants and derivatives or those that might arise, of the disclosed genes and proteins herein, is through defining the variants and derivatives in terms of homology to specific known sequences. This identity of particular sequences disclosed herein is also discussed elsewhere herein. In general, variants of genes and proteins herein disclosed typically have at least, about 70, 71, 72, 73, 74, 75, 76, 77, 78, 79, 80, 81, 82, 83, 84, 85, 86, 87, 88, 89, 90, 91, 92, 93, 94, 95, 96, 97, 98, or 99 percent homology to the stated sequence or the native sequence. Those of skill in the art readily understand how to determine the homology of two proteins or nucleic acids, such as genes. For example, the homology can be calculated after aligning the two sequences so that the homology is at its highest level.

Another way of calculating homology can be performed by published algorithms. Optimal alignment of sequences for comparison may be conducted by the local homology algorithm of Smith and Waterman Adv. Appl. Math. 2: 482 (1981), by the homology alignment algorithm of Needleman and Wunsch, J. MoL Biol. 48: 443 (1970), by the search for similarity method of Pearson and Lipman, Proc. Natl. Acad. Sci. U.S.A. 85: 2444 (1988), by computerized implementations of these algorithms (GAP, BESTFIT, FASTA, and TFASTA in the Wisconsin Genetics Software Package, Genetics Computer Group, 575 Science Dr., Madison, WI), or by inspection.

The same types of homology can be obtained for nucleic acids by for example the algorithms disclosed in Zuker, M. Science 244:48-52, 1989, Jaeger et al. Proc. Natl. Acad. Sci. USA 86:7706-7710, 1989, Jaeger et al. Methods Enzymol. 183:281-306, 1989 which are herein incorporated by reference for at least material related to nucleic acid alignment. It is understood that any of the methods typically can be used and that in certain instances the results of these various methods may differ, but the skilled artisan understands if identity is found with at least one of these methods, the sequences would be said to have the stated identity, and be disclosed herein.

For example, as used herein, a sequence recited as having a particular percent homology to another sequence refers to sequences that have the recited homology as calculated by any one or more of the calculation methods described above. For example, a first sequence has 80 percent homology, as defined herein, to a second sequence if the first sequence is calculated to have 80 percent homology to the second sequence using the Zuker calculation method even if the first sequence does not have 80 percent homology to the second sequence as calculated by any of the other calculation methods, As another example, a first sequence has 80 percent homology, as defined herein, to a second sequence if the first sequence is calculated to have 80 percent homology to the second sequence using both the Zuker calculation method and the Pearson and Lipman calculation method even if the first sequence does not have 80 percent homology to the second sequence as calculated by the Smith and Waterman calculation method, the Needleman and Wunsch calculation method, the Jaeger calculation methods, or any of the other calculation methods. As yet another example, a first sequence has 80 percent homology, as defined herein, to a second sequence if the first sequence is calculated to have 80 percent homology to the second sequence using each of calculation methods (although, in practice, the different calculation methods will often result in different calculated homology percentages).

It is also understood that functional fragments as well as antigenic fragments as well as fragments that can be used in selection protocols of the disclosed compositions are also disclosed. For example, integrins have domains that interact with the other integrins. It may be advantageous in certain embodiments to utilize just the integrin binding domain fragment of, for example, the beta4 integrin, in a selection protocol disclosed herein. By using this domain of the beta4 integrin as the selection target, for example, the selection protocol will be biased for molecules binding this domain of beta4 integrin.

### e) Hybridization/selective hybridization

The term hybridization typically means a sequence driven interaction between at least two nucleic acid molecules, such as a primer or a probe and a gene. Sequence driven interaction means an interaction that occurs between two nucleotides or nucleotide analogs or nucleotide derivatives in a nucleotide specific manner. For example, G interacting with C or A interacting with T are sequence driven interactions. Typically sequence driven interactions occur on the Watson-Crick face or Hoogsteen face of the nucleotide. The hybridization of two nucleic acids is affected by a number of conditions and parameters known to those of skill in the art. For example, the salt concentrations, pH, and temperature of the reaction all affect whether two nucleic acid molecules will hybridize.

Parameters for selective hybridization between two nucleic acid molecules are well known to those of skill in the art. For example, in some embodiments selective hybridization conditions can be defined as stringent hybridization conditions. For example, stringency of hybridization is controlled by both temperature and salt concentration of either or both of the hybridization and washing steps. For example, the conditions of hybridization to achieve selective hybridization may involve hybridization in high ionic strength solution (6X SSC or 6X SSPE) at a temperature that is about 12-25°C below the Tm (the melting temperature at which half of the molecules dissociate from their hybridization partners) followed by washing at a combination of temperature and salt concentration chosen so that the washing temperature is about 5°C to 20°C below the Tm. The temperature and salt conditions are readily determined empirically in preliminary experiments in which samples of reference DNA immobilized on filters are hybridized to a labeled nucleic acid of interest and then washed under conditions of different stringencies. Hybridization temperatures are typically higher for DNA-RNA and RNA-RNA hybridizations. The conditions can be used as described above to achieve stringency, or as is known in the art. (Sambrook et al., Molecular Cloning: A Laboratory Manual, 2nd Ed., Cold Spring Harbor Laboratory, Cold Spring Harbor, New York, 1989; Kunkel et al. Methods Enzymol. 1987:154:367, 1987 which is herein incorporated by reference for material at least related to hybridization of nucleic acids). A preferable stringent hybridization condition for a DNA:DNA hybridization can be at about 68°C (in aqueous solution) in 6X SSC or 6X SSPE followed by washing at 68°C. Stringency of hybridization and washing, if desired, can be reduced accordingly as the degree of complementarity desired is decreased, and further, depending upon the G-C or A-T richness of any area wherein variability is searched for. Likewise, stringency of hybridization and washing, if desired, can be increased accordingly as homology desired is increased, and further, depending upon the G-C or A-T richness of any area wherein high homology is desired, all as known in the art.

Another way to define selective hybridization is by looking at the amount (percentage) of one of the nucleic acids bound to the other nucleic acid. For example, in some embodiments selective hybridization conditions would be when at least about, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the limiting nucleic acid is bound to the non-limiting nucleic acid. Typically, the non-limiting primer is in for example, 10 or 100 or 1000 fold excess. This type of assay can be performed at under conditions where both the limiting and non-limiting primer are for example, 10 fold or 100 fold or 1000 fold below their k_{d}, or where only one of the nucleic acid molecules is 10 fold or 100 fold or 1000 fold or where one or both nucleic acid molecules are above their k_{d}.

Another way to define selective hybridization is by looking at the percentage of primer that gets enzymatically manipulated under conditions where hybridization is required to promote the desired enzymatic manipulation. For example, in some embodiments selective hybridization conditions would be when at least about, 60, 65, 70, 71, 72, 73, 74, 75, 76, 77, 78, 79, 80, 81. 82, 83, 84, 85, 86, 87, 88, 89, 90, 91, 92, 93, 94, 95, 96, 97, 98, 99, 100 percent of the primer is enzymatically manipulated under conditions which promote the enzymatic manipulation, for example if the enzymatic manipulation is DNA extension, then selective hybridization conditions would be when at least about 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the primer molecules are extended. Preferred conditions also include those suggested by the manufacturer or indicated in the art as being appropriate for the enzyme performing the manipulation.

Just as with homology, it is understood that there are a variety of methods herein disclosed for determining the level of hybridization between two nucleic acid molecules. It is understood that these methods and conditions may provide different percentages of hybridization between two nucleic acid molecules, but unless otherwise indicated meeting the parameters of any of the methods would be sufficient. For example if 80% hybridization was required and as long as hybridization occurs within the required parameters in any one of these methods it is considered disclosed herein.

It is understood that those of skill in the art understand that if a composition or method meets any one of these criteria for determining hybridization either collectively or singly it is a composition or method that is disclosed herein.

### f) Delivery of the compositions to cells

The disclosed compositions and methods often entail delivery of the compositions to cells. For example, antisense molecules directed to alpha6 mRNA or gamma2 mRNA can be delivered to cells via any method. A number of exemplary methods are disclosed herein. It is also understood that in certain embodiments, non-nucleic acid molecules will be and can be delivered to cells, for example an antibody to beta4 integrin, alpha6 integrin or, gamma2, or a small molecule, or a peptide. Delivery of these molecules can occur by any means, and exemplary compositions and methods for such delivery are disclosed herein.

There are a number of compositions and methods which can be used to deliver nucleic acids to cells, either in vitro or in vivo. These methods and compositions can largely be broken down into two classes: viral based delivery systems and non-viral based delivery systems. For example, the nucleic acids can be delivered through a number of direct delivery systems such as, electroporation, lipofection, calcium phosphate precipitation, plasmids, viral vectors, viral nucleic acids, phage nucleic acids, phages, cosmids, or via transfer of genetic material in cells or carriers such as cationic liposomes. Appropriate means for transfection, including viral vectors, chemical transfectants, or physico-mechanical methods such as electroporation and direct diffusion of DNA, are described by, for example, Wolff, J. A., et al., Science, 247, 1465-1468, (1990); and Wolff, J. A. Nature, 352, 815-818, (1991). Such methods are well known in the art and readily adaptable for use with the compositions and methods described herein. In certain cases, the methods will be modified to specifically function with large DNA molecules. Further, these methods can be used to target certain diseases and cell populations by using the targeting characteristics of the carrier.

### (1) Nucleic acid based delivery systems

Transfer vectors can be any nucleotide construction used to deliver genes into cells (e.g., a plasmid), or as part of a general strategy to deliver genes, e.g., as part of recombinant retrovirus or adenovirus (Ram et al. Cancer Res. 53:83-88, (1993)).

As used herein, plasmid or viral vectors are agents that transport the disclosed nucleic acids, such as gamma2 antisense producing molecules into the cell without degradation and include a promoter yielding expression of the gene in the cells into which it is delivered. In some embodiments the delivery vectors are derived from either a virus or a retrovirus. Viral vectors are, for example, Adenovirus, Adeno-associated virus, Herpes virus, Vaccinia virus, Polio virus, AIDS virus, neuronal trophic virus, Sindbis and other RNA viruses, including these viruses with the HIV backbone. Also preferred are any viral families which share the properties of these viruses which make them suitable for use as vectors. Retroviruses include Murine Moloney Leukemia virus, MMLV, and retroviruses that express the desirable properties of MMLV as a vector. Retroviral vectors are able to carry a larger genetic payload, i.e., a transgene or marker gene, than other viral vectors, and for this reason are a commonly used vector. However, other than Lentivirus vectors , they are typically not as useful in non-proliferating cells. Adenovirus vectors are relatively stable and easy to work with, have high titers, and can be delivered in aerosol formulation, and can transfect non-dividing cells. Pox viral vectors are large and have several sites for inserting genes, they are thermostable and can be stored at room temperature. A preferred embodiment is a viral vector which has been engineered so as to suppress the immune response of the host organism, elicited by the viral antigens. Preferred vectors of this type will carry coding regions for Interleukin 8 or 10.

Viral vectors can have higher transaction (ability to introduce genes) abilities than chemical or physical methods to introduce genes into cells. Typically, viral vectors contain, nonstructural early genes, structural late genes, an RNA polymerase III transcript, inverted terminal repeats necessary for replication and encapsidation, and promoters to control the transcription and replication of the viral genome. When engineered as vectors, viruses typically have one or more of the early genes removed and a gene or gene/promoter cassette is inserted into the viral genome in place of the removed viral DNA. Constructs of this type can carry up to about 8 kb of foreign genetic material. The necessary functions of the removed early genes are typically supplied by cell lines which have been engineered to express the gene products of the early genes in trans.

### (a) Retroviral Vectors

A retrovirus is an animal virus belonging to the virus family of Retroviridae, including any types, subfamilies, genus, or tropisms. Retroviral vectors, in general, are described by Verma, I.M., Retroviral vectors for gene transfer. In Microbiology-1985, American Society for Microbiology, pp. 229-232, Washington, (1985), which is incorporated by reference herein. Examples of methods for using retroviral vectors for gene therapy are described in U.S. Patent Nos. 4,868,116 and 4,980,286; PCT applications WO 90/02806 and WO 89/07136; and Mulligan, (Science 260:926-932 (1993)); the teachings of which are incorporated herein by reference.

A retrovirus is essentially a package which has packed into it nucleic acid cargo. The nucleic acid cargo carries with it a packaging signal, which ensures that the replicated daughter molecules will be efficiently packaged within the package coat. In addition to the package signal, there are a number of molecules which are needed in cis, for the replication, and packaging of the replicated virus. Typically a retroviral genome, contains the gag, pol, and env genes which are involved in the making of the protein coat. It is the gag, pol, and env genes which are typically replaced by the foreign DNA that it is to be transferred to the target cell. Retrovirus vectors typically contain a packaging signal for incorporation into the package coat, a sequence which signals the start of the gag transcription unit, elements necessary for reverse transcription, including a primer binding site to bind the tRNA primer of reverse transcription, terminal repeat sequences that guide the switch of RNA strands during DNA synthesis, a purine rich sequence 5' to the 3' LTR that serve as the priming site for the synthesis of the second strand of DNA synthesis, and specific sequences near the ends of the LTRs that enable the insertion of the DNA state of the retrovirus to insert into the host genome. The removal of the gag, pol, and env genes allows for about 8 kb of foreign sequence to be inserted into the viral genome, become reverse transcribed, and upon replication be packaged into a new retroviral particle. This amount of nucleic acid is sufficient for the delivery of a one to many genes depending on the size of each transcript. It is preferable to include either positive or negative selectable markers along with other genes in the insert.

Since the replication machinery and packaging proteins in most retroviral vectors have been removed (gag, pol, and env), the vectors are typically generated by placing them into a packaging cell line. A packaging cell line is a cell line which has been transfected or transformed with a retrovirus that contains the replication and packaging machinery, but lacks any packaging signal. When the vector carrying the DNA of choice is transfected into these cell lines, the vector containing the gene of interest is replicated and packaged into new retroviral particles, by the machinery provided in cis by the helper cell. The genomes for the machinery are not packaged because they lack the necessary signals.

### (b) Adenoviral Vectors

The construction of replication-defective adenoviruses has been described (Berkner et al., J. Virology 61:1213-1220 (1987); Massie et al., Mol. Cell. Biol. 6:2872-2883 (1986); Haj-Ahmad et al., J. Virology 57:267-274 (1986); Davidson et al., J. Virology 61:1226-1239 (1987); Zhang "Generation and identification of recombinant adenovirus by liposome-mediated transfection and PCR analysis" BioTechniques 15:868-872 (1993)). The benefit of the use of these viruses as vectors is that they are limited in the extent to which they can spread to other cell types, since they can replicate within an initial infected cell, but are unable to form new infectious viral particles. Recombinant adenoviruses have been shown to achieve high efficiency gene transfer after direct, in vivo delivery to airway epithelium, hepatocytes, vascular endothelium, CNS parenchyma and a number of other tissue sites (Morsy, J. Clin. Invest. 92:1580-1586 (1993); Kirshenbaum, J. Clin. Invest. 92:381-387 (1993); Roessler, J. Clin. Invest. 92:1085-1092 (1993); Moullier, Nature Genetics 4:154-159 (1993); La Salle, Science 259:988-990 (1993); Gomez-Foix, J. Biol. Chem. 267:25129-25134 (1992); Rich, Human Gene Therapy 4:461-476 (1993); Zabner, Nature Genetics 6:75-83 (1994); Guzman, Circulation Research 73:1201-1207 (1993); Bout, Human Gene Therapy 5:3-10 (1994); Zabner, Cell 75:207-216 (1993); Caillaud, Eur. J. Neuroscience 5:1287-1291 (1993); and Ragot, J. Gen. Virology 74:501-507 (1993)). Recombinant adenoviruses achieve gene transduction by binding to specific cell surface receptors, after which the virus is internalized by receptor-mediated endocytosis, in the same manner as wild type or replication-defective adenovirus (Chardonnet and Dales, Virology 40:462-477 (1970); Brown and Burlingham, J. Virology 12:386-396 (1973); Svensson and Persson, J. Virology 55:442-449 (1985); Seth, et al., J. Virol. 51:650-655 (1984); Seth, et al., Mol. Cell. Biol. 4:1528-1533 (1984); Varga et al., J. Virology 65:6061-6070 (1991); Wickham et al., Cell 73:309-319 (1993)).

A viral vector can be one based on an adenovirus which has had the E1 gene removed and these virons are generated in a cell line such as the human 293 cell line. In another preferred embodiment both the E1 and E3 genes are removed from the adenovirus genome.

### (c) Adeno-associated viral vectors

Another type of viral vector is based on an adeno-associated virus (AAV). This defective parvovirus is a preferred vector because it can infect many cell types and is nonpathogenic to humans. AAV type vectors can transport about 4 to 5 kb and wild type AAV is known to stably insert into chromosome 19. Vectors which contain this site specific integration property are preferred. An especially preferred embodiment of this type of vector is the P4.1 C vector produced by Avigcn, San Francisco, CA, which can contain the herpes simplex virus thymidine kinase gene, HSV-tk, and/or a marker gene, such as the gene encoding the green fluorescent protein, GFP.

In another type of AAV virus, the AAV contains a pair of inverted terminal repeats (ITRs) which flank at least one cassette containing a promoter which directs cell-specific expression operably linked to a heterologous gene. Heterologous in this context refers to any nucleotide sequence or gene which is not native to the AAV or B 19 parvovirus.

Typically the AAV and B19 coding regions have been deleted, resulting in a safe, noncytotoxic vector. The AAV ITRs, or modifications thereof, confer infectivity and site-specific integration, but not cytotoxicity, and the promoter directs cell-specific expression. United states Patent No. 6,261,834 is herein incorporated by reference for material related to the AAV vector.

The vectors of the present invention thus provide DNA molecules which are capable of integration into a mammalian chromosome without substantial toxicity.

The inserted genes in viral and retroviral usually contain promoters, and/or enhancers to help control the expression of the desired gene product. A promoter is generally a sequence or sequences of DNA that function when in a relatively fixed location in regard to the transcription start site. A promoter contains core elements required for basic interaction of RNA polymerase and transcription factors, and may contain upstream elements and response elements.

### (d) Large payload viral vectors

Molecular genetic experiments with large human herpesviruses have provided a means whereby large heterologous DNA fragments can be cloned, propagated and established in cells permissive for infection with herpesviruses (Sun et al., Nature genetics 8: 33-41, 1994; Cotter and Robertson,.Curr Opin Mol Ther 5: 633-644, 1999). These large DNA viruses (herpes simplex virus (HSV) and Epstein-Barr virus (EBV), have the potential to deliver fragments of human heterologous DNA > 150 kb to specific cells. EBV recombinants can maintain large pieces of DNA in the infected B-cells as episomal DNA. Individual clones carried human genomic inserts up to 330 kb appeared genetically stable The maintenance of these episomes requires a specific EBV nuclear protein, EBNA1, constitutively expressed during infection with EBV. Additionally, these vectors can be used for transfection, where large amounts of protein can be generated transiently in vitro. Herpesvirus amplicon systems are also being used to package pieces of DNA > 220 kb and to infect cells that can stably maintain DNA as episomes.

Other useful systems include, for example, replicating and host-restricted non-replicating vaccinia virus vectors.

### (2) Non-nucleic acid based systems

The disclosed compositions can be delivered to the target cells in a variety of ways. For example, the compositions can be delivered through electroporation, or through lipofection, or through calcium phosphate precipitation. The delivery mechanism chosen will depend in part on the type of cell targeted and whether the delivery is occurring for example in vivo or in vitro.

Thus, the compositions can comprise, in addition to the disclosed nucleic acids and proteins or vectors for example, lipids such as liposomes, such as cationic liposomes (e.g., DOTMA, DOPE, DC-cholesterol) or anionic liposomes. Liposomes can further comprise proteins to facilitate targeting a particular cell, if desired. Administration of a composition comprising a compound and a cationic liposome can be administered to the blood afferent to a target organ or inhaled into the respiratory tract to target cells of the respiratory tract. Regarding liposomes, see, e,g., Brigham et al. Am. J. Resp. Cell. Mol. Biol, 1:95-100 (1989); Felgner et al. Proc. Natl. Acad. Sci USA 84:7413-7417 (1987); U.S. Pat. No.4,897,355. Furthermore, the compound can be administered as a component of a microcapsule that can be targeted to specific cell types, such as macrophages, or where the diffusion of the compound or delivery of the compound from the microcapsule is designed for a specific rate or dosage.

In the methods described above which include the administration and uptake of exogenous DNA into the cells of a subject (i.e., gene transduction or transfection), delivery of the compositions to cells can be via a variety of mechanisms. As one example, delivery can be via a liposome, using commercially available liposome preparations such as LIPOFECTIN, LIPOFECTAMINE (GIBCO-BRL, Inc., Gaithersburg, MD), SUPERFECT (Qiagen, Inc. Hilden, Germany) and TRANSFECTAM (Promega Biotec, Inc., Madison, WI), as well as other liposomes developed according to procedures standard in the art. In addition, the nucleic acid or vector of this invention can be delivered *in vivo* by electroporation, the technology for which is available from Genetronics, Inc. (San Diego, CA) as well as by means of a SONOPORATION machine (ImaRx Pharmaceutical Corp., Tucson, AZ).

The materials may be in solution or suspension (for example, incorporated into microparticles, liposomes, or cells). These may be targeted to a particular cell type via antibodies, receptors, or receptor ligands. The following references are examples of the use of this technology to target specific proteins to tumor tissue (Senter, et al., Bioconjugate Chem., 2:447-451, (1991); Bagshawe, K.D., Br. J. Cancer, 60:275-281, (1989); Bagshawe, et al., Br. J. Cancer, 58:700-703, (1988); Senter, et al., Bioconjugate Chem., 4:3-9, (1993); Battelli, et al., Cancer Immunol. Immunother., 35:421-425, (1992); Pietersz and McKenzie, Immunolog. Reviews, 129:57-80, (1992); and Roffler, et al., Biochem. Pharmacol, 42:2062-2065, (1991)). These techniques can be used for a variety of other specific cell types. Vehicles, among others, include "stealth" and other antibody conjugated liposomes (including lipid mediated drug targeting to colonic carcinoma), receptor mediated targeting of DNA through cell specific ligands, lymphocyte directed tumor targeting, and highly specific therapeutic retroviral targeting of murine glioma cells *in* v*ivo.* The following references are examples of the use of this technology to target specific proteins to tumor tissue (Hughes et al., Cancer Research, 49:6214-6220, (1989); and Litzinger and Huang, Biochimica et Biophysica Acta, 1104:179-187, (1992)). In general, receptors are involved in pathways of endocytosis, either constitutive or ligand induced. These receptors cluster in elathrin-coated pits, enter the cell via clathrin-coated vesicles, pass through an acidified endosome in which the receptors are sorted, and then either recycle to the cell surface, become stored intracellularly, or are degraded in lysosomes. The internalization pathways serve a variety of functions, such as nutrient uptake, removal of activated proteins, clearance of macromolecules, opportunistic entry of viruses and toxins, dissociation and degradation of ligand, and receptor-level regulation. Many receptors follow more than one intracellular pathway, depending on the cell type, receptor concentration, type of ligand, ligand valency, and ligand concentration. Molecular and cellular mechanisms of receptor-mediated endocytosis has been reviewed (Brown and Greene, DNA and Cell Biology 10:6, 399-409 (1991)).

Nucleic acids that are delivered to cell which are to be integrated into the host cell genome, typically contain integration sequences. These sequences are often viral related sequences, particularly when viral based systems are used. These viral integration systems can also be incorporated into nucleic acids which are to be delivered using a non-nucleic acid based system of deliver, such as a liposome, so that the nucleic acid contained in the delivery system can be come integrated into the host genome.

Other general techniques for integration into the host genome include, for example, systems designed to promote homologous recombination with the host genome. These systems typically rely on sequence flanking the nucleic acid to be expressed that has enough homology with a target sequence within the host cell genome that recombination between the vector nucleic acid and the target nucleic acid takes place, causing the delivered nucleic acid to be integrated into the host genome. These systems and the methods necessary to promote homologous recombination are known to those of skill in the art.

### (3) In vivo/ex vivo

As described above, the compositions can be administered in a pharmaceutically acceptable carrier and can be delivered to the subject§s cells *in vivo* and/or *ex vivo* by a variety of mechanisms well known in the art (e.g., uptake of naked DNA, liposome fusion, intramuscular injection of DNA via a gene gun, endocytosis and the like).

If *ex vivo* methods are employed, cells or tissues can be removed and maintained outside the body according to standard protocols well known in the art. The compositions can be introduced into the cells via any gene transfer mechanism, such as, for example, calcium phosphate mediated gene delivery, electroporation, microinjection or proteoliposomes. The transduced cells can then be infused (e.g., in a pharmaceutically acceptable carrier) or homotopically transplanted back into the subject per standard methods for the cell or tissue type. Standard methods are known for transplantation or infusion of various cells into a subject.

It is understood that in certain embodiments, constructs which produce an integrin signal transduction inhibitor are driven by inducible promoters, rather than constitutive promoters. inducible systems provide certain advantages, to the expression of the disclosed constructs. Any inducible system can be used. Also disclosed are cells containing the inducible systems, described herein, and in the Examples. These cells, can be used as model systems in a wide variety of assays, as well as in vivo settings.

### g) Expression systems

The nucleic acids that are delivered to cells typically contain expression controlling systems. For example, the inserted genes in viral and retroviral systems usually contain promoters, and/or enhancers to help control the expression of the desired gene product. A promoter is generally a sequence or sequences of DNA that function when in a relatively fixed location in regard to the transcription start site. A promoter contains core elements required for basic interaction of RNA polymerase and transcription factors, and may contain upstream elements and response elements.

### (1) Viral Promoters and Enhancers

Preferred promoters controlling transcription from vectors in mammalian host cells may be obtained from various sources, for example, the genomes of viruses such as: polyoma, Simian Virus 40 (SV40), adenovirus, retroviruses, hepatitis-B virus and most preferably cytomegalovirus, or from heterologous mammalian promoters, e.g. beta actin promoter. The early and late promoters of the SV40 virus are conveniently obtained as an SV40 restriction fragment which also contains the SV40 viral origin of replication (Fiers et al., Nature, 273: 113 (1978)). The immediate early promoter of the human cytomegalovirus is conveniently obtained as a HindIII E restriction fragment (Greenway, P.J, et al., Gene 18: 355-360 (1982)). Of course, promoters from the host cell or related species also are useful herein.

Enhancer generally refers to a sequence of DNA that functions at no fixed distance from the transcription start site and can be either 5' (Laimins, L. et al., Proc. Natl. Acad. Sci 78: 993 (1981)) or 3' (Lusky, M.L., et al., Mol. Cell Bio. 3: 1108 (1983)) to the transcription unit. Furthermore, enhancers can be within an intron (Banerji, J.L. et al., Cell 33: 729 (1983)) as well as within the coding sequence itself (Osborne, T.F., et al., Mol. Cell Bio. 4: 1293 (1984)). They are usually between 10 and 300 bp in length, and they function in cis. Enhancers function to increase transcription from nearby promoters. Enhancers also often contain response elements that mediate the regulation of transcription. Promoters can also contain response elements that mediate the regulation of transcription. Enhancers often determine the regulation of expression of a gene. While many enhancer sequences are now known from mammalian genes (globin, elastase, albumin, α-fetoprotein and insulin), typically one will use an enhancer from a eukaryotic cell virus for general expression. Preferred examples are the SV40 enhancer on the late side of the replication origin (bp 100-270), the cytomegalovirus early promoter enhancer, the polyoma enhancer on the late side of the replication origin, and adenovirus enhancers.

The promoter and/or enhancer may be specifically activated either by light or specific chemical events which trigger their function. Systems can be regulated by reagents such as tetracycline and dexamethasone. There are also ways to enhance viral vector gene expression by exposure to irradiation, such as gamma irradiation, or alkylating chemotherapy drugs.

In certain embodiments the promoter and/or enhancer region can act as a constitutive promoter and/or enhancer to maximize expression of the region of the transcription unit to be transcribed. In certain constructs the promoter and/or enhancer region be active in all eukaryotic cell types, even if it is only expressed in a particular type of cell at a particular time. A preferred promoter of this type is the CMV promoter (650 bases). Other preferred promoters are SV40 promoters, cytomegalovirus (full length promoter), and retroviral vector LTF.

It has been shown that all specific regulatory elements can be cloned and used to construct expression vectors that are selectively expressed in specific cell types such as melanoma cells. The glial fibrillary acetic protein (GFAP) promoter has been used to selectively express genes in cells of glial origin.

Expression vectors used in eukaryotic host cells (yeast, fungi, insect, plant, animal, human or nucleated cells) may also contain sequences necessary for the termination of transcription which may affect mRNA expression. These regions are transcribed as polyadenylated segments in the untranslated portion of the mRNA encoding tissue factor protein. The 3' untranslated regions also include transcription termination sites. It is preferred that the transcription unit also contain a polyadenylation region. One benefit of this region is that it increases the likelihood that the transcribed unit will be processed and transported like mRNA. The identification and use of polyadenylation signals in expression constructs is well established. It is preferred that homologous polyadenylation signals be used in the transgene constructs. In certain transcription units, the polyadenylation region is derived from the SV40 early polyadenylation signal and consists of about 400 bases. It is also preferred that the transcribed units contain other standard sequences alone or in combination with the above sequences improve expression from, or stability of, the construct.

### (2) Markers

The viral vectors can include nucleic acid sequence encoding a marker product. This marker product is used to determine if the gene has been delivered to the cell and once delivered is being expressed. Preferred marker genes are the *E. Coli* lacZ gene, which encodes ß-galactosidase, and green fluorescent protein.

In some embodiments the marker may be a selectable marker. Examples of suitable selectable markers for mammalian cells are dihydrofolate reductase (DHFR), thymidine kinase, neomycin, neomycin analog G418, hydromycin, and puromycin. When such selectable markers are successfully transferred into a mammalian host cell, the transformed mammalian host cell can survive if placed under selective pressure. There are two widely used distinct categories of selective regimes. The first category is based on a cell's metabolism and the use of a mutant cell line which lacks the ability to grow independent of a supplemented media. Two examples are: CHO DHFR- cells and mouse LTK- cells. These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells which were not transformed with the DHFR or TK gene will not be capable of survival in non-supplemented media.

The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, (Southern P. and Berg, P., J. Molec. Appl. Genet. 1: 327 (1982)), myoophenolio acid, (Mulligan, R.C. and Berg, P. Science 209: 1422 (1980)) or hygromycin, (Sugden, B. et al., Mol. Cell. Biol. 5: 410-413 (1985)), The three examples employ bacterial genes under eukaryotic control to convey resistance to the appropriate drug G418 or neomycin (geneticin), xgpt (mycophenolic acid) or hygromycin, respectively. Others include the neomycin analog G418 and puromycin.

### h) Peptides

### (1) Protein variants

As discussed herein there are numerous variants of the beta4 integrin protein, alpha6 integrin protein, and gamma2 laminin5 protein, for example, that are known and herein contemplated. In addition, to the known functional homologue variants there are derivatives of the beta4, alpha6, and gamma2, and other disclosed proteins which also function in the disclosed methods and compositions. Protein variants and derivatives are well understood to those of skill in the art and in can involve amino acid sequence modifications. For example, amino acid sequence modifications typically fall into one or more of three classes: substitutional, insertional or deletional variants. Insertions include amino and/or carboxyl terminal fusions as well as intrasequence insertions of single or multiple amino acid residues. Insertions ordinarily will be smaller insertions than those of amino or carboxyl terminal fusions, for example, on the order of one to four residues. Immunogenic fusion protein derivatives are made by fusing a polypeptide sufficiently large to confer immunogenicity to the target sequence by cross-linking in vitro or by recombinant cell culture transformed with DNA encoding the fusion. Deletions are characterized by the removal of one or more amino acid residues from the protein sequence. Typically, no more than about from 2 to 6 residues are deleted at any one site within the protein molecule. These variants ordinarily are prepared by site specific mutagenesis of nucleotides in the DNA encoding the protein, thereby producing DNA encoding the variant, and thereafter expressing the DNA in recombinant cell culture. Techniques for making substitution mutations at predetermined sites in DNA having a known sequence are well known, for example M13 primer mutagenesis and PCR mutagenesis. Amino acid substitutions are typically of single residues, but can occur at a number of different locations at once; insertions usually will be on the order of about from 1 to 10 amino acid residues; and deletions will range about from 1 to 30 residues. Deletions or insertions preferably are made in adjacent pairs, i.e. a deletion of 2 residues or insertion of 2 residues. Substitutions, deletions, insertions or any combination thereof may be combined to arrive at a final construct. The mutations must not place the sequence out of reading frame and preferably will not create complementary regions that could produce secondary mRNA structure. Substitutional variants are those in which at least one residue has been removed and a different residue inserted in its place. Such substitutions generally are made in accordance with the following Tables 1 and 2 and are referred to as conservative substitutions.

**TABLE 1: Amino Acid Abbreviations**

| Amino Acid | Abbreviations | |
|---|---|---|
| alanine | Ala | A |
| allosoleucine | AIle | |
| arginine | Arg | R |
| asparagine | Asn | N |
| aspartic acid | Asp | D |
| cysteine | Cys | C |
| glutamic acid | Glu | E |
| glutamine | Gln | K |
| glycine | Gly | G |
| histidine | His | H |
| isolelucine | Ile | I |
| leucine | Leu | L |
| lysine | Lys | K |
| phenylalanine | Phe | F |
| proline | Pro | P |
| pyroglutamic acid | pGlu | |
| serine | Ser | S |
| threonine | Thr | T |
| tyrosine | Tyr | Y |
| tryptophan | Trp | W |
| valine | Val | V |

**TABLE 2: Amino Acid Substitutions**

| Original Residue | Exemplary Conservative Substitutions, others are known in the art. | |
|---|---|---|
| Ala | | ser |
| Arg | | lys, gln |
| Asn | | gln; his |
| Asp | | glu |
| Cys | | ser |
| Gln | | asn, lys |
| Glu | | asp |
| Gly | | pro |
| His | | asn;gln |
| Ile | | leu; val |
| Leu | | ile; val |
| Lys | | arg; gln; |
| Met | | Leu; ile |
| Phe | | met; leu; tyr |
| Ser | | thr |
| Thr | | ser |
| Trp | | tyr |
| Tyr | | trp; phe |
| Val | | ile; leu |

Substantial changes in function or immunological identity are made by selecting substitutions that are less conservative than those in Table 2, i.e., selecting residues that differ more significantly in their effect on maintaining (a) the structure of the polypeptide backbone in the area of the substitution, for example as a sheet or helical conformation, (b) the charge or hydrophobicity of the molecule at the target site or (c) the bulk of the side chain. The substitutions which in general are expected to produce the greatest changes in the protein properties will be those in which (a) a hydrophilic residue, e.g. seryl or threonyl, is substituted for (or by) a hydrophobic residue, e.g. leucyl, isoleucyl, phenylalanyl, valyl or alanyl; (b) a cysteine or proline is substituted for (or by) any other residue; (c) a residue having an electropositive side chain, e.g., lysyl, arginyl, or histidyl, is substituted for (or by) an electronegative residue, e.g., glutamyl or aspartyl; or (d) a residue having a bulky side chain, e.g., phenylalanine, is substituted for (or by) one not having a side chain, e.g., glycine, in this case, (e) by increasing the number of sites for sulfation and/or glycosylation.

For example, the replacement of one amino acid residue with another that is biologically and/or chemically similar is known to those skilled in the art as a conservative substitution. For example, a conservative substitution would be replacing one hydrophobic residue for another, or one polar residue for another. The substitutions include combinations such as, for example, Gly, Ala; Val, Ile, Leu: Asp, Glu; Asn, Gln; Ser, Thr; Lys, Arg; and Phe, Tyr. Such conservatively substituted variations of each explicitly disclosed sequence are included within the mosaic polypeptides provided herein.

Substitutional or deletional mutagenesis can be employed to insert sites for N-glycosylation (Asn-X-Thr/Ser) or O-glycosylation (Ser or Thr). Deletions of cysteine or other labile residues also may be desirable. Deletions or substitutions of potential proteolysis sites, e.g. Arg, is accomplished for example by deleting one of the basic residues or substituting one by glutaminyl or histidyl residues.

Certain post-translational derivatizations are the result of the action of recombinant host cells on the expressed polypeptide. Glutaminyl and asparaginyl residues are frequently post-translationally deamidated to the corresponding glutamyl and asparyl residues. Alternatively, these residues are deamidated under mildly acidic conditions. Other post-translational modifications include hydroxylation of proline and lysine, phosphorylation of hydroxyl groups of seryl or threonyl residues, methylation of the o-amino groups of lysine, arginine, and histidine side chains (T.E. Creighton, Proteins: Structure and Molecular Properties, W. H. Freeman & Co., San Francisco pp 79-86 [1983]), acetylation of the N-terminal amine and, in some instances, amidation of the C-terminal carboxyl.

It is understood that one way to define the variants and derivatives of the disclosed proteins herein is through defining the variants and derivatives in terms of homology/identity to specific known sequences. For example, SEQ ID NO:SEQ ID NO:5 sets forth a particular sequence of beta4 integrin cDNA and SEQ ID NO:6 sets forth a particular sequence of a beta4 integrin protein. Specifically disclosed are variants of these and other proteins herein disclosed which have at least, 70% or 75% or 80% or 85% or 90% or 95% homology to the stated sequence. Those of skill in the art readily understand how to determine the homology of two proteins. For example, the homology can be calculated after aligning the two sequences so that the homology is at its highest level.

Another way of calculating homology can be performed by published algorithms. Optimal alignment of sequences for comparison may be conducted by the local homology algorithm of Smith and Waterman Adv. Appl. Math. 2: 482 (1981), by the homology alignment algorithm of Needleman and Wunsch, J. MoL Biol. 48: 443 (1970), by the search for similarity method of Pearson and Lipman, Proc. Natl. Acad. Sci. U.S.A. 85: 2444 (1988), by computerized implementations of these algorithms (GAP, BESTFIT, FASTA, and TFASTA in the Wisconsin Genetics Software Package, Genetics Computer Group, 575 Science Dr., Madison, WI), or by inspection.

The same types of homology can be obtained for nucleic acids by for example the algorithms disclosed in Zuker, M. Science 244:48-52, 1989, Jaeger et al. Proc, Natl. Acad. Sci. USA 86:7706-7710, 1989, Jaeger et al. Methods Enzymol. 183:281-306, 1989 which are herein incorporated by reference for at least material related to nucleic acid alignment,

It is understood that the description of conservative mutations and homology can be combined together in any combination, such as embodiments that have at least 70% homology to a particular sequence wherein the variants are conservative mutations.

As this specification discusses various proteins and protein sequences it is understood that the nucleic acids that can encode those protein sequences are also disclosed. This would include all degenerate sequences related to a specific protein sequence, i.e. all nucleic acids having a sequence that encodes one particular protein sequence as well as all nucleic acids, including degenerate nucleic acids, encoding the disclosed variants and derivatives of the protein sequences. Thus, while each particular nucleic acid sequence may not be written out herein, it is understood that each and every sequence is in fact disclosed and described herein through the disclosed protein sequence. For example, one of the many nucleic acid sequences that can encode the protein sequence set forth in SEQ ID NO:6 is set forth in SEQ ID NO:5. Another nucleic acid sequence that encodes the same protein sequence set forth in SEQ ID NO:6 is set forth in SEQ ID NO: 16. In addition, for example, a disclosed conservative derivative of SEQ ID NO:2 is shown in SEQ ID NO: 17, where the valine (V) at position 34 is changed to a isoleucine (I). It is understood that for this mutation all of the nucleic acid sequences that encode this particular derivative of the beta4 integrin are also disclosed including for example SEQ ID NO: 18 and SEQ ID NO: 19 which set forth two of the degenerate nucleic acid sequences that encode the particular polypeptide set forth in SEQ ID NO: 17. It is also understood that while no amino acid sequence indicates what particular DNA sequence encodes that protein within an organism, where particular variants of a disclosed protein are disclosed herein, the known nucleic acid sequence that encodes that protein in the particular organism from which that protein arises is also known and herein disclosed and described.

### i) Pharmaceutical carriers/Delivery of pharmaceutical products

As described above, the compositions, for example, compositions that inhibit alpha6 function, beta4 function, or gamma2 function can also be administered *in vivo* in a pharmaceutically acceptable carrier. By "pharmaceutically acceptable" is meant a material that is not biologically or otherwise undesirable, i.e., the material may be administered to a subject, along with the nucleic acid or vector, without causing any undesirable biological effects or interacting in a deleterious manner with any of the other components of the pharmaceutical composition in which it is contained. The carrier would naturally be selected to minimize any degradation of the active ingredient and to minimize any adverse side effects in the subject, as would be well known to one of skill in the art.

The compositions may be administered orally, parenterally (e.g., intravenously), by intramuscular injection, by intraperitoneal injection, transdermally, extracorporeally, topically or the like, although topical intranasal administration or administration by inhalant is typically preferred. As used herein, "topical intranasal administration" means delivery of the compositions into the nose and nasal passages through one or both of the nares and can comprise delivery by a spraying mechanism or droplet mechanism, or through aerosolization of the nucleic acid or vector. The latter may be effective when a large number of animals is to be treated simultaneously. Administration of the compositions by inhalant can be through the nose or mouth via delivery by a spraying or droplet mechanism. Delivery can also be directly to any area of the respiratory system (e.g., lungs) via intubation. The exact amount of the compositions required will vary from subject to subject, depending on the species, age, weight and general condition of the subject, the severity of the allergic disorder being treated, the particular nucleic acid or vector used, its mode of administration and the like. Thus, it is not possible to specify an exact amount for every composition. However, an appropriate amount can be determined by one of ordinary skill in the art using only routine experimentation given the teachings herein.

Parenteral administration of the composition, if used, is generally characterized by injection. Injectables can be prepared in conventional forms, either as liquid solutions or suspensions, solid forms suitable for solution of suspension in liquid prior to injection, or as emulsions. A more recently revised approach for parenteral administration involves use of a slow release or sustained release system such that a constant dosage is maintained. See, e.g., U.S. Patent No. 3,610,795, which is incorporated by reference herein.

The materials may be in solution, suspension (for example, incorporated into microparticles, liposomes, or cells). These may be targeted to a particular cell type via antibodies, receptors, or receptor ligands. The following references are examples of the use of this technology to target specific proteins to tumor tissue (Senter, et al., Bioconiugate Chem., 2:447-451, (1991); Bagshawe, K.D., Br. J. Cancer, 60:275-281, (1989); Bagshawe, et al., Br. J._Cancer, 58:700-703, (1988); Senter, et al., Bioconjugate Chem., 4:3-9, (1993); Battelli, et al., Cancer Immunol. Immunother., 35:421-425, (1992); Pietersz and McKenzie, Immunolog, Reviews, 129:57-80, (1992); and Roffler, et al., Biochem. Pharmacol, 42:2062-2065, (1991)). Vehicles such as "stealth" and other antibody conjugated liposomes (including lipid mediated drug targeting to colonic carcinoma), receptor mediated targeting of DNA through cell specific ligands, lymphocyte directed tumor targeting, and highly specific therapeutic retroviral targeting of murine glioma cells *in vivo.* The following references are examples of the use of this technology to target specific proteins to tumor tissue (Hughes et al., Cancer Research, 49:6214-6220, (1989); and Litzinger and Huang, Biochimica et Biophysica Acta, 1104:179-187, (1992)). In general, receptors are involved in pathways of endocytosis, either constitutive or ligand induced. These receptors cluster in clathrin-coated pits, enter the cell via clathrin-coated vesicles, pass through an acidified endosome in which the receptors are sorted, and then either recycle to the cell surface, become stored intracellularly, or are degraded in lysosomes. The internalization pathways serve a variety of functions, such as nutrient uptake, removal of activated proteins, clearance of macromolecules, opportunistic entry of viruses and toxins, dissociation and degradation of ligand, and receptor-level regulation. Many receptors follow more than one intracellular pathway, depending on the cell type, receptor concentration, type of ligand, ligand valency, and ligand concentration. Molecular and cellular mechanisms of receptor-mediated endocytosis has been reviewed (Brown and Greene, DNA and Cell Biology 10:6, 399-409 (1991)).

### (1) Pharmaceutically Acceptable Carriers

The compositions, including antibodies, can be used therapeutically in combination with a pharmaceutically acceptable carrier.

Pharmaceutical carriers are known to those skilled in the art. These most typically would be standard carriers for administration of drugs to humans, including solutions such as sterile water, saline, and buffered solutions at physiological pH. The compositions can be administered intramuscularly or subcutaneously. Other compounds will be administered according to standard procedures used by those skilled in the art.

Pharmaceutical compositions may include carriers, thickeners, diluents, buffers, preservatives, surface active agents and the like in addition to the molecule of choice. Pharmaceutical compositions may also include one or more active ingredients such as antimicrobial agents, anti-inflammatory agents, anesthetics, and the like.

The pharmaceutical composition may be administered in a number of ways depending on whether local or systemic treatment is desired, and on the area to be treated. Administration may be topically (including ophthalmically, vaginally, rectally, intranasally), orally, by inhalation, or parenterally, for example by intravenous drip, subcutaneous, intraperitoneal or intramuscular injection. The disclosed antibodies can be administered intravenously, intraperitoneally, intramuscularly, subcutaneously, intracavity, or transdermally.

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

Formulations for topical administration may include ointments, lotions, creams, gels, drops, suppositories, sprays, liquids and powders. Conventional pharmaceutical carriers, aqueous, powder or oily bases, thickeners and the like may be necessary or desirable.

Compositions for oral administration include powders or granules, suspensions or solutions in water or non-aqueous media, capsules, sachets, or tablets. Thickeners, flavorings, diluents, emulsifiers, dispersing aids or binders may be desirable.

Some of the compositions may potentially be administered as a pharmaceutically acceptable acid- or base- addition salt, formed by reaction with inorganic acids such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mono-, di-, trialkyl and aryl amines and substituted ethanolamines.

### (2) Therapeutic Uses

The dosage ranges for the administration of the compositions are those large enough to produce the desired effect in which the symptoms disorder are effected. The dosage should not be so large as to cause adverse side effects, such as unwanted cross-reactions, anaphylactic reactions, and the like. Generally, the dosage will vary with the age, condition, sex and extent of the disease in the patient and can be determined by one of skill in the art. The dosage can be adjusted by the individual physician in the event of any counterindications, Dosage can vary, and can be administered in one or more dose administrations daily, for one or several days.

Other compositions which do not have a specific pharmaceutical function, but which may be used for tracking changes within cellular chromosomes or for the delivery of diagnostic tools for example can be delivered in ways similar to those described for the pharmaceutical products.

### i) Chips and micro arrays

Disclosed are chips where at least one address is the sequences or part of the sequences set forth in any of the nucleic acid sequences disclosed herein. Also disclosed are chips where at least one address is the sequences or portion of sequences set forth in any of the peptide sequences disclosed herein.

Also disclosed are chips where at least one address is a variant of the sequences or part of the sequences set forth in any of the nucleic acid sequences disclosed herein. Also disclosed are chips where at least one address is a variant of the sequences or portion of sequences set forth in any of the peptide sequences disclosed herein.

### k) Computer readable mediums

It is understood that the disclosed nucleic acids and proteins can be represented as a sequence consisting of the nucleotides of amino acids. There are a variety of ways to display these sequences, for example the nucleotide guanosine can be represented by G or g. Likewise the amino acid valine can be represented by Val or V. Those of skill in the art understand how to display and express any nucleic acid or protein sequence in any of the variety of ways that exist, each of which is considered herein disclosed. Specifically contemplated herein is the display of these sequences on computer readable mediums, such as, commercially available floppy disks, tapes, chips, hard drives, compact disks, and video disks, or other computer readable mediums. Also disclosed are the binary code representations of the disclosed sequences. Those of skill in the art understand what computer readable mediums. Thus, computer readable mediums on which the nucleic acids or protein sequences are recorded, stored, or saved.

Disclosed are computer readable mediums comprising the sequences and information regarding the sequences set forth herein. Also disclosed are computer readable mediums comprising the sequences and information regarding the sequences set forth herein wherein the sequences do not include SEQ ID NOs: SEQ ID NOs: 1-19.

### l) Kits

Disclosed herein are kits that are drawn to reagents that can be used in practicing the methods disclosed herein. The kits can include any reagent or combination of reagent discussed herein or that would be understood to be required or beneficial in the practice of the disclosed methods. For example, the kits could include primers to perform the amplification reactions discussed in certain embodiments of the methods, as well as the buffers and enzymes required to use the primers as intended. For example, disclosed is a kit for assessing a subject's risk for acquiring cancer, such as colon cancer, comprising the primers or probes that hybridize to the sequences set forth in SEQ ID NOs: 1, 3, 5, 7, 9, 11, and 13, for example.

### m) Compositions with similar functions

It is understood that the compositions disclosed herein have certain functions, such as inhibiting gamma2 function or binding alpha6 integrin or inhibiting beta4 function. Disclosed herein are certain structural requirements for performing the disclosed functions, and it is understood that there are a variety of structures which can perform the same function which are related to the disclosed structures, and that these structures will ultimately achieve the same result, for example stimulation or inhibition alpha6beta4 signaling or interruption of the alpha6beta4 signaling pathway.

### 2. Methods of making the compositions

The compositions disclosed herein and the compositions necessary to perform the disclosed methods can be made using any method known to those of skill in the art for that particular reagent or compound unless otherwise specifically noted.

### a) Nucleic acid synthesis

For example, the nucleic acids, such as, the oligonucleotides to be used as primers can be made using standard chemical synthesis methods or can be produced using enzymatic methods or any other known method. Such methods can range from standard enzymatic digestion followed by nucleotide fragment isolation (see for example, Sambrook et al., Molecular Cloning: A Laboratory Manual, 2nd Edition (Cold Spring Harbor Laboratory Press, Cold Spring Harbor, N.Y., 1989) Chapters 5, 6) to purely synthetic methods, for example, by the cyanoethyl phosphoramidite method using a Milligen or Beckman System 1Plus DNA synthesizer (for example, Model 8700 automated synthesizer of Milligen-Biosearch, Burlington, MA or ABI Model 380B). Synthetic methods useful for making oligonucleotides are also described by Ikuta et al., Ann. Rev. Biochem. 53:323-356 (1984), (phosphotriester and phosphite-triester methods), and Narang et al., Methods Enzymol., 65:610-620 (1980), (phosphotriester method). Protein nucleic acid molecules can be made using known methods such as those described by Nielsen et al., Bioconjug. Chem. 5:3-7 (1994).

### b) Peptide synthesis

One method of producing the disclosed proteins, or fragments of the disclosed proteins, such as a fragment of SEQ ID NO:6, is to link two or more peptides or polypeptides together by protein chemistry techniques. For example, peptides or polypeptides can be chemically synthesized using currently available laboratory equipment using either Fmoc (9-fluorenylmethyloxycarbonyl) or Boc (*tert* -butyloxycarbonoyl) chemistry. (Applied Biosystems, Inc., Foster City, CA). One skilled in the art can readily appreciate that a peptide or polypeptide corresponding to the disclosed proteins, for example, can be synthesized by standard chemical reactions. For example, a peptide or polypeptide can be synthesized and not cleaved from its synthesis resin whereas the other fragment of a peptide or protein can be synthesized and subsequently cleaved from the resin, thereby exposing a terminal group which is functionally blocked on the other fragment. By peptide condensation reactions, these two fragments can be covalently joined via a peptide bond at their carboxyl and amino termini, respectively, to form an antibody, or fragment thereof. (Grant GA (1992) Synthetic Peptides: A User Guide. W.H. Freeman and Co., N.Y. (1992); Bodansky M and Trost B., Ed. (1993) Principles of Peptide Synthesis. Springer-Verlag Inc., NY (which is herein incorporated by reference at least for material related to peptide synthesis). Alternatively, the peptide or polypeptide is independently synthesized *in vivo* as described herein. Once isolated, these independent peptides or polypeptides may be linked to form a peptide or fragment thereof via similar peptide condensation reactions.

For example, enzymatic ligation of cloned or synthetic peptide segments allow relatively short peptide fragments to be joined to produce larger peptide fragments, polypeptides or whole protein domains (Abrahmsen L et al., Biochemistry, 30:4151 (1991)). Alternatively, native chemical ligation of synthetic peptides can be utilized to synthetically construct large peptides or polypeptides from shorter peptide fragments. This method consists of a two step chemical reaction (Dawson et al. Synthesis of Proteins by Native Chemical Ligation. Science, 266:776-779 (1994)). The first step is the chemoselective reaction of an unprotected synthetic peptide--thioester with another unprotected peptide segment containing an amino-terminal Cys residue to give a thioester-linked intermediate as the initial covalent product. Without a change in the reaction conditions, this intermediate undergoes spontaneous, rapid intramolecular reaction to form a native peptide bond at the ligation site (Baggiolini M et al. (1992) FEBS Lett. 307:97-101; Clark-Lewis I et al., J.Biol.Chem., 269:16075 (1994); Clark-Lewis I et al., Biochemistry, 30:3128 (1991); Rajarathnam K et al., Biochemistry 33:6623-30 (1994)).

Alternatively, unprotected peptide segments are chemically linked where the bond formed between the peptide segments as a result of the chemical ligation is an unnatural (non-peptide) bond (Schnolzer, M et al. Science, 256:221 (1992)). This technique has been used to synthesize analogs of protein domains as well as large amounts of relatively pure proteins with full biological activity (deLisle Milton RC et al., Techniques in Protein Chemistry IV. Academic Press, New York, pp. 257-267 (1992)).

The disclosed proteins and polypeptides such as that for SEQ ID NO:6, beta4 integrin, can be made using any traditional recombinant biotechnology method. Examples of such methods can be found in Sambrook et al. which is herein incorporated by reference at least for material related to production of proteins and antibodies.

### c) Processes for making the compositions

Disclosed are processes for making the compositions as well as making the intermediates leading to the compositions. For example, disclosed are nucleic acids in SEQ ID NOs:1, 3, 5, 7, 9, 11, and 13. There are a variety of methods that can be used for making these compositions, such as synthetic chemical methods and standard molecular biology methods. It is understood that the methods of making these and the other disclosed compositions are specifically disclosed.

Disclosed are nucleic acid molecules produced by the process comprising linking in an operative way a nucleic acid molecule comprising the sequence set forth in SEQ ID Nos: 1, 3, 5, 7, 9, 11, or 13 or a fragment thereof, and a sequence controlling the expression of the nucleic acid.

Also disclosed are nucleic acid molecules produced by the process comprising linking in an operative way a nucleic acid molecule comprising a sequence having at least 80% identity to a sequence set forth in SEQ ID Nos:1, 3, 5, 7, 9, 11, or 13 or a fragment thereof, and a sequence controlling the expression of the nucleic acid.

Disclosed are nucleic acid molecules produced by the process comprising linking in an operative way a nucleic acid molecule comprising a sequence that hybridizes under stringent hybridization conditions to a nucleic acid molecule comprising the sequence set forth in SEQ ID Nos: 1, 3, 5, 7, 9, 11, or 13 or a fragment thereof, and a sequence controlling the expression of the nucleic acid.

Disclosed are nucleic acid molecules produced by the process comprising linking in an operative way a nucleic acid molecule comprising a sequence encoding a peptide set forth in SEQ ID Nos:2, 4, 6, 8, 10, 12, or 14 or a fragment thereof, and a sequence controlling an expression of the nucleic acid molecule.

Disclosed are nucleic acid molecules produced by the process comprising linking in an operative way a nucleic acid molecule comprising a sequence encoding a peptide having 80% identity to a peptide set forth in SEQ ID Nos:2, 4, 6, 8, 10, 12, or 14 or a fragment thereof and a sequence controlling an expression of the nucleic acid molecule.

Disclosed are nucleic acids produced by the process comprising linking in an operative way a nucleic acid molecule comprising a sequence encoding a peptide having 80% identity to a peptide set forth in SEQ ID Nos:2, 4, 6, 8, 10, 12, or 14 or a fragment thereof, wherein any change from the sequences set forth in SEQ ID Nos:2, 4, 6, 8, 10, 12, or 14 or a fragment thereof are conservative changes and a sequence controlling an expression of the nucleic acid molecule.

Disclosed are cells produced by the process of transforming the cell with any of the disclosed nucleic acids. Disclosed are cells produced by the process of transforming the cell with any of the non-naturally occurring disclosed nucleic acids.

Disclosed are any of the disclosed peptides produced by the process of expressing any of the disclosed nucleic acids. Disclosed are any of the non-naturally occurring disclosed peptides produced by the process of expressing any of the disclosed nucleic acids. Disclosed are any of the disclosed peptides produced by the process of expressing any of the non-naturally disclosed nucleic acids.

Disclosed are animals produced by the process oftransfecting a cell within the animal with any of the nucleic acid molecules disclosed herein. Disclosed are animals produced by the process of transfecting a cell within the animal any of the nucleic acid molecules disclosed herein, wherein the animal is a mammal. Also disclosed are animals produced by the process of transfecting a cell within the animal any of the nucleic acid molecules disclosed herein, wherein the mammal is mouse, rat, rabbit, cow, sheep, pig, or primate.

Also disclose are animals produced by the process of adding to the animal any of the cells disclosed herein.

### d) Products produced from selection protocols

Also disclosed are methods of obtaining molecules that act as functional regulators of integrin function, integrin receptor function, and functional regulators of signaling pathways related to integrin receptors, in particular integrin alpha6beta4.

Disclosed are methods for isolating molecules that interact with the proteins set forth in SEQ ID Nos:2, 4, 6, 8, 10, 12, or 14 or a fragment thereof comprising, interacting a library of molecules with the proteins set forth in SEQ ID Nos:2, 4, 6, 8, 10, 12, or 14 or a fragment thereof, removing the unbound molecules, and collecting the molecules that are bound to at least one of the proteins set forth in SEQ ID Nos:2, 4, 6, 8, 10, 12, or 14 or a fragment thereof.

### 3. Methods of using the compositions

### a) Methods of using the compositions as research tools

The compositions can be used for example as targets in combinatorial chemistry protocols or other screening protocols to isolate molecules that possess desired functional properties related to A6B4 and A6B1 signaling pathways.

The disclosed compositions can also be used diagnostic tools related to diseases such as cancer, such as colon cancer.

The disclosed compositions can be used as discussed herein as either reagents in micro arrays or as reagents to probe or analyze existing microarrays. The disclosed compositions can be used in any known method for isolating or identifying single nucleotide polymorphisms. The compositions can also be used in any method for determining allelic analysis of for example, beta4 alleles having varying function, particularly allelic analysis as it relates to beta4 signaling and functions. The compositions can also be used in any known method of screening assays, related to chip/micro arrays. The compositions can also be used in any known way of using the computer readable embodiments of the disclosed compositions, for example, to study relatedness or to perform molecular modeling analysis related to the disclosed compositions.

### b) Methods for affecting cancer

The disclosed compositions can be used to affect the growth of cancer cells because as disclosed herein, the disclosed relationships are fundamental to the ability of cancer cells to continue growing. The disclosed compositions, such as antisense constructs that will inhibit the production of either alpha6, beta4, or the gamma2 chain of laminin5 reduce the proliferation of cancer cells. In fact, the compositions cannot only reduce the proliferation of the cancer cells, but the compositions can kill the cancer cells, as shown herein.

It is understood that cancer is caused by a variety of cellular events, of which certain events related to alpha6 integrin (up regulated), beta4 integrin (upregulated) and gamma2 (upregulated) allow the continued viability of cancer cells, and that interference of these upregulated molecules inhibits the growth and kills the cancer cells. However, there are other known events that can cause non-cancerous cells to become oncogenio. For example, Abl, Ras, EGF receptor, ErB-2, APC, beta-catenin, Arf, Mdm2, p53, Rb, Myc are known to be involved in oncogenesis, and some of these molecules (For exmaple, Ras, APC loss, p53 loss) are directly related to the disclosed target signal transduction pathways mediated by the alpha6beta4 receptor. Just as the presently disclosed compositions can be used as therapeutics targeting the disclosed relationships, so to there are other targets (For example, Abl, ErB-2) for which pharmaceutical compositions have been developed (For example, Glivec, Herceptin, respectively).

It is understood that the disclosed anti-cancer compositions can be used in combination with other anti-cancer compositions. Great benefits can be obtained from using anti-cancer compositions that target different molecules, in the same signal transduction pathway as well as, or in addition to, targeting molecules in different signal transduction pathways than those disclosed herein. Thus, the disclosed compositions which can affect the growth of cancer cells, indeed kill cancer cells, can be used in conjunction with any other chemotherapy, radiation, or any other anti-cancer therapy.

Disclosed are methods of reducing the proliferation of a cancer cell which comprises inhibiting ligand binding to an integrin receptor on the cancer cell, wherein the integrin receptor comprises an integrin.

Also disclosed are methods of reducing the proliferation of a cancer cell which comprises reducing integrin-integrin interaction, integrin receptor clustering interaction, or integrin-non-integrin protein interaction.

Also disclosed are methods of selectively reducing the proliferation of cancer cells which comprises reducing integrins from interacting with one another, integrins from clustering, or integrins from interacting with other proteins associated with cancer cells.

Disclosed are methods of reducing the proliferation of a cancer cell which comprises reducing the production of an integrin by the cancer cell.

Also disclosed are methods of reducing the proliferation of a cancer cell which comprises reducing the production of an integrin receptor ligand by the cancer cell.

Disclosed are methods of reducing the proliferation of a cancer cell which comprises interfering with an integrin signaling pathway.

Also disclosed are methods of selectively killing or reducing the proliferation of cancer cells which comprises inhibiting ligand binding to integrin receptors on the cancer cells, wherein the integrin receptor comprises a B4 integrin.

Disclosed are methods of selectively killing or reducing the proliferation of cancer cells which comprises inhibiting ligand binding to integrin receptors on the cancer cells, wherein the ligand comprises a laminin5.

Also disclosed are methods of selectively killing or reducing the proliferation of cancer cells which comprises inhibiting ligand binding to integrin receptors on the cancer cells, wherein the ligand comprises the gamma2 subunit of the laminin L5.

Disclosed are methods of selectively killing or reducing the proliferation of cancer cells which comprises inhibiting ligand binding to integrin receptors on the cancer cells, wherein the integrin receptor comprises an alpha6 integrin.

Further disclosed are methods of selectively killing or reducing the proliferation of cancer cells which comprises preventing integrin receptor subunits from interacting with one another, preventing integrin clustering, or preventing integrin receptor subunits from interacting with other proteins on cancer cells, wherein the integrin receptor comprises a B4 integrin.

Disclosed are methods of selectively killing or reducing the proliferation of cancer cells which comprises reducing the production of laminin by the cancer cells, wherein the laminin comprises laminin5, and/or any of the subunits of laminin5, such as gamma2.

Also disclosed is a method of selectively killing or reducing the proliferation of cancer cells which comprises interfering with an integrin signaling pathway, wherein the integrin signaling pathway comprises a B4 integrin or an alpha6 integrin or a beta 1 integrin or a laminin or a laminin5 or the gamma2 subunit of laminin5.

Also dislcosed are methods, wherein the integrin receptor comprises integrin B4 and/or wherein the integrin receptor comprises integrin A6, and/or wherein the ligand that binds to the integrin receptor is laminin5, and/or wherein the integrin receptor is A6B4, and/or wherein the ligand comprises laminin5, and/or wherein the ligand comprises the gamma-2 subunit of laminin5, and/or wherein inhibiting ligand binding to an integrin receptor does not occur by using an antisense molecule to A6, and/or wherein inhibiting ligand binding to an integrin receptor comprises contacting a A6 integrin with a composition that inhibits ligand binding, and/or wherein inhibiting ligand binding to an integrin receptor comprises contacting a B4 integrin with a composition that inhibits ligand binding, and/or wherein inhibiting ligand binding to an integrin receptor comprises contacting a laminin5 with a composition that inhibits ligand binding, and/or wherein inhibiting ligand binding to an integrin receptor comprises contacting a gamma-2 subunit with a composition that inhibits ligand binding, and/or wherein reducing integrin-integrin interaction, integrin receptor clustering interaction, or integrin-non-integrin protein interaction does not occur by using a B4-delta-cyt, and/or wherein reducing integrin-integrin interaction, integrin receptor clustering interaction, or integrin-non-integrin protein interaction does not occur by using an antisense molecule to A6, and/or wherein reducing integrin-integrin interaction, integrin receptor clustering interaction, or integrin-non-integrin protein interaction comprises contacting a A6 integrin with a composition that inhibits an interaction between the B4 integrin and another integrin or protein molecule, and/or wherein reducing integrin-integrin interaction, integrin receptor clustering interaction, or integrin-non-integrin protein interaction comprises contacting a B4 integrin with a composition that inhibits the interaction between the B4 integrin and another integrin or protein molecule, and/or wherein reducing integrin-integrin interaction, integrin receptor clustering interaction, or integrin-non-integrin protein interaction comprises contacting a laminin5 with a composition that inhibits ligand binding, and/or wherein reducing integrin-integrin interaction, integrin receptor clustering interaction, or integrin-non-integrin protein interaction comprises contacting a gamma2 subunit with a composition that inhibits ligand binding, and/or wherein the non-integrin protein comprises a growth factor receptor, and/or wherein the non-integrin protein comprises a hemi-desomosome junction, and/or wherein the non-integrin protein comprises a SH2 domain, and/or wherein the non-integrin protein comprises a Shc protein, and/or wherein the non-integrin protein comprises a IRS-1 protein, and/or wherein the non-integrin protein comprises a IRS-2 protein, and/or wherein reducing the production of an integrin does not occur by using an antisense molecule to A6, and/or wherein the production of an integrin is reduced by inhibiting signaling leading to induction of expression of an integrin, and/or wherein reducing the production of an integrin comprises inhibiting alpha6 production, and/or wherein inhibiting alpha6 production further comprises using antisense molecules to alpha6 mRNA, and/or wherein reducing the production of an integrin comprises inhibiting beta4 production, and/or wherein inhibiting beta4 production further comprises using antisense molecules to beta4 mRNA, and/or wherein reducing the production of an integrin receptor ligand comprises inhibiting gamma2 production, and/or wherein reducing the production of an integrin receptor ligand comprises inhibiting laminin production, and/or wherein reducing the production of an integrin receptor ligand comprises inhibiting laminin5 production, and/or wherein interfering with an integrin signaling pathway does not occur by using a B4-delta-cyt, and/or wherein interfering with an integrin signaling pathway does not occur by using an antisense molecule to A6, and/or wherein interfering with an integrin signaling pathway comprises contacting an A6 integrin in the cell with a composition that inhibits ligand binding, and/or wherein interfering with an integrin signaling pathway comprises contacting a B4 integrin with a composition that inhibits ligand binding, and/or wherein interfering with an integrin signaling pathway comprises contacting a laminin5 with a composition that inhibits ligand binding, and/or wherein interfering with an integrin signaling pathway comprises contacting a gamma2 subunit with a composition that inhibits ligand binding, and/or wherein interfering with an integrin signaling pathway comprises contacting the cancer cell with a molecule that interferes with at least one of talin, paxillin, vinculin, a CAS family protein, CRX, NCK, FAK, 1LK, Src, Fyn, Shc, Grb-2, Guaning nucleotide exchange factors, SOS, DOCK 180,. Vav, Syk, P-1-3 kinase, AKT, Bad, Bid, Caspase 9, Cdc42, PAK, Rac, Rho, Rho kinase, Ras, Caveolin, Tetraspan, Receptor-type protein tyrosine phosphatase, SHP-2, Alpha-actinin, Filamin, Cytohesin, Beta3-endonexin, ICAP-1, RACK-1, CIB, actin, receptor tyrosine kinase, IRS-1 or IRS-2, and/or wherein interfering with an integrin signaling pathway comprises contacting the cancer cell with an agent that interferes with post-translational modification of integrins, and/or wherein the post translational modification is glycosylation or phosphorylation.

Also disclosed are methods, wherein the integrin comprises an A6 integrin, and/or wherein the integrin comprises a B4 integrin, and/or further comprising reducing a laminin5-integrin interaction, and/or further comprising reducing a laminin5 gamma2 integrin interaction, and/or wherein the cancer cell comprises normal p53, and/or wherein the proliferation of the cancer cells is not dependent on AKT/PKB, and/or wherein reducing the proliferation of the cancer cells is selective, and/or wherein the cancer cell is not an MDA-MB-435 cell, and/or wherein the cancer cell is not an HMT-3522 cell, and/or wherein the cancer cell is not an RKO colon carcinoma line, and/or wherein the cancer cell does not express exogenous B4 integrin, and/or further comprising contacting the cancer cells with a small molecule, peptide, peptide mimetic, or oligonucleotide or synthetic analog thereof, and/or wherein the cancer cells are contacted with dominant-negative beta 4 integrin, and/or wherein the cancer cell is contacted with an antisense molecule, and/or wherein the antisense molecule is linked to a leader sequence which enables translocation across a cell membrane, and/or wherein the leader sequence binds to a cell surface protein which facilitates internalization, and/or wherein the leader sequence is TAT or antennapedia, or fragment thereof, and/or wherein the antisense molecule is an alpha6 RNA antisense molecule, and/or wherein the small molecule peptide, peptide mimetic, or oligonucleotide or synthetic analog thereof is linked to a carrier, and/or wherein the carrier is at least one of a lipidic carrier, charged carrier, retroviral carrier, TAT or fragment thereof, antennapedia or fragment thereof, or polyethylene glycol, and/or further comprising contacting the cancer cell with another agent which modulates cell signaling, a chemotherapeutic drug, or treated with radiation or angiogenesis inhibitor, and/or wherein reducing the proliferation of cancer cell is *in vitro,* and/or wherein reducing the proliferation of the cancer cell is *in* vivo, and/or wherein the cancer cell is selected from the group consisting of melanoma, adenoma, lymphoma, myeloma, carcinoma, plasmocytoma, sarcoma, glioma, thyoma, leukemia, skin cancer, retinal cancer, breast cancer, prostate cancer, colon cancer, esophageal cancer, stomac cancer, pancreas cancer, brain tumors, lung cancer, ovarian cancer, cervical cancer, hepatic cancer, gastrointestinal cancer, and head and neck cancer cells, and/or wherein the cancer cell is killed, and/or wherein the cancer cell expresses a mutated Ras, and/or wherein the cancer cell expresses a mutated Ras and a mutated p53, and/or wherein the cancer cell expresses a mutated Ras and activates the AKT/PKB protein, and/or wherein the cancer cell expresses a mutated Ras, a mutated p53, and activates the AKT/PKB protein, and/or wherein the cancer cell expresses a mutated APC, and/or wherein the cancer cell expresses a mutated Ras and mutated APC.

Disclosed are methods of reducing the proliferation of a cancer cell in a patient which comprises administering to the patient a composition which inhibits ligand binding to an integrin receptor on the cancer cell, wherein the integrin receptor comprises an integrin.

Also disclosed are methods of reducing the proliferation of a cancer cell in a patient which comprises administering to the patient a composition which reduces integrin-integrin interaction, integrin receptor clustering interaction, or integrin-non-integrin protein interaction.

Also disclosed are methods of reducing the proliferation of a cancer cell in a patient which comprises administering to the patient a composition which reduces the production of an integrin or laminin by the cancer cell.

Further disclosed are methods of reducing the proliferation of a cancer cell in a patient which comprises administering to the patient a composition which interfers with an integrin signaling pathway.

Also disclsoed are methods, wherein the reduction in cancer cell proliferation is selective, and/or wherein the administering is local or systemic, and/or wherein the patient is additionally administered an agent which modulates cell signaling, a chemotherapeutic drug, or treated with radiation or angiogenesis inhibitor, and/or wherein the additional agent is administered serially or in combination, and/or wherein the local administering is direct application to cancer cells, and/or wherein the direct application to cancer cells is performed during surgery, and/or wherein the direct application to cancer cells is performed topically to cancerous tissue, and/or wherein the systemic administration is by subcutaneous, intraperitoneal, intra-arterial, intravenous, or bolus administration, or by application through a catheter or similar apparatus, and/or wherein the systemic administration comprises a long-term release formulation, and/or wherein the systemic administration is by oral administration, and/or wherein the oral administration comprises administering a pill, capsule, tablet, liquid or suspension.

Disclosed are methods of reducing the proliferation of a cancer cell in a patient which comprises administering to a patient a vector comprising coding sequence for a protein or peptide which inhibits ligand binding to integrin receptors on cancer cells wherein the coding sequence is under the control of a promoter which functions in mammalian cells.

Also disclosed are methods of reducing the proliferation of a cancer cell in a patient which comprises administering to a patient a vector comprising coding sequence for a protein or peptide which prevents integrin receptor subunits from interacting with one another, prevents integrin receptor clustering interaction, or prevents integrin receptor subunits from interacting with other proteins on cancer cells wherein the coding sequence is under the control of a promoter which functions in mammalian cells.

Further disclosed are methods of reducing the proliferation of a cancer cell in a patient which comprises administering to a patient a vector comprising coding sequence for a protein or peptide which prevents integrin receptor subunits from interacting with one another, prevents integrin receptor clustering interaction, or prevents integrin receptor subunits from interacting with other proteins in cancer cells wherein the coding sequence is under the control of a promoter which functions in mammalian cells.

Also disclosed are method of reducing the proliferation of a cancer cell in a patient which comprises administering to a patient a vector comprising coding sequence for a protein or peptide which interferes with integrin subunit or laminin production wherein the coding sequence is under the control of a promoter which functions in mammalian cells.

Further disclosed are methods of reducing the proliferation of a cancer cell in a patient which comprises administering to a patient a vector comprising coding sequence for a protein or peptide which interferes with an integrin signaling pathway of cells wherein the coding sequence is under the control of a promoter which functions in mammalian cells.

Also disclosed are methods, wherein the reduction in cancer cell proliferation is selective, and/or wherein the vector is administered directly to a cancer cell, and/or wherein the vector is administered directly to a normal cell, and/or wherein the vector is packaged in a viral vector or liposome, and/or wherein the vector is a retroviral vector, and/or wherein the vector is administered systemically, and/or wherein the direct administration is by topical application, and/or wherein the direct administration is by topical application, and/or wherein the direct administration is performed during surgery, and/or wherein the direct administration is performed during surgery, and/or wherein the patient is an animal, such as a mammal, mouse, rabbit, primate, chimp, ape, goriilla, and human, and/or wherein the cancer cells are selected from the group consisting of melanoma, adenoma, lymphoma, myeloma, carcinoma, plasmocytoma, sarcoma, glioma, thyoma, leukemia, skin cancer, retinal cancer, breast cancer, prostate cancer, colon cancer, esophageal cancer, stomac cancer, pancreas cancer, brain tumors, lung cancer, ovarian cancer, cervical cancer, hepatic cancer, gastrointestinal cancer, and head and neck cancer cells, and/or wherein the patient is additionally administered at least one of another agent which modulates cell signaling, a chemotherapeutic drug, an angiogenesis inhibitor or treated with radiation, and/or wherein the other agent which modifies cell signaling, chemotherapeutic drug, angiogenesis inhibitor or radiation treatment is administered serially or in combination.

### Methods of gene modification and gene disruption

The disclosed compositions and methods can be used in targeted gene disruption and modification in any animal that can undergo these events. Gene modification and gene disruption refer to the methods, techniques, and compositions that surround the selective removal or alteration of a gene or stretch of chromosome in an animal, such as a mammal, in a way that propagates the modification through the germ line of the mammal. In general, a cell is transformed with a vector which is designed to homologously recombine with a region of a particular chromosome contained within the cell, as for example, described herein. This homologous recombination event can produce a chromosome which has exogenous DNA introduced, for example in frame, with the surrounding DNA. This type of protocol allows for very specific mutations, such as point mutations, to be introduced into the genome contained within the cell. Methods for performing this type of homologous recombination are disclosed herein.

One of the preferred characteristics of performing homologous recombination in mammalian cells is that the cells should be able to be cultured, because the desired recombination event occur at a low frequency.

Once the cell is produced through the methods described herein, an animal can be produced from this cell through either stem cell technology or cloning technology. For example, if the cell into which the nucleic acid was transfected was a stem cell for the organism, then this cell, after transfection and culturing, can be used to produce an organism which will contain the gene modification or disruption in germ line cells, which can then in turn be used to produce another animal that possesses the gene modification or disruption in all of its cells. In other methods for production of an animal containing the gene modification or disruption in all of its cells, cloning technologies can be used. These technologies generally take the nucleus of the transfected cell and either through fusion or replacement fuse the transfected nucleus with an oocyte which can then be manipulated to produce an animal. The advantage of procedures that use cloning instead of ES technology is that cells other than ES cells can be transfected. For example, a fibroblast cell, which is very easy to culture can be used as the cell which is transfected and has a gene modification or disruption event take place, and then cells derived from this cell can be used to clone a whole animal. Conditional knockouts can also be made which will conditionally delete expression of the desired molecule, for example, an A6 or integrin or the gamma2 chain of laminin5.

### d) Methods of diagnosing cancer

Methods of diagnosing cancer using the disclosed information and the disclosed molecules. In particular disclosed are methdos of diagnoses that rely on the combined upregulation of both the ligand and the cogante integrin receptor or cognate integrins. It is understood that all of the methods of diagnosis disclosed herein, can be used with any of the disclosed compositions, but they also can be used in conjunction, where for example, both the ligand and the integrin receptor are monitored and correlated with cancer developing.

Disclosed are methods of assessing a subject's risk of developing cancer comprising determining the amount of A6 present in a target cell obtained from the subject, wherein a determination of increased levels of A6 correlates with an increased risk of cancer.

Disclosed are methods of assessing a subject's risk of acquiring cancer comprising determining the amount of B4 present in a target cell obtained from the subject, wherein a determination of increased levels of B4 correlates with an increased risk of cancer.

Disclosed are methods of assessing a subject's risk of acquiring cancer comprising determining the amount of laminin5 present in a target cell obtained from the subject, wherein a determination of increased levels of laminin5 correlates with an increased risk of cancer.

Disclosed are methods of assessing a subject's risk of acquiring cancer comprising determining the amount of gamma2 subunit present in a target cell obtained from the subject, wherein a determination of increased levels of gamma2 subunit correlates with an increased risk of cancer.

Also disclosed are methods, further comprising comparing the amount A6 present to the amount in a control cell, and wherein determining the amount of A6 present in the target cell comprises assaying the amount of A6 mRNA in the cell, and.or wherein the assaying the amount of mRNA in the cell comprises hybridizing a A6 probe to a sample of the subject's mRNA, and/or wherein the assaying the amount of mRNA in the cell comprises hybridizing a A6 primer to a sample of the subject's mRNA, and/or wherein the assaying the amount of mRNA further comprises performing an nucleic acid amplification reaction involving the primer, and/or wherein the nucleic acid amplification reaction comprises reverse transcription, producing a cDNA, and/or wherein the nucleic acid amplification reaction further comprises performing a polymerase chain reaction on the cDNA, and/or wherein determining the amount of A6 present in the target cell comprises assaying the amount of A6 protein in the cell, and/or further comprising comparing the amount B4 present to the amount in a control cell, and/or further comprising comparing the amount laminin5 present to the amount in a control cell, and/or further comprising comparing the amount gamma2 subunit present to the amount in a control cell.

### C. Examples

Throughout this application, various publications are referenced. The disclosures of these publications in their entireties are hereby incorporated by reference into this application in order to more fully describe the state of the art to which this invention pertains. The references disclosed are also individually and specifically incorporated by reference herein for the material contained in them that is discussed in the sentence in which the reference is relied upon.

It will be apparent to those skilled in the art that various modifications and variations can be made in the present invention without departing from the scope or spirit of the invention. Other embodiments of the invention will be apparent to those skilled in the art from consideration of the specification and practice of the invention disclosed herein. It is intended that the specification and examples be considered as exemplary only, with a true scope and spirit of the invention being indicated by the following claims.

The following examples are put forth so as to provide those of ordinary skill in the art with a complete disclosure and description of how the compounds, compositions, articles, devices and/or methods claimed herein are made and evaluated, and are intended to be purely exemplary of the invention and are not intended to limit the scope of what the inventors regard as their invention. Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric.

### 1. Example 1 Colonic epithelial cells dependent on alpha6beta4 receptor signal transduction for growth in the absence of ECM

Integrin alpha6 and integrin beta4 and laminin gamma2 chains are essential for tumor cell survival in vivo. The data disclosed herein indicate that interfering with integrin alpha6-mediated signaling, integrin beta 4signaling, and laminin gamma2 signaling can constitute an effective approach to induce programmed cell death in cancer cells without damaging normal cells. Ablation of integrin alpha6-dependent signaling, integrin beta4-dependent signaling, and laminin gamma2- dependent signaling in human cancer cell lines supports this.

The murine colonic epithelial transformation system introduced by D'Abaco et al. (1996) was used. In this system, control cells (control), cells containing an activated ras oncogene (Ras), a deletion in the APC gene (APCmin) or both alterations together (Ras+APCmin) can be compared with regard to their proliferation characteristics in tissue culture. The cells were derived from transgenic mice containing a temperature-sensitive allele of SV40 large T under the control of a gamma interferon-inducible promoter permitting conditional immortalization (Jat *et al.,* 1991), All experiments were carried out with the cells being kept at non-permissive temperature in the absence of gamma interferon. As shown previously (D'Abaco *et al.,* 1996), only the cells carrying activated Ras and the APCmin mutation were able to form colonies in soft agar in the absence of anchorage to a substratum. All other cell populations did not give rise to colonies under these conditions.

The frequency of tunel-staining cells (Fig.2A) and caspase 3 activity (Fig. 2B) in control, Ras, APCmin and Ras+APCmin colonic epithelial cells (D'Abaco *et al.,* 1996) in suspension was measured. Both assays show a strong suppression of apoptosis in Ras-APCmin cells, indicating that Ras and APCmin co-operate in preventing cell death in the absence of apparent ECM contacts. Activated Ras can protect cells from apoptosis via activation of the serine-threonine kinase AKT (Kauffmann-Zeh *et al.,* 1997; Khwaja *et al.,* 1997). However, both Ras and Ras+APCmin cells show equivalent levels of AKT phosphorylation (not shown), eliminating AKT as the relevant target of Ras+APCmin cooperation.

Although cancer cells have been thought to survive independently of integrin signaling, they frequently express high levels of alpha3 or alpha6 integrin receptors and/or their ligands (Dedhar *et al.,* 1993; Kennel *et al.,* 1989; Koshikawa *et al.,* 1999; Lohi *et al.,* 2000; Nejjari *et al.,* 1999; Van Waes and Carey, 1992). These integrins are suspected to play a role in invasion and metastasis (Mukhopadhyay *et al.,* 1999; Shaw *et al.,* 1997). Ras and APCmin mutations would induce alterations in the mRNA expression patterns of integrin and ECM components. Induction of alpha6 integrin alone, was specific to Ras+APCmin cells (Fig. 3A). In addition, we found elevated expression of the laminin alpha5, gamma2 and beta2 chains induced in Ras and Ras+APCmin cells (Fig. 3B). These chains are components of laminins 5, 10 and 11 (Malinda and Kleinman, 1996), which are ligands of integrin receptors alpha6/beta4 and alpha3/beta1(Kikkawa *et al.,* 2000; Niessen *et al.,* 1994). These results indicate that Ras and APCmin mutations can cooperate to induce autoorine activation of integrin receptors.

Alpha6 integrin can form functional receptors together with either beta1 or beta4 integrins (Clark and Brugge, 1995), leading to signaling through downstream effectors such as focal adhesion kinase (FAK) or the SH2 domain adapter Shc (Giancotti and Ruoslahti, 1999). Conversely, beta4 integrin only binds to alpha6 integrin (Clark and Brugge, 1995). In transformed colonic epithelial cells alpha6 integrin functions in conjunction with integrin beta4 and is engaged by the laminin gamma2 chain to activate Shc (see Fig. 4). Consistent with alpha6 integrin induction, only Ras+APCmin cells can bind a laminin gamma2-specific peptide in an alpha6 and beta4 integrin-specific manner. In contrast, an alpha3/betal and alpha6/beta1-specific peptide derived from the laminin gamma 1 chain, only binds to control cells (Fig. 4A). In addition, only in Ras+APCmin cells is Shc phosphorylated in response to clustering of the integrin alpha6/beta4 receptor by beta4-specific antibodies (Mainiero *el al.,* 1995) and by the laminin gamma2-specific peptide (Fig. 4B). In both cases p52^{Shc} is the major phosphorylated form. Moreover, alpha6 integrin is required for Shc activation, as Shc phosphorylation is inhibited in response to expression of a dominant-negative beta4 integrin mutant. This mutant lacks the cytoplasmic signaling domain but selectively binds to alpha6 integrin to form a ligand binding, yet signaling-defective alpha6/beta4 integrin (Spinardi *et al.,* 1993) (Fig. 4B)

Anti-sense RNAs specific for alpha6 integrin and gamma2 laminin, as well as dominant-negative beta4 integrin, were expressed and results indicated that integrin alpha6, integrin beta4, and laminin gamma2 expression are relevant for the survival of Ras+APCmin cells in Ras+APCmin, Ras, APC and control cells. All three constructs efficiently inhibit the growth of Ras+APCmin cells in soft agar (not shown) and when attached to plastic (Fig. 5A1). In contrast, the proliferation of wt, Ras and APCmin cells is not affected by this treatment (Fig.5A1). Importantly, the colony fonnation of Ras+APC cells exposed to any of the three inhibitory constructs can be rescued by co-expression of baculovirus p35, a potent inhibitor of caspase activity and of apoptosis (Resnicoff *et al.,* 1998) (Fig. 5A1). Similarly, co-expression of exogenous integrin alpha6 mRNA efficiently rescues Ras-APCmin cell proliferation inhibited by alpha6 anti-sense RNA (Fig. 5A1). This is mirrored by a rescue of alpha6 integrin expression on the cell surface of alpha6 anti-sense RNA expressing cells (Fig. 5B). Moreover, ectopic alpha6 integrin expression also rescues apoptosis induced by dominant-negative beta4 integrin (Fig. 5A1). The latter is expressed at constant levels (Fig. 5B). As one would expect, the cells expressing laminin gamma2 antisense RNA could not be rescued by integrin alpha6 overexpression, but showed significant rescue when plated on dishes coated with laminin gamma2 peptide (Fig. 5A1). Similarly, this peptide increases the survival of Ras+APCmin cells in suspension, as indicated by lower caspase 3 activity (Fig. 2C). In summary, the survival of Ras-APCmin cells depends on the expression of alpha6/beta4 integrin and the laminin gamma2 chain. Conversely, the inhibition of these gene activities leads to selective killing of transformed cells.

Data also indicate that cell death due to lack of alpha6/beta4 integrin receptor signaling in Ras-APCmin cells is independent of the death signaling pathway involving the induction of mitochondrial damage and caspase 9 activity. Instead, the data indicate an involvement of the Fas/TNF receptor/death domain protein/caspase8 pathway (Kruidering and Evan, 2000) in the control of tumor cell survival by alpha6 integrin-containing integrin receptors.

Control, APCmin and Ras cells, which lack alpha6/beta4 integrin receptor signaling activity, show high levels of caspase 8 activity when kept in suspension, while at the same time caspase 9 activity cannot be detected (Fig. 2D).

Although Ras-APCmin cells can be rescued from apoptosis by caspase inhibitor baculovirus p35 (Fig. 5 A1, A2 and text above), cell death induced by expression of dominant-negative beta4 integrin cannot be prevented by expression of the survival factor Bcl2 (see Fig. 5 A2). Bcl2 binds to and neutralizes BH3-domain killer proteins that cause mitochondrial damage, cytochrome C release and caspase 9 activation (Luo *et al.,* 1998).

Ras cells which lack alpha6/beta4 integrin receptor signaling activity and cannot survive in the absence of ECM contacts, and Ras+APCmin cells which depend on alpha6/beta4 integrin receptor signaling for survival, show equivalent levels of AKT phosphorylation (data not shown). This indicates that AKT does not serve as the key target for alpha6/beta4 integrin receptor signaling. AKT has been described to promote cell survival via phosphorylation and inactivation of Bad, a BH3-domain killer protein (Datta *et al.,* 1999).

### 2. Example 2 Other cancer cells dependent on alpha6beta4 receptor signal transduction for growth in the absence of ECM

The survival of highly transformed primary mouse embryo fibroblasts expressing Ras and Myc oncoproteins in conjunction with a homozygous ARF null mutation (Kamijo *et al.,* 1997) depends on expression and function of alpha6 integrin (Fig. 5C), thus demonstrating that this principle also may apply to transformed mesenchymal cells. Moreover, the human colon cancer cell line SW480 carrying multiple oncogenic mutations such as activated Ras (Fujita *et al.,* 1988), amplified c-Myc (Cherif *et al.,* 1988), a mutated APC allele (Munemitsu *et al.,* 1995) and a p53 mutation (Abarzua *el al.,* 1995) is effectively killed by anti-sense integrin alpha6 and laminin gamma2 RNAs as well as dominant-negative beta4 integrin (Fig. 4D). Similarly, integrin alpha6 ablation even leads to apoptosis of Ras/APCmin cells in the presence of active SV40 large T (not shown). Thus the survival of different types of highly transformed cells depends on alpha6 integrin expression, beta4 integrin expression and laminin5 expression irrespective of the status of major tumor supressor genes, such as arf, p53 or rb, and for at least sw480 cells, murine colon epitheilial cells, and the ras/APCmin cells, alpha6, beta4, and laminin5/gamma2 are required. The sensitivity of transformed cells to ablation of integrin signaling, for example, A6B4 integrin receptor signaling, is thus quite remarkable in its apparent generality.

Oncogenic mutations co-operate to engage autocrine integrin signaling. Importantly, this signaling mechanism, involving alpha6 integrin, beta4 integrin and the laminin gamma2 chain, becomes an essential component of the survival mechanism in transformed colonic epithelial cells. In addition, fibroblasts and human colon cancer cells also rely on integrin alpha6 for survival. In contrast, normal and partially transformed cells that express alpha6 integrin at low levels do not require this polypeptide for survival. Thus, integrin signaling inhibition can lead to selective killing of cancer cells.

The laminin-integrin receptor signaling loop is also relevant to the survival of cells transformed by other combinations of oncogenio lesions. Introduction of activated Ras together with dominant-negative p53 (Lloyd et al., 1997) into murine colonic epithelial cells (D'Abaco *et al.,* 1996; see also above) via retroviral infection supports this. Four distinct polyclonal pools of infected cells were derived by drug selection: I) Control (beo/neo) cells, infected with two retroviruses carrying neomycin (neo) or bleomycin (bleo) drug resistance markers, respectively; 2)Ras cells, infected with a Ras/neo virus and a virus with the bleo marker; 3) DNp53 cells, infected with a DNp53/bleo virus and a virus with the neo marker; and 4) Ras/DNp53 cells, infected with a Ras/neo virus and a DNp53/bleo virus.

As the colon epithelial cells contain temperature-sensitive SV40 large T under control of the gamma interferon promoter, all cell populations were drug-selected at the permissive temperature (33°C) in the presence of gamma interferon. All further experiments were carried out at the non-permissive temperature for SV40 large T in the absence of gamma interferon, as described above for Ras+APCmin cells.

Similar to Ras+APCmin cells, only Ras/DNp53 cells but neither bleo/neo, Ras/bleo or DNp53/neo cells were able to grow in the absence of ECM contact in soft agar (not shown). Moreover, Ras and DNp53 cooperate in the suppression of caspase 3 activity in suspension (Fig. 2B), and in the induction of alpha6 integrin expression (Fig. 4A). In Ras/DNp53 cells passaged through a single round of soft agar growth, alpha6 integrin is expressed at even higher levels, demonstrating a correlation between the ability of the cells to survive in the absence of ECM contact and alpha6 integrin expression. In Ras/DNp53 cells beta4 integrin expression levels are also increased, when compared to controls (Fig. 4A). As expected expression of the laminin gamma2 chain is induced in Ras/bleo and Ras/DNp53 cells (Fig. 4B).

Anti-sense RNAs specific for alpha6 integrin and gamma2 laminin, as well as dominant-negative beta4 integrin were expressed in Ras/DNp53, Ras/bleo, DNp53/neo and neo/bleo cells. All three constructs efficiently inhibit the growth of Ras/DNp53 cells when attached to plastic. In contrast, the proliferation of neo/bleo, Ras/bleo and DNp53/neo cells is not affected by this treatment (Fig.5A3). Anti-sense alpha6 integrin or dominant-negative beta4 integrin also inhibit the growth of Ras/DNp53 cells in soft agar (Fig. 5A4).
Integrin alpha6 expression can also be induced by activated Ras and DNp53 in primary murine colon crypt epithelial cells (not shown). Furthermore introduction of Ras and Myc oncoproteins into our control colon epithelial oells also leads to an induction of alpha6 expression (not shown), suggesting that induction of alpha6 integrin expression may be an integral component of distinct oncogene cooperation paradigms

### a) Cell types to be tested

As described herein, there are a variety of cancer and transformed cell types that express alpha6 integrin and laminins at high levels (Dedhar *et al.,* 1993; Koshikawa *et al.,* 1999; Lohi *et al.,* 2000; Van Waes and Carey, 1992). As disclosed herein the following cell lines other than the fibroblasts require alpha6 and beta4 integrin expression, as well as laminin gamma2 chain expression: (1) the human colon carcinoma cell line SW480 (data not shown), (2) Ras+APCmin-transformed murine colon epithelial cells (see Fig. 3A) Murine fibroblasts transformed by activated Ras and Myc in conjunction with homozygous Arf null mutation require alpha6 expression for their survival. These cells do not express integrin beta4, but express betal integrin, indicating the importance of alpha6/betal receptors in the transformed fibroblasts.

### b) xxxInducible expression of inhibitors

To interrupt alpha6 integrin and laminin gamma2 chain-dependent signaling in tumors regulatable alpha6 integrin and laminin gamma2 antisense mRNA expression as well as regulatable expression of the beta4 integrin the dominant-negative polypeptide described in Section C were used. Constitutive expression of these inhibitors leads to rapid cell death in tissue culture. In contrast, the establishment of clonal cell lines with inducible expression of anti-sense mRNAs or dominant-negative beta4 integrin minimize such limitations.

The doxycycline (dox)-inducible reverse tetracycline transactivator (rtTA) is frequently used to overexpress transgenes in a temporally regulated fashion in vitro and in vivo (Efrat *et al.,* 1995; Gossen *et al.,* 1995; Ray *et al.,* 1997). These systems are, however, often compromized by the levels of gene expression in the absence of dox administration. The tetracycline controlled transcriptional silencer (tTS), a fusion protein containing the tet repressor and the KRAB-AB domain of the kid-1 transcriptional repressor, is inhibited by doxycycline. As shown in tissue culture (Freundlieb *el al.,* 1999) and in transgenic mice (Zhu *et al.,* 2001), tTS tightens the control of transgene expression in rtTA-based systems, i.e. tTS effectively eliminates leaky baseline expression without altering the inducibility of rtTA-regulated genes. Thus optimal "off/on" regulation of gene expression can be accomplished with the combined use of tTS and rtTA. The complete expression system is commercially available from CLONTECH. This system can be used for the preparation of all inducible cell lines.

Another regulatable system comprising the estrogen-dependent transactivator GaIER-VP16 and a promoter under the control of Ga14 DNA binding sites (Braselmann *et al.,* 1993) can be used. (Perez-Roger *et al.,* 1997). This particular experimental set up, was used herein to show that the induction of the beta4 integrin dominant-negative mutant in SW480 colon cancer cells induces apoptosis in vitro, as measured by caspase 3 activation (Fig. 2B). For in vivo use, however, a point mutation has to be introduced into the GaIER-VP 16 transactivator that eliminates its sensitivity to estrogen while retaining its response to the anti-estrogen 40H-tamoxifen (Littlewood *et al.,* 1995). 4OH-tamoxifen has been demonstrated to reversibly regulate in vivo the activity of another regulatable transactivator, the MycER chimera (Pelengaris *et al.,* 1999).

Typically, inducible cell lines are first stably transfected with the anti-sense or dominant-negative constructs coupled to the regulatable promoters into the four test cell lines. Subsequently, the activator is introduced and the repressor, such as rtTA and tTS (see above) via infection using recombinant retroviruses with different selectable markers

### D. References

Abarzua, P., LoSardo, J.E., Gubler, M.L. and Neri, A. (1995) Microinjection of monoclonal antibody PAb421 into human SW480 colorectal carcinoma cells restores the transcription activation function to mutant p53. Cancer Res, 55, 3490-4.
Akiyama, S.K., Nagata, K. and Yamada, K.M. (1990) Cell surface receptors for extracellular matrix components. Biochim Biophys Acia, 1031, 91-110.
Braselmann, S., Graninger, P. and Busslinger, M. (1993) A selective transcriptional induction system for mammalian cells based on Gal4-estrogen receptor fusion proteins. Proc Natl Acad Sci USA, 90, 1657-61.
Cherif, D., Le Coniat, M., Suarez, H.G., Bernheim, A. and Berger, R. (1988) Chromosomal localization of amplified c-myc in a human colon adenocarcinoma cell line with a biotinylated probe. Cancer Genet Cytogenet, 33, 245-9.
Clark, E.A. and Brugge, J.S. (1995) Integrins and signal transduction pathways: the road taken. Science, 268, 233-9.
D'Abaco, G.M., Whitehead, R.H. and Burgess, A.W. (1996) Synergy between Apc min and an activated ras mutation is sufficient to induce colon carcinomas. Mol Cell Biol, 16, 884-91.
Dedhar, S., Saulnier, R., Nagle, R. and Overall, C.M. (1993) Specific alterations in the expression of alpha 3 beta 1 and alpha 6 beta 4 integrins in highly invasive and metastatic variants of human prostate carcinoma cells selected by in vitro invasion through reconstituted basement membrane. C/in Exp Metastasis, 11, 391-400.
Efrat, S., Fusco-DeMane, D., Lemberg, H., al Emran, O. and Wang, X. (1995) Conditional transformation of a pancreatic beta-cell line derived from transgenic mice expressing a tetracycline-regulated oncogene. Proc Natl Acad Sci USA, 92, 3576-80.
Freundlieb, S., Schirra-Muller, C. and Bujard, H. (1999) A tetracycline controlled activation/repression system with increased potential for gene transfer into mammalian cells. J Gene Med, 1, 4-12.
Fujita, J., Yoshida, O., Ebi, Y., Nakayama, H., Onoue, H., Rhim, J.S. and Kitamura, Y. (1988) Detection of ras oncogenes by analysis of p21 proteins in human tumor cell lines. Urol Res, 16, 415-8.
Giancotti, F.G. and Mainiero, F. (1994) Integrin-mediated adhesion and signaling in tumorigenesis. Biochim Biophys Acta, 1198, 47-64.
Giancotti, F.G. and Ruoslahti, E. (1999) Integrin signaling. Science, 285, 1028-32.
Gossen, M., Freundlieb, S., Bender, G., Muller, G., Hillen, W. and Bujard, H. (1995) Transcriptional activation by tetracyclines in mammalian cells. Science, 268, 1766-9.
He, T.C., Sparks, A.B., Rago, C., Hermeking, H., Zawel, L., da Costa, L.T., Morin, P.J., Vogelstein, B. and Kinzler, K.W. (1998) Identification of c-MYC as a target of the APC pathway. Science, 281, 1509-12.
Hynes, R.O. (1992) Integrins: versatility, modulation, and signaling in cell adhesion. Cell, 69, 11-25.
Jat, P.S., Noble, M.D., Ataliotis, P., Tanaka, Y., Yannoutsos, N., Larsen, L. and Kioussis, D. (1991) Direct derivation of conditionally immortal cell lines from an H-2Kb-tsA58 transgenic mouse. Proc Natl Acad Sci USA, 88, 5096-100.
Kamijo, T., Zindy, F., Roussel, M.F., Quelle, D.E., Downing, J.R., Ashmun, R.A., Grosveld, G. and Sherr, C.J. (1997) Tumor suppression at the mouse INK4a locus mediated by the alternative reading frame product p19ARF. Cell, 91, 649-59.
Kauffmann-Zeh, A., Rodriguez-Viciana, P., Ulrich, E., Gilbert, C., Coffer, P., Downward, J. and Evan, G. (1997) Suppression of c-Myc-induced apoptosis by Ras signalling through PI(3)K and PKB. Nature, 385, 544-8.
Kennel, S.J., Foote, L.J., Falcioni, R., Sonnenberg, A., Stringer, C.D., Crouse, C. and Hemler, M.E. (1989) Analysis of the tumor-associated antigen TSP-180. Identity with alpha 6-beta 4 in the integrin superfamily. J Biol Chem, 264, 15515-21.
Khwaja, A., Rodriguez-Viciana, P., Wennstrom, S., Wame, P.H. and Downward, J. (1997) Matrix adhesion and Ras transformation both activate a phosphoinositide 3-OH kinase and protein kinase B/Akt cellular survival pathway. Embo J, 16, 2783-93.
Kikkawa, Y., Sanzen, N., Fujiwara, H., Sonnenberg, A. and Sekiguchi, K. (2000) Integrin binding specificity of laminin-10/11: laminin-10/11 are recognized by alpha 3 beta 1, alpha 6 beta 1 and alpha 6 beta 4 integrins. J Cell Sci, 113, 869-76.
Koshikawa, N., Moriyama, K., Takamura, H., Mizushima, H., Nagashima, Y., Yanoma, S. and Miyazaki, K. (1999) Overexpression of laminin gamma2 chain monomer in invading gastric carcinoma cells. Cancer Res, 59, 5596-601.
Land, H., Parada, L.F. and Weinberg, R.A. (1983) Tumorigenic conversion of primary embryo fibroblasts requires at least two cooperating oncogenes. Nature, 304, 596-602.
Lee, J.W, and Juliano, R.L. (2000) alpha5betal integrin protects intestinal epithelial cells from apoptosis through a phosphatidylinositol 3-kinase and protein kinase B- dependent pathway. Mol Biol Cell, 11, 1973-87.
Lin, C.Q. and Bissell, M.J. (1993) Multi-faceted regulation of cell differentiation by extracellular matrix. Faseb J, 7, 737-43.
Littlewood, T.D., Hancock, D.C., Danielian, P.S., Parker, M.G. and Evan, G.I. (1995) A modified oestrogen receptor ligand-binding domain as an improved switch for the regulation of heterologous proteins. Nucleic Acids Res, 23, 1686-90.
Lloyd, A.C., Obermuller, F., Staddon, S., Barth, C.F., McMahon, M. and Land, H. (1997) Cooperating oncogenes converge to regulate cyclin/cdk complexes. Genes Dev, 11, 663-77.
Lohi, J., Oivula, J., Kivilaakso, E., Kiviluoto, T., Frojdman, K., Yamada, Y., Burgeson, R.E., Leivo, I, and Virtanen, I. (2000) Basement membrane laminin-5 is deposited in colorectal adenomas and carcinomas and serves as a ligand for alpha3betal integrin. Apmis, 108, 161-72.
Mainiero, F., Murgia, C., Wary, K.K., Curatola, A.M., Pepe, A., Blumemberg, M., Westwick, J.K., Der, C.J. and Giancotti, F.G. (1997) The coupling of alpha6beta4 integrin to Ras-MAP kinase pathways mediated by Shc controls keratinocyte proliferation. Embo J, 16, 2365-75.
Mainiero, F., Pepe, A., Wary, K.K., Spinardi, L., Mohammadi, M., Schlessinger, J. and Giancotti, F.G. (1995) Signal transduction by the alpha 6 beta 4 integrin: distinct beta 4 subunit sites mediate recruitment of Shc/Grb2 and association with the cytoskeleton of hemidesmosomes. Embo J, 14, 4470-81.
Malinda, K.M, and Kleinman, H.K. (1996) The laminins. Int J Biochem Cell Biol, 28, 957-9.
Marshall, M.S. (1995) Ras target proteins in eukaryotic cells. Faseb J, 9, 1311-8.
Morgenstern, J.P. and Land, H. (1990) Advanced mammalian gene transfer: high titre retroviral vectors with multiple drug selection markers and a complementary helper-free packaging cell line. Nucleic Acids Res, 18, 3587-96.
Mukhopadhyay, R., Theriault, R.L. and Price, J.E. (1999) Increased levels of alpha6 integrins are associated with the metastatic phenotype of human breast cancer cells. Clin Exp Melastasis, 17, 325-32.
Munemitsu, S., Albert, I., Souza, B., Rubinfeld, B. and Polakis, P. (1995) Regulation of intracellular beta-catenin levels by the adenomatous polyposis coli (APC) tumor-suppressor protein. Proc Natl Acad Sci USA, 92, 3046-50.
Nejjari, M., Hafdi, Z., Dumortier, J., Bringuier, A.F., Feldmann, G. and Scoazec, J.Y. (1999) alpha6betal integrin expression in hepatooarcinoma cells: regulation and role in cell adhesion and migration. Int J Cancer, 83, 518-25.
Niessen, C.M., Hogervorst, F., Jaspars, L.H., de Melker, A.A., Delwel, G.O., Hulsman, E.H., Kuikman, I. and Sonnenberg, A. (1994) The alpha 6 beta 4 integrin is a receptor for both laminin and kalinin. Exp Cell Res, 211, 360-7.
Parise, L.V., Lee, J. and Juliano, R.L. (2000) New aspects of integrin signaling in cancer. Semin Cancer Biol, 10, 407-14.
Pawson, T. and Scott, J.D. (1997) Signaling through scaffold, anchoring, and adaptor proteins. Science, 278, 2075-80.
Pelengaris, S., Littlewood, T., Khan, M., Elia, G. and Evan, G. (1999) Reversible activation of c-Myc in skin: induction of a complex neoplastic phenotype by a single oncogenic lesion. Mol Cell, 3, 565-77.
Perez-Roger, I., Kim, S.H., Griffiths, B., Sewing, A. and Land, H. (1999) Cyclins D1 and D2 mediate myc-induced proliferation via sequestration of p27(Kipl) and p21(Cipl). Embo J, 18, 5310-20.
Perez-Roger, I., Solomon, D.L., Sewing, A. and Land, H. (1997) Myc activation of cyclin E/Cdk2 kinase involves induction of cyclin E gene transcription and inhibition of p27(Kipl) binding to newly formed complexes. Oncogene, 14, 2373-81.
Ray, P., Tang, W., Wang, P., Homer, R., Kuhn, C., 3rd, Flavell, R.A. and Elias, J.A. (1997) Regulated overexpression of interleukin 11 in the lung. Use to dissociate development-dependent and -independent phenotypes. J Clin Invest, 100, 2501-11.
Resnicoff, M., Valentinis, B., Herbert, D., Abraham, D., Friesen, P.D., Alnemri, E.S. and Baserga, R. (1998) The baculovirus anti-apoptotic p35 protein promotes transformation of mouse embryo fibroblasts. J Biol Chem, 273, 10376-80.
Roper, E., Weinberg, W., Watt, F.M. and Land, H. (2001) p19ARF-independent induction of p53 and cell cycle arrest by Raf in murine keratinocytes. EMBO Rep, 2, 145-50.
Ruley, H.E. (1983) Adenovirus early region 1A enables viral and cellular transforming genes to transform primary cells in culture. Nature, 304, 602-6.
Sewing, A., Wiseman, B., Lloyd, A.C. and Land, H. (1997) High-intensity Raf signal causes cell cycle arrest mediated by p21Cipl. Mol Cell Biol, 17, 5588-97.
Shaw, L.M., Rabinovitz, I., Wang, H.H., Toker, A. and Mercurio, A.M. (1997) Activation of phosphoinositide 3-OH kinase by the alpha6beta4 integrin promotes carcinoma invasion. Cell, 91, 949-60.
Sonnenberg, A., Linders, C.J., Daams, J.H. and Kennel, S.J. (1990) The alpha 6 beta 1 (VLA-6) and alpha 6 beta 4 protein complexes: tissue distribution and biochemical properties. J Cell Sci, 96, 207-17.
Spinardi, L., Ren, Y.L., Sanders, R. and Giancotti, F.G. (1993) The beta 4 subunit cytoplasmic domain mediates the interaction of alpha 6 beta 4 integrin with the cytoskeleton of hemidesmosomes. Mol Biol Cell, 4, 871-84.
Thompson, T.C., Southgate, J., Kitchener, G. and Land, H. (1989) Multistage carcinogenesis induced by ras and myc oncogenes in a reconstituted organ. Cell, 56, 917-30.
Treisman, R. (1996) Regulation of transcription by MAP kinase cascades. Curr Opin Cell Biol, 8, 205-15.
Van Waes, C. and Carey, T.E. (1992) Overexpression of the A9 antigen/alpha 6 beta 4 integrin in head and neck cancer. Ololaryngol Clin North Am, 25, 1117-39.
Wary, K.K., Mainiero, F., Isakoff, S.J., Marcantonio, E.E. and Giancotti, F.G. (1996) The adaptor protein Shc couples a class of integrins to the control of cell cycle progression. Cell, 87, 733-43.
Zhu, Z., Ma, B., Homer, R.J., Zheng, T. and Elias, J.A. (2001) Use of the tetracycline controlled transcriptional silencer (tTS) to eliminate transgene leak in inducible overexpression transgenic mice. J Biol Chem, 30, 30.

### E. Sequences

### 1. SEQ ID NO:1 Human alpha6 integrin cds (acc# 4557674)

atggccgccgccgggcagctgtgcttgctctacctgtcggcggggctcctgtcccggctcggcgcagccttcaacttggac
actcgggaggacaacgtgatccggaaatatggagaccccgggagcctcttcggcttctcgctggccatgcactggcaactg
cagcccgaggacaagcggctgttgctcgtgggggccccgcgcggagaagcgcttccactgcagagagccaacagaacggga
gggctgtacagctgcgacatcaccgcccgggggccatgcacgcggatcgagtttgataacgatgctgaccccacgtcagaa
agcaaggaagatcagtggatgggggtcaccgtccagagccaaggtccagggggcaaggtcgtgacatgtgctcaccgatat
gaaaaaaggcagcatgttaatacgaagcaggaatcccgagacatctttggcggtgttatgtcctgagtcagaatctcagg

attgaagacgatatggatgggggagattggagcttttgtgatgggcgattgagaggccatgagaaatttggctcttgccag
caaggtgtagcagctacttttactaaagactttcattacattgtatttggagccccgggtacttataactggaaagggatt
gttcgtgtagagcaaaagaataacactttttttgacatgaacatctttgaagatgggccttatgaagttggtggagagact
gagcatgatgaaagtctcgttcctgttcctgctaacagttacttaggtttttctttggactcagggaaaggtattgtttct
aaagatgagatcacttttgtatctggtgctcccagagccaatcacagtggagccgtggttttgctgaagagagacatgaag
tctgcacatctcctccctgagcacatattcgatggagaaggtctggcctcttcatttggctatgatgtggcggtggtggac
ctcaacaaggatgggtggcaagatatagttattggagccccacagtattttgatagagatggagaagttggaggtgcagtg
tatgtctacatgaaccagcaaggcagatggaataatgtgaagccaattcgtcttaatggaaccaaagattctatgtttggc
attgcagtaaaaaatattggagatattaatcaagatggctacccagatattgcagttggagctccgtatgatgacttggga
aaggtttttatctatcatggatctgcaaatggaataaataccaaaccaacacaggttctcaagggtatatcaccttatttt
ggatattcaattgctggaaacatggaccttgatcgaaattcctaccctgatgttgctgttggttccctctcagattcagta
actattttcagatcccggcctgtgattaatattcagaaaaccatcacagtaactcctaacagaattgacctccgccagaaa
acagcgtgtggggcgcctagtgggatatgcctccaggttaaatcctgttttgaatatactgctaaccccgctggttataat
ccttcaatatcaattgtgggcacacttgaagctgaaaaagaaagaagaaaatctgggctatcctcaagagttcagtttcga
aaccaaggttctgagcccaaatatactcaagaactaactctgaagaggcagaaacagaaagtgtgcatggaggaaaccctg
tggctacaggataatatcagagataaactgcgtcccattcccataactgcctcagtggagatccaagagccaagctctcgt
aggcgagtgaattcacttccagaagttcttccaattctgaattcagatgaacccaagacagctcatattgatgttcacttc
ttaaaagagggatgtggagacgacaatgtatgtaacagcaaccttaaactagaatataaattttgcacccgagaaggaaat
caagacaaattttcttatttaccaattcaaaaaggtgtaccagaactagttctaaaagatcagaaggatattgctttagaa
ataacagtgacaaacagcccttccaacccaaggaatcccacaaaagatggcgatgacgcccatgaggctaaactgattgca
acgtttccagacactttaacctattctgcatatagagaactgagggctttccctgagaaacagttgagttgtgttgccaac
cagaatggctcgcaagctgactgtgagctcggaaatccttttaaaagaaattcaaatgtcacttttttatttggttttaagt
acaactgaagtcacctttgacaccccatatctggatattaatctgaagttagaaacaacaagcaatcaagataatttggct
ccaattacagctaaagcaaaagtggttattgaactgcttttatcggtctcgggagttgctaaaccttcccaggtgtatttt
ggaggtacagttgttggcgagcaagctatgaaatctgaagatgaagtgggaagtttaatagagtatgaattcagggtaata
aacttaggtaaacctcttacaaacctcggcacagcaaccttgaacattcagtggccaaaagaaattagcaatgggaaatgg
ttgctttatttggtgaaagtagaatccaaaggattggaaaaggtaacttgtgagccacaaaaggagataaactccctgaac
ctaacggagtctcacaactcaagaaagaaacgggaaattactgaaaaacagatagatgataacagaaaattttctttattt
gctgaaagaaaataccagactcttaactgtagcgtgaacgtgaactgtgtgaacatcagatgcccgctgcgggggctggac
agcaaggcgtctcttattttgcgctcgaggttatggaacagcacatttctagaggaatattccaaactgaactacttggac
attctcatgcgagccttcattgatgtgactgctgctgccgaaaatatcaggctgccaaatgcaggcactcaggttcgagtg
actgtgtttccctcaaagactgtagctcagtattcgggagtaccttggtggatcatcctagtggctattctcgctgggatc
ttgatgcttgctttattagtgtttatactatggaagtgtggtttcttcaagagaaataagaaagatcattatgatgccaca
tatcacaaggctgagatccatgctcagccatctgataaagagaggcttacttctgatgcatag

### 2. SEQ ID NO:2 Human integrin alpha6 protein sequence acc# NP 000201):

1 maaagqlcll ylsagllsrl gaafnldtre dnvirkygdp gslfgfslam hwqlqpedkr
61 lllvgaprge alplqranrt gglyscdita rgpctriefd ndadptsesk edqwmgvtvq
121 sqgpggkvvt cahryekrqh vntkqesrdi fgrcyvlsqn lrieddmdgg dwsfcdgrlr
181 ghekfgscqq gvaatftkdf hyivfgapgt ynwkgivrve qknntffdmn ifedgpyevg
241 getehdeslv pvpansylgf sldsgkgivs kdeitfvsga pranhsgavv llkrdmksah
301 llpehifdge glassfgydv avvdlnkdgw qdivigapqy fdrdgevgga vyvymnqqgr
361 wnnvkpirln gtkdsmfgia vknigdinqd gypdiavgap yddlgkvfiy hgsangintk
421 ptqvlkgisp yfgysiagnm dldrnsypdv avgslsdsvt ifrsrpvini qktitvtpnr
481 idlrqktacg apsgiclqvk scfeytanpa gynpsisivg tleaekerrk sglssrvqfr
541 nqgsepkytq eltlkrqkqk vcmeetlwlq dnirdklrpi pitasveiqe pssrrrvnsl
601 pevlpilnsd epktahidvh flkegcgddn vcnsnlkley kfctregnqd kfsylpiqkg
661 vpelvlkdqk dialeitvtn spsnprnptk dgddaheakl iatfpdtlty sayrelrafp
721 ekqlscvanq ngsqadcelg npfkrnsnvt fylvlsttev tfdtpyldin lklettsnqd
781 nlapitakak vvielllsvs gvakpsqvyf ggtvvgeqam ksedevgsli eyefrvinlg
841 kpltnlgtat lniqwpkeis ngkwllylvk veskglekvt cepqkeinsl nltesnnsrk
901 kreitekqid dnrkfslfae rkyqtlncsv nvncvnircp lrgldskasl ilrsrlwnst
961 fleeysklny ldilmrafid vtaaaenirl pnagtqvrvt vfpsktvaqy sgvpwwiilv
1021 ailagilmla llvfilwkcg ffkrnkkdhy datyhkaeih aqpsdkerlt sda

### 3-SEQ ID NO:3 Mus musculus alpha6 integrin cds (acc# 7110658):

atggccgtcgcgggccagttgtgcctgctctacctgtccgcggggcttctagcccggctgggtacagccttcaacctggac
acccgcgaggacaacgtgatccggaaatcgggggatcccgggagcctcttcggcttctcgctcgccatgcactggcagttg
cagccggaggacaagcggctgttgcttgtgggggcacctcgggcagaagcactcccgctgcagagggcgaacagaacaggg
ggcctgtacagctgtgacatcacctcccgaggaccttgtacacggattgaatttgataatgacgctgatcctatgtcagaa
agcaaggaagaccagtggatgggagtcactgtccagagccaaggtccagggggcaaagtggtgacgtgtgcacatcgatat
gagaaacggcagcacgtcaacacgaagcaggagtcgcgggatatctttggaagatgttatgtcctgagtcagaatctcaga
attgaagatgatatggacggaggagactggagtttctgcgatggccggttgagaggccatgaaaagtttggctcctgtcag
caaggagtagcggctactttcactaaggactttcattacattgttttggagccccaggacttacaactggaaagggatc
gtccgtgtagaacaaaagaataacactttttttgacatgaacatctttgaagatgggccctatgaagttggtggagagaca
gatcatgatgaaagtctcgtgcccgttcctgctaacagttacctaggcttttcgctggactcagggaagggtattgtttct

aaagatgacatcactttgtgtctggtgctccaagagccaatcacagtggggctgtagtgttgctaaaaagagacatgaag
tccgcacatctgctccctgagtatatatttgacggagaaggcctggcttcctcgtttggctatgatgtggcagtggtggac
ctcaatgcagatgggtggcaagacatcgttatcggagctccacagtattttgatagggatggtgaagtcggggtgcagtt
tacgtctacattaaccagcaaggcaaatggagtaatgtgaagccgattcgtctaaatggaccaaagactcgatgtttgga
atctctgtgaaaaatataggtgatattaaccaagatggctatccagatattgctgttggagctccctatgatgatctgggg
aaggtttttatctatcatggatccccgactggcataattaccaagccaacacaggttctcgaggggacatcgccttacttc
ggctattcaatcgctggaatatggacctggatcggaattcctaccccgaccttgctgtgggctccctctcagactcggtc
actattttcagatcccggccagtgattaacattctaaaaaccatcacagtgactcctaacagaattgacctccgccagaag
tccatgtgtggctcacctagcgggatatgcctcaaggttaaagcctgttttgaatatactgcgaaaccttccggttataac
cctccaatatcaatttttgggtattcttgaagctgaaaaagaaagaagaaaatcaggttgtcatcgagagttcagtttcga
aaccaaggttccgagccaaagtatactcaggagctgaccctgaatcggcagaagcagcgggcgtgcatggaggagaccctc
tggctgcaggagaacatcagagacaagctgcgtcccatccccatcacggcttctgtggagatccaggagcccacgtctcgc
cggcgggtgaactcactccccgaagttcttcccatcctgaattcaaatgaagccaaaacggtccagacagatgtccacttc
ttaaaggaaggatgtggagacgacaatgtctgtaacagcaaccttaagctagagtataaatttggtacccgagaaggaaat
caagacaaattctcttaccttccaattcaaaaaggcatcccagaattagtcctaaaagatcagaaagatatagctctggaa
ataacggtgaccaacagcccttcggatccaaggaatccccggaaagatggcgacgatgcccatgaagccaaactcatcgcc
acgtttccagacactctgacatattccgcttacagagaactgagggctttccctgagaagcagctgagctgtgtggccaac
cagaatggctcccaagccgactgtgagctcggaaatcctttcaagagaaattccagtgttactttctatctgatttaagt
acaaccgaggtcacctttgacaccacagatctggatattaatctgaagttggaaacaacaagcaatcaggataaattggct
ccaattacagcgaaggcaaaagtggttattgaattgcttttatccctctccggagtcgctaagccttcgcaggtgtatttt
ggaggtacagttgttggtgagcaagctatgaaatctgaagatgaagtaggaagtttaatagagtatgaatttagggtgatt
aacttaggcaagcctcttaaaaacctcggcacagcaaccttgaatatacagtggcccaaggagattagcaatggcaaatgg
ttgctttatttgatgaaagttgaatccaaaggtttggagcagattgtttgtgagccacacaatgaaataaactacctgaag
ctgaaggagtctcacaactcaagaaagaaacggaacttcctgaaaaacagatagatgacagcaggaaatttctttattt
cctgaaagaaaataccagactctcaactgcagcgtcaacgtcaggtgtgtgaacatcaggtgcccactgcgagggctggac
acgaaggcctctctcgttctgtgttccaggttgtggaacagcacatttctagaggaatattccaaactgaactacttggac
attctcgtgagggcttccatagatgtcaccgctgctgctcagaatatcaagctccctcacgcgggcactcaggttcgagtg
acggtgtttccctcaaagactgtagctcagtattcaggagtagcttggtggatcatcctcctggctgttcttgccgggatt
ctgatgctggctctattagtgttttactgtggaagtgtggcttcttcaagagaaataagaaagatcattacgatgccacc
tatcacaaggctgagatccatactcagccgtctgataaagagaggcttacttccgatgcatag

### 4. SEQ ID NO:4 Mus musculus alpha6 integrin protein sequence (acc# NP 032423)

1 mavagqlcll ylsagllarl gtafnldtre dnvirksgdp gslfgfslam hwqlqpedkr
61 lllvgaprae alplqranrt gglyscdits rgpctriefd ndadpmsesk edqwmgvtvq
121 sqgpggkvvt cahryekrqh vntkqesrdi fgrcyvlsqn lrieddmdgg dwsfcdgrlr
181 ghekfgscqq gvaatftkdf hyivfgapgt ynwkgivrve qknntffdmn ifedgpyevg
241 getdhdeslv pvpansylgf sldsgkgivs kdditfvsga pranhsgavv llkrdmksah
301 llpeyifdge glassfgydv avvdlnadgw qdivigapqy fdrdgevgga vyvyinqqgk
361 wsnvkpirln gtkdsmfgis vknigdinqd gypdiavgap yddlgkvfiy hgsptgiitk
421 ptqvlegtsp yfgysiagnm dldrnsypdl avgslsdsvt ifrsrpvini lktitvtpnr
481 idlrqksmcg spsgiclkvk acfeytakps gynppisilg ileaekerrk sglssrvqfr
541 nqgsepkytq eltlnrqkqr acmeetlwlq enirdklrpi pitasveiqe ptsrrrvnsl
601 pevlpilnsn eaktvqtdvh flkegcgddn vcnsnlkley kfgtregnqd kfsylpiqkg
661 ipelvlkdqk dialeitvtn spsdprnprk dgddaheakl iatfpdtlty sayrelrafp
721 ekqlscvanq ngsqadcelg npfkrnssvt fylilsttev tfdttdldin lklettsnqd
781 klapitakak vvielllsls gvakpsqvyf ggtvvgeqam ksedevgsli eyefrvinlg
841 kplknlgtat lniqwpkeis ngkwllylmk veskgleqiv cephneinyl klkeshnsrk
901 krelpekqid dsrkfslfpe rkyqtlncsv nvrcvnircp lrgldtkasl vlcsrlwnst
961 fleeysklny ldilvrasid vtaaaqnikl phagtqvrvt vfpsktvaqy sgvawwiill
1021 avlagilmla llvfllwkcg ffkrnkkdhy datyhkaeih tqpsdkerlt sda

### 5. SEQ ID NO:5 Human integrin beta4 subunit cds (acc# 6453379):

atggcagggccacgccccagcccatggccaggctgctcctggcagccttgatcagcgtcagcctctctgggaccttgaac
cgctgcaagaaggccccagtgaagagctgcacggagtgtgtccgtgtggataaggactgcgcctactgcacagacgagatg
ttcagggaccggcgctgcaacacccaggcggagctgctggccgcgggctgccagcgggagagcatcgtggtcatggagagc
agcttccaaatcacagaggagacccagattgacaccaccctgcggcgcagccagatgtcccccaaggcctgcgggtccgt
ctgcggcccggtgaggagcggcattttgagctggaggtgtttgagccactggagagccccgtggacctgtacatcctcatg
gacttctccaactccatgtccgatgatctggacaacctcaagaagatggggcagaacctggctcgggtcctgagccagctc
accagcgactacactattggatttggcaagtttgtggacaaagtcagcgtcccgcagacggacatgaggcctgagaagctg
aaggagccttggcccaacagtgacccccccttctccttcaagaacgtcatcagcctgacagaagatgtggatgagttccgg
aataaactgcagggagagcggatctcaggcaacctggatgctcctgagggcggcttcgatgccatcctgcagacagctgtg
tgcacgagggacattggctggcgcccggacagcacccacctgctggtcttctccaccgagtcagccttccactatgaggct
gatggcgccaacgtgctggctggcatcatgagccgcaacgatgaacggtgccacctggacaccacgggcacctacacccag
tacaggacacaggactacccgtcggtgcccaccctggtgcgcctgctcgccaagcacaacatcatccccatctttgctgtc
accaactactcctatagctactacgagaagcttcacacctatttccctgtctcctcactgggggtgctgcaggaggactcg
tccaacatcgtggagctgctggaggaggccttcaatcggatccgctccaacctggacatccgggccctagacagcccccga

ggccttcggacagaggtcacctccaagatgttccagaagacgaggactgggtcctttcacatccggcgggggaagtgggt
atataccaggtgcagctgcgggcccttgagcacgtggatgggacgcacgtgtgccagctgccggaggaccagaagggcaac
atccatctgaaaccttccttctccgacggcctcaagatggacgcgggcatcatctgtgatgtgtgcacctgcgagctgcaa
aaagaggtgcggtcagctcgctgcagcttcaacggagacttcgtgtgcggacagtgtgtgtgcagcgagggctggagtggc
cagacctgcaactgctccaccggctctctgagtgacattcagccctgcctgcgggagggcgaggacaagccgtgctccggc
cgtggggagtgccagtgcgggcactgtgtgtgctacggcgaaggccgctacgagggtcagttctgcgagtatgacaacttc
cagtgtccccgcacttccgggttcctctgcaatgaccgaggacgctgctccatgggccagtgtgtgtgtgagcctggttgg
acaggcccaagctgtgactgtcccctcagcaatgccacctgcatcgacagcaatgggggcatctgtaatggacgtggccac
tgtgagtgtggccgctgccactgccaccagcagtcgctctacacggacaccatctgcgagatcaactactcggcgtccacc
cgggcctctgcgaggacctacgctcctgcgtgcagtgccaggcgtggggcaccggcgagaagaagggcgcacgtgtgagg
aatgcaacttcaaggtcaagatggtggacgagcttaagagaggcgaggaggtggtggtgcgctgctccttccgggacgagg
atgacgactgcacctacagctacaccatggaaggtgacggcgcccctgggcccaacagcactgtcctggtgcacaagaaga
agggactgccctccgggctccttctggtggctcatcccccctgctcctcctcctcctgccgctcctggccctgctactgctg
ctatgctggaagtactgtgcctgctgcaaggcctgcctggcacttctcccgtgctgcaaccgaggtcacatggtgggcttt
aaggaagaccactacatgctgcgggagaacctgatggcctctgaccacttggacacgcccatgctgcgcagcgggaacctc
aagggccgtgacgtggtccgctggaaggtcaccaacaacatgcagcggcctggctttgccactcatgccgccagcatcaac
cccacagagctggtgccctacgggctgtccttgcgcctggcccgcctttgcaccgagaacctgctgaagcctgacactcgg
gagtgcgcccagctgcgccaggaggtggaggagaacctgaacgaggtctacaggcagatctccggtgtacacaagctccag
cagaccaagttccggcagcagcccaatgccgggaaaaagcaagaccacaccattgtggacacagtgctgatggcgccccgc
tcggccaagccggccctgctgaagcttacagagaagcaggtggaacagagggccttccacgacctcaaggtggcccccggc
tactacaccctcactgcagaccaggacgcccggggcatggtggagttccaggagggcgtggagctggtggacgtacgggtg
cccctctttatccggcctgaggatgacgacgagaagcagctgctggtggaggccatcgacgtgcccgcaggcactgccacc
ctcggccgccgcctggtaaacatcaccatcatcaaggagcaagccagagacgtggtgtcctttgagcagcctgagttctcg
gtcagccgcggggaccaggtggcccgcatccctgtcatccggcgtgtcctggacggcgggaagtcccaggtctcctaccgc
acacaggatggcaccgcgcagggcaaccgggactacatccccgtggagggtgagctgctgttccagcctggggaggcctgg
aaagagctgcaggtgaagctcctggagctgcaagaagttgactccctcctgcggggccgccaggtccgccgtttccacgtc
cagctcagcaaccctaagtttggggcccacctgggccagccccactccaccaccatcatcatcagggacccagatgaactg
gaccggagcttcacgagtcagatgttgtcatcacagccacccctcacggcgacctgggcgccccgcagaaccccaatgct
aaggccgctgggtccaggaagatccatttcaactggctgcccccttctggcaagccaatggggtacagggtaaagtactgg
attcagggtgactccgaatccgaagcccacctgctcgacagcaaggtgccctcagtggagctcaccaacctgtacccgtat
tgcgactatgagatgaaggtgtgcgcctacggggctcagggcgagggaccctacagctccctggtgtcctgccgcacccac
caggaagtgcccagcgagccagggcgtctggccttcaatgtcgtctcctccacggtgacccagctgagctgggctgagccg
gctgagaccaacggtgagatcacagcctacgaggtctgctatggcctggtcaacgatgacaaccgacctattgggcccatg
aagaaagtgctggttgacaaccctaagaaccggatgctgcttattgagaaccttcgggagtcccagccctaccgctacacg
gtgaaggcgcgcaacggggccggctgggggcctgagcgggaggccatcatcaacctggccacccagcccaagaggcccatg
tccatccccatcatccctgacatccctatcgtggacgcccagagcggggaggactacgacagcttccttatgtacagcgat
gacgttctacgctctccatcgggcagccagaggcccagcgtctccgatgacactggctgcggctggaagttcgagcccctg
ctgggggaggagctggacctgcggcgcgtcacgtggcggctgcccccggagctcatcccgcgcctgtcggccagcagcggg
cgctcctccgacgccgaggcgccccacggcccccggacgacggcggcgcgggcgggaagggcggcagcctgccccgcagt
gcgacacccgggcccccggagagcacctggtgaatggccggatggactttgccttcccgggcagcaccaactccctgcac
aggatgaccacgaccagtgctgctgcctatggcacccacctgagcccacacgtgccccaccgcgtgctaagcacatcctcc
accctcacacgggactacaactcactgacccgctcagaacactcacactcgaccacactgccgagggactactccaccctc
acctccgtctcctcccacggcctccctcccatctgggaacacgggaggagcaggcttccgctgtcctgggccctggggtcc
cggagtcgggctcagatgaaagggttccccccttccaggggcccacgagactctataatcctggctgggaggccagcagcg
ccctcctgggcccagactctcgcctgactgctggtgtgcccgacacgcccacccgcctggtgttctctgccctggggccc
acatctctcagagtgagctggcaggagccgcggtgcgagcggccgctgcagggctacagtgtggagtaccagctgctgaac
ggcggtgagctgcatcggctcaacatccccaaccctgcccagacctcggtggtggtggaagacctcctgcccaaccactcc
tacgtgttccgcgtgcgggcccagagccaggaaggctggggccgagagcgtgagggtgtcatcaccattgaatcccaggtg
cacccgcagagcccactgtgtcccctgccaggctccgccttcactttgagcactcccagtgccccaggcccgctggtgttc
actgccctgagcccagactcgctgcagctgagctgggagcggccacggaggcccaatggggatatcgtcggctacctggtg
acctgtgagatggcccaaggaggaggtccagccaccgcattccgggtggatggagacagccccgagagccggctgaccgtg
ccgggcctcagcgagaacgtgccctacaagttcaaggtgcaggccaggaccactgagggcttcgggccagagcgcgagggc
atcatcaccatagagtcccaggatggaggtcccttcccgcagctgggcagccgtgccgggctcttccagcacccgctgcaa
agcgagtacagcagcatctccaccacccacaccagcgccaccgagcccttcctagtgggtccgaccctgggggcccagcac
ctggaggcaggcggctccctcacccggcatgtgacccaggagtttgtgagccggacactgaccaccagcggaacccttagc
acccacatggaccaacagttcttccaaacttga

### 6. SEQ ID NO:6 Human beta4 integrin protein sequence (acc# CAB61345):

1 magprpspwa rlllaalisv slsgtlnrck kapvksctec vrvdkdcayc tdemfrdrrc
61 ntqaellaag cqresivvme ssfqiteetq idttlrrsqm spqglrvrlr pgeerhfele
121 vfeplespvd lyilmdfsns msddldnlkk mgqnlarvls qltsdytigf gkfvdkvsvp
181 qtdmrpeklk epwpnsdppf sfknvislte dvdefrnklq gerisgnlda peggfdailq
241 tavctrdigw rpdsthllvf stesafhyea dganvlagim srnderchld ttgtytqyrt
301 qdypsvptlv rllakhniip ifavtnysys yyeklhtyfp vsslgvlqed ssnivellee
361 afnrirsnld iraldsprgl rtevtskmfq ktrtgsfhir rgevgiyqvq lralehvdgt
421 hvcqlpedqk gnihlkpsfs dglkmdagii cdvctcelqk evrsarcsfn gdfvcgqcvc
481 segwsgqtcn cstgslsdiq pclregedkp csgrgecqcg hcvcygegry egqfceydnf

541 qcprtsgflc ndrgrcsmgq cvcepgwtgp scdcplsnat cidsnggicn grghcecgrc
601 hchqqslytd ticeinysas trasartyap acsarrgapa rrrgarvrna tsrsrwwtsl
661 rearrwwcaa psgtrmttap tatpwkvtap lgptalswct rrrdcppgsf wwlipllll
721 lpllalllll cwkycaccka clallpccnr ghmvgfkedh ymlrenlmas dhldtpmlrs
781 gnlkgrdvvr wkvtnnmqrp gfathaasin ptelvpygls lrlarlcten llkpdtreca
841 qlrqeveenl nevyrqisgv hklqqtkfrq qpnagkkqdh tivdtvlmap rsakpallkl
901 tekqveqraf hdlkvapgyy tltadqdarg mvefqegvel vdvrvplfir pedddekqll
961 veaidvpagt atlgrrlvni tiikeqardv vsfeqpefsv srgdqvarip virrvldggk
1021 sqvsyrtqdg taqgnrdyip vegellfqpg eawkelqvkl lelqevdsll rgrqvrrfhv
1081 qlsnpkfgah lgqphsttii irdpdeldrs ftsqmlssqp pphgdlgapq npnakaagsr
1141 kihfnwlpps gkpmgyrvky wiqgdsesea hlldskvpsv eltnlypycd yemkvcayga
1201 qgegpysslv scrthqevps epgrlafnvv sstvtqlswa epaetngeit ayevcyglvn
1261 ddnrpigpmk kvlvdnpknr mllienlres qpyrytvkar ngagwgpere aiinlatqpk
1321 rpmsipiipd ipivdaqsge dydsflmysd dvlrspsgsq rpsvsddtgc gwkfepllge
1381 eldlrrvtwr lppeliprls assgrssdae aphgppddgg aggkggslpr satpgppgeh
1441 lvngrmdfaf pgstnslhrm tttsaaaygt hlsphvphrv lstsstltrd ynsltrsehs
1501 hsttlprdys tltsvsshgl ppiwehgrsr lplswalgsr sraqmkgfpp srgprdsiil
1561 agrpaapswg pdsrltagvp dtptrlvfsa lgptslrvsw qeprcerplq gysveyqlln
1621 ggelhrlnip npaqtsvvve dllpnhsyvf rvraqsqegw greregviti esqvhpqspl
1681 cplpgsaftl stpsapgplv ftalspdslq lswerprrpn gdivgylvtc emaqgggpat
1741 afrvdgdspe srltvpglse nvpykfkvqa rttegfgper egiitiesqd ggpfpqlgsr
1801 aglfqhplqs eyssistth t satepflvgp tlgaqhleag gsltrhvtqe fvsrtlttsg
1861 tlsthmdqqf fqt

### 7. SEO ID NO:7 Mouse integrin beta4 subunit mRNA (not only eds) (acc# L04678):

ggaccgtcgaggcagcgggactgacccagctgggctcactgtattaagaagcggacccgcgacccggagcgcccggggacc
cgatctgggagcctggacgggtgcagcgcgcaggaatgcagtccgcctgactcaccagcgcctccttcctacctgcgccgc
ccgtccataaagcgctgctcgtcccgcccgccgccgccgccctgctgtcccgccgggctcgcccgcgcgctcagctcgacc
caacgcagcccaagtccgaggtagtctcactaaggaggaggaggatggcagggccctgttgcagcccatgggtgaagctgc
tgctgctggcacgaatgctgagtgccagcctccctggagacctggccaaccgctgcaagaaggctcaggtgaagagctgta
ccgagtgcatccgggtggacaagagctgtgcctactgcacagacgagctgttcaaggagaggcgctgcaacacccaggcgg
acgttctggctgcaggctgcaggggagagagcatcctggtcatggagagcagccttgaaatcacagagaacacccagatcg
tcaccagcctgcaccgcagccaggtatctccccaaggcctgcaagtccggctgcggcggggtgaggagcgcacgtttgtgt
tccaggtctttgagcccctggagagccccgtggatctgtatatcctcatggacttctccaactccatgtctgacgatctgg
acaacctcaagcagatggggcagaacctggccaagatcctgcgccagctcaccagcgactacaccattggatttggaaagt
ttgtggacaaagtcagcgtcccacagacagacatgaggcccgagaaactgaaggagccctggcccaacagtgatcccccgt
tctccttcaagaacgttatcagcttaacggagaatgtggaagaattctggaacaaactgcaaggagaacgcatctcaggca
acctggacgctcctgaagggggctttgatgccatcctgcagacagctgtgtgcacaaggacattggctggaggctgaca
gcacccacctgctggtgttctccaccgagtctgccttccactacgaggctgatggtgccaacgttctggccggcatcatga
accgcaatgatgagaaatgccacctggacgcctcgggcgcctacacccaatacaagacacaggactacccatcagtgccca
cgctggttcgcctgcttgccaagcataacatcatccccatctttgctgtcaccaactactcttacagctactatgagaagc
tccataagtatttccccgtctcctctctgggcgtcctgcaggaggattcatccaacatcgtggagctgctggaggaggcct
tctatcgaattcgctccaacctggacatccgggctctggacagccccagaggcctgagaacagaggtcacctccgatactc
tccagaagacggagactgggtcctttcacatcaagcgggggggaagtgggcacatacaatgtgcatctccggcagtggagg
acatagatgggacacatgtgtgccagctggctaaagaagaccaaggggggcaacatccacctgaaaccctccttctctgatg
gcctccggatggacgcgagtgtgatctgtgacgtgtgcccctgtgagctgcaaaaggaagttcgatcagctcgctgtcact
tcagaggagacttcatgtgtggacactgtgtgtgcaatgagggctggagtggcaaaacctgcaactgctccaccggctctc
tgagtgacacacagccctgcctgcgtgagggtgaggacaaaccgtgctcgggccacggcgagtgccagtgcggacgctgtg
tgtgctatggtgaaggccgctacgagggtcacttctgcgagtatgacaacttccagtgtccccggacctctggattcctgt
gcaatgaccggggacgctgttctatgggagagtgtgtgtgtgagcctggttggacaggccgcagctgcgactgtcccctca
gcaatgccacctgcatcgatagcaacgggggcatctgcaacggccgaggctactgtgagtgtggccgttgtcactgcaacc
agcagtcgctctacacggacaccacctgtgagatcaactactctgcgatactgggtctctgtgaggatctccgctcctgcg
tacagtgccaggcctggggcaccggggagaagaaagggcgcgtgtgacgattgcccctttaaagtcaagatggtagacg
agcttaagaaagaagaggtggtggagtactgctccttccgggatgaggatgacgactgcacttacagctacaacgtggagg
gcgacggcagccctgggcccaacagcacagtcctggtccacaaaaagaaagactgcctcccggctccttcctggtggctca
tcccccctgctcatcttcctcctgttgctcctggcgttgcttctgctgctctgctggaaatactgtgcctgctgcaaagcct
gcctggggcttcttccttgctgcaaccgaggtcacatggtgggctttaaggaagatcactatatgcttcgggagaacctga
tggcctctgaccacctggacacgcccatgctacgaagcgggaacctcaagggacgagacacagtccgctggaagatcacca
acaatgtgcagcgccctggctttgccacccatgccgccagcaccagccccacggagctcgtaccctacgggctgtccctgc
gccttggccgcctctgcactgagaaccttatgaagccgggcacccgagagtgtgaccagctacgccaggaggtggaggaaa
atctgaatgaggtgtatagacaggtcagcggcgcacacaagctccagcagacgaagttccgacagcagcccaacgccggga
aaaagcaagaccacaccattgtggacacagtgttgctggcgccccgctccgccaagcagatgctgctgaagctgacagaga
agcaggtggagcaggggtccttccatgaactgaaggtggcccctggctactacactgtcacggcagagcaggatgcccggg
gcatggtggagttccaggagggcgtggagctggtggatgtgcgagtgcccctcttcatccggcctgaggatgatgatgaga
agcagctgctggtggaggccattgatgtccctgtgagcactgccacccttggtcgccgtctggtaaacatcaccattatca
aggaacaagctagtggggtagtgtccttcgagcagcctgaatactcggtgagtcgtggagaccaggtggcccgcatccctg

tcatccggcacatcctggacaatggcaagtcccaggtctcctatagcacacaggataatacagcacacggacaccgggatt
atgttcccgtggagggagagctgctgttccatcctggggaaacctggaaggagttgcaggtgaagctactggagctgcagg
aggttgactccctcctgcgtggccgccaggtccgccgcttccaagtccaactcagcaaccccaagttcggagcccgcctgg
gccagcccagcacaaccaccgttattctcgatgaaacggacaggagtctcataaatcaaacactttcatcgcctccgccac
cccatggagacctgggcgccccacagaaccccaatgccaaggctgccggatccaggaagatccatttcaactggctgcccc
ctcctggcaagccaatggggtacagggtgaagtactggatccagggcgactctgaatctgaagcccaccttctagatagca
aggtgccctcagtggaactcaccaacctgtatccctattgcgactacgaaatgaaggtgtgtgcctatggggccaagggtg
aggggccctatagctcactggtgtcctgccgcacccaccaggaagtacccagtgagccagggaggctggctttcaatgtag
tctcttctacggtgactcagctgagctgggcagagccagctgagaccaatggcgagatcacagcctacgaggtctgctatg
gactggtcaatgaggacaacagacccattggacctatgaagaaggtgctcgtggacaaccccaagaaccggatgctgctca
ttgagaatctgcgagattcccagccataccgatacacggttaaggcgcgcaatggggcaggatggggacccgagagagagg
ctatcatcaacctggctacacagcccaagcggcccatgtccatccctatcatcccagacatccccatagtggacgcccagg
gtggagaagactacgaaaacttccttatgtacagtgatgacgtcctgcggtccccagccagcagccagaggcccagcgttt
ctgatgacactgagcacctggtgaatggccggatggactttgcctatccaggcagcgccaactccctgcacagaatgactg
cagccaatgtggcctatggcacgcatctgagcccacacctgtcccaccgagtgctgagcacgtcctccacccttacccggg
actaccactctctgacacgcacagagcactcccactcaggcacacttcccagggactactccaccctcacttccctttcct
cccaagcctccctcctatctgggaagatgggaggagcaggcttccgctgtcctggactcttgggtccttgagccgggctca
catgaagggtgtgcccgcatccaggggttcaccagactctataatcctggccgggcagtcagcagcaccctcctggggtac
aggattcccgtggggctgtgggtgtgcctgacacacccactcggctggtgttctctgccctggggcgcacgtctttgaagg
tgagctggcaggagccacagtgtgatcggacgctgctgggctacagtgtggaataccagctactaacgtgcgtcgagatgc
atcggctcaacatccctaaccctggccaaacctcggtggtggtagaggatctcctgcctaactactctcatgtgttccggg
tacgggcacagagccaggagggctggggtcgagagcgagagggtgtcatcaccatcgagtcccaggtgcacccgcagagcc
ctctctgcccccctgccaggctcagccttcactctgagcaccccagcgccccaggaccactggtgttcactgccctaagcc
cagactccccgcagctcagctgggagcggccgaggagccgcaatggagatatccttggctacctggtgacctgtgagatgg
cccaaggaggagcaccagccaggaccttccgggtggacggagacaaccctgagagccggttgactgtacctggcctcagtg
agaacgttccttacaagttcaaggttcaggccaggacgaccgagggctttgggccagagcgtgagggtatcatcaccatcg
agtctcaggttggaggccccttcccacagctgggcagcaattctgggctcttccagaacccagtgcaaagcgagttcagca
gcgtgaccagcacgcacagcaccacgactgagcccttcctcatggatggtctaaccctggggacccagcgcctggaagcag
gaggctccctcacccggcatgtgacccaggaattcgtgacccggaccttaacggccagtggctctctcagcactcatatgg
accaacagttcttccaaacctgaacctcccccgcgccccagccacctggcccctccttgcctcctctcctagcgccttct
tcctctgctgctctacccacgagcttgctgaccacagagccagcccctgtagtcagagagcaggggtaggtgctgtccagg
aaccataaagtgggtagaggtgatacaaggtctttctgactgcatcccaccctgggtccaatcccacatgtaacc

### 8. SEQ ID NO:8 Murine integrin beta4 protein sequence:

MAGPCCSPWV KLLLLARMLS ASLPGDLANR CKKAQVKSCT ECIRVDKSCA YCTDELFKERRCNTQADVLA
AGCRGESILV MESSLEITEN TQIVTSLHRS QVSPQGLQVR LRRGEERTFVFQVFEPLESP VDLYILMDFS
NSMSDDLDNL KQMGQNLAKI LRQLTSDYTI GFGKFVDKVSVPQTDMRPEK LKEPWPNSDP PFSFKNVISL
TENVEEFWNK LQGERISGNL DAPEGGFDAILQTAVCTRDI GWRADSTHLL VFSTESAFHY EADGANVLAG
IMNRNDEKCH LDASGAYTQY
KTQDYPSVPT LVRLLAKHNI IPIFAVTNYS YSYYEKLHKY FPVSSLGVLQ EDSSNIVELLEEAFYRIRSN
LDIRALDSPR GLRTEVTSDT LQKTETGSFH IKRGEVGTYN VHLRAVEDIDGTHVCQLAKE DQGGNIHLKP
SFSDGLRMDA SVICDVCPCE LQKEVRSARC HFRGDFMCGHCVCNEGWSGK TCNCSTGSLS DTQPCLREGE
DKPCSGHGEC QCGRCVCYGE GRYEGHFCEYDNFQCPRTSG FLCNDRGRCS MGECVCEPGW TGRSCDCPLS
NATCIDSNGG ICNGRGYCEC
GRCHCNQQSL YTDTTCEINY SAILGLCEDL RSCVQCQAWG TGEKKGRACD DCPFKVKMVDELKKEEVVEY
CSFRDEDDDC TYSYNVEGDG SPGPNSTVLV HKKKDCLPAP SWWLIPLLIFLLLLLALLLL LCWKYCACCK
ACLGLLPCCN RGHMVGFKED HYMLRENLMA SDHLDTPMLRSGNLKGRDTV RWKITNNVQR PGFATHAAST
SPTELVPYGL SLRLGRLCTE NLMKPGTRECDQLRQEVEEN LNEVYRQVSG AHKLQQTKFR QQPNAGKKQD
HTIVDTVLLA PRSAKQMLLK
LTEKQVEQGS FHELKVAPGY YTVTAEQDAR GMVEFQEGVE LVDVRVPLFI RPEDDDEKQLLVEAIDVPVS
TATLGRRLVN ITIIKEQASG VVSFEQPEYS VSRGDQVARI PVIRHILDNGKSQVSYSTQD NTAHGHRDYV
PVEGELLFHP GETWKELQVK LLELQEVDSL LRGRQVRRFQVQLSNPKFGA RLGQPSTTTV ILDETDRSLI
NQTLSSPPPP HGDLGAPQNP NAKAAGSRKIHFNWLPPPGK PMGYRVKYWI QGDSESEAHL LDSKVPSVEL
TNLYPYCDYE MKVCAYGAKG
EGPYSSLVSC RTHQEVPSEP GRLAFNVVSS TVTQLSWAEP AETNGEITAY EVCYGLVNEDNRPIGPMKKV
LVDNPKNRML LIENLRDSQP YRYTVKARNG AGWGPEREAI INLATQPKRPMSIPIIPDIP IVDAQGGEDY
ENFLMYSDDV LRSPASSQRP SVSDDTEHLV NGRMDFAYPGSANSLHRMTA ANVAYGTHLS PHLSHRVLST
SSTLTRDYHS LTRTEHSHSG TLPRDYSTLTSLSSQASLLS GKMGGAGFRC PGLLGP

### 9. SEQ ID NO:9 Mouse beta4 dominant negative cds:

atggcagggccctgttgcagcccatgggtgaagctgctgctgctggcacgaatgctgagtgccagcctccctggagacctg
gccaaccgctgcaagaaggctcaggtgaagagctgtaccgagtgcatccgggtggacaagagctgtgcctactgcacagac
gagctgttcaaggagaggcgctgcaacacccaggcggacgttctggctgcaggctgcaggggagagagcatcctggtcatg
gagagcagccttgaaatcacagagaacacccagatcgtcaccagcctgcaccgcagccaggtatctccccaaggcctgcaa
gtccggctgcggcggggtgaggagcgcacgtttgtgttccaggtctttgagcccctggagagccccgtggatctgtatatc
ctcatggacttctccaactccatgtctgacgatctggacaacctcaagcagatggggcagaacctggccaagatcctgcgc
cagctcaccagcgactacaccattggatttggaaagtttgtggacaaagtcagcgtcccacagacagacatgaggcccgag

aaactgaaggagccctggcccaacagtgatccccgttctccttcaagaacgttatcagcttaacggagaatgtggaagaa
ttctggaacaaactgcaaggagaacgcatctcaggcaacctggacgctcctgaaggggctttgatgccatcctgcagaca
gctgtgtgcacaagggacattggctggagggctgacagcacccacctgctggtgttctccaccgagtctgccttccactac
gaggctgatggtgccaacgttctggccggcatcatgaaccgcaatgatgagaaatgccacctggacgcctcgggcgcctac
acccaatacaagacacaggactacccatcagtgcccacgctggttcgcctgcttgccaagcataacatcatccccatcttt
gctgtcaccaactactcttacagctactatgagaagctccataagtatttccccgtctcctctctgggcgtcctgcaggag
gattcatccaacatcgtggagctgctggaggaggccttctatcgaattcgctccaacctggacatccgggctctggacagc
cccagaggcctgagaacagaggtcacctccgatactctccagaagacggagactgggtcctttcacatcaagcggggggaa
gtgggcacatacaatgtgcatctccgggcagtggaggacatagatgggacacatgtgtgccagctggctaaagaagaccaa
gggggcaacatccacctgaaaccctccttctctgatggcctccggatggacgcgagtgtgatctgtgacgtgtgcccctgt
gagctgcaaaaggaagttcgatcagctcgctgtcacttcagaggagacttcatgtgtggacactgtgtgtgcaatgagggc
tggagtggcaaaacctgcaactgctccaccggctctctgagtgacacacagccctgcctgcgtgagggtgaggacaaaccg
tgctcgggccacggcgagtgccagtgcggacgctgtgtgtgctatggtgaaggccgctacgagggtcacttctgcgagtat
gacaacttccagtgtccccggacctctggattcctgtgcaatgaccggggacgctgttctatgggagagtgtgtgtgtgag
cctggttggacaggccgcagctgcgactgtcccctcagcaatgccacctgcatcgatagcaacgggggcatctgcaacggc
cgaggctactgtgagtgtggccgttgtcactgcaaccagcagtcgctctacacggacaccacctgtgagatcaactactct
gcgatactgggtctctgtgaggatctccgctcctgcgtacagtgccaggcctggggcaccggggagaagaaagggcgcgcg
tgtgacgattgcccctttaaagtcaagatggtagacgagcttaagaaagaagaggtggtggagtactgctccttccgggat
gaggatgacgactgcacttacagctacaacgtggagggcgacggcagccctgggcccaacagcacagtcctggtccacaaa
aagaaagactgcctcccggctccttcctggtggctcatcccccctgctcatcttcctcctgttgctcctggcgttgcttctg
ctgctctgctggaaatga

### 10. SEQ ID NO:10 Mouse beta4 dominant negative protein sequence:

MAGPCCSPWV KLLLLARMLS ASLPGDLANR CKKAQVKSCT ECIRVDKSCA YCTDELFKERRCNTQADVLA
AGCRGESILV MESSLEITEN TQIVTSLHRS QVSPQGLQVR LRRGEERTFVFQVFEPLESP VDLYILMDFS
NSMSDDLDNL KQMGQNLAKI LRQLTSDYTI GFGKFVDKVSVPQTDMRPEK LKEPWPNSDP PFSFKNVISL
TENVEEFWNK LQGERISGNL DAPEGGFDAILQTAVCTRDI GWRADSTHLL VFSTESAFHY EADGANVLAG
IMNRNDEKCH LDASGAYTQY
KTQDYPSVPT LVRLLAKHNI IPIFAVTNYS YSYYEKLHKY FPVSSLGVLQ EDSSNIVELLEEAFYRIRSN
LDIRALDSPR GLRTEVTSDT LQKTETGSFH IKRGEVGTYN VHLRAVEDIDGTHVCQLAKE DQGGNIHLKP
SFSDGLRMDA SVICDVCPCE LQKEVRSARC HFRGDFMCGHCVCNEGWSGK TCNCSTGSLS DTQPCLREGE
DKPCSGHGEC QCGRCVCYGE GRYEGHFCEYDNFQCPRTSG FLCNDRGRCS MGECVCEPGW TGRSCDCPLS
NATCIDSNGG ICNGRGYCEC
GRCHCNQQSL YTDTTCEINY SAILGLCEDL RSCVQCQAWG TGEKKGRACD DCPFKVKMVDELKKEEVVEY
CSFRDEDDDC TYSYNVEGDG SPGPNSTVLV HKKKDCLPAP SWWLIPLLIFLLLLLALLLLLCWK

### 11. SEQ ID NO:11 Murine laminin gamma2 chain complete cds (acc# U43327):

atgcctgcgctctggctcagctgctgcctcggtgtcgcgctcctgctgcccgccagccaggccacctccagga
gggaagtctgtgattgcaatggaagtccaggcaatgtgtctttgatcaggagctccatcgacaagcaggcag
cgggttccgttgcctcaactgcaatgacaatacagcgggggttcactgcgagcggtcgagggaggggttttac
cagcatcagagcaagagccgctgcctaccctgcaactgccactcaaagggttccctcagtgctggatgtgaca
actctggacaatgcaggtgtaagccaggtgtgacaggacaaagatgtgaccagtgtcagccaggcttccatat
gctcaccgatgctggatgcacccgagaccaggggcaactagattccaagtgtgactgtgacccagctggcatc
tctggaccctgtgattctggccgatgtgtctgcaaaccagccgtcactggagagcgctgtgataggtgccgac
cacgtgactatcatctggaccgggcaaaccctgagggctgtacccagtgtttctgctatgggcattcagccag
ctgccacgcctctgccgacttcagtgtccacaaaatcacttcaactttcagtcaggatgtggatggttggaag
gcggttcagagaaacggggcacctgcaaaactccactggtcacagcgccatcggacgtgtttagttctgccc
gaagatcagaccccgtctatttcgtggcccctgccaaattcctcggtaaccagcaagtgagttacggcagag
cctgtcttttgactaccgcgtggacagaggaggtagacagccgtctgcctacgatgtgatcctggaaggtgct
ggtctacagatcagagctcctctgatggctccaggcaagacacttccttgtgggatcacaaagacttacacat
tcagactgaatgaacatccaagcagtcactggagtccccagctgagttatttcgaatatcgaaggttactgcg
gaacctcacagccctcctgatgatccgagctacgtacggagaatatagtacagggtacattgataacgtgacc
ctggtttcagcccgccctgtccttggagccccagccccttgggttgaacgttgtgtatgcctgctggggtaca
agggacaattctgccaggaatgtgcttctggttacaaaagagattcggcaagattggcgcttttggcgcctg
tgttccctgtaactgccaagggggagggggcctgtgatccagacacgggagattgctactcgggggacgagaat
cctgacattgagtgtgctgactgtcccatcggtttctacaatgacccacatgacccccgcagctgcaagccat
gtccctgtcacaatgggttcagctgttcagtgatgcctgagacagaggaggtggtgtgtaacaactgtccccc
tggggtcacaggtgcccgctgtgagctctgtgctgatggcttctttggggatccctttgggaacatggccca
gtgaggccttgtcaacgctgccaatgcaacaacaacgtggaccccaatgcctctgggaactgtgaccagttga
caggcagatgcttgaaatgtatctacaacacggccggtgtctactgtgaccagtgcaaagcaggttactttgg
agacccattggctcccaacccagcagacaagtgtcgagcttgcaactgcagccccatgggtgcggagcctgga
gagtgtcgaggtgatggcagctgtgtttgcaagccaggctttggcgccttcaactgtgatcacgcagccctaa
ccagttgtcctgcttgctacaatcaagtgaagattcagatggaccagtttacccagcagctccagagcctgga

ggccctggtttcaaaggctcagggtggtggtggtggtggtacagtcccagtgcagctggaaggcaggatcgag
caggctgagcaggcccttcaggacattctgggagaagctcagatttcagaaggggcaatgagagccgttgctg
tccggctggccaaggcaaggagccaagagaacgactacaagacccgcctggatgacctcaagatgactgcaga
aaggatccgggccctgggcagtcagcatcagaacagagttcaggatacgagcagactcatctctcagatgcgc
ctgagtctggcaggaagcgaagctctcttggaaaacactaatatccattcttctgagcactacgtggggccga
atgattttaaaagtctggctcaggaggctacaagaaaggcagacagccacgctgagtcagctaacgcaatgaa
gcaactagcaagggaaactgaggactactccaaacaagcactttcattggcccgcaagctcttgagtggagga
ggcggaagtggctcttgggacagctccgtggtacaaggtcttatgggaaaattagagaaaaccaagtccctga
gccagcagctgtcattggaggcacccaagccgacattgaagctgataggtcgtatcagcacagtctccgcct
cctggattctgcctctcagcttcagggagtcagtgatctgtcctttcaggtggaagcaaagaggatcagacaa
aaggctgattctctctcaaacctggtgaccagacaaacggatgcattcacgcgtgtgcgaaacaatctggga
actgggaaaaagaaacacggcagcttttacagactggaaaggataggagacagacttcagatcagctgctttc
ccgtgccaaccttgctaaaaacagagcccaagaagcgctaagtatgggcaatgccacttttttatgaagttgag
aacatcctgaagaacctccgagagtttgatctgcaggttgaagacagaaaagcagaggctgaagaggccatga
agagactctcctctattagccagaaggttgcggatgccagtgacaagacccagcaagcagaaacggccctggg
gagcgccactgccgacacccaacgggcaaagaacgcagctagggaggccctggagatcagcagcgagatagag
ctggagatagggagtctgaacttggaagctaatgtgacagcagatggggccttggccatggagaaaggactg
ccactctgaagagcgagatgagagagatgattgagctggccagaaaggagctggagtttgacacggataagga
cacggtgcagctggtgattactgaagcccagcaagctgatgccagagccacgagtgccggagttaccatccaa
gacacrctcaacacattggacggcatcctacacctcatagaccagcctggcagtgtggatgaagaaggatga
tgctattagaacaaggcttttccaagccaagacccagatcaacagtcgacttcggcccttgatgtctgacct
ggaggagagggtgcgtcggcagaggaaccacctccatctgctggagactagcatagatggaattcttgctgat
gtgaagaacctggagaacattcgagacaacctgcccccaggctgctacaatacccaagctcttgagcaacagt
ga

### 12. SEQ ID NO:12 Murine laminin gamma2 chain protein sequence (acc# AAA85256)

1 mpalwlsccl gvalllpasq atsrrevcdc ngksrqcvfd qelhrqagsg frclncndnt
61 agvhcersre gfyqhqsksr clpcnchskg slsagcdnsg qcrckpgvtg qrcdqcqpgf
121 hmltdagctr dqgqldskcd cdpagisgpc dsgrcvckpa vtgercdrcr prdyhldran
181 pegctqcfcy ghsaschasa dfsvhkitst fsqdvdgwka vqrngapakl hwsqrhrdvf
241 ssarrsdpvy fvapakflgn qqvsygqsls fdyrvdrggr qpsaydvile gaglqirapl
301 mapgktlpcg itktytfrln ehpsshwspq lsyfeyrrll rnltallmir atygeystgy
361 idnvtlvsar pvlgapapwv ercvcllgyk gqfcqecasg ykrdsarlga fgacvpcncq
421 gegacdpdtg dcysgdenpd iecadcpigf yndphdprsc kpcpchngfs csvmpeteev
481 vcnncppgvt garcelcadg ffgdpfgehg pvrpcqrcqc nnnvdpnasg ncdqltgrcl
541 kciyntagvy cdqckagyfg dplapnpadk cracncspmg aepgecrgdg scvckpgfga
601 fncdhaalts cpacynqvki qmdqftqqlq slealvskaq ggggggtvpv qlegrieqae
661 qalqdilgea qisegamrav avrlakarsq endyktrldd lkmtaerira lgsqhqnrvq
721 dtsrlisqmr lslagseall entnihsseh yvgpndfksl aqeatrkads haesanamkg
781 laretedysk qalslarkll sggggsgswd ssvvqglmgk lektkslsqq lslegtqadi
841 eadrsyqhsl rlldsasqlq gvsdlsfqve akrirqkads lsnlvtrqtd aftrvrnnlg
901 nweketrqll qtgkdrrqts dqllsranla knraqealsm gnatfyeven ilknlrefdl
961 qvedrkaeae eamkrlssis qkvadasdkt qqaetalgsa tadtqrakna arealeisse
1021 ieleigslnl eanvtadgal amekgtatlk semremiela rkelefdtdk dtvqlvitea
1081 qqadaratsa gvtiqdtlnt ldgilhlidq pgsvdeegmm lleqglfqak tqinsrlrpl
1141 msdleervrr qrnhlhllet sidgiladvk nlenirdnlp pgcyntqale qq

### 13. SEQ ID NO:13 Human laminin gamma2 chain complete cds (acc# AH006634)

atgcctgcgctctggctgggctgctgcctctgcttctcgctcctcctgcccgcagcccgggccacctccaggagggaagtc
tgtgattgcaatgggaagtccaggcagtgtatctttgatcgggaacttcacagacaaactggtaatggattccgctgcctc
aactgcaatgacaacactgatggcattcactgcgagaagtgcaagaatggcttttaccggcacagagaaagggaccgctgt
ttgccctgcaattgtaactccaaaggttctcttagtgctcgatgtgacaactctggacggtgcagctgtaaaccaggtgtg
acaggagccagatgcgaccgatgtctgccaggcttccacatgctcacggatgcggggtgcacccaagaccagagactgcta
gactccaagtgtgactgtgacccagctggcatcgcagggccctgtgacgcgggccgctgtgtctgcaagccagctgttact
ggagaacgctgtgataggtgtcgatcaggttactataatctggatgggggaaccctgagggctgtacccagtgtttctgc
tatgggcattcagccagctgccgcagctctgcagaatacagtgtccataagatcacctctacctttcatcagatgttgat
ggctggaaggctgtccaacgaaatgggtctcctgcaaagctccaatggtcacagcgccatcaagatgtgtttagctcagcc
caacgactagaccctgtctattttgtggctcctgccaaatttcttgggaatcaacaggtgagctatgggcaaagcctgtcc
tttgactaccgtgtggacagaggaggcagacacccatctgcccatgatgtgattctggaaggtgctggtctacggatcaca
gctcccttgatgccacttggcaagacactgccttgtgggctcaccaagacttacacattcaggttaaatgagcatccaagc
aataattggagcccccagctgagttactttgagtatcgaaggttactgcggaatctcacagccctccgcatccgagctaca
tatggagaatacagtactgggtacattgacaatgtgaccctgatttcagcccgccctgtctctggagccccagcaccctgg

gttgaacagtgtatatgtcctgttgggtacaaggggcaattctgccaggattgtgcttctggctacaagagagattcagcg
agactggggccttttggcacctgtattccttgtaactgtcaaggggagggcctgtgatccagacacaggagattgttat
tcaggggatgagaatcctgacattgagtgtgctgactgcccaattggtttctacaacgatccgcacgacccccgcagctgc
aagccatgtccctgtcataacgggttcagctgctcagtgatgccggagacggaggaggtggtgtgcaataactgccctccc
ggggtcaccggtgcccgctgtgagctctgtgctgatggctactttggggacccctttggtgaacatggcccagtgaggcct
tgtcagccctgtcaatgcaacaacaatgtggaccccagtgcctctgggaattgtgaccggctgacaggcaggtgtttgaag
tgtatccacaacacagccggcatctactgcgaccagtgcaaagcaggctacttcggggacccattggctcccaacccagca
gacaagtgtcgagcttgcaactgtaaccccatgggctcagagcctgtaggatgtcgaagtgatggcacctgtgtttgcaag
ccaggatttggtggccccaactgtgagcatggagcattcagctgtccagcttgctataatcaagtgaagattcagatggat
cagtttatgcagcagcttcagagaatggaggccctgatttcaaaggctcagggtggtgatggagtagtacctgatacagag
ctggaaggcaggatgcagcaggctgagcaggcccttcaggacattctgagagatgcccagatttcagaaggtgctagcaga
tcccttggtctccagttggccaaggtgaggagccaagagaacagctaccagagccgcctggatgacctcaagatgactgtg
gaaagagttcgggctctgggaagtcagtaccagaaccgagttcgggatactcacaggctcatcactcagatgcagctgagc
ctggcagaaagtgaagcttccttgggaaacactaacattcctgcctcagaccactacgtggggccaaatggctttaaaagt
ctggctcaggaggccacaagattagcagaaagccacgttgagtcagccagtaacatggagcaactgacaaggaaactgag
gactattccaaacaagccctctcactggtgcgcaaggccctgcatgaaggagtcggaagcggaagcggtagcccggacggt
gctgtggtgcaagggcttgtggaaaaattggagaaaaccaagtccctggcccagcagttgacaagggaggccactcaagcg
gaaattgaagcagataggtcttatcagcacagtctccgcctcctggattcagtgtctcggcttcagggagtcagtgatcag
tcctttcaggtggaagaagcaaagaggatcaaacaaaaagcggattcactctcaacgctggtaaccaggcatatggatgag
ttcaagcgtacacaaaagaatctgggaaactggaaagaagaagcacagcagctcttacagaatggaaaaagtgggagagag
aaatcagatcagctgctttcccgtgccaatcttgctaaaagcagagcacaagaagcactgagtatgggcaatgccactttt
tatgaagttgagagcatccttaaaaacctcagagagtttgacctgcaggtggacaacagaaaagcagaagctgaagaagcc
atgaagagactctcctacatcagccagaaggtttcagatgccagtgacaagacccagcaagcagaaagagccctggggagc
gctgctgctgatgcacagagggcaaagaatggggccggggaggccctggaaatctccagtgagattgaacaggagattggg
agtctgaacttggaagccaatgtgacagcagatggagccttggccatggaaaagggactggcctctctgaagagtgagatg
agggaagtggaaggagagctggaaaggaaggagctggagtttgacacgaatatggatgcagtacagatggtgattacagaa
gcccagaaggttgataccagagccaagaacgctggggttacaatccaagacacactcaacacattagacggcctcctgcat
ctgatggaccagcctctcagtgtagatgaagaggggctggtcttactggagcagaagctttcccgagccaagacccagatc
aacagccaactgcggcccatgatgtcagagctggaagagagggcacgtcagcagaggggccacctccatttgctggagaca
agcatagatgggattctggctgatgtgaagaacttggagaacattagggacaacctgcccccaggctgctacaatacccag
gctcttgagcaacagtga

### 14. SEQ ID NO:14 Human laminin gamma2 chain protein sequence (acc# AAC50457) Alternative splice form

1 mpalwlgccl cfslllpaar atsrrevcdc ngksrqcifd relhrqtgng frclncndnt
61 dgihcekckn gfyrhrerdr clpcncnskg slsarcdnsg rcsckpgvtg arcdrclpgf
121 hmltdagctq dqrlldskcd cdpagiagpc dagrcvckpa vtgercdrcr sgyynldggn
181 pegctqcfcy ghsascrssa eysvhkitst fhqdvdgwka vqrngspakl qwsqrhqdvf
241 ssaqrldpvy fvapakflgn qqvsygqsls fdyrvdrggr hpsahdvile gaglritapl
301 mplgktlpcg ltktytfrln ehpsnnwspq lsyfeyrrll rnltalrira tygeystgyi
361 dnvtlisarp vsgapapwve qcicpvgykg qfcqdcasgy krdsarlgpf gtcipcncqg
421 ggacdpdtgd cysgdenpdi ecadcpigfy ndphdprsck pcpchngfsc svmpeteevv
481 cnncppgvtg arcelcadgy fgdpfgehgp vrpcqpcqcn nnvdpsasgn cdrltgrclk
541 cihntagiyc dqckagyfgd plapnpadkc racncnpmgs epvgcrsdgt cvckpgfggp
601 ncehgafscp acynqvkiqm dqfmqqlqrm ealiskaqgg dgvvpdtele grmqqaeqal
661 qdilrdaqis egasrslglq lakvrsqens yqsrlddlkm tvervralgs qyqnrvrdth
721 rlitqmqlsl aeseaslgnt nipasdhyvg pngfkslaqe atrlaeshve sasnmeqltr
781 etedyskqal slvrkalheg vgsgsgspdg avvqglvekl ektkslaqql treatqaeie
841 adrsyqhslr lldsvsrlqg vsdqsfqvee akrikqkads lstlvtrhmd efkrtqknlg
901 nwkeeaqqll qngksgreks dqllsranla ksraqealsm gnatfyeves ilknlrefdl
961 qvdnrkaeae eamkrlsyis qkvsdasdkt qqaeralgsa aadaqrakng agealeisse
1021 ieqeigslnl eanvtadgal amekglaslk semrevegel erkelefdtn mdavqmvite
1081 aqkvdtrakn agvtiqdtln tldgllhlmd qplsvdeegl vlleqklsra ktqinsqlrp
1141 mmseleerar qqrghlhlle tsidgiladv knlenirdnl ppgcyntqal eqq

### 15. SEQ ID NO:15 Human laminin gamma2 chain protein sequence (acc# AAC50456) Alternative splice form

1 mpalwlgccl cfslllpaar atsrrevcdc ngksrqcifd relhrqtgng frclncndnt
61 dgihcekckn gfyrhrerdr clpcncnskg slsarcdnsg rcsckpgvtg arcdrclpgf
121 hmltdagctq dqrlldskcd cdpagiagpc dagrcvckpa vtgercdrcr sgyynldggn
181 pegctqcfcy ghsascrssa eysvhkitst fhqdvdgwka vqrngspakl qwsqrhqdvf
241 ssaqrldpvy fvapakflgn qqvsygqsls fdyrvdrggr hpsahdvile gaglritapl
301 mplgktlpcg ltktytfrln ehpsnnwspq lsyfeyrrll rnltalrira tygeystgyi
361 dnvtlisarp vsgapapwve qcicpvgykg qfcqdcasgy krdsarlgpf gtcipcncqg
421 ggacdpdtgd cysgdenpdi ecadcpigfy ndphdprsck pcpchngfsc svmpeteevv
481 cnncppgvtg arcelcadgy fgdpfgehgp vrpcqpcqcn nnvdpsasgn cdrltgrclk
541 cihntagiyc dqckagyfgd plapnpadkc racncnpmgs epvgcrsdgt cvckpgfggp

601 ncehgafscp acynqvkiqm dqfmqqlqrm ealiskaqgg dgvvpdtele grmqqaeqal
661 qdilrdaqis egasrslglq lakvrsqens yqsrlddlkm tvervralgs qygnrvrdth
721 rlitqmqlsl aeseaslgnt nipasdhyvg pngfkslaqe atrlaeshve sasnmeqltr
781 etedyskqal slvrkalheg vgsgsgspdg avvqglvekl ektkslaqql treatqaeie
841 adrsyqhslr lldsvsrlqg vsdqsfqvee akrikqkads lstlvtrhmd efkrtqknlg
901 nwkeeaqqll qngksgreks dqllsranla ksraqealsm gnatfyeves ilknlrefdl
961 qvdnrkaeae eamkrlsyis qkvsdasdkt qqaeralgsa aadaqrakng agealeisse
1021 ieqeigslnl eanvtadgal amekglaslk semrevegel erkelefdtn mdavqmvite
1081 aqkvdtrakn agvtiqdtln tldgllhlmg m

### 16. Degenerate Human integrin beta4 subunit cds

atg gca gCg cca cgc ccc agc cca tgg gcc agg ctg ctc ctg gca gcc ttg atc agc gtc
agc ctc tct ggg acc ttg aac cgc tgc aag aag gcc cca gtg aag agc tgc acg gag tgt
gtc cgt gtg gat aag gac tgc gcc tac tgc aca gac gag atg ttc agg
gaccggcgctgcaacacccaggcggagctgctggccgcgggctgccagcgggagagcatcgtggtcatggagagcagcttc
caaatcacagaggagacccagattgacaccaccctgcggcgcagccagatgtcccccaaggcctgcgggtccgtctgcgg
cccggtgaggagcggcattttgagctggaggtgtttgagccactggagagccccgtggacctgtacatcctcatggacttc
tccaactccatgtccgatgatctggacaacctcaagaagatggggcagaacctggctcgggtcctgagccagctcaccagc
gactacactattggatttggcaagtttgtggacaaagtcagcgtcccgcagacggacatgaggcctgagaagctgaaggag
ccttggcccaacagtgacccccccttctccttcaagaacgtcatcagcctgacagaagatgtggatgagttccggaataaa
ctgcagggagagcggatctcaggcaacctggatgctcctgagggcggcttcgatgccatcctgcagacagctgtgtgcacg
agggacattggctggcgcccggacagcacccacctgctggtcttctccaccgagtcagccttccactatgaggctgatggc
gccaacgtgctggctggcatcatgagccgcaacgatgaacggtgccacctggacaccacgggcacctacacccagtacagg
acacaggactacccgtcggtgcccaccctggtgcgcctgctcgccaagcacaacatcatccccatctttgctgtcaccaac
tactcctatagctactacgagaagcttcacacctatttccctgtctcctcactgggggtgctgcaggaggactcgtccaac
atcgtggagctgctggaggaggccttcaatcggatccgctccaacctggacatccgggccctagacagcccccgaggcctt
cggacagaggtcacctccaagatgttccagaagacgaggactgggtcctttcacatccggcgggggaagtgggtatatac
caggtgcagctgcgggcccttgagcacgtggatgggacgcacgtgtgccagctgccggaggaccagaagggcaacatccat
ctgaaaccttccttctccgacggcctcaagatggacgcgggcatcatctgtgatgtgtgcacctgcgagctgcaaaaagag
gtgcggtcagctcgctgcagcttcaacggagacttcgtgtgcggacagtgtgtgtgcagcgagggctggagtggccagacc
tgcaactgctccaccggctctctgagtgacattcagccctgcctgcgggagggcgaggacaagccgtgctccggccgtggg
gagtgccagtgcgggcactgtgtgtgctacggcgaaggccgctacgagggtcagttctgcgagtatgacaacttccagtgt
ccccgcacttccgggttcctctgcaatgaccgaggacgctgctccatgggccagtgtgtgtgtgagcctggttggacaggc
ccaagctgtgactgtcccctcagcaatgccacctgcatcgacagcaatgggggcatctgtaatggacgtggccactgtgag
tgtggccgctgccactgccaccagcagtcgctctacacggacaccatctgcgagatcaactactcggcgtccacccggcc
tctgcgaggacctacgctcctgcgtgcagtgccaggcgtggggcaccggcgagaagaagggcgcacgtgtgaggaatgca
acttcaaggtcaagatggtggacgagcttaagagaggcgaggaggtggtggtgcgctgctccttccgggacgaggatgacg
actgcacctacagctacaccatggaaggtgacggcgcccctgggcccaacagcactgtcctggtgcacaagaagaaggac
tgccctccgggctccttctggtggctcatccccctgctcctcctcctgccgctcctggccctgctactgctgctatgc
tggaagtactgtgcctgctgcaaggcctgcctggcacttctcccgtgctgcaaccgaggtcacatggtgggctttaaggaa
gaccactacatgctgcgggagaacctgatggcctctgaccacttggacacgcccatgctgcgcagcgggaacctcaaggc
cgtgacgtggtccgctggaaggtcaccaacaacatgcagcggcctggctttgccactcatgccgccagcatcaaccccaca
gagctggtgccctacgggctgtccttgcgcctggcccgcctttgcaccgagaacctgctgaagcctgacactcgggagtgc
gcccagctgcgccaggaggtggaggagaacctgaacgaggtctacaggcagatctccggtgtacacaagctccagcagacc
aagttccggcagcagcccaatgccgggaaaaagcaagaccacaccattgtggacacagtgctgatggcgccccgctcggcc
aagccggccctgctgaagcttacagagaagcaggtggaacagagggccttccacgacctcaaggtggcccccggctactac
accctcactgcagaccaggacgcccggggcatggtggagttccaggagggcgtggagctggtggacgtacgggtgcccctc
tttatccggcctgaggatgacgacgagaagcagctgctggtggaggccatcgacgtgcccgcaggcactgccaccctcggc
cgccgcctggtaaacatcaccatcatcaaggagcaagccagagacgtggtgtcctttgagcagcctgagttctcggtcagc
cgcggggaccaggtggcccgcatccctgtcatccggcgtgtcctggacggcgggaagtcccaggtctcctaccgcacacag
gatggcaccgcgcagggcaaccggactacatccccgtggagggtgagctgctgttccagcctggggaggcctggaaagag
ctgcaggtgaagctcctggagctgcaagaagttgactccctcctgcggggccgccaggtccgccgtttccacgtccagctc
agcaaccctaagtttggggcccacctgggccagccccactccaccaccatcatcatcagggacccagatgaactggaccgg
agcttcacgagtcagatgttgtcatcacagccacccctcacggcgacctggcgccccgcagaaccccaatgctaaggcc
gctgggtccaggaagatccatttcaactggctgcccccttctggcaagccaatggggtacagggtaaagtactggattcag
ggtgactccgaatccgaagcccacctgctcgacagcaaggtgccctcagtggagctcaccaacctgtacccgtattgcgac
tatgagatgaaggtgtgcgcctacggggctcagggcgagggaccctacagctccctggtgtcctgccgcacccaccaggaa
gtgcccagcgagccagggcgtctggccttcaatgtcgtctcctccacggtgacccagctgagctgggctgagccggctgag
accaacggtgagatcacagcctacgaggtctgctatggcctggtcaacgatgacaaccgacctattgggcccatgaagaa
gtgctggttgacaaccctaagaaccggatgctgcttattgagaaccttcgggagtcccagccctaccgctacacggtgaag
gcgcgcaacggggccggctgggggcctgagcgggaggccatcatcaacctggccacccagcccaagaggcccatgtccatc
cccatcatccctgacatccctatcgtggacgcccagagcggggaggactacgacagcttccttatgtacagcgatgacgtt
ctacgctctccatcggcagccagaggcccagcgtctccgatgacactggctgcggctggaagttcgagcccctgctgggg
gaggagctggacctgcggcgcgtcacgtggcggctgcccccggagctcatcccgcgcctgtcggccagcagcgggcgctcc
tccgacgccgaggcgccccacggccccggacgacggcggcgcgggcgggaagggcggcagcctgccccgcagtgcgaca
cccgggcccccggagagcacctggtgaatggccggatggactttgccttcccgggcagcaccaactccctgcacaggatg
accacgaccagtgctgctgcctatggcacccacctgagcccacacgtgccccaccgcgtgctaagcacatcctccaccctc

acacgggactacaactcactgacccgctcagaacactcacactcgaccacactgccgaggactactccaccctcacctcc
gtctcctcccacggcctccctcccatctgggaacacggaggagcaggcttccgctgtcctggccctggggtcccggagt
cgggctcagatgaaagggttccccccttccaggggcccacgagactctataatcctggctgggaggccagcagcgccctcc
tggggcccagactctcgcctgactgctggtgtgcccgacacgcccacccgcctggtgttctctgccctggggcccacatct
ctcagagtgagctggcaggagccgcggtgcgagcggccgctgcagggctacagtgtggagtaccagctgctgaacggcggt
gagctgcatcggctcaacatccccaaccctgcccagacctcggtggtggtggaagacctcctgcccaaccactcctacgtg
ttccgcgtgcgggcccagagccaggaaggctggggccgagagcgtgagggtgtcatcaccattgaatcccaggtgcacccg
cagagcccactgtgtcccctgccaggctccgccttcactttgagcactcccagtgccccaggcccgctggtgttcactgcc
ctgagcccagactcgctgcagctgagctgggagcggccacggaggcccaatggggatatcgtcggctacctggtgacctgt
gagatggcccaaggaggaggtccagccaccgcattccgggtggatggagacagccccgagagccggctgaccgtgccgggc
ctcagcgagaacgtgccctacaagttcaaggtgcaggccaggaccactgagggcttcgggccagagcgcgagggcatcatc
accatagagtcccaggatggaggtcccttcccgcagctgggcagccgtgccgggctcttccagcacccgctgcaaagcgag
tacagcagcatctccaccacccacaccagcgccaccgagcccttcctagtgggtccgaccctgggggcccagcacctggag
gcaggcggctccctcacccggcatgtgacccaggagtttgtgagccggacactgaccaccagcggaacccttagcacccac
atggaccaacagttcttccaaacttga

### 17. SEQ ID NO:17 Human beta4 integrin protein sequence Variant V at 34 to I:

1 magprpspwa rlllaalisv slsgtlnrck kaplksctec vrvdkdcayc tdemfrdrrc
61 ntqaellaag cqresivvme ssfqiteetq idttlrrsqm spqglrvrlr pgeerhfele
121 vfeplespvd lyilmdfsns msddldnlkk mggnlarvls qltsdytigf gkfvdkvsvp
181 qtdmrpeklk epwpnsdppf sfknvislte dvdefrnklq gerisgnlda peggfdailq
241 tavctrdigw rpdsthllvf stesafhyea dganvlagim srnderchld ttgtytqyrt
301 qdypsvptlv rllakhniip ifavtnysys yyeklhtyfp vsslgvlqed ssnivellee
361 afnrirsnld iraldsprgl rtevtskmfq ktrtgsfhir rgevgiyqvq lralehvdgt
421 hvcqlpedqk gnihlkpsfs dglkmdagii cdvctcelqk evrsarcsfn gdfvcgqcvc
481 segwsgqtcn cstgslsdiq pclregedkp csgrgecqcg hcvcygegry egqfceydnf
541 qcprtsgflc ndrgrcsmgq cvcepgwtgp scdcplsnat cidsnggicn grghcecgrc
601 hchqqslytd ticeinysas trasartyap acsarrgapa rrrgarvrna tsrsrwwtsl
661 rearrwwcaa psgtrmttap tatpwkvtap lgptalswct rrrdcppgsf wwlipllll
721 lpllalllll cwkycaccka clallpccnr ghmvgfkedh ymlrenlmas dhldtpmlrs
781 gnlkgrdvvr wkvtnnmqrp gfathaasin ptelvpygls lrlarlcten llkpdtreca
841 qlrqeveenl nevyrqisgv hklqqtkfrq qpnagkkqdh tivdtvlmap rsakpallkl
901 tekqveqraf hdlkvapgyy tltadqdarg mvefqegvel vdvrvplfir pedddekqll
961 veaidvpagt atlgrrlvni tiikeqardv vsfeqpefsv srgdqvarip virrvldggk
1021 sqvsyrtqdg taqgnrdyip vegellfqpg eawkelqvkl lelqevdsll rgrqvrrfhv
1081 qlsnpkfgah lgqphsttii irdpdeldrs ftsqmlssqp pphgdlgapq npnakaagsr
1141 kihfnwlpps gkpmgyrvky wiqgdsesea hlldskvpsv eltnlypycd yemkvcayga
1201 qgegpysslv scrthqevps epgrlafnvv sstvtqlswa epaetngeit ayevcyglvn
1261 ddnrpigpmk kvlvdnpknr mllienlres qpyrytvkar ngagwgpere aiinlatqpk
1321 rpmsipiipd ipivdaqsge dydsflmysd dvlrspsgsq rpsvsddtgc gwkfepllge
1381 eldlrrvtwr lppeliprls assgrssdae aphgppddgg aggkggslpr satpgppgeh
1441 lvngrmdfaf pgstnslhrm tttsaaaygt hlsphvphrv lstsstltrd ynsltrsehs
1501 hsttlprdys tltsvsshgl ppiwehgrsr lplswalgsr sraqmkgfpp srgprdsiil
1561 agrpaapswg pdsrltagvp dtptrlvfsa lgptslrvsw qeprcerplq gysveyqlln
1621 ggelhrlnip npaqtsvvve dllpnhsyvf rvraqsqegw greregviti esqvhpqspl
1681 cplpgsaftl stpsapgplv ftalspdslq lswerprrpn gdivgylvtc emaqgggpat
1741 afrvdgdspe srltvpglse nvpykfkvqa rttegfgper egiitiesqd ggpfpqlgsr
1801 aglfqhplqs eyssisttht satepflvgp tlgaqhleag gsltrhvtqe fvsrtlttsg
1861 tlsthmdqqf fqt

### 18. SEQ ID NO:18 Human integrin beta4 subunit cds Variant at 34 V to I:

atg gca ggg cca cgc ccc agc cca tgg gcc agg ctg ctc ctg gca gcc ttg atc agc gtc
agc ctc tct ggg acc ttg aac cgc tgc aag aag gcc cca AtA aag agc tgc acg gag tgt
gtc cgt gtg gat aag gac tgc gcc tac tgc aca gac gag atg ttc agg
gaccggcgctgcaacacccaggcggagctgctggccgcgggctgccagcgggagagcatcgtggtcatggagagcagcttc
caaatcacagaggagacccagattgacaccaccctgcggcgcagccagatgtcccccaaggcctgcgggtccgtctgcgg
cccggtgaggagcggcattttgagctggaggtgtttgagccactggagagccccgtggacctgtacatcctcatggacttc
tccaactccatgtccgatgatctggacaacctcaagaagatggggcagaacctggctcgggtcctgagccagctcaccagc
gactacactattggatttggcaagtttgtggacaaagtcagcgtcccgcagacggacatgaggcctgagaagctgaaggag
ccttggcccaacagtgacccccccttctccttcaagaacgtcatcagcctgacagaagatgtggatgagttccggaataaa
ctgcagggagagcggatctcaggcaacctggatgctcctgagggcggcttcgatgccatcctgcagacagctgtgtgcacg
agggacattggctggcgcccggacagcacccacctgctggtcttctccaccgagtcagccttccactatgaggctgatggc
gccaacgtgctggctggcatcatgagccgcaacgatgaacggtgccacctggacaccacgggcacctacacccagtacagg
acacaggactacccgtcggtgcccaccctggtgcgcctgctcgccaagcacaacatcatccccatctttgctgtcaccaac
tactcctatagctactacgagaagcttcacacctatttccctgtctcctcactgggggtgctgcaggaggactcgtccaac
atcgtggagctgctggaggaggccttcaatcggatccgctccaacctggacatccgggccctagacagcccccgaggcctt

cggacagaggtcacctccaagatgttccagaagacgaggactgggtcctttcacatccggcgggggaagtgggtatatac
caggtgcagctgcgggcccttgagcacgtggatgggacgcacgtgtgccagctgccggaggaccagaagggcaacatccat
ctgaaaccttccttctccgacggcctcaagatggacgcgggcatcatctgtgatgtgtgcacctgcgagctgcaaaaagag
gtgcggtcagctcgctgcagcttcaacggagacttcgtgtgcggacagtgtgtgtgcagcgagggctggagtggccagacc
tgcaactgctccaccggctctctgagtgacattcagccctgcctgcgggagggcgaggacaagccgtgctccggccgtggg
gagtgccagtgcgggcactgtgtgtgctacggcgaaggccgctacgagggtcagttctgcgagtatgacaacttccagtgt
ccccgcacttccgggttcctctgcaatgaccgaggacgctgctccatgggccagtgtgtgtgtgagcctggttggacaggc
ccaagctgtgactgtcccctcagcaatgccacctgcatcgacagcaatggggggcatctgtaatggacgtggccactgtgag
tgtggccgctgccactgccaccagcagtcgctctacacggacaccatctgcgagatcaactactcggcgtccacccgggcc
tctgcgaggacctacgctcctgcgtgcagtgccaggcgtggggcaccggcgagaagaagggggcgcacgtgtgaggaatgca
acttcaaggtcaagatggtggacgagcttaagagaggcgaggaggtggtggtgcgctgctccttccgggacgaggatgacg
actgcacctacagctacaccatggaaggtgacggcgcccctgggcccaacagcactgtcctggtgcacaagaagaagggac
tgccctccgggctccttctggtggctcatcccctgctcctcctcctcctgccgctcctggccctgctactgctgctatgc
tggaagtactgtgcctgctgcaaggcctgcctggcacttctcccgtgctgcaaccgaggtcacatggtgggctttaaggaa
gaccactacatgctgcgggagaacctgatggcctctgaccacttggacacgcccatgctgcgcagcgggaacctcaagggc
cgtgacgtggtccgctggaaggtcaccaacaacatgcagcggcctggctttgccactcatgccgccagcatcaaccccaca
gagctggtgccctacgggctgtccttgcgcctggcccgcctttgcaccgagaacctgctgaagcctgacactcgggagtgc
gcccagctgcgccaggaggtggaggagaacctgaacgaggtctacaggcagatctccggtgtacacaagctccagcagacc
aagttccggcagcagcccaatgccgggaaaaagcaagaccacaccattgtggacacagtgctgatggcgccccgctcggcc
aagccggccctgctgaagcttacagagaagcaggtggaacagagggccttccacgacctcaaggtggcccccggctactac
accctcactgcagaccaggacgcccggggcatggtggagttccaggagggcgtggagctggtggacgtacgggtgcccctc
tttatccggcctgaggatgacgacgagaagcagctgctggtggaggccatcgacgtgcccgcaggcactgccaccctcggc
cgccgcctggtaaacatcaccatcatcaaggagcaagccagagacgtggtgtcctttgagcagcctgagttctcggtcagc
cgcggggaccaggtggcccgcatccctgtcatccggcgtgtcctggacggcgggaagtcccaggtctcctaccgcacacag
gatggcaccgcgcagggcaaccgggactacatccccgtggagggtgagctgctgttccagcctggggaggcctggaaagag
ctgcaggtgaagctcctggagctgcaagaagttgactccctcctgcggggccgccaggtccgccgtttccacgtccagctc
agcaaccctaagtttggggcccacctgggccagcccactccaccaccatcatcatcagggacccagatgaactggaccgg
agcttcacgagtcagatgttgtcatcacagccacccctcacggcgacctggcgccccgcagaaccccaatgctaaggcc
gctgggtccaggaagatccatttcaactggctgcccccttctggcaagccaatggggtacagggtaaagtactggattcag
ggtgactccgaatccgaagcccacctgctcgacagcaaggtgccctcagtggagctcaccaacctgtacccgtattgcgac
tatgagatgaaggtgtgcgcctacggggctcagggcgagggaccctacagctccctggtgtcctgccgcacccaccaggaa
gtgcccagcgagccagggcgtctggccttcaatgtcgtctcctccacggtgacccagctgagctgggctgagccggctgag
accaacggtgagatcacagcctacgaggtctgctatggcctggtcaacgatgacaaccgacctattgggcccatgaagaa
gtgctggttgacaaccctaagaaccggatgctgcttattgagaaccttcgggagtcccagccctaccgctacacggtgaag
gcgcgcaacggggccggctgggggcctgagcgggaggccatcatcaacctggccacccagcccaagaggcccatgtccatc
cccatcatccctgacatccctatcgtggacgcccagagcggggaggactacgacagcttccttatgtacagcgatgacgtt
ctacgctctccatcgggcagccagaggcccagcgtctccgatgacactggctgcggctggaagttcgagcccctgctgggg
gaggagctggacctgcggcgcgtcacgtggcggctgcccccggagctcatcccgcgcctgtcggccagcagcgggcgctcc
tccgacgccgaggcgccccacggcccccggacgacggcggcgcgggcgggaagggcggcagcctgccccgcagtgcgaca
cccgggcccccccggagagcacctggtgaatggccggatggactttgccttcccgggcagcaccaactccctgcacaggatg
accacgaccagtgctgctgcctatggcacccacctgagcccacacgtgccccaccgcgtgctaagcacatcctccaccctc
acacgggactacaactcactgacccgctcagaacactcacactcgaccacactgccgagggactactccaccctcacctcc
gtctcctcccacggcctccctcccatctgggaacacgggaggagcaggcttccgctgtcctgggccctggggtcccggagt
cgggctcagatgaaagggttccccccttccagggcccacgagactctataatcctggctgggaggccagcagcgccctcc
tggggcccagactctcgcctgactgctggtgtgcccgacacgcccacccgcctggtgttctctgccctggggcccacatct
ctcagagtgagctggcaggagccgcggtgcgagcggccgctgcagggctacagtgtggagtaccagctgctgaacggcggt
gagctgcatcggctcaacatccccaaccctgcccagacctcggtggtggtggaagacctcctgcccaaccactcctacgtg
ttccgcgtgcgggcccagagccaggaaggctggggccgagagcgtgagggtgtcatcaccattgaatcccaggtgcacccg
cagagcccactgtgtcccctgccaggctccgccttcactttgagcactcccagtgccccaggcccgctggtgttcactgcc
ctgagcccagactcgctgcagctgagctgggagcggccacggaggcccaatggggatatcgtcggctacctggtgacctgt
gagatggcccaaggaggaggtccagccaccgcattccgggtggatggagacagccccgagagccggctgaccgtgccgggc
ctcagcgagaacgtgccctacaagttcaaggtgcaggccaggaccactgagggcttcggcccagagcgcgagggcatcatc
accatagagtcccaggatggaggtcccttcccgcagctgggcagccgtgccgggctcttccagcacccgctgcaaagcgag
tacagcagcatctccaccacccacaccagcgccaccgagcccttcctagtgggtccgaccctgggggcccagcacctggag
gcaggcggctccctcacccggcatgtgacccaggagtttgtgagccggacactgaccaccagcggaacccttagcacccac
atggaccaacagttcttccaaacttga

### 19. SEQ ID NO:19 Degenerate Human integrin beta4 subunit cds variant at 34 V to I:

atg gca ggg cca cgG ccc agc cca tgg gcc agg ctg ctc ctg gca gcc ttg atc agc gtc
agc ctc tct ggg acc ttg aac cgc tgc aag aag gcc cca AtA aag agc tgc acg gag tgt
gtc cgt gtg gat aag gac tgc gcc tac tgc aca gac gag atg ttc agg
gaccggcgctgcaacacccaggcggagctgctggccgcgggctgccagcgggagagcatcgtggtcatggagagcagcttc
caaatcacagaggagacccagattgacaccaccctgcggcgcagccagatgtcccccaaggcctgcgggtccgtctgcgg
cccggtgaggagcggcattttgagctggaggtgtttgagccactggagagccccgtggacctgtacatcctcatggacttc
tccaactccatgtccgatgatctggacaacctcaagaagatggggcagaacctggctcgggtcctgagccagctcaccagc
gactacactattggatttggcaagtttgtggacaaagtcagcgtcccgcagacggacatgaggcctgagaagctgaaggag

cctt ggcccaacagtgacccccccttctccttcaagaacgtcatcagcctgacagaagatgtggatgagttccggaataa
ctgcagggagagcggatctcaggcaacctggatgctcctgagggcggcttcgatgccatcctgcagacagctgtgtgcacg
agggacattggctggcgcccggacagcacccacctgctggtcttctccaccgagtcagccttccactatgaggctgatggc
gccaacgtgctggctggcatcatgagccgcaacgatgaacggtgccacctggacaccacgggcacctacacccagtacagg
acacaggactacccgtcggtgcccaccctggtgcgcctgctcgccaagcacaacatcatccccatctttgctgtcaccaac
tactcctatagctactacgagaagcttcacacctatttccctgtctcctcactgggggtgctgcaggaggactcgtccaac
atcgtggagctgctggaggaggccttcaatcggatccgctccaacctggacatccgggccctagacagcccccgaggcctt
cggacagaggtcacctccaagatgttccagaagacgaggactgggtcctttcacatccggcgggggaagtgggtatatac
caggtgcagctgcgggcccttgagcacgtggatgggacgcacgtgtgccagctgccggaggaccagaagggcaacatccat
ctgaaaccttccttctccgacggcctcaagatggacgcgggcatcatctgtgatgtgtgcacctgcgagctgcaaaaagag
gtgcggtcagctcgctgcagcttcaacggagacttcgtgtgcggacagtgtgtgtgcagcgagggctggagtggccagacc
tgcaactgctccaccggctctctgagtgacattcagccctgcctgcgggagggcgaggacaagccgtgctccggccgtggg
gagtgccagtgcgggcactgtgtgtgctacggcgaaggccgctacgagggtcagttctgcgagtatgacaacttccagtgt
ccccgcacttccgggttcctctgcaatgaccgaggacgctgctccatgggccagtgtgtgtgtgagcctggttggacaggc
ccaagctgtgactgtcccctcagcaatgccacctgcatcgacagcaatgggggcatctgtaatggacgtggccactgtgag
tgtggccgctgccactgccaccagcagtcgctctacacggacaccatctgcgagatcaactactcggcgtccacccgggcc
tctgcgaggacctacgctcctgcgtgcagtgccaggcgtggggcaccggcgagaagaagggcgcacgtgtgaggaatgca
acttcaaggtcaagatggtggacgagcttaagagaggcgaggaggtggtggtgcgctgctccttccgggacgaggatgacg
actgcacctacagctacaccatggaaggtgacggcgcccctgggcccaacagcactgtcctggtgcacaagaagaagggac
tgccctccgggctccttctggtggctcatcccctgctcctcctcctgccgctcctggccctgctactgctgctatgc
tggaagtactgtgcctgctgcaaggcctgcctggcacttctcccgtgctgcaaccgaggtcacatggtgggctttaaggaa
gaccactacatgctgcgggagaacctgatggcctctgaccacttggacacgcccatgctgcgcagcgggaacctcaagggc
cgtgacgtggtccgctggaaggtcaccaacaacatgcagcggcctggctttgccactcatgccgccagcatcaaccccaca
gagctggtgccctacgggctgtccttgcgcctggcccgcctttgcaccgagaacctgctgaagcctgacactcgggagtgc
gcccagctgcgccaggaggtggaggagaacctgaacgaggtctacaggcagatctccggtgtacacaagctccagcagacc
aagttccggcagcagcccaatgccgggaaaaagcaagaccacaccattgtggacacagtgctgatggcgccccgctcggcc
aagccggccctgctgaagcttacagagaagcaggtggaacagagggccttccacgacctcaaggtggcccccggctactac
accctcactgcagaccaggacgcccggggcatggtggagttccaggagggcgtggagctggtggacgtacgggtgcccctc
tttatccggcctgaggatgacgacgagaagcagctgctggtggaggccatcgacgtgcccgcaggcactgccaccctcggc
cgccgcctggtaaacatcaccatcatcaaggagcaagccagagacgtggtgtcctttgagcagcctgagttctcggtcagc
cgcggggaccaggtggcccgcatccctgtcatccggcgtgtcctggacggcgggaagtcccaggtctcctaccgcacacag
gatggcaccgcgcagggcaaccgggactacatccccgtggagggtgagctgctgttccagcctggggaggcctggaaagag
ctgcaggtgaagctcctggagctgcaagaagttgactccctcctgcggggccgccaggtccgccgtttccacgtccagctc
agcaaccctaagtttggggcccacctgggccagccccactccaccaccatcatcatcagggacccagatgaactggaccgg
agcttcacgagtcagatgttgtcatcacagccacccccctcacggcgacctgggcgccccgcagaaccccaatgctaaggcc
gctgggtccaggaagatccatttcaactggctgcccccttctggcaagccaatggggtacagggtaaagtactggattcag
ggtgactccgaatccgaagcccacctgctcgacagcaaggtgccctcagtggagctcaccaacctgtacccgtattgcgac
tatgagatgaaggtgtgcgcctacggggctcagggcgagggaccctacagctccctggtgtcctgccgcacccaccaggaa
gtgcccagcgagccagggcgtctggccttcaatgtcgtctcctccacggtgacccagctgagctgggctgagccggctgag
accaacggtgagatcacagcctacgaggtctgctatggcctggtcaacgatgacaaccgacctattgggcccatgaagaa
gtgctggttgacaaccctaagaaccggatgctgcttattgagaaccttcgggagtcccagccctaccgctacacggtgaag
gcgcgcaacggggccggctgggggcctgagcgggaggccatcatcaacctggccacccagcccaagaggcccatgtccatc
cccatcatccctgacatccctatcgtggacgcccagagcggggaggactacgacagcttccttatgtacagcgatgacgtt
ctacgctctccatcgggcagccagaggcccagcgtctccgatgacactggctgcggctggaagttcgagcccctgctgggg
gaggagctggacctgcggcgcgtcacgtggcggctgcccccggagctcatcccgcgcctgtcggccagcagcgggcgctcc
tccgacgccgaggcgccccacgggcccccggacgacggcggcgcgggcgggaagggcggcagcctgccccgcagtgcgaca
cccgggcccccccggagagcacctggtgaatggccggatggactttgccttcccgggcagcaccaactccctgcacaggatg
accacgaccagtgctgctgcctatggcacccacctgagcccacacgtgccccaccgcgtgctaagcacatcctccaccctc
acacgggactacaactcactgacccgctcagaacactcacactcgaccacactgccgagggactactccaccctcacctcc
gtctcctcccacggcctccctcccatctgggaacacgggaggagcaggcttccgctgtcctgggccctggggtcccggagt
cgggctcagatgaaaggttccccccttccagggcccacgagactctataatcctggctgggaggccagcagcgccctcc
tggggcccagactctcgcctgactgctggtgtgcccgacacgcccacccgcctggtgttctctgccctggggcccacatct
ctcagagtgagctggcaggagccgcggtgcgagcggccgctgcagggctacagtgtggagtaccagctgctgaacggcggt
gagctgcatcggctcaacatccccaaccctgcccagacctcggtggtggtggaagacctcctgcccaaccactcctacgtg
ttccgcgtgcgggcccagagccaggaaggctggggccgagagcgtgagggtgtcatcaccattgaatcccaggtgcacccg
cagagcccactgtgtcccctgccaggctccgccttcactttgagcactcccagtgccccaggcccgctggtgttcactgcc
ctgagcccagactcgctgcagctgagctgggagcggccacggaggcccaatggggatatcgtcggctacctggtgacctgt
gagatggcccaaggaggaggtccagccaccgcattccgggtggatggagacagccccgagagccggctgaccgtgccgggc
ctcagcgagaacgtgccctacaagttcaaggtgcaggccaggaccactgagggcttcgggccagagcgcgagggcatcatc
accatagagtcccaggatggaggtcccttcccgcagctgggcagccgtgccgggctcttccagcacccgctgcaaagcgag
tacagcagcatctccaccacccacaccagcgccaccgagcccttcctagtgggtccgaccctgggggcccagcacctggag
gcaggcggctccctcacccggcatgtgacccaggagtttgtgagccggacactgaccaccagcggaacccttagcacccac
atggaccaacagttcttccaaacttga

Preferred embodiments are outlined in the following paragraphs:

### Embodiments:

1. A method of reducing the proliferation of a cancer cell which comprises inhibiting ligand binding to an integrin receptor on the cancer cell, wherein the integrin receptor comprises an integrin.
2. The method of paragraph 1, wherein the integrin receptor comprises integrin B4.
3. The method of paragraph 1, wherein the integrin receptor comprises integrin A6.
4. The method of paragraph 1, wherein the ligand that binds to the integrin receptor is laminin5.
5. The method of paragraph 1, wherein the integrin receptor is A6B4.
6. The method of paragraph 1, wherein the ligand comprises laminin5.
7. The method of paragraph 1, wherein the ligand comprises the gamma-2 subunit of laminin5.
8. The method of paragraph 1, wherein inhibiting ligand binding to an integrin receptor comprises contacting a A6 integrin with a composition that inhibits ligand binding.
9. The method of paragraph 1, wherein inhibiting ligand binding to an integrin receptor comprises contacting a B4 integrin with a composition that inhibits ligand binding.
10. The method of paragraph 1, wherein inhibiting ligand binding to an integrin receptor comprises contacting a laminin5 with a composition that inhibits ligand binding.
11. The method of paragraph 1, wherein inhibiting ligand binding to an integrin receptor comprises contacting a gamma-2 subunit with a composition that inhibits ligand binding.
12. A method of reducing the proliferation of a cancer cell which comprises reducing integrin-integrin interaction, integrin receptor clustering interaction, or integrin-non-integrin protein interaction.
13. The method of paragraph 12, wherein reducing integrin-integrin interaction, integrin receptor clustering interaction, or integrin-non-integrin protein interaction comprises contacting a A6 integrin with a composition that inhibits an interaction between the B4 integrin and another integrin or protein molecule.
14. The method of paragraph 12, wherein reducing integrin-integrin interaction, integrin receptor clustering interaction, or integrin-non-integrin protein interaction comprises contacting a B4 integrin with a composition that inhibits the interaction between the B4 integrin and another integrin or protein molecule.
15. The method of paragraph 12, wherein reducing integrin-integrin interaction, integrin receptor clustering interaction, or integrin-non-integrin protein interaction comprises contacting a laminin5 with a composition that inhibits ligand binding.
16. The method of paragraph 12, wherein reducing integrin-integrin interaction, integrin receptor clustering interaction, or integrin-non-integrin protein interaction comprises contacting a gamma2 subunit with a composition that inhibits ligand binding.
17. The method of paragraph 12, wherein the non-integrin protein comprises a growth factor receptor.
18. The method of paragraph 12, wherein the non-integrin protein comprises a hemi-desomosome junction.
19. The method of paragraph 12, wherein the non-integrin protein comprises a SH2 domain.
20. The method of paragraph 19, wherein the non-integrin protein comprises a Shc protein.
21. The method of paragraph 12, wherein the non-integrin protein comprises a IRS-1 protein.
22. The method of paragraph 12, wherein the non-integrin protein comprises a IRS-2 protein.
23. A method of reducing the proliferation of a cancer cell which comprises reducing the production of an integrin by the cancer cell.
24. The method of paragraph 23, wherein the production of an integrin is reduced by inhibiting signaling leading to induction of expression of an integrin.
25. The method of paragraph 23, wherein reducing the production of an integrin comprises inhibiting alpha6 production.
26. The method of paragraph 25, wherein inhibiting alpha6 production further comprises using antisense molecules to alpha6 mRNA.
27. The method of paragraph 23, wherein reducing the production of an integrin comprises inhibiting beta4 production.
28. The method of paragraph 27, wherein inhibiting beta4 production further comprises using antisense molecules to beta4 mRNA.
29. A method of reducing the proliferation of a cancer cell which comprises reducing the production of an integrin receptor ligand by the cancer cell.
30. The method of paragraph 29, wherein reducing the production of an integrin receptor ligand comprises inhibiting gamma2 production.
31. The method of paragraph 29, wherein reducing the production of an integrin receptor ligand comprises inhibiting laminin production.
32. The method of paragraph 29, wherein reducing the production of an integrin receptor ligand comprises inhibiting laminin5 production.
33. A method of reducing the proliferation of a cancer cell which comprises interfering with an integrin signaling pathway.
34. The method of paragraph 33, wherein interfering with an integrin signaling pathway comprises contacting an A6 integrin in the cell with a composition that inhibits ligand binding.
35. The method of paragraph 33, wherein interfering with an integrin signaling pathway comprises contacting a B4 integrin with a composition that inhibits ligand binding.
36. The method of paragraph 33, wherein interfering with an integrin signaling pathway comprises contacting a laminin5 with a composition that inhibits ligand binding.
37. The method of paragraph 33, wherein interfering with an integrin signaling pathway comprises contacting a gamma2 subunit with a composition that inhibits ligand binding.
38. The method of paragraph 33, wherein interfering with an integrin signaling pathway comprises contacting the cancer cell with a molecule that interferes with at least one of talin, paxillin, vinculin, a CAS family protein, CRX, NCK, FAK, ILK, Src, Fyn, Shc, Grb-2, Guaning nucleotide exchange factors, SOS, DOCK 180,. Vav, Syk, P-1-3 kinase, AKT, Bad, Bid, Caspase 9, Cdc42, PAK, Rac, Rho, Rho kinase, Ras, Caveolin, Tetraspan, Receptor-type protein tyrosine phosphatase, SHP-2, Alpha-actinin, Filamin, Cytohesin, Beta3-endonexin, ICAP-1, RACK-1, CIB, actin, receptor tyrosine kinase, IRS-1 or IRS-2.
39. The method of paragraph 33, wherein interfering with an integrin signaling pathway comprises contacting the cancer cell with an agent that interferes with post-translational modification of integrins.
40. The method of paragraph 39, wherein the post translational modification is glycosylation or phosphorylation.
41. The method of paragraphs 1,12,23,29,33, wherein the integrin comprises an A6 integrin.
42. The method of paragraphs 1,12,23,29,33, wherein the integrin comprises a B4 integrin.
43. The method of paragraphs 1,12,23,29,33, further comprising reducing a laminin5-integrin interaction.
44. The method of paragraphs 1,12,23,29,33, further comprising reducing a laminin5 gamma2 integrin interaction.
45. The method of paragraphs 1,12,23,29,33, wherein reducing the proliferation of the cancer cells is selective.
46. The method of paragraphs 1,12,23,29,33, wherein the cancer cell is not an MDA-MB-435 cell.
47. The method of paragraphs 1,12,23,29,33, wherein the cancer cell is not an HMT-3522 cell.
48. The method of paragraphs 1,12,23,29,33, wherein the cancer cell is not an RKO colon carcinoma line.
49. The method of paragraphs 1,12, 23,29,33, wherein the cancer cell does not express exogenous B4 integrin.
50. The method of paragraphs 1,12,23,29,33, further comprising contacting the cancer cells with a small molecule, peptide, peptide mimetic, or oligonucleotide or synthetic analog thereof.
51. The method of paragraph 50, wherein the cancer cells are contacted with dominant-negative beta 4 integrin.
52. The method of paragraph 50, wherein the cancer cell is contacted with an antisense molecule.
53. The method of paragraph 52, wherein the antisense molecule is linked to a leader sequence which enables translocation across a cell membrane.
54. The method of paragraph 53, wherein the leader sequence binds to a cell surface protein which facilitates internalization.
55. The method of paragraph 54, wherein the leader sequence is TAT or antennapedia, or fragment thereof.
56. The method of paragraph 52, wherein the antisense molecule is an alpha6 RNA antisense molecule.
57. The method of paragraph 47, wherein the small molecule peptide, peptide mimetic, or oligonucleotide or synthetic analog thereof is linked to a carrier.
58. The method of paragraph 57, wherein the carrier is at least one of a lipidic carrier, charged carrier, retroviral carrier, TAT or fragment thereof, antennapedia or fragment thereof, or polyethylene glycol.
59. The method of any one of paragraphs 1,12,23,29,33, further comprising contacting the cancer cell with another agent which modulates cell signaling, a chemotherapeutic drug, or treated with radiation or angiogenesis inhibitor.
60. The method of any of paragraphs 1, 12, 23, 29, 33, wherein reducing the proliferation of cancer cell is *in vitro.*
61. The method of any of paragraphs 1, 12,23,29,33, wherein reducing the proliferation of the cancer cell is *in vivo.*
62. The method of any of paragraphs 1, 12, 23, 29, 33, wherein the cancer cell is selected from the group consisting of melanoma, adenoma, lymphoma, myeloma, carcinoma, plasmocytoma, sarcoma, glioma, thyoma, leukemia, skin cancer, retinal cancer, breast cancer, prostate cancer, colon cancer, esophageal cancer, stomac cancer, pancreas cancer, brain tumors, lung cancer, ovarian cancer, cervical cancer, hepatic cancer, gastrointestinal cancer, and head and neck cancer cells.
63. The method of paragraphs 1,12,23,29,33, wherein the cancer cell is killed.
64. The method of paragraphs 1,12,23,29,33, wherein the cancer cell expresses a mutated Ras.
65. The method of paragraphs 1,12,23,29,33, wherein the cancer cell expresses a mutated Ras and a mutated p53.
66. The method of paragraphs 1,12,23,29,33, wherein the cancer cell expresses a mutated Ras and activates the AKT/PKB protein.
67. The method of paragraphs 1,12,23,29,33, wherein the cancer cell expresses a mutated Ras, a mutated p53, and activates the AKT/PKB protein.
68. The method of paragraphs 1,12,23,29,33, wherein the cancer cell expresses a mutated APC.
69. The method of paragraphs 1,12,23,29,33, wherein the cancer cell expresses a mutated Ras and mutated APC.
70. A method of isolating molecules that bind with a target molecule selected from the group consisting, B4 integrin, alpha6 integrin, and the gamma2 subunit of laminin5 comprising 1) contacting a library of molecules with the target molecule and 2) collecting molecules that bind the target molecule producing an enriched population of molecules.
71. The method of paragraph 70, further comprising the step of repeating steps 1 and 2 with the enriched population of molecules.
72. The method of paragraph 70, wherein the library comprises a small molecule, peptide, peptide mimetic, or oligonucleotide.
73. A method of assessing a subject's risk of developing cancer comprising determining the amount of A6 present in a target cell obtained from the subject, wherein a determination of increased levels of A6 correlates with an increased risk of cancer.
74. The method of paragraph 73, further comprising comparing the amount A6 present to the amount in a control cell.
75. The method of paragraph 73, wherein determining the amount of A6 present in the target cell comprises assaying the amount of A6 mRNA in the cell.
76. The method of paragraph 75, wherein the assaying the amount of mRNA in the cell comprises hybridizing a A6 probe to a sample of the subject's mRNA.
77. The method paragraph 75, wherein the assaying the amount of mRNA in the cell comprises hybridizing a A6 primer to a sample of the subject's mRNA.
78. The method paragraph 77, wherein the assaying the amount of mRNA further comprises performing an nucleic acid amplification reaction involving the primer.
79. The method of paragraph 78, wherein the nucleic acid amplification reaction comprises reverse transcription, producing a cDNA.
80. The method of paragraph 79, wherein the nucleic acid amplification reaction further comprises performing a polymerase chain reaction on the cDNA.
81. The method of paragraph 73, wherein determining the amount of A6 present in the target cell comprises assaying the amount of A6 protein in the cell.
82. A method of assessing a subject's risk of acquiring cancer comprising determining the amount of B4 present in a target cell obtained from the subject, wherein a determination of increased levels of B4 correlates with an increased risk of cancer.
83. The method of paragraph 82, further comprising comparing the amount B4 present to the amount in a control cell.
84. A method of assessing a subject's risk of acquiring cancer comprising determining the amount of laminin5 present in a target cell obtained from the subject, wherein a determination of increased levels of laminin5 correlates with an increased risk of cancer.
85. The method of paragraph 84, further comprising comparing the amount laminin5 present to the amount in a control cell.
86. A method of assessing a subject's risk of acquiring cancer comprising determining the amount of gamma2 subunit present in a target cell obtained from the subject, wherein a determination of increased levels of gamma2 subunit correlates with an increased risk of cancer.
87. The method of paragraph 86, further comprising comparing the amount gamma2 subunit present to the amount in a control cell.
88. A method of reducing the proliferation of a cancer cell in a patient which comprises administering to the patient a composition which inhibits ligand binding to an integrin receptor on the cancer cell, wherein the integrin receptor comprises an integrin.
89. A method of reducing the proliferation of a cancer cell in a patient which comprises administering to the patient a composition which reduces integrin-integrin interaction, integrin receptor clustering interaction, or integrin-non-integrin protein interaction.
90. A method of reducing the proliferation of a cancer cell in a patient which comprises administering to the patient a composition which reduces the production of an integrin or laminin by the cancer cell.
91. A method of reducing the proliferation of a cancer cell in a patient which comprises administering to the patient a composition which interfers with an integrin signaling pathway.
92. The methods of paragraphs 88-91, wherein the reduction in cancer cell proliferation is selective.
93. The method of any of paragraphs 88-91, wherein the administering is local or systemic.
94. The method of any of paragraphs 88-91, wherein the patient is additionally administered an agent which modulates cell signaling, a chemotherapeutic drug, or treated with radiation or angiogenesis inhibitor.
95. The method of paragraph 94, wherein the additional agent is administered serially or in combination.
96. The method of paragraph 93, wherein the local administering is direct application to cancer cells.
97. The method of paragraph 96, wherein the direct application to cancer cells is performed during surgery.
98. The method of paragraph 97, wherein the direct application to cancer cells is performed topically to cancerous tissue.
99. The method of paragraph 94, wherein the systemic administration is by subcutaneous, intraperitoneal, intra-arterial, intravenous, or bolus administration, or by application through a catheter or similar apparatus.
100. The method of paragraph 94, wherein the systemic administration comprises a long-term release formulation.
101. The method of paragraph 94, wherein the systemic administration is by oral administration.
102. The method of paragraph 101 wherein the oral administration comprises administering a pill, capsule, tablet, liquid or suspension.
103.A method of reducing the proliferation of a cancer cell in a patient which comprises administering to a patient a vector comprising coding sequence for a protein or peptide which inhibits ligand binding to integrin receptors on cancer cells wherein the coding sequence is under the control of a promoter which functions in mammalian cells.
104.A method of reducing the proliferation of a cancer cell in a patient which comprises administering to a patient a vector comprising coding sequence for a protein or peptide which prevents integrin receptor subunits from interacting with one another, prevents integrin receptor clustering interaction, or prevents integrin receptor subunits from interacting with other proteins on cancer cells wherein the coding sequence is under the control of a promoter which functions in mammalian cells.
105. A method of reducing the proliferation of a cancer cell in a patient which comprises administering to a patient a vector comprising coding sequence for a protein or peptide which prevents integrin receptor subunits from interacting with one another, prevents integrin receptor clustering interaction, or prevents integrin receptor subunits from interacting with other proteins in cancer cells wherein the coding sequence is under the control of a promoter which functions in mammalian cells.
106. A method of reducing the proliferation of a cancer cell in a patient which comprises administering to a patient a vector comprising coding sequence for a protein or peptide which interferes with integrin subunit or laminin production wherein the coding sequence is under the control of a promoter which functions in mammalian cells.
107. A method of reducing the proliferation of a cancer cell in a patient which comprises administering to a patient a vector comprising coding sequence for a protein or peptide which interferes with an integrin signaling pathway of cells wherein the coding sequence is under the control of a promoter which functions in mammalian cells.
108.The methods of paragraphs 103-107, wherein the reduction in cancer cell proliferation is selective.
109.The method of paragraphs 103-107 wherein the vector is administered directly to a cancer cell.
110.The method of paragraphs 103-107, wherein the vector is administered directly to a normal cell.
111.The method of paragraphs 103-107, wherein the vector is packaged in a viral vector or liposome.
112.The method of paragraph 88 wherein the vector is a retroviral vector.
113.The method of any one of paragraphs 103-107, wherein the vector is administered systemically.
114.The method of paragraph 109, wherein the direct administration is by topical application.
115.The method of paragraph 111, wherein the direct administration is by topical application.
116.The method of paragraph 114, wherein the direct administration is performed during surgery.
117.The method of paragraph 115, wherein the direct administration is performed during surgery.
118.The method of any of paragraphs 103-107, wherein the patient is an animal.
119.The method of paragraph 118 wherein the animal is a mammal.
120.The method of paragraph 119, wherein the mammal is human.
121.The method of paragraphs 103-107, wherein the cancer cells are selected from the group consisting of melanoma, adenoma, lymphoma, myeloma, carcinoma, plasmocytoma, sarcoma, glioma, thyoma, leukemia, skin cancer, retinal cancer, breast cancer, prostate cancer, colon cancer, esophageal cancer, stomac cancer, pancreas cancer, brain tumors, lung cancer, ovarian cancer, cervical cancer, hepatic cancer, gastrointestinal cancer, and head and neck cancer cells.
122.The method of any of paragraphs 103-107 wherein the patient is additionally administered at least one of another agent which modulates cell signaling, a chemotherapeutic drug, an angiogenesis inhibitor or treated with radiation.
123.The method of paragraph 120 wherein the other agent which modifies cell signaling, chemotherapeutic drug, angiogenesis inhibitor or radiation treatment is administered serially or in combination.

## Claims

1. An in-vitro method of reducing the proliferation of a cancer cell which comprises reducing the production of an integrin by the cancer cell, wherein the integrin comprises A6 integrin, and wherein the cancer cell comprises a mutated p53 and mutated Ras; or
an in-vitro method of reducing the proliferation of a cancer cell which comprises reducing the production of an integrin receptor ligand by the cancer cell wherein the cancer cell comprises a mutated p53 and mutated Ras; or
an in-vitro method of reducing the proliferation of a cancer cell which comprises interfering with an integrin signaling pathway, wherein the integrin comprises A6 integrin, and wherein the cancer cell comprises a mutated p53 and mutated Ras.

2. A composition for reducing the production of an integrin by a cancer cell, for use for reducing the proliferation of the cancer cell, wherein the integrin comprises A6 integrin, and wherein the cancer cell comprises a mutated p53 and mutated Ras;
or a composition for reducing the production of an integrin receptor ligand by a cancer cell for use for reducing the proliferation of the cancer cell wherein the cancer cell comprises a mutated p53 and mutated Ras; or
a composition for interfering with an integrin signaling pathway for use for reducing the proliferation of a cancer cell, wherein the integrin comprises A6 integrin, and wherein the cancer cell comprises a mutated p53 and mutated Ras.

3. The method of claim 1 or the composition of claim 2 of reducing the proliferation of a cancer cell which comprises reducing the production of an integrin by the cancer cell, wherein the production of an integrin is reduced by inhibiting signaling leading to induction of expression of an integrin.; or wherein reducing the production of an integrin comprises inhibiting alpha6 or beta4 production; or wherein inhibiting alpha6 or beta4 production further comprises using antisense molecules to alpha6 mRNA or beta4 mRNA, respectively.

4. The method of claim 1 for the composition of claim 2 of reducing the production of an integrin receptor ligand by the cancer cell, wherein reducing the production of an integrin receptor ligand comprises inhibiting gamma2 production; or wherein reducing the production of an integrin receptor ligand comprises inhibiting laminin5 production.

5. The method of claim 1 or the composition of claim 2 of interfering with an integrin signaling pathway, wherein interfering with an integrin signaling pathway comprises contacting the cancer cell with a molecule that interferes with at least one of talin, paxillin, vinculin, a CAS family protein, CRX, NCK, FAK, ILK, Src, Fyn, Shc, Grb-2, Guaning nucleotide exchange factors, SOS, DOCK 180, Vav, Syk, P-1-3 kinase, AKT, Bad, Bid, Caspase 9, Cdc42, PAK, Rac, Rho, Rho kinase, Ras, Caveolin, Tetraspan, Receptor-type protein tyrosine phosphatase, SHP-2, Alpha-actinin, Filamin, Cytohesin, Beta3-endonexin, ICAP-1, RACK-1, CLB, actin, receptor tyrosine kinase, IRS-1 or IRS-2; or
wherein interfering with an integrin signaling pathway comprises contacting the cancer cell with an agent that interferes with post-translational modification of integrins, wherein preferably the post translational modification is glycosylation or phosphorylation.

6. The method of claim 1 or the composition of claim 2, wherein the integrin comprises a B4 integrin; or
further comprising reducing a laminin5- integrin or laminin5-gamma2 integrin interaction; or
wherein reducing the proliferation of the cancer cells is selective; or wherein the cancer cell does not express exogenous B4 integrin.

7. The method of claim 1 or the composition of claim 2, further comprising contacting the cancer cells with a small molecule, peptide, peptide mimetic, or oligonucleotide or synthetic analog thereof.

8. The method or the composition of claim 7,
a) wherein the cancer cells are contacted with dominant- negative beta 4 integrin; or
b) wherein the cancer cell is contacted with an antisense molecule,
i) the antisense molecule being preferably linked to a leader sequence which enables translocation across a cell membrane, which leader sequence binds preferably to a cell surface protein which facilitates internalization, said leader sequence being preferably TAT or antennapedia, or fragment thereof; or
ii) the antisense molecule being an alpha6 RNA antisense molecule.

9. The method of claim 1 or the composition of claim 2, wherein the cancer cell is not an HMT-3522 cell and wherein the small molecule peptide, peptide mimetic, or oligonucleotide or synthetic analog thereof is linked to a carrier.

10. The method or the composition of claim 9, wherein the carrier is at least one of a lipidic carrier, charged carrier, retroviral carrier, TAT or fragment thereof, antennapedia or fragment thereof, or polyethylene glycol.

11. The method of claim 1 or the composition of claim 2, further comprising contacting the cancer cell with another agent which modulates cell signaling, a chemotherapeutic drug, or treated with radiation or anglogenesis inhibitor.

12. The method of claim 1 or the composition of claim 2, wherein reducing the proliferation of the cancer cell is in vivo; or
wherein the cancer cell is selected from the group consisting of melanoma, adenoma, lymphom, myeloma, carcinoma, plasmocytoma, sarcoma, glioma, thyoma, leukemia, skin cancer, retinal cancer, breast cancer, prostate cancer, colon cancer, esophageal cancer, stomac cancer, pancreas cancer, brain tumors, lung cancer, ovarian cancer, cervical cancer, hepatic cancer, gastrointestinal cancer, and head and neck cancer cells.


SEQUENCE LISTING

<110> University of Rochester Medical Center

      Deleu, Laurent
      Land, Hartmut

<120> COMPOSITIONS THAT INHIBIT PROLIFERATION
  OF CANCER CELLS

<130> 21108.0005P1

<150> 60/239,705
<151> 2000-10-12

<150> 60/242,812
<151> 2000-10-24

<160> 19

<170> FastSEQ for Windows Version 4.0

<210> 1
<211> 3222
<212> DNA
<213> Artificial Sequence

<220>
<223> Description of Artificial Sequence; Note =
      synthetic construct

<400> 1
atggccgccg ccgggcagct gtgcttgctc tacctgtcgg cggggctcct gtcccggctc      60
ggcgcagcct tcaacttgga cactcgggag gacaacgtga tccggaaata tggagacccc     120
gggagcctct tcggcttctc gctggccatg cactggcaac tgcagcccga ggacaagcgg     180
ctgttgctcg tgggggcccc gcgcggagaa gcgcttccac tgcagagagc caacagaacg     240
ggagggctgt acagctgcga catcaccgcc cgggggccat gcacgcggat cgagtttgat     300
aacgatgctg accccacgtc agaaagcaag gaagatcagt ggatgggggt caccgtccag     360
agccaaggtc caggggggcaa ggtcgtgaca tgtgctcacc gatatgaaaa aaggcagcat     420
gttaatacga agcaggaatc ccgagacatc tttgggcggt gttatgtcct gagtcagaat     480
ctcaggattg aagacgatat ggatggggga gattggagct tttgtgatgg gcgattgaga     540
ggccatgaga aatttggctc ttgccagcaa ggtgtagcag ctacttttac taaagacttt     600
cattacattg tatttggagc cccgggtact tataactgga aagggattgt tcgtgtagag     660
caaaagaata acactttttt tgacatgaac atctttgaag atgggcctta tgaagttggt     720
ggagagactg agcatgatga aagtctcgtt cctgttcctg ctaacagtta cttaggtttt     780
tctttggact cagggaaagg tattgtttct aaagatgaga tcacttttgt atctggtgct     840
cccagagcca atcacagtgg agccgtggtt ttgctgaaga gagacatgaa gtctgcacat     900
ctcctccctg agcacatatt cgatggagaa ggtctggcct cttcatttgg ctatgatgtg     960
gcggtggtgg acctcaacaa ggatgggtgg caagatatag ttattggagc cccacagtat    1020
tttgatagag atggagaagt tggaggtgca gtgtatgtct acatgaacca gcaaggcaga    1080
tggaataatg tgaagccaat tcgtcttaat ggaaccaaag attctatgtt tggcattgca    1140
gtaaaaaata ttggagatat taatcaagat ggctacccag atattgcagt tggagctccg    1200
tatgatgact tgggaaaggt ttttatctat catggatctg caaatggaat aaataccaaa    1260
ccaacacagg ttctcaaggg tatatcacct tattttggat attcaattgc tggaaacatg    1320
gaccttgatc gaaattccta ccctgatgtt gctgttggtt ccctctcaga ttcagtaact    1380
attttcagat cccggcctgt gattaatatt cagaaaacca tcacagtaac tcctaacaga    1440
attgacctcc gccagaaaac agcgtgtggg gcgcctagtg ggatatgcct ccaggttaaa    1500
tcctgttttg aatatactgc taaccccgct ggttataatc cttcaatatc aattgtgggc    1560
acacttgaag ctgaaaaaga aagaagaaaa tctgggctat cctcaagagt tcagtttcga    1620
aaccaaggtt ctgagcccaa atatactcaa gaactaactc tgaagaggca gaaacagaaa    1680
gtgtgcatgg aggaaaccct gtggctacag gataatatca gagataaact gcgtcccatt    1740
cccataactg cctcagtgga gatccaagag ccaagctctc gtaggcgagt gaattcactt    1800
ccagaagttc ttccaattct gaattcagat gaacccaaga cagctcatat tgatgttcac    1860
ttcttaaaag agggatgtgg agacgacaat gtatgtaaca gcaaccttaa actagaatat    1920
aaattttgca cccgagaagg aaatcaagac aaattttctt atttaccaat tcaaaaaggt    1980
gtaccagaac tagttctaaa agatcagaag gatattgctt tagaaataac agtgacaaac    2040
agcccttcca acccaaggaa tcccacaaaa gatggcgatg acgcccatga ggctaaactg    2100

attgcaacgt ttccagacac tttaacctat tctgcatata gagaactgag ggctttccct 2160
gagaaacagt tgagttgtgt tgccaaccag aatggctcgc aagctgactg tgagctcgga 2220
aatcctttta aaagaaattc aaatgtcact ttttatttgg ttttaagtac aactgaagtc 2280
acctttgaca ccccatatct ggatattaat ctgaagttag aaacaacaag caatcaagat 2340
aatttggctc caattacagc taaagcaaaa gtggttattg aactgctttt atcggtctcg 2400
ggagttgcta aaccttccca ggtgtatttt ggaggtacag ttgttggcga gcaagctatg 2460
aaatctgaag atgaagtggg aagtttaata gagtatgaat tcagggtaat aaacttaggt 2520
aaacctctta caaacctcgg cacagcaacc ttgaacattc agtggccaaa agaaattagc 2580
aatgggaaat ggttgcttta tttggtgaaa gtagaatcca aaggattgga aaaggtaact 2640
tgtgagccac aaaaggagat aaactccctg aacctaacgg agtctcacaa ctcaagaaag 2700
aaacgggaaa ttactgaaaa acagatagat gataacagaa aattttcttt atttgctgaa 2760
agaaaatacc agactcttaa ctgtagcgtg aacgtgaact gtgtgaacat cagatgcccg 2820
ctgcgggggc tggacagcaa ggcgtctctt attttgcgct cgaggttatg gaacagcaca 2880
tttctagagg aatattccaa actgaactac ttggacattc tcatgcgagc cttcattgat 2940
gtgactgctg ctgccgaaaa tatcaggctg ccaaatgcag gcactcaggt tcgagtgact 3000
gtgtttccct caaagactgt agctcagtat tcgggagtac cttggtggat catcctagtg 3060
gctattctcg ctgggatctt gatgcttgct ttattagtgt ttatactatg gaagtgtggt 3120
ttcttcaaga gaaataagaa agatcattat gatgccacat atcacaaggc tgagatccat 3180
gctcagccat ctgataaaga gaggcttact tctgatgcat ag 3222

<210> 2
<211> 1072
<212> PRT
<213> Artificial Sequence

<220>
<223> Description of Artificial Sequence; Note =
      synthetic construct

<400> 2
Met Ala Ala Ala Gly Gln Leu Cys Leu Leu Tyr Leu Ser Ala Gly Leu
1               5                   10                  15
Leu Ser Arg Leu Gly Ala Ala Phe Asn Leu Asp Thr Arg Glu Asp Asn
            20                  25                  30
Val Ile Arg Lys Tyr Gly Asp Pro Gly Ser Leu Phe Gly Phe Ser Leu
        35                  40                  45
Ala Met His Trp Gln Leu Gln Pro Glu Asp Lys Arg Leu Leu Val Gly
    50                  55                  60
Ala Pro Arg Gly Glu Ala Leu Pro Leu Gln Arg Ala Asn Arg Thr Gly
65                  70                  75                  80
Gly Leu Tyr Ser Cys Asp Ile Thr Ala Arg Gly Pro Cys Thr Arg Ile
                85                  90                  95
Glu Phe Asp Asn Asp Ala Asp Pro Thr Ser Glu Ser Lys Glu Asp Gln
            100                 105                 110
Trp Met Gly Val Thr Val Gln Ser Gln Gly Pro Gly Gly Lys Val Val
        115                 120                 125
Thr Cys Ala His Arg Tyr Glu Lys Arg Gln His Val Asn Thr Lys Gln
    130                 135                 140
Glu Ser Arg Asp Ile Phe Gly Arg Cys Tyr Val Leu Ser Gln Asn Leu
145                 150                 155                 160
Arg Ile Glu Asp Asp Met Asp Gly Gly Asp Trp Ser Phe Cys Asp Gly
                165                 170                 175
Arg Leu Arg Gly His Glu Lys Phe Gly Ser Cys Gln Gln Gly Val Ala
            180                 185                 190
Ala Thr Phe Thr Lys Asp Phe His Tyr Ile Val Phe Gly Ala Pro Gly
        195                 200                 205
Thr Tyr Asn Trp Lys Gly Ile Val Arg Val Glu Gln Lys Asn Asn Thr
    210                 215                 220
Phe Phe Asp Met Asn Ile Phe Glu Asp Gly Pro Tyr Glu Val Gly Gly
225                 230                 235                 240
Glu Thr Glu His Asp Glu Ser Leu Val Pro Val Pro Ala Asn Ser Tyr
                245                 250                 255
Leu Gly Phe Ser Leu Asp Ser Gly Lys Gly Ile Val Ser Lys Asp Glu
            260                 265                 270
Ile Thr Phe Val Ser Gly Ala Pro Arg Ala Asn His Ser Gly Ala Val
        275                 280                 285
Val Leu Leu Lys Arg Asp Met Lys Ser Ala His Leu Leu Pro Glu His
    290                 295                 300

Ile Phe Asp Gly Glu Gly Leu Ala Ser Ser Phe Gly Tyr Asp Val Ala
305                 310                 315                 320
Val Val Asp Leu Asn Lys Asp Gly Trp Gln Asp Ile Val Ile Gly Ala
                325                 330                 335
Pro Gln Tyr Phe Asp Arg Asp Gly Glu Val Gly Gly Ala Val Tyr Val
            340                 345                 350
Tyr Met Asn Gln Gln Gly Arg Trp Asn Asn Val Lys Pro Ile Arg Leu
        355                 360                 365
Asn Gly Thr Lys Asp Ser Met Phe Gly Ile Ala Val Lys Asn Ile Gly
    370                 375                 380
Asp Ile Asn Gln Asp Gly Tyr Pro Asp Ile Ala Val Gly Ala Pro Tyr
385                 390                 395                 400
Asp Asp Leu Gly Lys Val Phe Ile Tyr His Gly Ser Ala Asn Gly Ile
                405                 410                 415
Asn Thr Lys Pro Thr Gln Val Leu Lys Gly Ile Ser Pro Tyr Phe Gly
            420                 425                 430
Tyr Ser Ile Ala Gly Asn Met Asp Leu Asp Arg Asn Ser Tyr Pro Asp
        435                 440                 445
Val Ala Val Gly Ser Leu Ser Asp Ser Val Thr Ile Phe Arg Ser Arg
    450                 455                 460
Pro Val Ile Asn Ile Gln Lys Thr Ile Thr Val Thr Pro Asn Arg Ile
465                 470                 475                 480
Asp Leu Arg Gln Lys Thr Ala Cys Gly Ala Pro Ser Gly Ile Cys Leu
                485                 490                 495
Gln Val Lys Ser Cys Phe Glu Tyr Thr Ala Asn Pro Ala Gly Tyr Asn
            500                 505                 510
Pro Ser Ile Ser Ile Val Gly Thr Leu Glu Ala Glu Lys Glu Arg Arg
        515                 520                 525
Lys Ser Gly Leu Ser Ser Arg Val Gln Phe Arg Asn Gln Gly Ser Glu
    530                 535                 540
Pro Lys Tyr Thr Gln Glu Leu Thr Leu Lys Arg Gln Lys Gln Lys Val
545                 550                 555                 560
Cys Met Glu Glu Thr Leu Trp Leu Gln Asp Asn Ile Arg Asp Lys Leu
                565                 570                 575
Arg Pro Ile Pro Ile Thr Ala Ser Val Glu Ile Gln Glu Pro Ser Ser
            580                 585                 590
Arg Arg Arg Val Asn Ser Leu Pro Glu Val Leu Pro Ile Leu Asn Ser
        595                 600                 605
Asp Glu Pro Lys Thr Ala His Ile Asp Val His Phe Leu Lys Glu Gly
    610                 615                 620
Cys Gly Asp Asp Asn Val Cys Asn Ser Asn Leu Lys Leu Glu Tyr Lys
625                 630                 635                 640
Phe Cys Thr Arg Glu Gly Asn Gln Asp Lys Phe Ser Tyr Leu Pro Ile
                645                 650                 655
Gln Lys Gly Val Pro Glu Leu Val Leu Lys Asp Gln Lys Asp Ile Ala
            660                 665                 670
Leu Glu Ile Thr Val Thr Asn Ser Pro Ser Asn Pro Arg Asn Pro Thr
        675                 680                 685
Lys Asp Gly Asp Asp Ala His Glu Ala Lys Leu Ile Ala Thr Phe Pro
    690                 695                 700
Asp Thr Leu Thr Tyr Ser Ala Tyr Arg Glu Leu Arg Ala Phe Pro Glu
705                 710                 715                 720
Lys Gln Leu Ser Cys Val Ala Asn Gln Asn Gly Ser Gln Ala Asp Cys
                725                 730                 735
Glu Leu Gly Asn Pro Phe Lys Arg Asn Ser Asn Val Thr Phe Tyr Leu
            740                 745                 750
Val Leu Ser Thr Thr Glu Val Thr Phe Asp Thr Pro Tyr Leu Asp Ile
        755                 760                 765
Asn Leu Lys Leu Glu Thr Thr Ser Asn Gln Asp Asn Leu Ala Pro Ile
    770                 775                 780
Thr Ala Lys Ala Lys Val Val Ile Glu Leu Leu Leu Ser Val Ser Gly
785                 790                 795                 800
Val Ala Lys Pro Ser Gln Val Tyr Phe Gly Gly Thr Val Val Gly Glu
                805                 810                 815
Gln Ala Met Lys Ser Glu Asp Glu Val Gly Ser Leu Ile Glu Tyr Glu
            820                 825                 830
Phe Arg Val Ile Asn Leu Gly Lys Pro Leu Thr Asn Leu Gly Thr Ala
        835                 840                 845

Thr Leu Asn Ile Gln Trp Pro Lys Glu Ile Ser Asn Gly Lys Trp Leu
    850                 855                 860
Leu Tyr Leu Val Lys Val Glu Ser Lys Gly Leu Glu Lys Val Thr Cys
865                 870                 875                 880
Glu Pro Gln Lys Glu Ile Asn Ser Leu Asn Leu Thr Glu Ser His Asn
                885                 890                 895
Ser Arg Lys Lys Arg Glu Ile Thr Glu Lys Gln Ile Asp Asp Asn Arg
            900                 905                 910
Lys Phe Ser Leu Phe Ala Glu Arg Lys Tyr Gln Thr Leu Asn Cys Ser
        915                 920                 925
Val Asn Val Asn Cys Val Asn Ile Arg Cys Pro Leu Arg Gly Leu Asp
    930                 935                 940
Ser Lys Ala Ser Leu Ile Leu Arg Ser Arg Leu Trp Asn Ser Thr Phe
945                 950                 955                 960
Leu Glu Glu Tyr Ser Lys Leu Asn Tyr Leu Asp Ile Leu Met Arg Ala
                965                 970                 975
Phe Ile Asp Val Thr Ala Ala Ala Glu Asn Ile Arg Leu Pro Asn Ala
            980                 985                 990
Gly Thr Gln Val Arg Val Thr Val Phe Pro Ser Lys Thr Val Ala Gln
        995                 1000                1005
Tyr Ser Gly Val Pro Trp Trp Ile Ile Leu Val Ala Ile Leu Ala Gly
    1010                1015                1020
Ile Leu Met Leu Ala Leu Leu Val Phe Ile Leu Trp Lys Cys Gly Phe
1025                1030                1035                1040
Phe Lys Arg Asn Lys Lys Asp His Tyr Asp Ala Thr Tyr His Lys Ala
                1045                1050                1055
Glu Ile His Ala Gln Pro Ser Asp Lys Glu Arg Leu Thr Ser Asp Ala
            1060                1065                1070

<210> 3
<211> 3222
<212> DNA
<213> Artificial Sequence

<220>
<223> Description of Artificial Sequence; Note =
synthetic construct

<400> 3
atggccgtcg cgggccagtt gtgcctgctc tacctgtccg cggggcttct agcccggctg 60
ggtacagcct tcaacctgga cacccgcgag gacaacgtga tccggaaatc gggggatccc 120
gggagcctct tcggcttctc gctcgccatg cactggcagt tgcagccgga ggacaagcgg 180
ctgttgcttg tgggggcacc tcgggcagaa gcactcccgc tgcagagggc gaacagaaca 240
gggggcctgt acagctgtga catcacctcc cgaggacctt gtacacggat tgaatttgat 300
aatgacgctg atcctatgtc agaaagcaag gaagaccagt ggatgggagt cactgtccag 360
agccaaggtc caggggggcaa agtggtgacg tgtgcacatc gatatgagaa acggcagcac 420
gtcaacacga agcaggagtc gcgggatatc tttggaagat gttatgtcct gagtcagaat 480
ctcagaattg aagatgatat ggacggagga gactggagtt tctgcgatgg ccggttgaga 540
ggccatgaaa agtttggctc ctgtcagcaa ggagtagcgg ctactttcac taaggacttt 600
cattacattg tttttggagc cccagggact tacaactgga aagggatcgt ccgtgtagaa 660
caaaagaata acactttttt tgacatgaac atctttgaag atgggcccta tgaagttggt 720
ggagagacag atcatgatga aagtctcgtg cccgttcctg ctaacagtta cctaggcttt 780
tcgctggact cagggaaggg tattgtttct aaagatgaca tcacttttgt gtctggtgct 840
ccaagagcca atcacagtgg ggctgtagtg ttgctaaaaa gagacatgaa gtccgcacat 900
ctgctccctg agtatatatt tgacggagaa ggcctggctt cctcgtttgg ctatgatgtg 960
gcagtggtgg acctcaatgc agatgggtgg caagacatcg ttatcggagc tccacagtat 1020
tttgataggg atggtgaagt cgggggtgca gtttacgtct acattaacca gcaaggcaaa 1080
tggagtaatg tgaagccgat tcgtctaaat gggaccaaag actcgatgtt tggaatctct 1140
gtgaaaaata taggtgatat taaccaagat ggctatccag atattgctgt tggagctccc 1200
tatgatgatc tggggaaggt ttttatctat catggatccc cgactggcat aattaccaag 1260
ccaacacagg ttctcgaggg gacatcgcct tacttcggct attcaatcgc tgggaatatg 1320
gacctggatc ggaattccta ccccgacctt gctgtgggct ccctctcaga ctcggtcact 1380
attttcagat cccggccagt gattaacatt ctaaaaacca tcacagtgac tcctaacaga 1440
attgacctcc gccagaagtc catgtgtggc tcacctagcg ggatatgcct caaggttaaa 1500
gcctgttttg aatatactgc gaaaccttcc ggttataacc ctccaatatc aattttgggt 1560
attcttgaag ctgaaaaaga aagaagaaaa tcagggttgt catcgagagt tcagtttcga 1620
aaccaaggtt ccgagccaaa gtatactcag gagctgaccc tgaatcggca gaagcagcgg 1680
gcgtgcatgg aggagaccct ctggctgcag gagaacatca gagacaagct gcgtcccatc 1740

cccatcacgg cttctgtgga gatccaggag cccacgtctc gccggcgggt gaactcactc 1800
cccgaagttc ttcccatcct gaattcaaat gaagccaaaa cggtccagac agatgtccac 1860
ttcttaaagg aaggatgtgg agacgacaat gtctgtaaca gcaaccttaa gctagagtat 1920
aaatttggta cccgagaagg aaatcaagac aaattctctt accttccaat tcaaaaaggc 1980
atcccagaat tagtcctaaa agatcagaaa gatatagctc tggaaataac ggtgaccaac 2040
agcccttcgg atccaaggaa tccccggaaa gatggcgacg atgcccatga agccaaactc 2100
atcgccacgt ttccagacac tctgacatat tccgcttaca gagaactgag ggctttccct 2160
gagaagcagc tgagctgtgt ggccaaccag aatggctccc aagccgactg tgagctcgga 2220
aatcctttca agagaaattc cagtgttact ttctatctga ttttaagtac aaccgaggtc 2280
acctttgaca ccacagatct ggatattaat ctgaagttgg aaacaacaag caatcaggat 2340
aaattggctc caattacagc gaaggcaaaa gtggttattg aattgctttt atccctctcc 2400
ggagtcgcta agccttcgca ggtgtatttt ggaggtacag ttgttggtga gcaagctatg 2460
aaatctgaag atgaagtagg aagtttaata gagtatgaat ttagggtgat taacttaggc 2520
aagcctctta aaaacctcgg cacagcaacc ttgaatatac agtggcccaa ggagattagc 2580
aatggcaaat ggttgcttta tttgatgaaa gttgaatcca aaggtttgga gcagattgtt 2640
tgtgagccac acaatgaaat aaactacctg aagctgaagg agtctcacaa ctcaagaaag 2700
aaacgggaac ttcctgaaaa acagatagat gacagcagga aatttctttt atttcctgaa 2760
agaaaatacc agactctcaa ctgcagcgtc aacgtcaggt gtgtgaacat caggtgccca 2820
ctgcgagggc tggacacgaa ggcctctctc gttctgtgtt ccaggttgtg gaacagcaca 2880
tttctagagg aatattccaa actgaactac ttggacattc tcgtgagggc ttccatagat 2940
gtcaccgctg ctgctcagaa tatcaagctc cctcacgcgg gcactcaggt tcgagtgacg 3000
gtgtttccct caaagactgt agctcagtat tcaggagtag cttggtggat catcctcctg 3060
gctgttcttg ccgggattct gatgctggct ctattagtgt ttttactgtg gaagtgtggc 3120
ttcttcaaga gaaataagaa agatcattac gatgccacct atcacaaggc tgagatccat 3180
actcagccgt ctgataaaga gaggcttact tccgatgcat ag 3222

<210> 4
<211> 1073
<212> PRT
<213> Artificial Sequence

<220>
<223> Description of Artificial Sequence; Note =
      synthetic construct

<400> 4
Met Ala Val Ala Gly Gln Leu Cys Leu Leu Tyr Leu Ser Ala Gly Leu
1               5                   10                  15
Leu Ala Arg Leu Gly Thr Ala Phe Asn Leu Asp Thr Arg Glu Asp Asn
            20                  25                  30
Val Ile Arg Lys Ser Gly Asp Pro Gly Ser Leu Phe Gly Phe Ser Leu
        35                  40                  45
Ala Met His Trp Gln Leu Gln Pro Glu Asp Lys Arg Leu Leu Leu Val
    50                  55                  60
Gly Ala Pro Arg Ala Glu Ala Leu Pro Leu Gln Arg Ala Asn Arg Thr
65                  70                  75                  80
Gly Gly Leu Tyr Ser Cys Asp Ile Thr Ser Arg Gly Pro Cys Thr Arg
                85                  90                  95
Ile Glu Phe Asp Asn Asp Ala Asp Pro Met Ser Glu Ser Lys Glu Asp
            100                 105                 110
Gln Trp Met Gly Val Thr Val Gln Ser Gln Gly Pro Gly Gly Lys Val
        115                 120                 125
Val Thr Cys Ala His Arg Tyr Glu Lys Arg Gln His Val Asn Thr Lys
    130                 135                 140
Gln Glu Ser Arg Asp Ile Phe Gly Arg Cys Tyr Val Leu Ser Gln Asn
145                 150                 155                 160
Leu Arg Ile Glu Asp Asp Met Asp Gly Gly Asp Trp Ser Phe Cys Asp
                165                 170                 175
Gly Arg Leu Arg Gly His Glu Lys Phe Gly Ser Cys Gln Gln Gly Val
            180                 185                 190
Ala Ala Thr Phe Thr Lys Asp Phe His Tyr Ile Val Phe Gly Ala Pro
        195                 200                 205
Gly Thr Tyr Asn Trp Lys Gly Ile Val Arg Val Glu Gln Lys Asn Asn
    210                 215                 220
Thr Phe Phe Asp Met Asn Ile Phe Glu Asp Gly Pro Tyr Glu Val Gly
225                 230                 235                 240
Gly Glu Thr Asp His Asp Glu Ser Leu Val Pro Val Pro Ala Asn Ser
                245                 250                 255

Tyr Leu Gly Phe Ser Leu Asp Ser Gly Lys Gly Ile Val Ser Lys Asp
            260                 265                 270
Asp Ile Thr Phe Val Ser Gly Ala Pro Arg Ala Asn His Ser Gly Ala
        275                 280                 285
Val Val Leu Leu Lys Arg Asp Met Lys Ser Ala His Leu Leu Pro Glu
    290                 295                 300
Tyr Ile Phe Asp Gly Glu Gly Leu Ala Ser Ser Phe Gly Tyr Asp Val
305                 310                 315                 320
Ala Val Val Asp Leu Asn Ala Asp Gly Trp Gln Asp Ile Val Ile Gly
                325                 330                 335
Ala Pro Gln Tyr Phe Asp Arg Asp Gly Glu Val Gly Gly Ala Val Tyr
            340                 345                 350
Val Tyr Ile Asn Gln Gln Gly Lys Trp Ser Asn Val Lys Pro Ile Arg
        355                 360                 365
Leu Asn Gly Thr Lys Asp Ser Met Phe Gly Ile Ser Val Lys Asn Ile
    370                 375                 380
Gly Asp Ile Asn Gln Asp Gly Tyr Pro Asp Ile Ala Val Gly Ala Pro
385                 390                 395                 400
Tyr Asp Asp Leu Gly Lys Val Phe Ile Tyr His Gly Ser Pro Thr Gly
                405                 410                 415
Ile Ile Thr Lys Pro Thr Gln Val Leu Glu Gly Thr Ser Pro Tyr Phe
            420                 425                 430
Gly Tyr Ser Ile Ala Gly Asn Met Asp Leu Asp Arg Asn Ser Tyr Pro
        435                 440                 445
Asp Leu Ala Val Gly Ser Leu Ser Asp Ser Val Thr Ile Phe Arg Ser
    450                 455                 460
Arg Pro Val Ile Asn Ile Leu Lys Thr Ile Thr Val Thr Pro Asn Arg
465                 470                 475                 480
Ile Asp Leu Arg Gln Lys Ser Met Cys Gly Ser Pro Ser Gly Ile Cys
                485                 490                 495
Leu Lys Val Lys Ala Cys Phe Glu Tyr Thr Ala Lys Pro Ser Gly Tyr
            500                 505                 510
Asn Pro Pro Ile Ser Ile Leu Gly Ile Leu Glu Ala Glu Lys Glu Arg
        515                 520                 525
Arg Lys Ser Gly Leu Ser Ser Arg Val Gln Phe Arg Asn Gln Gly Ser
    530                 535                 540
Glu Pro Lys Tyr Thr Gln Glu Leu Thr Leu Asn Arg Gln Lys Gln Arg
545                 550                 555                 560
Ala Cys Met Glu Glu Thr Leu Trp Leu Gln Glu Asn Ile Arg Asp Lys
                565                 570                 575
Leu Arg Pro Ile Pro Ile Thr Ala Ser Val Glu Ile Gln Glu Pro Thr
            580                 585                 590
Ser Arg Arg Arg Val Asn Ser Leu Pro Glu Val Leu Pro Ile Leu Asn
        595                 600                 605
Ser Asn Glu Ala Lys Thr Val Gln Thr Asp Val His Phe Leu Lys Glu
    610                 615                 620
Gly Cys Gly Asp Asp Asn Val Cys Asn Ser Asn Leu Lys Leu Glu Tyr
625                 630                 635                 640
Lys Phe Gly Thr Arg Glu Gly Asn Gln Asp Lys Phe Ser Tyr Leu Pro
                645                 650                 655
Ile Gln Lys Gly Ile Pro Glu Leu Val Leu Lys Asp Gln Lys Asp Ile
            660                 665                 670
Ala Leu Glu Ile Thr Val Thr Asn Ser Pro Ser Asp Pro Arg Asn Pro
        675                 680                 685
Arg Lys Asp Gly Asp Asp Ala His Glu Ala Lys Leu Ile Ala Thr Phe
    690                 695                 700
Pro Asp Thr Leu Thr Tyr Ser Ala Tyr Arg Glu Leu Arg Ala Phe Pro
705                 710                 715                 720
Glu Lys Gln Leu Ser Cys Val Ala Asn Gln Asn Gly Ser Gln Ala Asp
                725                 730                 735
Cys Glu Leu Gly Asn Pro Phe Lys Arg Asn Ser Ser Val Thr Phe Tyr
            740                 745                 750
Leu Ile Leu Ser Thr Thr Glu Val Thr Phe Asp Thr Thr Asp Leu Asp
        755                 760                 765
Ile Asn Leu Lys Leu Glu Thr Thr Ser Asn Gln Asp Lys Leu Ala Pro
    770                 775                 780
Ile Thr Ala Lys Ala Lys Val Val Ile Glu Leu Leu Leu Ser Leu Ser
785                 790                 795                 800

Gly Val Ala Lys Pro Ser Gln Val Tyr Phe Gly Gly Thr Val Val Gly
                805                 810                 815
Glu Gln Ala Met Lys Ser Glu Asp Glu Val Gly Ser Leu Ile Glu Tyr
            820                 825                 830
Glu Phe Arg Val Ile Asn Leu Gly Lys Pro Leu Lys Asn Leu Gly Thr
        835                 840                 845
Ala Thr Leu Asn Ile Gln Trp Pro Lys Glu Ile Ser Asn Gly Lys Trp
    850                 855                 860
Leu Leu Tyr Leu Met Lys Val Glu Ser Lys Gly Leu Glu Gln Ile Val
865                 870                 875                 880
Cys Glu Pro His Asn Glu Ile Asn Tyr Leu Lys Leu Lys Glu Ser His
                885                 890                 895
Asn Ser Arg Lys Lys Arg Glu Leu Pro Glu Lys Gln Ile Asp Asp Ser
            900                 905                 910
Arg Lys Phe Ser Leu Phe Pro Glu Arg Lys Tyr Gln Thr Leu Asn Cys
        915                 920                 925
Ser Val Asn Val Arg Cys Val Asn Ile Arg Cys Pro Leu Arg Gly Leu
    930                 935                 940
Asp Thr Lys Ala Ser Leu Val Leu Cys Ser Arg Leu Trp Asn Ser Thr
945                 950                 955                 960
Phe Leu Glu Glu Tyr Ser Lys Leu Asn Tyr Leu Asp Ile Leu Val Arg
                965                 970                 975
Ala Ser Ile Asp Val Thr Ala Ala Ala Gln Asn Ile Lys Leu Pro His
            980                 985                 990
Ala Gly Thr Gln Val Arg Val Thr Val Phe Pro Ser Lys Thr Val Ala
        995                 1000                1005
Gln Tyr Ser Gly Val Ala Trp Trp Ile Ile Leu Leu Ala Val Leu Ala
    1010                1015                1020
Gly Ile Leu Met Leu Ala Leu Leu Val Phe Leu Leu Trp Lys Cys Gly
1025                1030                1035                1040
Phe Phe Lys Arg Asn Lys Lys Asp His Tyr Asp Ala Thr Tyr His Lys
                1045                1050                1055
Ala Glu Ile His Thr Gln Pro Ser Asp Lys Glu Arg Leu Thr Ser Asp
            1060                1065                1070
Ala

<210> 5
<211> 5622
<212> DNA
<213> Artificial Sequence

<220>
<223> Description of Artificial Sequence; Note =
      synthetic construct

<400> 5
   atggcagggc cacgccccag cccatgggcc aggctgctcc tggcagcctt gatcagcgtc      60
   agcctctctg ggaccttgaa ccgctgcaag aaggccccag tgaagagctg cacggagtgt     120
   gtccgtgtgg ataaggactg cgcctactgc acagacgaga tgttcaggga ccggcgctgc     180
   aacacccagg cggagctgct ggccgcgggc tgccagcggg agagcatcgt ggtcatggag     240
   agcagcttcc aaatcacaga ggagacccag attgacacca ccctgcggcg cagccagatg     300
   tccccccaag gcctgcgggt ccgtctgcgg cccggtgagg agcggcattt tgagctggag     360
   gtgtttgagc cactggagag ccccgtggac ctgtacatcc tcatggactt ctccaactcc     420
   atgtccgatg atctggacaa cctcaagaag atggggcaga acctggctcg ggtcctgagc     480
   cagctcacca gcgactacac tattggattt ggcaagtttg tggacaaagt cagcgtcccg     540
   cagacggaca tgaggcctga gaagctgaag gagccttggc ccaacagtga cccccccttc     600
   tccttcaaga acgtcatcag cctgacagaa gatgtggatg agttccggaa taaactgcag     660
   ggagagcgga tctcaggcaa cctggatgct cctgagggcg gcttcgatgc catcctgcag     720
   acagctgtgt gcacgaggga cattggctgg cgcccggaca gcacccacct gctggtcttc     780
   tccaccgagt cagccttcca ctatgaggct gatggcgcca acgtgctggc tggcatcatg     840
   agccgcaacg atgaacggtg ccacctggac accacgggca cctacaccca gtacaggaca     900
   caggactacc cgtcggtgcc caccctggtg cgcctgctcg ccaagcacaa catcatcccc     960
   atctttgctg tcaccaacta ctcctatagc tactacgaga agcttcacac ctatttccct    1020
   gtctcctcac tgggggtgct gcaggaggac tcgtccaaca tcgtggagct gctggaggag    1080
   gccttcaatc ggatccgctc caacctggac atccgggccc tagacagccc ccgaggcctt    1140
   cggacagagg tcacctccaa gatgttccag aagacgagga ctgggtcctt tcacatccgg    1200
   cggggggaag tgggtatata ccaggtgcag ctgcgggccc ttgagcacgt ggatgggacg    1260
   cacgtgtgcc agctgccgga ggaccagaag ggcaacatcc atctgaaacc ttccttctcc 1320
   gacggcctca agatggacgc gggcatcatc tgtgatgtgt gcacctgcga gctgcaaaaa 1380
   gaggtgcggt cagctcgctg cagcttcaac ggagacttcg tgtgcggaca gtgtgtgtgc 1440
   agcgagggct ggagtggcca gacctgcaac tgctccaccg gctctctgag tgacattcag 1500
   ccctgcctgc gggagggcga ggacaagccg tgctccggcc gtggggagtg ccagtgcggg 1560
   cactgtgtgt gctacggcga aggccgctac gagggtcagt tctgcgagta tgacaacttc 1620
   cagtgtcccc gcacttccgg gttcctctgc aatgaccgag gacgctgctc catgggccag 1680
   tgtgtgtgtg agcctggttg gacaggccca agctgtgact gtcccctcag caatgccacc 1740
   tgcatcgaca gcaatggggg catctgtaat ggacgtggcc actgtgagtg tggccgctgc 1800
   cactgccacc agcagtcgct ctacacggac accatctgcg agatcaacta ctcggcgtcc 1860
   acccgggcct ctgcgaggac ctacgctcct gcgtgcagtg ccaggcgtgg ggcaccggcg 1920
   agaagaaggg gcgcacgtgt gaggaatgca acttcaaggt caagatggtg gacgagctta 1980
   agagaggcga ggaggtggtg gtgcgctgct ccttccggga cgaggatgac gactgcacct 2040
   acagctacac catggaaggt gacggcgccc ctgggcccaa cagcactgtc ctggtgcaca 2100
   agaagaaggg actgccctcc gggctccttc tggtggctca tccccctgct cctcctcctc 2160
   ctgccgctcc tggccctgct actgctgcta tgctggaagt actgtgcctg ctgcaaggcc 2220
   tgcctggcac ttctcccgtg ctgcaaccga ggtcacatgg tgggctttaa ggaagaccac 2280
   tacatgctgc gggagaacct gatggcctct gaccacttgg acacgcccat gctgcgcagc 2340
   gggaacctca agggccgtga cgtggtccgc tggaaggtca ccaacaacat gcagcggcct 2400
   ggctttgcca ctcatgccgc cagcatcaac cccacagagc tggtgcccta cgggctgtcc 2460
   ttgcgcctgg cccgcctttg caccgagaac ctgctgaagc ctgacactcg ggagtgcgcc 2520
   cagctgcgcc aggaggtgga ggagaacctg aacgaggtct acaggcagat ctccggtgta 2580
   cacaagctcc agcagaccaa gttccggcag cagcccaatg ccgggaaaaa gcaagaccac 2640
   accattgtgg acacagtgct gatggcgccc cgctcggcca agccggccct gctgaagctt 2700
   acagagaagc aggtggaaca gagggccttc cacgacctca aggtggcccc cggctactac 2760
   accctcactg cagaccagga cgcccggggc atggtggagt tccaggaggg cgtggagctg 2820
   gtggacgtac gggtgcccct ctttatccgg cctgaggatg acgacgagaa gcagctgctg 2880
   gtggaggcca tcgacgtgcc cgcaggcact gccaccctcg gccgccgcct ggtaaacatc 2940
   accatcatca aggagcaagc cagagacgtg gtgtcctttg agcagcctga gttctcggtc 3000
   agccgcgggg accaggtggc ccgcatccct gtcatccggc gtgtcctgga cggcgggaag 3060
   tcccaggtct cctaccgcac acaggatggc accgcgcagg gcaaccggga ctacatcccc 3120
   gtggagggtg agctgctgtt ccagcctggg gaggcctgga aagagctgca ggtgaagctc 3180
   ctggagctgc aagaagttga ctccctcctg cggggccgcc aggtccgccg tttccacgtc 3240
   cagctcagca accctaagtt tggggcccac ctgggccagc cccactccac caccatcatc 3300
   atcagggacc cagatgaact ggaccggagc ttcacgagtc agatgttgtc atcacagcca 3360
   ccccctcacg gcgacctggg cgccccgcag aaccccaatg ctaaggccgc tgggtccagg 3420
   aagatccatt tcaactggct gcccccttct ggcaagccaa tggggtacag ggtaaagtac 3480
   tggattcagg gtgactccga atccgaagcc cacctgctcg acagcaaggt gccctcagtg 3540
   gagctcacca acctgtaccc gtattgcgac tatgagatga aggtgtgcgc ctacggggct 3600
   cagggcgagg gaccctacag ctccctggtg tcctgccgca cccaccagga agtgcccagc 3660
   gagccagggc gtctggcctt caatgtcgtc tcctccacgg tgacccagct gagctgggct 3720
   gagccggctg agaccaacgg tgagatcaca gcctacgagg tctgctatgg cctggtcaac 3780
   gatgacaacc gacctattgg gcccatgaag aaagtgctgg ttgacaaccc taagaaccgg 3840
   atgctgctta ttgagaacct tcgggagtcc cagccctacc gctacacggt gaaggcgcgc 3900
   aacggggccg gctgggggcc tgagcgggag gccatcatca acctggccac ccagcccaag 3960
   aggcccatgt ccatccccat catccctgac atccctatcg tggacgccca gagcggggag 4020
   gactacgaca gcttccttat gtacagcgat gacgttctac gctctccatc gggcagccag 4080
   aggcccagcg tctccgatga cactggctgc ggctggaagt tcgagcccct gctgggggag 4140
   gagctggacc tgcggcgcgt cacgtggcgg ctgcccccgg agctcatccc gcgcctgtcg 4200
   gccagcagcg ggcgctcctc cgacgccgag gcgccccacg ggcccccgga cgacggcggc 4260
   gcgggcggga agggcggcag cctgccccgc agtgcgacac ccgggccccc cggagagcac 4320
   ctggtgaatg gccggatgga ctttgccttc ccgggcagca ccaactccct gcacaggatg 4380
   accacgacca gtgctgctgc ctatggcacc cacctgagcc cacacgtgcc ccaccgcgtg 4440
   ctaagcacat cctccaccct cacacgggac tacaactcac tgacccgctc agaacactca 4500
   cactcgacca cactgccgag ggactactcc accctcacct ccgtctcctc ccacggcctc 4560
   cctcccatct gggaacacgg gaggagcagg cttccgctgt cctgggccct ggggtcccgg 4620
   agtcgggctc agatgaaagg gttcccccct tccaggggcc cacgagactc tataatcctg 4680
   gctgggaggc cagcagcgcc ctcctggggc ccagactctc gcctgactgc tggtgtgccc 4740
   gacacgccca cccgcctggt gttctctgcc ctggggccca catctctcag agtgagctgg 4800
   caggagccgc ggtgcgagcg gccgctgcag ggctacagtg tggagtacca gctgctgaac 4860
   ggcggtgagc tgcatcggct caacatcccc aaccctgccc agacctcggt ggtggtggaa 4920
   gacctcctgc ccaaccactc ctacgtgttc cgcgtgcggg cccagagcca ggaaggctgg 4980
   ggccgagagc gtgagggtgt catcaccatt gaatcccagg tgcacccgca gagcccactg 5040
   tgtcccctgc caggctccgc cttcactttg agcactccca gtgccccagg cccgctggtg 5100
   ttcactgccc tgagcccaga ctcgctgcag ctgagctggg agcggccacg gaggcccaat 5160
   ggggatatcg tcggctacct ggtgacctgt gagatggccc aaggaggagg tccagccacc 5220
   gcattccggg tggatggaga cagccccgag agccggctga ccgtgccggg cctcagcgag 5280
   aacgtgccct acaagttcaa ggtgcaggcc aggaccactg agggcttcgg gccagagcgc 5340
   gagggcatca tcaccataga gtcccaggat ggaggtccct tcccgcagct gggcagccgt 5400
   gccgggctct tccagcaccc gctgcaaagc gagtacagca gcatctccac cacccacacc 5460
   agcgccaccg agcccttcct agtgggtccg accctggggg cccagcacct ggaggcaggc 5520
   ggctccctca cccggcatgt gacccaggag tttgtgagcc ggacactgac caccagcgga 5580
   acccttagca cccacatgga ccaacagttc ttccaaactt ga 5622

<210> 6
<211> 1873
<212> PRT
<213> Artificial Sequence

<220>
<223> Description of Artificial Sequence; Note =
      synthetic construct

<400> 6

<210> 7
<211> 5907
<212> DNA
<213> Artificial Sequence

<220>
<223> Description of Artificial Sequence; Note =
      synthetic construct

<400> 7
ggaccgtcga ggcagcggga ctgacccagc tgggctcact gtattaagaa gcggacccgc      60
gacccggagc gcccggggac ccgatctggg agcctggacg ggtgcagcgc gcaggaatgc     120
agtccgcctg actcaccagc gcctccttcc tacctgcgcc gcccgtccat aaagcgctgc     180
tcgtcccgcc cgccgccgcc gccctgctgt cccgccgggc tcgcccgcgc gctcagctcg     240
acccaacgca gcccaagtcc gaggtagtct cactaaggag gaggaggatg gcagggccct     300
gttgcagccc atgggtgaag ctgctgctgc tggcacgaat gctgagtgcc agcctccctg     360
gagacctggc caaccgctgc aagaaggctc aggtgaagag ctgtaccgag tgcatccggg     420

tggacaagag ctgtgcctac tgcacagacg agctgttcaa ggagaggcgc tgcaacaccc 480
aggcggacgt tctggctgca ggctgcaggg gagagagcat cctggtcatg gagagcagcc 540
ttgaaatcac agagaacacc cagatcgtca ccagcctgca ccgcagccag gtatctcccc 600
aaggcctgca agtccggctg cggcggggtg aggagcgcac gtttgtgttc caggtctttg 660
agcccctgga gagccccgtg gatctgtata tcctcatgga cttctccaac tccatgtctg 720
acgatctgga caacctcaag cagatggggc agaacctggc caagatcctg cgccagctca 780
ccagcgacta caccattgga tttggaaagt ttgtggacaa agtcagcgtc ccacagacag 840
acatgaggcc cgagaaactg aaggagccct ggcccaacag tgatcccccg ttctccttca 900
agaacgttat cagcttaacg gagaatgtgg aagaattctg gaacaaactg caaggagaac 960
gcatctcagg caacctggac gctcctgaag gggctttga tgccatcctg cagacagctg 1020
tgtgcacaag ggacattggc tggagggctg acagcaccca cctgctggtg ttctccaccg 1080
agtctgcctt ccactacgag gctgatggtg ccaacgttct ggccggcatc atgaaccgca 1140
atgatgagaa atgccacctg gacgcctcgg gcgcctacac ccaatacaag acacaggact 1200
acccatcagt gcccacgctg gttcgcctgc ttgccaagca taacatcatc cccatctttg 1260
ctgtcaccaa ctactcttac agctactatg agaagctcca taagtatttc cccgtctcct 1320
ctctgggcgt cctgcaggag gattcatcca acatcgtgga gctgctggag gaggccttct 1380
atcgaattcg ctccaacctg gacatccggg ctctggacag ccccagaggc ctgagaacag 1440
aggtcacctc cgatactctc cagaagacgg agactgggtc ctttcacatc aagcgggggg 1500
aagtgggcac atacaatgtg catctccggg cagtggagga catagatggg acacatgtgt 1560
gccagctggc taaagaagac caagggggca acatccacct gaaaccctcc ttctctgatg 1620
gcctccggat ggacgcgagt gtgatctgtg acgtgtgccc ctgtgagctg caaaaggaag 1680
ttcgatcagc tcgctgtcac ttcagaggag acttcatgtg tggacactgt gtgtgcaatg 1740
agggctggag tggcaaaacc tgcaactgct ccaccggctc tctgagtgac acacagccct 1800
gcctgcgtga gggtgaggac aaaccgtgct cgggccacgg cgagtgccag tgcggacgct 1860
gtgtgtgcta tggtgaaggc cgctacgagg gtcacttctg cgagtatgac aacttccagt 1920
gtccccggac ctctggattc ctgtgcaatg accggggacg ctgttctatg ggagagtgtg 1980
tgtgtgagcc tggttggaca ggccgcagct gcgactgtcc cctcagcaat gccacctgca 2040
tcgatagcaa cgggggcatc tgcaacggcc gaggctactg tgagtgtggc cgttgtcact 2100
gcaaccagca gtcgctctac acggacacca cctgtgagat caactactct gcgatactgg 2160
gtctctgtga ggatctccgc tcctgcgtac agtgccaggc ctggggcacc ggggagaaga 2220
aagggcgcgc gtgtgacgat tgccccttta aagtcaagat ggtagacgag cttaagaaag 2280
aagaggtggt ggagtactgc tccttccggg atgaggatga cgactgcact tacagctaca 2340
acgtggaggg cgacggcagc cctgggccca acagcacagt cctggtccac aaaaagaaag 2400
actgcctccc ggctccttcc tggtggctca tccccctgct catcttcctc ctgttgctcc 2460
tggcgttgct tctgctgctc tgctggaaat actgtgcctg ctgcaaagcc tgcctggggc 2520
ttcttccttg ctgcaaccga ggtcacatgg tgggctttaa ggaagatcac tatatgcttc 2580
gggagaacct gatggcctct gaccacctgg acacgcccat gctacgaagc gggaacctca 2640
agggacgaga cacagtccgc tggaagatca ccaacaatgt gcagcgccct ggctttgcca 2700
cccatgccgc cagcaccagc cccacggagc tcgtacccta cgggctgtcc ctgcgccttg 2760
gccgcctctg cactgagaac cttatgaagc cgggcacccg agagtgtgac cagctacgcc 2820
aggaggtgga ggaaaatctg aatgaggtgt atagacaggt cagcggcgca cacaagctcc 2880
agcagacgaa gttccgacag cagcccaacg ccgggaaaaa gcaagaccac accattgtgg 2940
acacagtgtt gctggcgccc cgctccgcca agcagatgct gctgaagctg acagagaagc 3000
aggtggagca ggggtccttc catgaactga aggtggcccc tggctactac actgtcacgg 3060
cagagcagga tgcccggggc atggtggagt tccaggaggg cgtggagctg gtggatgtgc 3120
gagtgcccct cttcatccgg cctgaggatg atgatgagaa gcagctgctg gtggaggcca 3180
ttgatgtccc tgtgagcact gccacccttg gtcgccgtct ggtaaacatc accattatca 3240
aggaacaagc tagtggggta gtgtccttcg agcagcctga atactcggtg agtcgtggag 3300
accaggtggc ccgcatccct gtcatccggc acatcctgga caatggcaag tcccaggtct 3360
cctatagcac acaggataat acagcacacg gacaccggga ttatgttccc gtggagggag 3420
agctgctgtt ccatcctggg gaaacctgga aggagttgca ggtgaagcta ctggagctgc 3480
aggaggttga ctccctcctg cgtggccgcc aggtccgccg cttccaagtc caactcagca 3540
accccaagtt cggagcccgc ctgggccagc ccagcacaac caccgttatt ctcgatgaaa 3600
cggacaggag tctcataaat caaacacttt catcgcctcc gccaccccat ggagacctgg 3660
gcgccccaca gaaccccaat gccaaggctg ccggatccag gaagatccat ttcaactggc 3720
tgcccccctcc tggcaagcca atggggtaca gggtgaagta ctggatccag ggcgactctg 3780
aatctgaagc ccaccttcta gatagcaagg tgccctcagt ggaactcacc aacctgtatc 3840
cctattgcga ctacgaaatg aaggtgtgtg cctatggggc caagggtgag gggccctata 3900
gctcactggt gtcctgccgc acccaccagg aagtacccag tgagccaggg aggctggctt 3960
tcaatgtagt ctcttctacg gtgactcagc tgagctgggc agagccagct gagaccaatg 4020
gcgagatcac agcctacgag gtctgctatg gactggtcaa tgaggacaac agacccattg 4080
gacctatgaa gaaggtgctc gtggacaacc ccaagaaccg gatgctgctc attgagaatc 4140
tgcgagattc ccagccatac cgatacacgg ttaaggcgcg caatggggca ggatggggac 4200
ccgagagaga ggctatcatc aacctggcta cacagcccaa gcggcccatg tccatcccta 4260
tcatcccaga catccccata gtggacgccc agggtggaga agactacgaa aacttcctta 4320
tgtacagtga tgacgtcctg cggtccccag ccagcagcca gaggcccagc gtttctgatg 4380
acactgagca cctggtgaat ggccggatgg actttgccta tccaggcagc gccaactccc 4440
tgcacagaat gactgcagcc aatgtggcct atggcacgca tctgagccca cacctgtccc 4500

accgagtgct gagcacgtcc tccaccctta cccgggacta ccactctctg acacgcacag 4560
agcactccca ctcaggcaca cttcccaggg actactccac cctcacttcc ctttcctccc 4620
aagcctccct cctatctggg aagatgggag gagcaggctt ccgctgtcct ggactcttgg 4680
gtccttgagc cgggctcaca tgaagggtgt gcccgcatcc aggggttcac cagactctat 4740
aatcctggcc gggcagtcag cagcaccctc ctggggtaca ggattcccgt ggggctgtgg 4800
gtgtgcctga cacacccact cggctggtgt tctctgccct ggggcgcacg tctttgaagg 4860
tgagctggca ggagccacag tgtgatcgga cgctgctggg ctacagtgtg gaataccagc 4920
tactaacgtg cgtcgagatg catcggctca acatccctaa ccctggccaa acctcggtgg 4980
tggtagagga tctcctgcct aactactctc atgtgttccg ggtacgggca cagagccagg 5040
agggctgggg tcgagagcga gagggtgtca tcaccatcga gtcccaggtg cacccgcaga 5100
gccctctctg ccccctgcca ggctcagcct tcactctgag cacccccagc gccccaggac 5160
cactggtgtt cactgcccta agcccagact ccccgcagct cagctgggag cggccgagga 5220
gccgcaatgg agatatcctt ggctacctgg tgacctgtga gatggcccaa ggaggagcac 5280
cagccaggac cttccgggtg gacggagaca accctgagag ccggttgact gtacctggcc 5340
tcagtgagaa cgttccttac aagttcaagg ttcaggccag gacgaccgag ggctttgggc 5400
cagagcgtga gggtatcatc accatcgagt ctcaggttgg aggccccttc ccacagctgg 5460
gcagcaattc tgggctcttc cagaacccag tgcaaagcga gttcagcagc gtgaccagca 5520
cgcacagcac cacgactgag cccttcctca tggatggtct aaccctgggg acccagcgcc 5580
tggaagcagg aggctccctc acccggcatg tgacccagga attcgtgacc cggaccttaa 5640
cggccagtgg ctctctcagc actcatatgg accaacagtt cttccaaacc tgaacctccc 5700
ccgcgcccca gccacctggg cccctccttg cctcctctcc tagcgccttc ttcctctgct 5760
gctctaccca cgagcttgct gaccacagag ccagcccctg tagtcagaga gcaggggtag 5820
gtgctgtcca ggaaccataa agtgggtaga ggtgatacaa ggtctttctg actgcatccc 5880
accctgggtc caatcccaca tgtaacc 5907

<210> 8
<211> 1466
<212> PRT
<213> Artificial Sequence

<220>
<223> Description of Artificial Sequence; Note =
      synthetic construct

<400> 8
Met Ala Gly Pro Cys Cys Ser Pro Trp Val Lys Leu Leu Leu Leu Ala
1               5                   10                  15
Arg Met Leu Ser Ala Ser Leu Pro Gly Asp Leu Ala Asn Arg Cys Lys
            20                  25                  30
Lys Ala Gln Val Lys Ser Cys Thr Glu Cys Ile Arg Val Asp Lys Ser
        35                  40                  45
Cys Ala Tyr Cys Thr Asp Glu Leu Phe Lys Glu Arg Arg Cys Asn Thr
    50                  55                  60
Gln Ala Asp Val Leu Ala Ala Gly Cys Arg Gly Glu Ser Ile Leu Val
65                  70                  75                  80
Met Glu Ser Ser Leu Glu Ile Thr Glu Asn Thr Gln Ile Val Thr Ser
                85                  90                  95
Leu His Arg Ser Gln Val Ser Pro Gln Gly Leu Gln Val Arg Leu Arg
            100                 105                 110
Arg Gly Glu Glu Arg Thr Phe Val Phe Gln Val Phe Glu Pro Leu Glu
        115                 120                 125
Ser Pro Val Asp Leu Tyr Ile Leu Met Asp Phe Ser Asn Ser Met Ser
    130                 135                 140
Asp Asp Leu Asp Asn Leu Lys Gln Met Gly Gln Asn Leu Ala Lys Ile
145                 150                 155                 160
Leu Arg Gln Leu Thr Ser Asp Tyr Thr Ile Gly Phe Gly Lys Phe Val
                165                 170                 175
Asp Lys Val Ser Val Pro Gln Thr Asp Met Arg Pro Glu Lys Leu Lys
            180                 185                 190
Glu Pro Trp Pro Asn Ser Asp Pro Pro Phe Ser Phe Lys Asn Val Ile
        195                 200                 205
Ser Leu Thr Glu Asn Val Glu Glu Phe Trp Asn Lys Leu Gln Gly Glu
    210                 215                 220
Arg Ile Ser Gly Asn Leu Asp Ala Pro Glu Gly Gly Phe Asp Ala Ile
225                 230                 235                 240
Leu Gln Thr Ala Val Cys Thr Arg Asp Ile Gly Trp Arg Ala Asp Ser

                    245                     250                     255
Thr His Leu Leu Val Phe Ser Thr Glu Ser Ala Phe His Tyr Glu Ala
                260                     265                     270
Asp Gly Ala Asn Val Leu Ala Gly Ile Met Asn Arg Asn Asp Glu Lys
            275                     280                     285
Cys His Leu Asp Ala Ser Gly Ala Tyr Thr Gln Tyr Lys Thr Gln Asp
        290                     295                     300
Tyr Pro Ser Val Pro Thr Leu Val Arg Leu Leu Ala Lys His Asn Ile
305                     310                     315                     320
Ile Pro Ile Phe Ala Val Thr Asn Tyr Ser Tyr Ser Tyr Tyr Glu Lys
                325                     330                     335
Leu His Lys Tyr Phe Pro Val Ser Ser Leu Gly Val Leu Gln Glu Asp
            340                     345                     350
Ser Ser Asn Ile Val Glu Leu Leu Glu Glu Ala Phe Tyr Arg Ile Arg
        355                     360                     365
Ser Asn Leu Asp Ile Arg Ala Leu Asp Ser Pro Arg Gly Leu Arg Thr
    370                     375                     380
Glu Val Thr Ser Asp Thr Leu Gln Lys Thr Glu Thr Gly Ser Phe His
385                     390                     395                     400
Ile Lys Arg Gly Glu Val Gly Thr Tyr Asn Val His Leu Arg Ala Val
                405                     410                     415
Glu Asp Ile Asp Gly Thr His Val Cys Gln Leu Ala Lys Glu Asp Gln
            420                     425                     430
Gly Gly Asn Ile His Leu Lys Pro Ser Phe Ser Asp Gly Leu Arg Met
        435                     440                     445
Asp Ala Ser Val Ile Cys Asp Val Cys Pro Cys Glu Leu Gln Lys Glu
    450                     455                     460
Val Arg Ser Ala Arg Cys His Phe Arg Gly Asp Phe Met Cys Gly His
465                     470                     475                     480
Cys Val Cys Asn Glu Gly Trp Ser Gly Lys Thr Cys Asn Cys Ser Thr
                485                     490                     495
Gly Ser Leu Ser Asp Thr Gln Pro Cys Leu Arg Glu Gly Glu Asp Lys
            500                     505                     510
Pro Cys Ser Gly His Gly Glu Cys Gln Cys Gly Arg Cys Val Cys Tyr
        515                     520                     525
Gly Glu Gly Arg Tyr Glu Gly His Phe Cys Glu Tyr Asp Asn Phe Gln
    530                     535                     540
Cys Pro Arg Thr Ser Gly Phe Leu Cys Asn Asp Arg Gly Arg Cys Ser
545                     550                     555                     560
Met Gly Glu Cys Val Cys Glu Pro Gly Trp Thr Gly Arg Ser Cys Asp
                565                     570                     575
Cys Pro Leu Ser Asn Ala Thr Cys Ile Asp Ser Asn Gly Gly Ile Cys
            580                     585                     590
Asn Gly Arg Gly Tyr Cys Glu Cys Gly Arg Cys His Cys Asn Gln Gln
        595                     600                     605
Ser Leu Tyr Thr Asp Thr Thr Cys Glu Ile Asn Tyr Ser Ala Ile Leu
    610                     615                     620
Gly Leu Cys Glu Asp Leu Arg Ser Cys Val Gln Cys Gln Ala Trp Gly
625                     630                     635                     640
Thr Gly Glu Lys Lys Gly Arg Ala Cys Asp Asp Cys Pro Phe Lys Val
                645                     650                     655
Lys Met Val Asp Glu Leu Lys Lys Glu Glu Val Val Glu Tyr Cys Ser
            660                     665                     670
Phe Arg Asp Glu Asp Asp Asp Cys Thr Tyr Ser Tyr Asn Val Glu Gly
        675                     680                     685
Asp Gly Ser Pro Gly Pro Asn Ser Thr Val Leu Val His Lys Lys Lys
    690                     695                     700
Asp Cys Leu Pro Ala Pro Ser Trp Trp Leu Ile Pro Leu Leu Ile Phe
705                     710                     715                     720
Leu Leu Leu Leu Leu Ala Leu Leu Leu Leu Leu Cys Trp Lys Tyr Cys
                725                     730                     735
Ala Cys Cys Lys Ala Cys Leu Gly Leu Leu Pro Cys Cys Asn Arg Gly
            740                     745                     750
His Met Val Gly Phe Lys Glu Asp His Tyr Met Leu Arg Glu Asn Leu
        755                     760                     765
Met Ala Ser Asp His Leu Asp Thr Pro Met Leu Arg Ser Gly Asn Leu
    770                     775                     780
Lys Gly Arg Asp Thr Val Arg Trp Lys Ile Thr Asn Asn Val Gln Arg

785                 790                 795                 800
Pro Gly Phe Ala Thr His Ala Ala Ser Thr Ser Pro Thr Glu Leu Val
                805                 810                 815
Pro Tyr Gly Leu Ser Leu Arg Leu Gly Arg Leu Cys Thr Glu Asn Leu
            820                 825                 830
Met Lys Pro Gly Thr Arg Glu Cys Asp Gln Leu Arg Gln Glu Val Glu
        835                 840                 845
Glu Asn Leu Asn Glu Val Tyr Arg Gln Val Ser Gly Ala His Lys Leu
    850                 855                 860
Gln Gln Thr Lys Phe Arg Gln Gln Pro Asn Ala Gly Lys Lys Gln Asp
865                 870                 875                 880
His Thr Ile Val Asp Thr Val Leu Leu Ala Pro Arg Ser Ala Lys Gln
                885                 890                 895
Met Leu Leu Lys Leu Thr Glu Lys Gln Val Glu Gln Gly Ser Phe His
            900                 905                 910
Glu Leu Lys Val Ala Pro Gly Tyr Tyr Thr Val Thr Ala Glu Gln Asp
        915                 920                 925
Ala Arg Gly Met Val Glu Phe Gln Glu Gly Val Glu Leu Val Asp Val
    930                 935                 940
Arg Val Pro Leu Phe Ile Arg Pro Glu Asp Asp Asp Glu Lys Gln Leu
945                 950                 955                 960
Leu Val Glu Ala Ile Asp Val Pro Val Ser Thr Ala Thr Leu Gly Arg
                965                 970                 975
Arg Leu Val Asn Ile Thr Ile Ile Lys Glu Gln Ala Ser Gly Val Val
            980                 985                 990
Ser Phe Glu Gln Pro Glu Tyr Ser Val Ser Arg Gly Asp Gln Val Ala
        995                 1000                1005
Arg Ile Pro Val Ile Arg His Ile Leu Asp Asn Gly Lys Ser Gln Val
    1010                1015                1020
Ser Tyr Ser Thr Gln Asp Asn Thr Ala His Gly His Arg Asp Tyr Val
1025                1030                1035                1040
Pro Val Glu Gly Glu Leu Leu Phe His Pro Gly Glu Thr Trp Lys Glu
                1045                1050                1055
Leu Gln Val Lys Leu Leu Glu Leu Gln Glu Val Asp Ser Leu Leu Arg
            1060                1065                1070
Gly Arg Gln Val Arg Arg Phe Gln Val Gln Leu Ser Asn Pro Lys Phe
        1075                1080                1085
Gly Ala Arg Leu Gly Gln Pro Ser Thr Thr Thr Val Ile Leu Asp Glu
    1090                1095                1100
Thr Asp Arg Ser Leu Ile Asn Gln Thr Leu Ser Ser Pro Pro Pro Pro
1105                1110                1115                1120
His Gly Asp Leu Gly Ala Pro Gln Asn Pro Asn Ala Lys Ala Ala Gly
                1125                1130                1135
Ser Arg Lys Ile His Phe Asn Trp Leu Pro Pro Pro Gly Lys Pro Met
            1140                1145                1150
Gly Tyr Arg Val Lys Tyr Trp Ile Gln Gly Asp Ser Glu Ser Glu Ala
        1155                1160                1165
His Leu Leu Asp Ser Lys Val Pro Ser Val Glu Leu Thr Asn Leu Tyr
    1170                1175                1180
Pro Tyr Cys Asp Tyr Glu Met Lys Val Cys Ala Tyr Gly Ala Lys Gly
1185                1190                1195                1200
Glu Gly Pro Tyr Ser Ser Leu Val Ser Cys Arg Thr His Gln Glu Val
                1205                1210                1215
Pro Ser Glu Pro Gly Arg Leu Ala Phe Asn Val Val Ser Ser Thr Val
            1220                1225                1230
Thr Gln Leu Ser Trp Ala Glu Pro Ala Glu Thr Asn Gly Glu Ile Thr
        1235                1240                1245
Ala Tyr Glu Val Cys Tyr Gly Leu Val Asn Glu Asp Asn Arg Pro Ile
    1250                1255                1260
Gly Pro Met Lys Lys Val Leu Val Asp Asn Pro Lys Asn Arg Met Leu
1265                1270                1275                1280
Leu Ile Glu Asn Leu Arg Asp Ser Gln Pro Tyr Arg Tyr Thr Val Lys
                1285                1290                1295
Ala Arg Asn Gly Ala Gly Trp Gly Pro Glu Arg Glu Ala Ile Ile Asn
            1300                1305                1310
Leu Ala Thr Gln Pro Lys Arg Pro Met Ser Ile Pro Ile Ile Pro Asp
        1315                1320                1325
Ile Pro Ile Val Asp Ala Gln Gly Gly Glu Asp Tyr Glu Asn Phe Leu

1330                1335                1340
Met Tyr Ser Asp Asp Val Leu Arg Ser Pro Ala Ser Ser Gln Arg Pro
1345                1350                1355                1360
Ser Val Ser Asp Asp Thr Glu His Leu Val Asn Gly Arg Met Asp Phe
                1365                1370                1375
Ala Tyr Pro Gly Ser Ala Asn Ser Leu His Arg Met Thr Ala Ala Asn
            1380                1385                1390
Val Ala Tyr Gly Thr His Leu Ser Pro His Leu Ser His Arg Val Leu
        1395                1400                1405
Ser Thr Ser Ser Thr Leu Thr Arg Asp Tyr His Ser Leu Thr Arg Thr
    1410                1415                1420
Glu His Ser His Ser Gly Thr Leu Pro Arg Asp Tyr Ser Thr Leu Thr
1425                1430                1435                1440
Ser Leu Ser Ser Gln Ala Ser Leu Leu Ser Gly Lys Met Gly Gly Ala
                1445                1450                1455
Gly Phe Arg Cys Pro Gly Leu Leu Gly Pro
            1460                1465

<210> 9
<211> 2205
<212> DNA
<213> Artificial Sequence

<220>
<223> Description of Artificial Sequence; Note =
      synthetic construct

<400> 9
atggcagggc cctgttgcag cccatgggtg aagctgctgc tgctggcacg aatgctgagt      60
gccagcctcc ctggagacct ggccaaccgc tgcaagaagg ctcaggtgaa gagctgtacc     120
gagtgcatcc gggtggacaa gagctgtgcc tactgcacag acgagctgtt caaggagagg     180
cgctgcaaca cccaggcgga cgttctggct gcaggctgca ggggagagag catcctggtc     240
atggagagca gccttgaaat cacagagaac acccagatcg tcaccagcct gcaccgcagc     300
caggtatctc cccaaggcct gcaagtccgg ctgcggcggg gtgaggagcg cacgtttgtg     360
ttccaggtct ttgagcccct ggagagcccc gtggatctgt atatcctcat ggacttctcc     420
aactccatgt ctgacgatct ggacaacctc aagcagatgg ggcagaacct ggccaagatc     480
ctgcgccagc tcaccagcga ctacaccatt ggatttggaa agtttgtgga caaagtcagc     540
gtcccacaga cagacatgag gcccgagaaa ctgaaggagc cctggcccaa cagtgatccc     600
ccgttctcct tcaagaacgt tatcagctta acggagaatg tggaagaatt ctggaacaaa     660
ctgcaaggag aacgcatctc aggcaacctg gacgctcctg aagggggctt tgatgccatc     720
ctgcagacag ctgtgtgcac aagggacatt ggctggaggg ctgacagcac ccacctgctg     780
gtgttctcca ccgagtctgc cttccactac gaggctgatg gtgccaacgt tctggccggc     840
atcatgaacc gcaatgatga gaaatgccac ctggacgcct cgggcgccta cacccaatac     900
aagacacagg actacccatc agtgcccacg ctggttcgcc tgcttgccaa gcataacatc     960
atccccatct ttgctgtcac caactactct tacagctact atgagaagct ccataagtat    1020
ttccccgtct cctctctggg cgtcctgcag gaggattcat ccaacatcgt ggagctgctg    1080
gaggaggcct tctatcgaat tcgctccaac ctggacatcc gggctctgga cagccccaga    1140
ggcctgagaa cagaggtcac ctccgatact ctccagaaga cggagactgg gtcctttcac    1200
atcaagcggg gggaagtggg cacatacaat gtgcatctcc gggcagtgga ggacatagat    1260
gggacacatg tgtgccagct ggctaaagaa gaccaagggg gcaacatcca cctgaaaccc    1320
tccttctctg atggcctccg gatggacgcg agtgtgatct gtgacgtgtg cccctgtgag    1380
ctgcaaaagg aagttcgatc agctcgctgt cacttcagag gagacttcat gtgtggacac    1440
tgtgtgtgca atgagggctg gagtggcaaa acctgcaact gctccaccgg ctctctgagt    1500
gacacacagc cctgcctgcg tgagggtgag gacaaaccgt gctcgggcca cggcgagtgc    1560
cagtgcggac gctgtgtgtg ctatggtgaa ggccgctacg agggtcactt ctgcgagtat    1620
gacaacttcc agtgtccccg gacctctgga ttcctgtgca atgaccgggg acgctgttct    1680
atgggagagt gtgtgtgtga gcctggttgg acaggccgca gctgcgactg tcccctcagc    1740
aatgccacct gcatcgatag caacgggggc atctgcaacg gccgaggcta ctgtgagtgt    1800
ggccgttgtc actgcaacca gcagtcgctc tacacggaca ccacctgtga gatcaactac    1860
tctgcgatac tgggtctctg tgaggatctc cgctcctgcg tacagtgcca ggcctggggc    1920
accggggaga agaaagggcg cgcgtgtgac gattgcccct ttaaagtcaa gatggtagac    1980
gagcttaaga aagaagaggt ggtggagtac tgctccttcc gggatgagga tgacgactgc    2040
acttacagct acaacgtgga gggcgacggc agccctgggc ccaacagcac agtcctggtc    2100
cacaaaaaga aagactgcct cccggctcct tcctggtggc tcatccccct gctcatcttc    2160
ctcctgttgc tcctggcgtt gcttctgctg ctctgctgga aatga                    2205

<210> 10
<211> 734

<212> PRT
<213> Artificial Sequence

<220>
<223> Description of Artificial Sequence; Note =
      synthetic construct

<400> 10
Met Ala Gly Pro Cys Cys Ser Pro Trp Val Lys Leu Leu Leu Leu Ala
1               5                   10                  15
Arg Met Leu Ser Ala Ser Leu Pro Gly Asp Leu Ala Asn Arg Cys Lys
            20                  25                  30
Lys Ala Gln Val Lys Ser Cys Thr Glu Cys Ile Arg Val Asp Lys Ser
        35                  40                  45
Cys Ala Tyr Cys Thr Asp Glu Leu Phe Lys Glu Arg Arg Cys Asn Thr
    50                  55                  60
Gln Ala Asp Val Leu Ala Ala Gly Cys Arg Gly Glu Ser Ile Leu Val
65                  70                  75                  80
Met Glu Ser Ser Leu Glu Ile Thr Glu Asn Thr Gln Ile Val Thr Ser
                85                  90                  95
Leu His Arg Ser Gln Val Ser Pro Gln Gly Leu Gln Val Arg Leu Arg
            100                 105                 110
Arg Gly Glu Glu Arg Thr Phe Val Phe Gln Val Phe Glu Pro Leu Glu
        115                 120                 125
Ser Pro Val Asp Leu Tyr Ile Leu Met Asp Phe Ser Asn Ser Met Ser
    130                 135                 140
Asp Asp Leu Asp Asn Leu Lys Gln Met Gly Gln Asn Leu Ala Lys Ile
145                 150                 155                 160
Leu Arg Gln Leu Thr Ser Asp Tyr Thr Ile Gly Phe Gly Lys Phe Val
                165                 170                 175
Asp Lys Val Ser Val Pro Gln Thr Asp Met Arg Pro Glu Lys Leu Lys
            180                 185                 190
Glu Pro Trp Pro Asn Ser Asp Pro Pro Phe Ser Phe Lys Asn Val Ile
        195                 200                 205
Ser Leu Thr Glu Asn Val Glu Glu Phe Trp Asn Lys Leu Gln Gly Glu
    210                 215                 220
Arg Ile Ser Gly Asn Leu Asp Ala Pro Glu Gly Gly Phe Asp Ala Ile
225                 230                 235                 240
Leu Gln Thr Ala Val Cys Thr Arg Asp Ile Gly Trp Arg Ala Asp Ser
                245                 250                 255
Thr His Leu Leu Val Phe Ser Thr Glu Ser Ala Phe His Tyr Glu Ala
            260                 265                 270
Asp Gly Ala Asn Val Leu Ala Gly Ile Met Asn Arg Asn Asp Glu Lys
        275                 280                 285
Cys His Leu Asp Ala Ser Gly Ala Tyr Thr Gln Tyr Lys Thr Gln Asp
    290                 295                 300
Tyr Pro Ser Val Pro Thr Leu Val Arg Leu Leu Ala Lys His Asn Ile
305                 310                 315                 320
Ile Pro Ile Phe Ala Val Thr Asn Tyr Ser Tyr Ser Tyr Tyr Glu Lys
                325                 330                 335
Leu His Lys Tyr Phe Pro Val Ser Ser Leu Gly Val Leu Gln Glu Asp
            340                 345                 350
Ser Ser Asn Ile Val Glu Leu Leu Glu Glu Ala Phe Tyr Arg Ile Arg
        355                 360                 365
Ser Asn Leu Asp Ile Arg Ala Leu Asp Ser Pro Arg Gly Leu Arg Thr
    370                 375                 380
Glu Val Thr Ser Asp Thr Leu Gln Lys Thr Glu Thr Gly Ser Phe His
385                 390                 395                 400
Ile Lys Arg Gly Glu Val Gly Thr Tyr Asn Val His Leu Arg Ala Val
                405                 410                 415
Glu Asp Ile Asp Gly Thr His Val Cys Gln Leu Ala Lys Glu Asp Gln
            420                 425                 430
Gly Gly Asn Ile His Leu Lys Pro Ser Phe Ser Asp Gly Leu Arg Met
        435                 440                 445
Asp Ala Ser Val Ile Cys Asp Val Cys Pro Cys Glu Leu Gln Lys Glu
    450                 455                 460
Val Arg Ser Ala Arg Cys His Phe Arg Gly Asp Phe Met Cys Gly His
465                 470                 475                 480

Cys Val Cys Asn Glu Gly Trp Ser Gly Lys Thr Cys Asn Cys Ser Thr
                485                 490                 495
Gly Ser Leu Ser Asp Thr Gln Pro Cys Leu Arg Glu Gly Glu Asp Lys
            500                 505                 510
Pro Cys Ser Gly His Gly Glu Cys Gln Cys Gly Arg Cys Val Cys Tyr
        515                 520                 525
Gly Glu Gly Arg Tyr Glu Gly His Phe Cys Glu Tyr Asp Asn Phe Gln
    530                 535                 540
Cys Pro Arg Thr Ser Gly Phe Leu Cys Asn Asp Arg Gly Arg Cys Ser
545                 550                 555                 560
Met Gly Glu Cys Val Cys Glu Pro Gly Trp Thr Gly Arg Ser Cys Asp
                565                 570                 575
Cys Pro Leu Ser Asn Ala Thr Cys Ile Asp Ser Asn Gly Gly Ile Cys
            580                 585                 590
Asn Gly Arg Gly Tyr Cys Glu Cys Gly Arg Cys His Cys Asn Gln Gln
        595                 600                 605
Ser Leu Tyr Thr Asp Thr Thr Cys Glu Ile Asn Tyr Ser Ala Ile Leu
    610                 615                 620
Gly Leu Cys Glu Asp Leu Arg Ser Cys Val Gln Cys Gln Ala Trp Gly
625                 630                 635                 640
Thr Gly Glu Lys Lys Gly Arg Ala Cys Asp Asp Cys Pro Phe Lys Val
                645                 650                 655
Lys Met Val Asp Glu Leu Lys Lys Glu Glu Val Val Glu Tyr Cys Ser
            660                 665                 670
Phe Arg Asp Glu Asp Asp Asp Cys Thr Tyr Ser Tyr Asn Val Glu Gly
        675                 680                 685
Asp Gly Ser Pro Gly Pro Asn Ser Thr Val Leu Val His Lys Lys Lys
    690                 695                 700
Asp Cys Leu Pro Ala Pro Ser Trp Trp Leu Ile Pro Leu Leu Ile Phe
705                 710                 715                 720
Leu Leu Leu Leu Leu Ala Leu Leu Leu Leu Leu Cys Trp Lys
                725                 730

<210> 11
<211> 3579
<212> DNA
<213> Artificial Sequence

<220>
<223> Description of Artificial Sequence; Note =
      synthetic construct

<400> 11
atgcctgcgc tctggctcag ctgctgcctc ggtgtcgcgc tcctgctgcc cgccagccag      60
gccacctcca ggagggaagt ctgtgattgc aatgggaagt ccaggcaatg tgtctttgat     120
caggagctcc atcgacaagc aggcagcggg ttccgttgcc tcaactgcaa tgacaataca     180
gcgggggttc actgcgagcg gtcgagggag gggttttacc agcatcagag caagagccgc     240
tgcctaccct gcaactgcca ctcaaagggt tccctcagtg ctggatgtga caactctgga     300
caatgcaggt gtaagccagg tgtgacagga caaagatgtg accagtgtca gccaggcttc     360
catatgctca ccgatgctgg atgcacccga gaccaggggc aactagattc caagtgtgac     420
tgtgacccag ctggcatctc tggaccctgt gattctggcc gatgtgtctg caaaccagcc     480
gtcactggag agcgctgtga taggtgccga ccacgtgact atcatctgga ccgggcaaac     540
cctgagggct gtacccagtg tttctgctat gggcattcag ccagctgcca cgcctctgcc     600
gacttcagtg tccacaaaat cacttcaact ttcagtcagg atgtggatgg ttggaaggcg     660
gttcagagaa acggggcacc tgcaaaactc cactggtcac agcgccatcg ggacgtgttt     720
agttctgccc gaagatcaga ccccgtctat ttcgtggccc ctgccaaatt cctcggtaac     780
cagcaagtga gttacgggca gagcctgtct tttgactacc gcgtggacag aggaggtaga     840
cagccgtctg cctacgatgt gatcctggaa ggtgctggtc tacagatcag agctcctctg     900
atggctccag gcaagacact tccttgtggg atcacaaaga cttacacatt cagactgaat     960
gaacatccaa gcagtcactg gagtccccag ctgagttatt tcgaatatcg aaggttactg    1020
cggaacctca cagccctcct gatgatccga gctacgtacg gagaatatag tacagggtac    1080
attgataacg tgaccctggt ttcagcccgc cctgtccttg gagccccagc cccttgggtt    1140
gaacgttgtg tatgcctgct ggggtacaag ggacaattct gccaggaatg tgcttctggt    1200
tacaaaagag attcggcaag attgggcgct tttggcgcct gtgttccctg taactgccaa    1260
ggggaggggg cctgtgatcc agacacggga gattgctact cgggggacga gaatcctgac    1320
attgagtgtg ctgactgtcc catcggtttc tacaatgacc cacatgaccc ccgcagctgc    1380
aagccatgtc cctgtcacaa tgggttcagc tgttcagtga tgcctgagac agaggaggtg    1440
gtgtgtaaca actgtccccc tggggtcaca ggtgcccgct gtgagctctg tgctgatggc    1500

ttctttgggg atccctttgg ggaacatggc ccagtgaggc cttgtcaacg ctgccaatgc 1560
aacaacaacg tggaccccaa tgcctctggg aactgtgacc agttgacagg cagatgcttg 1620
aaatgtatct acaacacggc cggtgtctac tgtgaccagt gcaaagcagg ttactttgga 1680
gacccattgg ctcccaaccc agcagacaag tgtcgagctt gcaactgcag ccccatgggt 1740
gcggagcctg gagagtgtcg aggtgatggc agctgtgttt gcaagccagg ctttggcgcc 1800
ttcaactgtg atcacgcagc cctaaccagt tgtcctgctt gctacaatca agtgaagatt 1860
cagatggacc agtttaccca gcagctccag agcctggagg ccctggtttc aaaggctcag 1920
ggtggtggtg gtggtggtac agtcccagtg cagctggaag gcaggatcga gcaggctgag 1980
caggcccttc aggacattct gggagaagct cagatttcag aaggggcaat gagagccgtt 2040
gctgtccggc tggccaaggc aaggagccaa gagaacgact acaagacccg cctggatgac 2100
ctcaagatga ctgcagaaag gatccgggcc ctgggcagtc agcatcagaa cagagttcag 2160
gatacgagca gactcatctc tcagatgcgc ctgagtctgg caggaagcga agctctcttg 2220
gaaaacacta atatccattc ttctgagcac tacgtggggc cgaatgattt taaaagtctg 2280
gctcaggagg ctacaagaaa ggcagacagc cacgctgagt cagctaacgc aatgaagcaa 2340
ctagcaaggg aaactgagga ctactccaaa caagcacttt cattggcccg caagctcttg 2400
agtggaggag gcggaagtgg ctcttgggac agctccgtgg tacaaggtct tatgggaaaa 2460
ttagagaaaa ccaagtccct gagccagcag ctgtcattgg agggcaccca agccgacatt 2520
gaagctgata ggtcgtatca gcacagtctc cgcctcctgg attctgcctc tcagcttcag 2580
ggagtcagtg atctgtcctt tcaggtggaa gcaaagagga tcagacaaaa ggctgattct 2640
ctctcaaacc tggtgaccag acaaacggat gcattcacgc gtgtgcgaaa caatctgggg 2700
aactgggaaa aagaaacacg gcagctttta cagactggaa aggataggag acagacttca 2760
gatcagctgc tttcccgtgc caaccttgct aaaaacagag cccaagaagc gctaagtatg 2820
ggcaatgcca ctttttatga agttgagaac atcctgaaga acctccgaga gtttgatctg 2880
caggttgaag acagaaaagc agaggctgaa gaggccatga agagactctc ctctattagc 2940
cagaaggttg cggatgccag tgacaagacc cagcaagcag aaacggccct ggggagcgcc 3000
actgccgaca cccaacgggc aaagaacgca gctagggagg ccctggagat cagcagcgag 3060
atagagctgg agatagggag tctgaacttg gaagctaatg tgacagcaga tggggccttg 3120
gccatggaga aggggactgc cactctgaag agcgagatga gagagatgat tgagctggcc 3180
agaaaggagc tggagtttga cacggataag gacacggtgc agctggtgat tactgaagcc 3240
cagcaagctg atgccagagc cacgagtgcc ggagttacca tccaagacac rctcaacaca 3300
ttggacggca tcctacacct catagaccag cctggcagtg tggatgaaga agggatgatg 3360
ctattagaac aagggctttt ccaagccaag acccagatca acagtcgact tcggcccttg 3420
atgtctgacc tggaggagag ggtgcgtcgg cagaggaacc acctccatct gctggagact 3480
agcatagatg gaattcttgc tgatgtgaag aacctggaga acattcgaga caacctgccc 3540
ccaggctgct acaataccca agctcttgag caacagtga 3579

<210> 12
<211> 1192
<212> PRT
<213> Artificial Sequence

<220>
<223> Description of Artificial Sequence; Note =
synthetic construct

<400> 12
Met Pro Ala Leu Trp Leu Ser Cys Cys Leu Gly Val Ala Leu Leu Leu
1               5                   10                  15
Pro Ala Ser Gln Ala Thr Ser Arg Arg Glu Val Cys Asp Cys Asn Gly
            20                  25                  30
Lys Ser Arg Gln Cys Val Phe Asp Gln Glu Leu His Arg Gln Ala Gly
        35                  40                  45
Ser Gly Phe Arg Cys Leu Asn Cys Asn Asp Asn Thr Ala Gly Val His
    50                  55                  60
Cys Glu Arg Ser Arg Glu Gly Phe Tyr Gln His Gln Ser Lys Ser Arg
65                  70                  75                  80
Cys Leu Pro Cys Asn Cys His Ser Lys Gly Ser Leu Ser Ala Gly Cys
                85                  90                  95
Asp Asn Ser Gly Gln Cys Arg Cys Lys Pro Gly Val Thr Gly Gln Arg
            100                 105                 110
Cys Asp Gln Cys Gln Pro Gly Phe His Met Leu Thr Asp Ala Gly Cys
        115                 120                 125
Thr Arg Asp Gln Gly Gln Leu Asp Ser Lys Cys Asp Cys Asp Pro Ala
    130                 135                 140
Gly Ile Ser Gly Pro Cys Asp Ser Gly Arg Cys Val Cys Lys Pro Ala
145                 150                 155                 160
Val Thr Gly Glu Arg Cys Asp Arg Cys Arg Pro Arg Asp Tyr His Leu
                165                 170                 175

Asp Arg Ala Asn Pro Glu Gly Cys Thr Gln Cys Phe Cys Tyr Gly His
                180                 185                 190
Ser Ala Ser Cys His Ala Ser Ala Asp Phe Ser Val His Lys Ile Thr
            195                 200                 205
Ser Thr Phe Ser Gln Asp Val Asp Gly Trp Lys Ala Val Gln Arg Asn
    210                 215                 220
Gly Ala Pro Ala Lys Leu His Trp Ser Gln Arg His Arg Asp Val Phe
225                 230                 235                 240
Ser Ser Ala Arg Arg Ser Asp Pro Val Tyr Phe Val Ala Pro Ala Lys
                245                 250                 255
Phe Leu Gly Asn Gln Gln Val Ser Tyr Gly Gln Ser Leu Ser Phe Asp
            260                 265                 270
Tyr Arg Val Asp Arg Gly Gly Arg Gln Pro Ser Ala Tyr Asp Val Ile
        275                 280                 285
Leu Glu Gly Ala Gly Leu Gln Ile Arg Ala Pro Leu Met Ala Pro Gly
    290                 295                 300
Lys Thr Leu Pro Cys Gly Ile Thr Lys Thr Tyr Thr Phe Arg Leu Asn
305                 310                 315                 320
Glu His Pro Ser Ser His Trp Ser Pro Gln Leu Ser Tyr Phe Glu Tyr
                325                 330                 335
Arg Arg Leu Leu Arg Asn Leu Thr Ala Leu Leu Met Ile Arg Ala Thr
            340                 345                 350
Tyr Gly Glu Tyr Ser Thr Gly Tyr Ile Asp Asn Val Thr Leu Val Ser
        355                 360                 365
Ala Arg Pro Val Leu Gly Ala Pro Ala Pro Trp Val Glu Arg Cys Val
    370                 375                 380
Cys Leu Leu Gly Tyr Lys Gly Gln Phe Cys Gln Glu Cys Ala Ser Gly
385                 390                 395                 400
Tyr Lys Arg Asp Ser Ala Arg Leu Gly Ala Phe Gly Ala Cys Val Pro
                405                 410                 415
Cys Asn Cys Gln Gly Glu Gly Ala Cys Asp Pro Asp Thr Gly Asp Cys
            420                 425                 430
Tyr Ser Gly Asp Glu Asn Pro Asp Ile Glu Cys Ala Asp Cys Pro Ile
        435                 440                 445
Gly Phe Tyr Asn Asp Pro His Asp Pro Arg Ser Cys Lys Pro Cys Pro
    450                 455                 460
Cys His Asn Gly Phe Ser Cys Ser Val Met Pro Glu Thr Glu Glu Val
465                 470                 475                 480
Val Cys Asn Asn Cys Pro Pro Gly Val Thr Gly Ala Arg Cys Glu Leu
                485                 490                 495
Cys Ala Asp Gly Phe Phe Gly Asp Pro Phe Gly Glu His Gly Pro Val
            500                 505                 510
Arg Pro Cys Gln Arg Cys Gln Cys Asn Asn Asn Val Asp Pro Asn Ala
        515                 520                 525
Ser Gly Asn Cys Asp Gln Leu Thr Gly Arg Cys Leu Lys Cys Ile Tyr
    530                 535                 540
Asn Thr Ala Gly Val Tyr Cys Asp Gln Cys Lys Ala Gly Tyr Phe Gly
545                 550                 555                 560
Asp Pro Leu Ala Pro Asn Pro Ala Asp Lys Cys Arg Ala Cys Asn Cys
                565                 570                 575
Ser Pro Met Gly Ala Glu Pro Gly Glu Cys Arg Gly Asp Gly Ser Cys
            580                 585                 590
Val Cys Lys Pro Gly Phe Gly Ala Phe Asn Cys Asp His Ala Ala Leu
        595                 600                 605
Thr Ser Cys Pro Ala Cys Tyr Asn Gln Val Lys Ile Gln Met Asp Gln
    610                 615                 620
Phe Thr Gln Gln Leu Gln Ser Leu Glu Ala Leu Val Ser Lys Ala Gln
625                 630                 635                 640
Gly Gly Gly Gly Gly Gly Thr Val Pro Val Gln Leu Glu Gly Arg Ile
                645                 650                 655
Glu Gln Ala Glu Gln Ala Leu Gln Asp Ile Leu Gly Glu Ala Gln Ile
            660                 665                 670
Ser Glu Gly Ala Met Arg Ala Val Ala Val Arg Leu Ala Lys Ala Arg
        675                 680                 685
Ser Gln Glu Asn Asp Tyr Lys Thr Arg Leu Asp Asp Leu Lys Met Thr
    690                 695                 700
Ala Glu Arg Ile Arg Ala Leu Gly Ser Gln His Gln Asn Arg Val Gln
705                 710                 715                 720

Asp Thr Ser Arg Leu Ile Ser Gln Met Arg Leu Ser Leu Ala Gly Ser
                725                 730                 735
Glu Ala Leu Leu Glu Asn Thr Asn Ile His Ser Ser Glu His Tyr Val
            740                 745                 750
Gly Pro Asn Asp Phe Lys Ser Leu Ala Gln Glu Ala Thr Arg Lys Ala
        755                 760                 765
Asp Ser His Ala Glu Ser Ala Asn Ala Met Lys Gln Leu Ala Arg Glu
    770                 775                 780
Thr Glu Asp Tyr Ser Lys Gln Ala Leu Ser Leu Ala Arg Lys Leu Leu
785                 790                 795                 800
Ser Gly Gly Gly Gly Ser Gly Ser Trp Asp Ser Ser Val Val Gln Gly
                805                 810                 815
Leu Met Gly Lys Leu Glu Lys Thr Lys Ser Leu Ser Gln Gln Leu Ser
            820                 825                 830
Leu Glu Gly Thr Gln Ala Asp Ile Glu Ala Asp Arg Ser Tyr Gln His
        835                 840                 845
Ser Leu Arg Leu Leu Asp Ser Ala Ser Gln Leu Gln Gly Val Ser Asp
    850                 855                 860
Leu Ser Phe Gln Val Glu Ala Lys Arg Ile Arg Gln Lys Ala Asp Ser
865                 870                 875                 880
Leu Ser Asn Leu Val Thr Arg Gln Thr Asp Ala Phe Thr Arg Val Arg
                885                 890                 895
Asn Asn Leu Gly Asn Trp Glu Lys Glu Thr Arg Gln Leu Leu Gln Thr
            900                 905                 910
Gly Lys Asp Arg Arg Gln Thr Ser Asp Gln Leu Leu Ser Arg Ala Asn
        915                 920                 925
Leu Ala Lys Asn Arg Ala Gln Glu Ala Leu Ser Met Gly Asn Ala Thr
    930                 935                 940
Phe Tyr Glu Val Glu Asn Ile Leu Lys Asn Leu Arg Glu Phe Asp Leu
945                 950                 955                 960
Gln Val Glu Asp Arg Lys Ala Glu Ala Glu Glu Ala Met Lys Arg Leu
                965                 970                 975
Ser Ser Ile Ser Gln Lys Val Ala Asp Ala Ser Asp Lys Thr Gln Gln
            980                 985                 990
Ala Glu Thr Ala Leu Gly Ser Ala Thr Ala Asp Thr Gln Arg Ala Lys
        995                 1000                1005
Asn Ala Ala Arg Glu Ala Leu Glu Ile Ser Ser Glu Ile Glu Leu Glu
    1010                1015                1020
Ile Gly Ser Leu Asn Leu Glu Ala Asn Val Thr Ala Asp Gly Ala Leu
1025                1030                1035                1040
Ala Met Glu Lys Gly Thr Ala Thr Leu Lys Ser Glu Met Arg Glu Met
                1045                1050                1055
Ile Glu Leu Ala Arg Lys Glu Leu Glu Phe Asp Thr Asp Lys Asp Thr
            1060                1065                1070
Val Gln Leu Val Ile Thr Glu Ala Gln Gln Ala Asp Ala Arg Ala Thr
        1075                1080                1085
Ser Ala Gly Val Thr Ile Gln Asp Thr Leu Asn Thr Leu Asp Gly Ile
    1090                1095                1100
Leu His Leu Ile Asp Gln Pro Gly Ser Val Asp Glu Glu Gly Met Met
1105                1110                1115                1120
Leu Leu Glu Gln Gly Leu Phe Gln Ala Lys Thr Gln Ile Asn Ser Arg
                1125                1130                1135
Leu Arg Pro Leu Met Ser Asp Leu Glu Glu Arg Val Arg Arg Gln Arg
            1140                1145                1150
Asn His Leu His Leu Leu Glu Thr Ser Ile Asp Gly Ile Leu Ala Asp
        1155                1160                1165
Val Lys Asn Leu Glu Asn Ile Arg Asp Asn Leu Pro Pro Gly Cys Tyr
    1170                1175                1180
Asn Thr Gln Ala Leu Glu Gln Gln
1185                1190

<210> 13
<211> 3582
<212> DNA
<213> Artificial Sequence

<220>
<223> Description of Artificial Sequence; Note =

synthetic construct

<400> 13
atgcctgcgc tctggctggg ctgctgcctc tgcttctcgc tcctcctgcc cgcagcccgg 60
gccacctcca ggagggaagt ctgtgattgc aatgggaagt ccaggcagtg tatctttgat 120
cgggaacttc acagacaaac tggtaatgga ttccgctgcc tcaactgcaa tgacaacact 180
gatggcattc actgcgagaa gtgcaagaat ggcttttacc ggcacagaga aagggaccgc 240
tgtttgccct gcaattgtaa ctccaaaggt tctcttagtg ctcgatgtga caactctgga 300
cggtgcagct gtaaaccagg tgtgacagga gccagatgcg accgatgtct gccaggcttc 360
cacatgctca cggatgcggg gtgcacccaa gaccagagac tgctagactc caagtgtgac 420
tgtgacccag ctggcatcgc agggccctgt gacgcgggcc gctgtgtctg caagccagct 480
gttactggag aacgctgtga taggtgtcga tcaggttact ataatctgga tgggggaac 540
cctgagggct gtacccagtg tttctgctat gggcattcag ccagctgccg cagctctgca 600
gaatacagtg tccataagat cacctctacc tttcatcaag atgttgatgg ctggaaggct 660
gtccaacgaa atgggtctcc tgcaaagctc caatggtcac agcgccatca agatgtgttt 720
agctcagccc aacgactaga ccctgtctat tttgtggctc ctgccaaatt tcttgggaat 780
caacaggtga gctatgggca aagcctgtcc tttgactacc gtgtggacag aggaggcaga 840
cacccatctg cccatgatgt gattctggaa ggtgctggtc tacggatcac agctcccttg 900
atgccacttg gcaagacact gccttgtggg ctcaccaaga cttacacatt caggttaaat 960
gagcatccaa gcaataattg gagcccccag ctgagttact ttgagtatcg aaggttactg 1020
cggaatctca cagccctccg catccgagct acatatggag aatacagtac tgggtacatt 1080
gacaatgtga ccctgatttc agcccgccct gtctctggag ccccagcacc ctgggttgaa 1140
cagtgtatat gtcctgttgg gtacaagggg caattctgcc aggattgtgc ttctggctac 1200
aagagagatt cagcgagact ggggcctttt ggcacctgta ttccttgtaa ctgtcaaggg 1260
ggaggggcct gtgatccaga cacaggagat tgttattcag gggatgagaa tcctgacatt 1320
gagtgtgctg actgcccaat tggtttctac aacgatccgc acgacccccg cagctgcaag 1380
ccatgtccct gtcataacgg gttcagctgc tcagtgatgc cggagacgga ggaggtggtg 1440
tgcaataact gccctcccgg ggtcaccggt gcccgctgtg agctctgtgc tgatggctac 1500
tttggggacc cctttggtga acatggccca gtgaggcctt gtcagccctg tcaatgcaac 1560
aacaatgtgg accccagtgc ctctgggaat tgtgaccggc tgacaggcag gtgtttgaag 1620
tgtatccaca acacagccgg catctactgc gaccagtgca aagcaggcta cttcggggac 1680
ccattggctc ccaacccagc agacaagtgt cgagcttgca actgtaaccc catgggctca 1740
gagcctgtag gatgtcgaag tgatggcacc tgtgtttgca agccaggatt tggtggcccc 1800
aactgtgagc atggagcatt cagctgtcca gcttgctata atcaagtgaa gattcagatg 1860
gatcagttta tgcagcagct tcagagaatg gaggccctga tttcaaaggc tcagggtggt 1920
gatggagtag tacctgatac agagctggaa ggcaggatgc agcaggctga gcaggccctt 1980
caggacattc tgagagatgc ccagatttca gaaggtgcta gcagatccct tggtctccag 2040
ttggccaagg tgaggagcca agagaacagc taccagagcc gcctggatga cctcaagatg 2100
actgtggaaa gagttcgggc tctgggaagt cagtaccaga accgagttcg ggatactcac 2160
aggctcatca ctcagatgca gctgagcctg gcagaaagtg aagcttcctt gggaaacact 2220
aacattcctg cctcagacca ctacgtgggg ccaaatggct ttaaaagtct ggctcaggag 2280
gccacaagat tagcagaaag ccacgttgag tcagccagta acatggagca actgacaagg 2340
gaaactgagg actattccaa acaagccctc tcactggtgc gcaaggccct gcatgaagga 2400
gtcggaagcg gaagcggtag cccggacggt gctgtggtgc aagggcttgt ggaaaaattg 2460
gagaaaacca agtccctggc ccagcagttg acaagggagg ccactcaagc ggaaattgaa 2520
gcagataggt cttatcagca cagtctccgc ctcctggatt cagtgtctcg gcttcaggga 2580
gtcagtgatc agtcctttca ggtggaagaa gcaaagagga tcaaacaaaa agcggattca 2640
ctctcaacgc tggtaaccag gcatatggat gagttcaagc gtacacaaaa gaatctggga 2700
aactggaaag aagaagcaca gcagctctta cagaatggaa aaagtgggag agagaaatca 2760
gatcagctgc tttcccgtgc caatcttgct aaaagcagag cacaagaagc actgagtatg 2820
ggcaatgcca ctttttatga agttgagagc atccttaaaa acctcagaga gtttgacctg 2880
caggtggaca acagaaaagc agaagctgaa gaagccatga agagactctc ctacatcagc 2940
cagaaggttt cagatgccag tgacaagacc cagcaagcag aaagagccct ggggagcgct 3000
gctgctgatg cacagagggc aaagaatggg gccggggagg ccctggaaat ctccagtgag 3060
attgaacagg agattgggag tctgaacttg gaagccaatg tgacagcaga tggagccttg 3120
gccatggaaa agggactggc ctctctgaag agtgagatga gggaagtgga aggagagctg 3180
gaaaggaagg agctggagtt tgacacgaat atggatgcag tacagatggt gattacagaa 3240
gcccagaagg ttgataccag agccaagaac gctggggtta caatccaaga cacactcaac 3300
acattagacg gcctcctgca tctgatggac cagcctctca gtgtagatga agaggggctg 3360
gtcttactgg agcagaagct ttcccgagcc aagacccaga tcaacagcca actgcggccc 3420
atgatgtcag agctggaaga gagggcacgt cagcagaggg gccacctcca tttgctggag 3480
acaagcatag atgggattct ggctgatgtg aagaacttgg agaacattag ggacaacctg 3540
cccccaggct gctacaatac ccaggctctt gagcaacagt ga 3582

<210> 14
<211> 1193
<212> PRT
<213> Artificial Sequence

<220>
<223> Description of Artificial Sequence; Note =
      synthetic construct

<400> 14
Met Pro Ala Leu Trp Leu Gly Cys Cys Leu Cys Phe Ser Leu Leu Leu
1               5                   10                  15
Pro Ala Ala Arg Ala Thr Ser Arg Arg Glu Val Cys Asp Cys Asn Gly
            20                  25                  30
Lys Ser Arg Gln Cys Ile Phe Asp Arg Glu Leu His Arg Gln Thr Gly
        35                  40                  45
Asn Gly Phe Arg Cys Leu Asn Cys Asn Asp Asn Thr Asp Gly Ile His
    50                  55                  60
Cys Glu Lys Cys Lys Asn Gly Phe Tyr Arg His Arg Glu Arg Asp Arg
65                  70                  75                  80
Cys Leu Pro Cys Asn Cys Asn Ser Lys Gly Ser Leu Ser Ala Arg Cys
                85                  90                  95
Asp Asn Ser Gly Arg Cys Ser Cys Lys Pro Gly Val Thr Gly Ala Arg
            100                 105                 110
Cys Asp Arg Cys Leu Pro Gly Phe His Met Leu Thr Asp Ala Gly Cys
        115                 120                 125
Thr Gln Asp Gln Arg Leu Leu Asp Ser Lys Cys Asp Cys Asp Pro Ala
    130                 135                 140
Gly Ile Ala Gly Pro Cys Asp Ala Gly Arg Cys Val Cys Lys Pro Ala
145                 150                 155                 160
Val Thr Gly Glu Arg Cys Asp Arg Cys Arg Ser Gly Tyr Tyr Asn Leu
                165                 170                 175
Asp Gly Gly Asn Pro Glu Gly Cys Thr Gln Cys Phe Cys Tyr Gly His
            180                 185                 190
Ser Ala Ser Cys Arg Ser Ser Ala Glu Tyr Ser Val His Lys Ile Thr
        195                 200                 205
Ser Thr Phe His Gln Asp Val Asp Gly Trp Lys Ala Val Gln Arg Asn
    210                 215                 220
Gly Ser Pro Ala Lys Leu Gln Trp Ser Gln Arg His Gln Asp Val Phe
225                 230                 235                 240
Ser Ser Ala Gln Arg Leu Asp Pro Val Tyr Phe Val Ala Pro Ala Lys
                245                 250                 255
Phe Leu Gly Asn Gln Gln Val Ser Tyr Gly Gln Ser Leu Ser Phe Asp
            260                 265                 270
Tyr Arg Val Asp Arg Gly Gly Arg His Pro Ser Ala His Asp Val Ile
        275                 280                 285
Leu Glu Gly Ala Gly Leu Arg Ile Thr Ala Pro Leu Met Pro Leu Gly
    290                 295                 300
Lys Thr Leu Pro Cys Gly Leu Thr Lys Thr Tyr Thr Phe Arg Leu Asn
305                 310                 315                 320
Glu His Pro Ser Asn Asn Trp Ser Pro Gln Leu Ser Tyr Phe Glu Tyr
                325                 330                 335
Arg Arg Leu Leu Arg Asn Leu Thr Ala Leu Arg Ile Arg Ala Thr Tyr
            340                 345                 350
Gly Glu Tyr Ser Thr Gly Tyr Ile Asp Asn Val Thr Leu Ile Ser Ala
        355                 360                 365
Arg Pro Val Ser Gly Ala Pro Ala Pro Trp Val Glu Gln Cys Ile Cys
    370                 375                 380
Pro Val Gly Tyr Lys Gly Gln Phe Cys Gln Asp Cys Ala Ser Gly Tyr
385                 390                 395                 400
Lys Arg Asp Ser Ala Arg Leu Gly Pro Phe Gly Thr Cys Ile Pro Cys
                405                 410                 415
Asn Cys Gln Gly Gly Gly Ala Cys Asp Pro Asp Thr Gly Asp Cys Tyr
            420                 425                 430
Ser Gly Asp Glu Asn Pro Asp Ile Glu Cys Ala Asp Cys Pro Ile Gly
        435                 440                 445
Phe Tyr Asn Asp Pro His Asp Pro Arg Ser Cys Lys Pro Cys Pro Cys
    450                 455                 460
His Asn Gly Phe Ser Cys Ser Val Met Pro Glu Thr Glu Glu Val Val
465                 470                 475                 480
Cys Asn Asn Cys Pro Pro Gly Val Thr Gly Ala Arg Cys Glu Leu Cys
                485                 490                 495

Ala Asp Gly Tyr Phe Gly Asp Pro Phe Gly Glu His Gly Pro Val Arg
            500                 505                 510
Pro Cys Gln Pro Cys Gln Cys Asn Asn Asn Val Asp Pro Ser Ala Ser
        515                 520                 525
Gly Asn Cys Asp Arg Leu Thr Gly Arg Cys Leu Lys Cys Ile His Asn
    530                 535                 540
Thr Ala Gly Ile Tyr Cys Asp Gln Cys Lys Ala Gly Tyr Phe Gly Asp
545                 550                 555                 560
Pro Leu Ala Pro Asn Pro Ala Asp Lys Cys Arg Ala Cys Asn Cys Asn
                565                 570                 575
Pro Met Gly Ser Glu Pro Val Gly Cys Arg Ser Asp Gly Thr Cys Val
            580                 585                 590
Cys Lys Pro Gly Phe Gly Gly Pro Asn Cys Glu His Gly Ala Phe Ser
        595                 600                 605
Cys Pro Ala Cys Tyr Asn Gln Val Lys Ile Gln Met Asp Gln Phe Met
    610                 615                 620
Gln Gln Leu Gln Arg Met Glu Ala Leu Ile Ser Lys Ala Gln Gly Gly
625                 630                 635                 640
Asp Gly Val Val Pro Asp Thr Glu Leu Glu Gly Arg Met Gln Gln Ala
                645                 650                 655
Glu Gln Ala Leu Gln Asp Ile Leu Arg Asp Ala Gln Ile Ser Glu Gly
            660                 665                 670
Ala Ser Arg Ser Leu Gly Leu Gln Leu Ala Lys Val Arg Ser Gln Glu
        675                 680                 685
Asn Ser Tyr Gln Ser Arg Leu Asp Asp Leu Lys Met Thr Val Glu Arg
    690                 695                 700
Val Arg Ala Leu Gly Ser Gln Tyr Gln Asn Arg Val Arg Asp Thr His
705                 710                 715                 720
Arg Leu Ile Thr Gln Met Gln Leu Ser Leu Ala Glu Ser Glu Ala Ser
                725                 730                 735
Leu Gly Asn Thr Asn Ile Pro Ala Ser Asp His Tyr Val Gly Pro Asn
            740                 745                 750
Gly Phe Lys Ser Leu Ala Gln Glu Ala Thr Arg Leu Ala Glu Ser His
        755                 760                 765
Val Glu Ser Ala Ser Asn Met Glu Gln Leu Thr Arg Glu Thr Glu Asp
    770                 775                 780
Tyr Ser Lys Gln Ala Leu Ser Leu Val Arg Lys Ala Leu His Glu Gly
785                 790                 795                 800
Val Gly Ser Gly Ser Gly Ser Pro Asp Gly Ala Val Val Gln Gly Leu
                805                 810                 815
Val Glu Lys Leu Glu Lys Thr Lys Ser Leu Ala Gln Gln Leu Thr Arg
            820                 825                 830
Glu Ala Thr Gln Ala Glu Ile Glu Ala Asp Arg Ser Tyr Gln His Ser
        835                 840                 845
Leu Arg Leu Leu Asp Ser Val Ser Arg Leu Gln Gly Val Ser Asp Gln
    850                 855                 860
Ser Phe Gln Val Glu Glu Ala Lys Arg Ile Lys Gln Lys Ala Asp Ser
865                 870                 875                 880
Leu Ser Thr Leu Val Thr Arg His Met Asp Glu Phe Lys Arg Thr Gln
                885                 890                 895
Lys Asn Leu Gly Asn Trp Lys Glu Glu Ala Gln Gln Leu Leu Gln Asn
            900                 905                 910
Gly Lys Ser Gly Arg Glu Lys Ser Asp Gln Leu Leu Ser Arg Ala Asn
        915                 920                 925
Leu Ala Lys Ser Arg Ala Gln Glu Ala Leu Ser Met Gly Asn Ala Thr
    930                 935                 940
Phe Tyr Glu Val Glu Ser Ile Leu Lys Asn Leu Arg Glu Phe Asp Leu
945                 950                 955                 960
Gln Val Asp Asn Arg Lys Ala Glu Ala Glu Glu Ala Met Lys Arg Leu
                965                 970                 975
Ser Tyr Ile Ser Gln Lys Val Ser Asp Ala Ser Asp Lys Thr Gln Gln
            980                 985                 990
Ala Glu Arg Ala Leu Gly Ser Ala Ala Ala Asp Ala Gln Arg Ala Lys
        995                 1000                1005
Asn Gly Ala Gly Glu Ala Leu Glu Ile Ser Ser Glu Ile Glu Gln Glu
    1010                1015                1020
Ile Gly Ser Leu Asn Leu Glu Ala Asn Val Thr Ala Asp Gly Ala Leu
1025                1030                1035                1040

Ala Met Glu Lys Gly Leu Ala Ser Leu Lys Ser Glu Met Arg Glu Val
                1045                1050                1055
Glu Gly Glu Leu Glu Arg Lys Glu Leu Glu Phe Asp Thr Asn Met Asp
            1060                1065                1070
Ala Val Gln Met Val Ile Thr Glu Ala Gln Lys Val Asp Thr Arg Ala
        1075                1080                1085
Lys Asn Ala Gly Val Thr Ile Gln Asp Thr Leu Asn Thr Leu Asp Gly
    1090                1095                1100
Leu Leu His Leu Met Asp Gln Pro Leu Ser Val Asp Glu Glu Gly Leu
1105                1110                1115                1120
Val Leu Leu Glu Gln Lys Leu Ser Arg Ala Lys Thr Gln Ile Asn Ser
                1125                1130                1135
Gln Leu Arg Pro Met Met Ser Glu Leu Glu Glu Arg Ala Arg Gln Gln
            1140                1145                1150
Arg Gly His Leu His Leu Leu Glu Thr Ser Ile Asp Gly Ile Leu Ala
        1155                1160                1165
Asp Val Lys Asn Leu Glu Asn Ile Arg Asp Asn Leu Pro Pro Gly Cys
    1170                1175                1180
Tyr Asn Thr Gln Ala Leu Glu Gln Gln
1185                1190

<210> 15
<211> 1111
<212> PRT
<213> Artificial Sequence

<220>
<223> Description of Artificial Sequence; Note =
      synthetic construct

<400> 15
Met Pro Ala Leu Trp Leu Gly Cys Cys Leu Cys Phe Ser Leu Leu Leu
 1               5                  10                  15
Pro Ala Ala Arg Ala Thr Ser Arg Arg Glu Val Cys Asp Cys Asn Gly
            20                  25                  30
Lys Ser Arg Gln Cys Ile Phe Asp Arg Glu Leu His Arg Gln Thr Gly
        35                  40                  45
Asn Gly Phe Arg Cys Leu Asn Cys Asn Asp Asn Thr Asp Gly Ile His
    50                  55                  60
Cys Glu Lys Cys Lys Asn Gly Phe Tyr Arg His Arg Glu Arg Asp Arg
65                  70                  75                  80
Cys Leu Pro Cys Asn Cys Asn Ser Lys Gly Ser Leu Ser Ala Arg Cys
                85                  90                  95
Asp Asn Ser Gly Arg Cys Ser Cys Lys Pro Gly Val Thr Gly Ala Arg
            100                 105                 110
Cys Asp Arg Cys Leu Pro Gly Phe His Met Leu Thr Asp Ala Gly Cys
        115                 120                 125
Thr Gln Asp Gln Arg Leu Leu Asp Ser Lys Cys Asp Cys Asp Pro Ala
    130                 135                 140
Gly Ile Ala Gly Pro Cys Asp Ala Gly Arg Cys Val Cys Lys Pro Ala
145                 150                 155                 160
Val Thr Gly Glu Arg Cys Asp Arg Cys Arg Ser Gly Tyr Tyr Asn Leu
                165                 170                 175
Asp Gly Gly Asn Pro Glu Gly Cys Thr Gln Cys Phe Cys Tyr Gly His
            180                 185                 190
Ser Ala Ser Cys Arg Ser Ser Ala Glu Tyr Ser Val His Lys Ile Thr
        195                 200                 205
Ser Thr Phe His Gln Asp Val Asp Gly Trp Lys Ala Val Gln Arg Asn
    210                 215                 220
Gly Ser Pro Ala Lys Leu Gln Trp Ser Gln Arg His Gln Asp Val Phe
225                 230                 235                 240
Ser Ser Ala Gln Arg Leu Asp Pro Val Tyr Phe Val Ala Pro Ala Lys
                245                 250                 255
Phe Leu Gly Asn Gln Gln Val Ser Tyr Gly Gln Ser Leu Ser Phe Asp
            260                 265                 270
Tyr Arg Val Asp Arg Gly Gly Arg His Pro Ser Ala His Asp Val Ile
        275                 280                 285
Leu Glu Gly Ala Gly Leu Arg Ile Thr Ala Pro Leu Met Pro Leu Gly

290                 295                 300
Lys Thr Leu Pro Cys Gly Leu Thr Lys Thr Tyr Thr Phe Arg Leu Asn
305                 310                 315                 320
Glu His Pro Ser Asn Asn Trp Ser Pro Gln Leu Ser Tyr Phe Glu Tyr
                325                 330                 335
Arg Arg Leu Leu Arg Asn Leu Thr Ala Leu Arg Ile Arg Ala Thr Tyr
            340                 345                 350
Gly Glu Tyr Ser Thr Gly Tyr Ile Asp Asn Val Thr Leu Ile Ser Ala
        355                 360                 365
Arg Pro Val Ser Gly Ala Pro Ala Pro Trp Val Glu Gln Cys Ile Cys
    370                 375                 380
Pro Val Gly Tyr Lys Gly Gln Phe Cys Gln Asp Cys Ala Ser Gly Tyr
385                 390                 395                 400
Lys Arg Asp Ser Ala Arg Leu Gly Pro Phe Gly Thr Cys Ile Pro Cys
                405                 410                 415
Asn Cys Gln Gly Gly Gly Ala Cys Asp Pro Asp Thr Gly Asp Cys Tyr
            420                 425                 430
Ser Gly Asp Glu Asn Pro Asp Ile Glu Cys Ala Asp Cys Pro Ile Gly
        435                 440                 445
Phe Tyr Asn Asp Pro His Asp Pro Arg Ser Cys Lys Pro Cys Pro Cys
    450                 455                 460
His Asn Gly Phe Ser Cys Ser Val Met Pro Glu Thr Glu Glu Val Val
465                 470                 475                 480
Cys Asn Asn Cys Pro Pro Gly Val Thr Gly Ala Arg Cys Glu Leu Cys
                485                 490                 495
Ala Asp Gly Tyr Phe Gly Asp Pro Phe Gly Glu His Gly Pro Val Arg
            500                 505                 510
Pro Cys Gln Pro Cys Gln Cys Asn Asn Asn Val Asp Pro Ser Ala Ser
        515                 520                 525
Gly Asn Cys Asp Arg Leu Thr Gly Arg Cys Leu Lys Cys Ile His Asn
    530                 535                 540
Thr Ala Gly Ile Tyr Cys Asp Gln Cys Lys Ala Gly Tyr Phe Gly Asp
545                 550                 555                 560
Pro Leu Ala Pro Asn Pro Ala Asp Lys Cys Arg Ala Cys Asn Cys Asn
                565                 570                 575
Pro Met Gly Ser Glu Pro Val Gly Cys Arg Ser Asp Gly Thr Cys Val
            580                 585                 590
Cys Lys Pro Gly Phe Gly Gly Pro Asn Cys Glu His Gly Ala Phe Ser
        595                 600                 605
Cys Pro Ala Cys Tyr Asn Gln Val Lys Ile Gln Met Asp Gln Phe Met
    610                 615                 620
Gln Gln Leu Gln Arg Met Glu Ala Leu Ile Ser Lys Ala Gln Gly Gly
625                 630                 635                 640
Asp Gly Val Val Pro Asp Thr Glu Leu Glu Gly Arg Met Gln Gln Ala
                645                 650                 655
Glu Gln Ala Leu Gln Asp Ile Leu Arg Asp Ala Gln Ile Ser Glu Gly
            660                 665                 670
Ala Ser Arg Ser Leu Gly Leu Gln Leu Ala Lys Val Arg Ser Gln Glu
        675                 680                 685
Asn Ser Tyr Gln Ser Arg Leu Asp Asp Leu Lys Met Thr Val Glu Arg
    690                 695                 700
Val Arg Ala Leu Gly Ser Gln Tyr Gln Asn Arg Val Arg Asp Thr His
705                 710                 715                 720
Arg Leu Ile Thr Gln Met Gln Leu Ser Leu Ala Glu Ser Glu Ala Ser
                725                 730                 735
Leu Gly Asn Thr Asn Ile Pro Ala Ser Asp His Tyr Val Gly Pro Asn
            740                 745                 750
Gly Phe Lys Ser Leu Ala Gln Glu Ala Thr Arg Leu Ala Glu Ser His
        755                 760                 765
Val Glu Ser Ala Ser Asn Met Glu Gln Leu Thr Arg Glu Thr Glu Asp
    770                 775                 780
Tyr Ser Lys Gln Ala Leu Ser Leu Val Arg Lys Ala Leu His Glu Gly
785                 790                 795                 800
Val Gly Ser Gly Ser Gly Ser Pro Asp Gly Ala Val Val Gln Gly Leu
                805                 810                 815
Val Glu Lys Leu Glu Lys Thr Lys Ser Leu Ala Gln Gln Leu Thr Arg
            820                 825                 830
Glu Ala Thr Gln Ala Glu Ile Glu Ala Asp Arg Ser Tyr Gln His Ser

835                 840                 845
Leu Arg Leu Leu Asp Ser Val Ser Arg Leu Gln Gly Val Ser Asp Gln
    850                 855                 860
Ser Phe Gln Val Glu Glu Ala Lys Arg Ile Lys Gln Lys Ala Asp Ser
865                 870                 875                 880
Leu Ser Thr Leu Val Thr Arg His Met Asp Glu Phe Lys Arg Thr Gln
                885                 890                 895
Lys Asn Leu Gly Asn Trp Lys Glu Glu Ala Gln Gln Leu Leu Gln Asn
            900                 905                 910
Gly Lys Ser Gly Arg Glu Lys Ser Asp Gln Leu Leu Ser Arg Ala Asn
        915                 920                 925
Leu Ala Lys Ser Arg Ala Gln Glu Ala Leu Ser Met Gly Asn Ala Thr
    930                 935                 940
Phe Tyr Glu Val Glu Ser Ile Leu Lys Asn Leu Arg Glu Phe Asp Leu
945                 950                 955                 960
Gln Val Asp Asn Arg Lys Ala Glu Ala Glu Glu Ala Met Lys Arg Leu
                965                 970                 975
Ser Tyr Ile Ser Gln Lys Val Ser Asp Ala Ser Asp Lys Thr Gln Gln
            980                 985                 990
Ala Glu Arg Ala Leu Gly Ser Ala Ala Ala Asp Ala Gln Arg Ala Lys
        995                 1000                1005
Asn Gly Ala Gly Glu Ala Leu Glu Ile Ser Ser Glu Ile Glu Gln Glu
    1010                1015                1020
Ile Gly Ser Leu Asn Leu Glu Ala Asn Val Thr Ala Asp Gly Ala Leu
1025                1030                1035                1040
Ala Met Glu Lys Gly Leu Ala Ser Leu Lys Ser Glu Met Arg Glu Val
                1045                1050                1055
Glu Gly Glu Leu Glu Arg Lys Glu Leu Glu Phe Asp Thr Asn Met Asp
            1060                1065                1070
Ala Val Gln Met Val Ile Thr Glu Ala Gln Lys Val Asp Thr Arg Ala
        1075                1080                1085
Lys Asn Ala Gly Val Thr Ile Gln Asp Thr Leu Asn Thr Leu Asp Gly
    1090                1095                1100
Leu Leu His Leu Met Gly Met
1105                1110

<210> 16
<211> 5622
<212> DNA
<213> Artificial Sequence

<220>
<223> Description of Artificial Sequence; Note =
      synthetic construct

<400> 16
atggcagcgc cacgccccag cccatgggcc aggctgctcc tggcagcctt gatcagcgtc      60
agcctctctg ggaccttgaa ccgctgcaag aaggccccag tgaagagctg cacggagtgt     120
gtccgtgtgg ataaggactg cgcctactgc acagacgaga tgttcaggga ccggcgctgc     180
aacacccagg cggagctgct ggccgcgggc tgccagcggg agagcatcgt ggtcatggag     240
agcagcttcc aaatcacaga ggagacccag attgacacca ccctgcggcg cagccagatg     300
tccccccaag gcctgcgggt ccgtctgcgg cccggtgagg agcggcattt tgagctggag     360
gtgtttgagc cactggagag ccccgtggac ctgtacatcc tcatggactt ctccaactcc     420
atgtccgatg atctggacaa cctcaagaag atggggcaga acctggctcg ggtcctgagc     480
cagctcacca gcgactacac tattggattt ggcaagtttg tggacaaagt cagcgtcccg     540
cagacggaca tgaggcctga gaagctgaag gagccttggc ccaacagtga cccccccttc     600
tccttcaaga acgtcatcag cctgacagaa gatgtggatg agttccggaa taaactgcag     660
ggagagcgga tctcaggcaa cctggatgct cctgagggcg gcttcgatgc catcctgcag     720
acagctgtgt gcacgaggga cattggctgg cgcccggaca gcacccacct gctggtcttc     780
tccaccgagt cagccttcca ctatgaggct gatggcgcca acgtgctggc tggcatcatg     840
agccgcaacg atgaacggtg ccacctggac accacgggca cctacaccca gtacaggaca     900
caggactacc cgtcggtgcc caccctggtg cgcctgctcg ccaagcacaa catcatcccc     960
atctttgctg tcaccaacta ctcctatagc tactacgaga agcttcacac ctatttccct    1020
gtctcctcac tgggggtgct gcaggaggac tcgtccaaca tcgtggagct gctggaggag    1080
gccttcaatc ggatccgctc caacctggac atccgggccc tagacagccc ccgaggcctt    1140
cggacagagg tcacctccaa gatgttccag aagacgagga ctgggtcctt tcacatccgg    1200
cggggggaag tgggtatata ccaggtgcag ctgcgggccc ttgagcacgt ggatgggacg    1260
cacgtgtgcc agctgccgga ggaccagaag ggcaacatcc atctgaaacc ttccttctcc    1320

gacggcctca agatggacgc gggcatcatc tgtgatgtgt gcacctgcga gctgcaaaaa 1380
gaggtgcggt cagctcgctg cagcttcaac ggagacttcg tgtgcggaca gtgtgtgtgc 1440
agcgagggct ggagtggcca gacctgcaac tgctccaccg gctctctgag tgacattcag 1500
ccctgcctgc gggagggcga ggacaagccg tgctccggcc gtggggagtg ccagtgcggg 1560
cactgtgtgt gctacggcga aggccgctac gagggtcagt tctgcgagta tgacaacttc 1620
cagtgtcccc gcacttccgg gttcctctgc aatgaccgag gacgctgctc catgggccag 1680
tgtgtgtgtg agcctggttg gacaggccca agctgtgact gtcccctcag caatgccacc 1740
tgcatcgaca gcaatggggg catctgtaat ggacgtggcc actgtgagtg tggccgctgc 1800
cactgccacc agcagtcgct ctacacggac accatctgcg agatcaacta ctcggcgtcc 1860
acccgggcct ctgcgaggac ctacgctcct gcgtgcagtg ccaggcgtgg ggcaccggcg 1920
agaagaaggg gcgcacgtgt gaggaatgca acttcaaggt caagatggtg gacgagctta 1980
agagaggcga ggaggtggtg gtgcgctgct ccttccggga cgaggatgac gactgcacct 2040
acagctacac catggaaggt gacggcgccc ctgggcccaa cagcactgtc ctggtgcaca 2100
agaagaaggg actgccctcc gggctccttc tggtggctca tccccctgct cctcctcctc 2160
ctgccgctcc tggccctgct actgctgcta tgctggaagt actgtgcctg ctgcaaggcc 2220
tgcctggcac ttctcccgtg ctgcaaccga ggtcacatgg tgggctttaa ggaagaccac 2280
tacatgctgc gggagaacct gatggcctct gaccacttgg acacgcccat gctgcgcagc 2340
gggaacctca agggccgtga cgtggtccgc tggaaggtca ccaacaacat gcagcggcct 2400
ggctttgcca ctcatgccgc cagcatcaac cccacagagc tggtgcccta cgggctgtcc 2460
ttgcgcctgg cccgcctttg caccgagaac ctgctgaagc ctgacactcg ggagtgcgcc 2520
cagctgcgcc aggaggtgga ggagaacctg aacgaggtct acaggcagat ctccggtgta 2580
cacaagctcc agcagaccaa gttccggcag cagcccaatg ccgggaaaaa gcaagaccac 2640
accattgtgg acacagtgct gatggcgccc cgctcggcca agccggccct gctgaagctt 2700
acagagaagc aggtggaaca gagggccttc cacgacctca aggtggcccc cggctactac 2760
accctcactg cagaccagga cgcccggggc atggtggagt tccaggaggg cgtggagctg 2820
gtggacgtac gggtgcccct ctttatccgg cctgaggatg acgacgagaa gcagctgctg 2880
gtggaggcca tcgacgtgcc cgcaggcact gccaccctcg gccgccgcct ggtaaacatc 2940
accatcatca aggagcaagc cagagacgtg gtgtcctttg agcagcctga gttctcggtc 3000
agccgcgggg accaggtggc ccgcatccct gtcatccggc gtgtcctgga cggcgggaag 3060
tcccaggtct cctaccgcac acaggatggc accgcgcagg gcaaccggga ctacatcccc 3120
gtggagggtg agctgctgtt ccagcctggg gaggcctgga aagagctgca ggtgaagctc 3180
ctggagctgc aagaagttga ctccctcctg cggggccgcc aggtccgccg tttccacgtc 3240
cagctcagca accctaagtt tggggcccac ctgggccagc cccactccac caccatcatc 3300
atcagggacc cagatgaact ggaccggagc ttcacgagtc agatgttgtc atcacagcca 3360
ccccctcacg gcgacctggg cgccccgcag aaccccaatg ctaaggccgc tgggtccagg 3420
aagatccatt tcaactggct gcccccttct ggcaagccaa tggggtacag ggtaaagtac 3480
tggattcagg gtgactccga atccgaagcc cacctgctcg acagcaaggt gccctcagtg 3540
gagctcacca acctgtaccc gtattgcgac tatgagatga aggtgtgcgc ctacggggct 3600
cagggcgagg gaccctacag ctccctggtg tcctgccgca cccaccagga agtgcccagc 3660
gagccagggc gtctggcctt caatgtcgtc tcctccacgg tgacccagct gagctgggct 3720
gagccggctg agaccaacgg tgagatcaca gcctacgagg tctgctatgg cctggtcaac 3780
gatgacaacc gacctattgg gcccatgaag aaagtgctgg ttgacaaccc taagaaccgg 3840
atgctgctta ttgagaacct tcgggagtcc cagccctacc gctacacggt gaaggcgcgc 3900
aacggggccg gctgggggcc tgagcgggag gccatcatca acctggccac ccagcccaag 3960
aggcccatgt ccatccccat catccctgac atccctatcg tggacgccca gagcggggag 4020
gactacgaca gcttccttat gtacagcgat gacgttctac gctctccatc gggcagccag 4080
aggcccagcg tctccgatga cactggctgc ggctggaagt tcgagcccct gctgggggag 4140
gagctggacc tgcggcgcgt cacgtggcgg ctgcccccgg agctcatccc gcgcctgtcg 4200
gccagcagcg ggcgctcctc cgacgccgag gcgccccacg ggcccccgga cgacggcggc 4260
gcgggcggga agggcggcag cctgccccgc agtgcgacac ccgggccccc cggagagcac 4320
ctggtgaatg gccggatgga ctttgccttc ccgggcagca ccaactccct gcacaggatg 4380
accacgacca gtgctgctgc ctatggcacc cacctgagcc cacacgtgcc ccaccgcgtg 4440
ctaagcacat cctccaccct cacacgggac tacaactcac tgacccgctc agaacactca 4500
cactcgacca cactgccgag ggactactcc accctcacct ccgtctcctc ccacggcctc 4560
cctcccatct gggaacacgg gaggagcagg cttccgctgt cctgggccct ggggtcccgg 4620
agtcgggctc agatgaaagg gttcccccct tccaggggcc cacgagactc tataatcctg 4680
gctgggaggc cagcagcgcc ctcctggggc ccagactctc gcctgactgc tggtgtgccc 4740
gacacgccca cccgcctggt gttctctgcc ctggggccca catctctcag agtgagctgg 4800
caggagccgc ggtgcgagcg gccgctgcag ggctacagtg tggagtacca gctgctgaac 4860
ggcggtgagc tgcatcggct caacatcccc aaccctgccc agacctcggt ggtggtggaa 4920
gacctcctgc ccaaccactc ctacgtgttc cgcgtgcggg cccagagcca ggaaggctgg 4980
ggccgagagc gtgagggtgt catcaccatt gaatcccagg tgcacccgca gagcccactg 5040
tgtcccctgc caggctccgc cttcactttg agcactccca gtgccccagg cccgctggtg 5100
ttcactgccc tgagcccaga ctcgctgcag ctgagctggg agcggccacg gaggcccaat 5160
ggggatatcg tcggctacct ggtgacctgt gagatggccc aaggaggagg tccagccacc 5220
gcattccggg tggatggaga cagccccgag agccggctga ccgtgccggg cctcagcgag 5280
aacgtgccct acaagttcaa ggtgcaggcc aggaccactg agggcttcgg gccagagcgc 5340
gagggcatca tcaccataga gtcccaggat ggaggtccct tcccgcagct gggcagccgt 5400

gccgggctct tccagcaccc gctgcaaagc gagtacagca gcatctccac cacccacacc 5460
agcgccaccg agcccttcct agtgggtccg accctggggg cccagcacct ggaggcaggc 5520
ggctccctca cccggcatgt gacccaggag tttgtgagcc ggacactgac caccagcgga 5580
acccttagca cccacatgga ccaacagttc ttccaaactt ga 5622

<210> 17
<211> 1873
<212> PRT
<213> Artificial Sequence

<220>
<223> Description of Artificial Sequence; Note =
      synthetic construct

<400> 17
Met Ala Gly Pro Arg Pro Ser Pro Trp Ala Arg Leu Leu Leu Ala Ala
1               5                   10                  15
Leu Ile Ser Val Ser Leu Ser Gly Thr Leu Asn Arg Cys Lys Lys Ala
            20                  25                  30
Pro Ile Lys Ser Cys Thr Glu Cys Val Arg Val Asp Lys Asp Cys Ala
        35                  40                  45
Tyr Cys Thr Asp Glu Met Phe Arg Asp Arg Arg Cys Asn Thr Gln Ala
    50                  55                  60
Glu Leu Leu Ala Ala Gly Cys Gln Arg Glu Ser Ile Val Val Met Glu
65                  70                  75                  80
Ser Ser Phe Gln Ile Thr Glu Glu Thr Gln Ile Asp Thr Thr Leu Arg
                85                  90                  95
Arg Ser Gln Met Ser Pro Gln Gly Leu Arg Val Arg Leu Arg Pro Gly
            100                 105                 110
Glu Glu Arg His Phe Glu Leu Glu Val Phe Glu Pro Leu Glu Ser Pro
        115                 120                 125
Val Asp Leu Tyr Ile Leu Met Asp Phe Ser Asn Ser Met Ser Asp Asp
    130                 135                 140
Leu Asp Asn Leu Lys Lys Met Gly Gln Asn Leu Ala Arg Val Leu Ser
145                 150                 155                 160
Gln Leu Thr Ser Asp Tyr Thr Ile Gly Phe Gly Lys Phe Val Asp Lys
                165                 170                 175
Val Ser Val Pro Gln Thr Asp Met Arg Pro Glu Lys Leu Lys Glu Pro
            180                 185                 190
Trp Pro Asn Ser Asp Pro Pro Phe Ser Phe Lys Asn Val Ile Ser Leu
        195                 200                 205
Thr Glu Asp Val Asp Glu Phe Arg Asn Lys Leu Gln Gly Glu Arg Ile
    210                 215                 220
Ser Gly Asn Leu Asp Ala Pro Glu Gly Gly Phe Asp Ala Ile Leu Gln
225                 230                 235                 240
Thr Ala Val Cys Thr Arg Asp Ile Gly Trp Arg Pro Asp Ser Thr His
                245                 250                 255
Leu Leu Val Phe Ser Thr Glu Ser Ala Phe His Tyr Glu Ala Asp Gly
            260                 265                 270
Ala Asn Val Leu Ala Gly Ile Met Ser Arg Asn Asp Glu Arg Cys His
        275                 280                 285
Leu Asp Thr Thr Gly Thr Tyr Thr Gln Tyr Arg Thr Gln Asp Tyr Pro
    290                 295                 300
Ser Val Pro Thr Leu Val Arg Leu Leu Ala Lys His Asn Ile Ile Pro
305                 310                 315                 320
Ile Phe Ala Val Thr Asn Tyr Ser Tyr Ser Tyr Tyr Glu Lys Leu His
                325                 330                 335
Thr Tyr Phe Pro Val Ser Ser Leu Gly Val Leu Gln Glu Asp Ser Ser
            340                 345                 350
Asn Ile Val Glu Leu Leu Glu Glu Ala Phe Asn Arg Ile Arg Ser Asn
        355                 360                 365
Leu Asp Ile Arg Ala Leu Asp Ser Pro Arg Gly Leu Arg Thr Glu Val
    370                 375                 380
Thr Ser Lys Met Phe Gln Lys Thr Arg Thr Gly Ser Phe His Ile Arg
385                 390                 395                 400
Arg Gly Glu Val Gly Ile Tyr Gln Val Gln Leu Arg Ala Leu Glu His

                405                 410                 415
Val Asp Gly Thr His Val Cys Gln Leu Pro Glu Asp Gln Lys Gly Asn
            420                 425                 430
Ile His Leu Lys Pro Ser Phe Ser Asp Gly Leu Lys Met Asp Ala Gly
        435                 440                 445
Ile Ile Cys Asp Val Cys Thr Cys Glu Leu Gln Lys Glu Val Arg Ser
    450                 455                 460
Ala Arg Cys Ser Phe Asn Gly Asp Phe Val Cys Gly Gln Cys Val Cys
465                 470                 475                 480
Ser Glu Gly Trp Ser Gly Gln Thr Cys Asn Cys Ser Thr Gly Ser Leu
                485                 490                 495
Ser Asp Ile Gln Pro Cys Leu Arg Glu Gly Glu Asp Lys Pro Cys Ser
            500                 505                 510
Gly Arg Gly Glu Cys Gln Cys Gly His Cys Val Cys Tyr Gly Glu Gly
        515                 520                 525
Arg Tyr Glu Gly Gln Phe Cys Glu Tyr Asp Asn Phe Gln Cys Pro Arg
    530                 535                 540
Thr Ser Gly Phe Leu Cys Asn Asp Arg Gly Arg Cys Ser Met Gly Gln
545                 550                 555                 560
Cys Val Cys Glu Pro Gly Trp Thr Gly Pro Ser Cys Asp Cys Pro Leu
                565                 570                 575
Ser Asn Ala Thr Cys Ile Asp Ser Asn Gly Gly Ile Cys Asn Gly Arg
            580                 585                 590
Gly His Cys Glu Cys Gly Arg Cys His Cys His Gln Gln Ser Leu Tyr
        595                 600                 605
Thr Asp Thr Ile Cys Glu Ile Asn Tyr Ser Ala Ser Thr Arg Ala Ser
    610                 615                 620
Ala Arg Thr Tyr Ala Pro Ala Cys Ser Ala Arg Arg Gly Ala Pro Ala
625                 630                 635                 640
Arg Arg Arg Gly Ala Arg Val Arg Asn Ala Thr Ser Arg Ser Arg Trp
                645                 650                 655
Trp Thr Ser Leu Arg Glu Ala Arg Arg Trp Trp Cys Ala Ala Pro Ser
            660                 665                 670
Gly Thr Arg Met Thr Thr Ala Pro Thr Ala Thr Pro Trp Lys Val Thr
        675                 680                 685
Ala Pro Leu Gly Pro Thr Ala Leu Ser Trp Cys Thr Arg Arg Arg Asp
    690                 695                 700
Cys Pro Pro Gly Ser Phe Trp Trp Leu Ile Pro Leu Leu Leu Leu Leu
705                 710                 715                 720
Leu Pro Leu Leu Ala Leu Leu Leu Leu Leu Cys Trp Lys Tyr Cys Ala
                725                 730                 735
Cys Cys Lys Ala Cys Leu Ala Leu Leu Pro Cys Cys Asn Arg Gly His
            740                 745                 750
Met Val Gly Phe Lys Glu Asp His Tyr Met Leu Arg Glu Asn Leu Met
        755                 760                 765
Ala Ser Asp His Leu Asp Thr Pro Met Leu Arg Ser Gly Asn Leu Lys
    770                 775                 780
Gly Arg Asp Val Val Arg Trp Lys Val Thr Asn Asn Met Gln Arg Pro
785                 790                 795                 800
Gly Phe Ala Thr His Ala Ala Ser Ile Asn Pro Thr Glu Leu Val Pro
                805                 810                 815
Tyr Gly Leu Ser Leu Arg Leu Ala Arg Leu Cys Thr Glu Asn Leu Leu
            820                 825                 830
Lys Pro Asp Thr Arg Glu Cys Ala Gln Leu Arg Gln Glu Val Glu Glu
        835                 840                 845
Asn Leu Asn Glu Val Tyr Arg Gln Ile Ser Gly Val His Lys Leu Gln
    850                 855                 860
Gln Thr Lys Phe Arg Gln Gln Pro Asn Ala Gly Lys Lys Gln Asp His
865                 870                 875                 880
Thr Ile Val Asp Thr Val Leu Met Ala Pro Arg Ser Ala Lys Pro Ala
                885                 890                 895
Leu Leu Lys Leu Thr Glu Lys Gln Val Glu Gln Arg Ala Phe His Asp
            900                 905                 910
Leu Lys Val Ala Pro Gly Tyr Tyr Thr Leu Thr Ala Asp Gln Asp Ala
        915                 920                 925
Arg Gly Met Val Glu Phe Gln Glu Gly Val Glu Leu Val Asp Val Arg
    930                 935                 940
Val Pro Leu Phe Ile Arg Pro Glu Asp Asp Asp Glu Lys Gln Leu Leu

945                 950                 955                 960
Val Glu Ala Ile Asp Val Pro Ala Gly Thr Ala Thr Leu Gly Arg Arg
                965                 970                 975
Leu Val Asn Ile Thr Ile Ile Lys Glu Gln Ala Arg Asp Val Val Ser
            980                 985                 990
Phe Glu Gln Pro Glu Phe Ser Val Ser Arg Gly Asp Gln Val Ala Arg
        995                 1000                1005
Ile Pro Val Ile Arg Arg Val Leu Asp Gly Gly Lys Ser Gln Val Ser
    1010                1015                1020
Tyr Arg Thr Gln Asp Gly Thr Ala Gln Gly Asn Arg Asp Tyr Ile Pro
1025                1030                1035                1040
Val Glu Gly Glu Leu Leu Phe Gln Pro Gly Glu Ala Trp Lys Glu Leu
                1045                1050                1055
Gln Val Lys Leu Leu Glu Leu Gln Glu Val Asp Ser Leu Leu Arg Gly
            1060                1065                1070
Arg Gln Val Arg Arg Phe His Val Gln Leu Ser Asn Pro Lys Phe Gly
        1075                1080                1085
Ala His Leu Gly Gln Pro His Ser Thr Thr Ile Ile Ile Arg Asp Pro
    1090                1095                1100
Asp Glu Leu Asp Arg Ser Phe Thr Ser Gln Met Leu Ser Ser Gln Pro
1105                1110                1115                1120
Pro Pro His Gly Asp Leu Gly Ala Pro Gln Asn Pro Asn Ala Lys Ala
                1125                1130                1135
Ala Gly Ser Arg Lys Ile His Phe Asn Trp Leu Pro Pro Ser Gly Lys
            1140                1145                1150
Pro Met Gly Tyr Arg Val Lys Tyr Trp Ile Gln Gly Asp Ser Glu Ser
        1155                1160                1165
Glu Ala His Leu Leu Asp Ser Lys Val Pro Ser Val Glu Leu Thr Asn
    1170                1175                1180
Leu Tyr Pro Tyr Cys Asp Tyr Glu Met Lys Val Cys Ala Tyr Gly Ala
1185                1190                1195                1200
Gln Gly Glu Gly Pro Tyr Ser Ser Leu Val Ser Cys Arg Thr His Gln
                1205                1210                1215
Glu Val Pro Ser Glu Pro Gly Arg Leu Ala Phe Asn Val Val Ser Ser
            1220                1225                1230
Thr Val Thr Gln Leu Ser Trp Ala Glu Pro Ala Glu Thr Asn Gly Glu
        1235                1240                1245
Ile Thr Ala Tyr Glu Val Cys Tyr Gly Leu Val Asn Asp Asp Asn Arg
    1250                1255                1260
Pro Ile Gly Pro Met Lys Lys Val Leu Val Asp Asn Pro Lys Asn Arg
1265                1270                1275                1280
Met Leu Leu Ile Glu Asn Leu Arg Glu Ser Gln Pro Tyr Arg Tyr Thr
                1285                1290                1295
Val Lys Ala Arg Asn Gly Ala Gly Trp Gly Pro Glu Arg Glu Ala Ile
            1300                1305                1310
Ile Asn Leu Ala Thr Gln Pro Lys Arg Pro Met Ser Ile Pro Ile Ile
        1315                1320                1325
Pro Asp Ile Pro Ile Val Asp Ala Gln Ser Gly Glu Asp Tyr Asp Ser
    1330                1335                1340
Phe Leu Met Tyr Ser Asp Asp Val Leu Arg Ser Pro Ser Gly Ser Gln
1345                1350                1355                1360
Arg Pro Ser Val Ser Asp Asp Thr Gly Cys Gly Trp Lys Phe Glu Pro
                1365                1370                1375
Leu Leu Gly Glu Glu Leu Asp Leu Arg Arg Val Thr Trp Arg Leu Pro
            1380                1385                1390
Pro Glu Leu Ile Pro Arg Leu Ser Ala Ser Ser Gly Arg Ser Ser Asp
        1395                1400                1405
Ala Glu Ala Pro His Gly Pro Pro Asp Asp Gly Gly Ala Gly Gly Lys
    1410                1415                1420
Gly Gly Ser Leu Pro Arg Ser Ala Thr Pro Gly Pro Pro Gly Glu His
1425                1430                1435                1440
Leu Val Asn Gly Arg Met Asp Phe Ala Phe Pro Gly Ser Thr Asn Ser
                1445                1450                1455
Leu His Arg Met Thr Thr Thr Ser Ala Ala Ala Tyr Gly Thr His Leu
            1460                1465                1470
Ser Pro His Val Pro His Arg Val Leu Ser Thr Ser Ser Thr Leu Thr
        1475                1480                1485
Arg Asp Tyr Asn Ser Leu Thr Arg Ser Glu His Ser His Ser Thr Thr

1490                1495                1500
Leu Pro Arg Asp Tyr Ser Thr Leu Thr Ser Val Ser Ser His Gly Leu
1505                1510                1515                1520
Pro Pro Ile Trp Glu His Gly Arg Ser Arg Leu Pro Leu Ser Trp Ala
                1525                1530                1535
Leu Gly Ser Arg Ser Arg Ala Gln Met Lys Gly Phe Pro Pro Ser Arg
            1540                1545                1550
Gly Pro Arg Asp Ser Ile Ile Leu Ala Gly Arg Pro Ala Ala Pro Ser
        1555                1560                1565
Trp Gly Pro Asp Ser Arg Leu Thr Ala Gly Val Pro Asp Thr Pro Thr
    1570                1575                1580
Arg Leu Val Phe Ser Ala Leu Gly Pro Thr Ser Leu Arg Val Ser Trp
1585                1590                1595                1600
Gln Glu Pro Arg Cys Glu Arg Pro Leu Gln Gly Tyr Ser Val Glu Tyr
                1605                1610                1615
Gln Leu Leu Asn Gly Gly Glu Leu His Arg Leu Asn Ile Pro Asn Pro
            1620                1625                1630
Ala Gln Thr Ser Val Val Val Glu Asp Leu Leu Pro Asn His Ser Tyr
        1635                1640                1645
Val Phe Arg Val Arg Ala Gln Ser Gln Glu Gly Trp Gly Arg Glu Arg
    1650                1655                1660
Glu Gly Val Ile Thr Ile Glu Ser Gln Val His Pro Gln Ser Pro Leu
1665                1670                1675                1680
Cys Pro Leu Pro Gly Ser Ala Phe Thr Leu Ser Thr Pro Ser Ala Pro
                1685                1690                1695
Gly Pro Leu Val Phe Thr Ala Leu Ser Pro Asp Ser Leu Gln Leu Ser
            1700                1705                1710
Trp Glu Arg Pro Arg Arg Pro Asn Gly Asp Ile Val Gly Tyr Leu Val
        1715                1720                1725
Thr Cys Glu Met Ala Gln Gly Gly Gly Pro Ala Thr Ala Phe Arg Val
    1730                1735                1740
Asp Gly Asp Ser Pro Glu Ser Arg Leu Thr Val Pro Gly Leu Ser Glu
1745                1750                1755                1760
Asn Val Pro Tyr Lys Phe Lys Val Gln Ala Arg Thr Thr Glu Gly Phe
                1765                1770                1775
Gly Pro Glu Arg Glu Gly Ile Ile Thr Ile Glu Ser Gln Asp Gly Gly
            1780                1785                1790
Pro Phe Pro Gln Leu Gly Ser Arg Ala Gly Leu Phe Gln His Pro Leu
        1795                1800                1805
Gln Ser Glu Tyr Ser Ser Ile Ser Thr Thr His Thr Ser Ala Thr Glu
    1810                1815                1820
Pro Phe Leu Val Gly Pro Thr Leu Gly Ala Gln His Leu Glu Ala Gly
1825                1830                1835                1840
Gly Ser Leu Thr Arg His Val Thr Gln Glu Phe Val Ser Arg Thr Leu
                1845                1850                1855
Thr Thr Ser Gly Thr Leu Ser Thr His Met Asp Gln Gln Phe Phe Gln
            1860                1865                1870
Thr


<210> 18
<211> 5622
<212> DNA
<213> Artificial Sequence

<220>
<223> Description of Artificial Sequence; Note =
      synthetic construct

<400> 18
   atggcagggc cacgccccag cccatgggcc aggctgctcc tggcagcctt gatcagcgtc      60
   agcctctctg ggaccttgaa ccgctgcaag aaggccccaa taaagagctg cacggagtgt     120
   gtccgtgtgg ataaggactg cgcctactgc acagacgaga tgttcaggga ccggcgctgc     180
   aacacccagg cggagctgct ggccgcgggc tgccagcggg agagcatcgt ggtcatggag     240
   agcagcttcc aaatcacaga ggagacccag attgacacca ccctgcggcg cagccagatg     300
   tccccccaag gcctgcgggt ccgtctgcgg cccggtgagg agcggcattt tgagctggag     360
   gtgtttgagc cactggagag ccccgtggac ctgtacatcc tcatggactt ctccaactcc     420
   atgtccgatg atctggacaa cctcaagaag atggggcaga acctggctcg ggtcctgagc     480
   cagctcacca gcgactacac tattggattt ggcaagtttg tggacaaagt cagcgtcccg 540
   cagacggaca tgaggcctga gaagctgaag gagccttggc ccaacagtga cccccccttc 600
   tccttcaaga acgtcatcag cctgacagaa gatgtggatg agttccggaa taaactgcag 660
   ggagagcgga tctcaggcaa cctggatgct cctgagggcg gcttcgatgc catcctgcag 720
   acagctgtgt gcacgaggga cattggctgg cgcccggaca gcacccacct gctggtcttc 780
   tccaccgagt cagccttcca ctatgaggct gatggcgcca acgtgctggc tggcatcatg 840
   agccgcaacg atgaacggtg ccacctggac accacgggca cctacaccca gtacaggaca 900
   caggactacc cgtcggtgcc caccctggtg cgcctgctcg ccaagcacaa catcatcccc 960
   atctttgctg tcaccaacta ctcctatagc tactacgaga agcttcacac ctatttccct 1020
   gtctcctcac tgggggtgct gcaggaggac tcgtccaaca tcgtggagct gctggaggag 1080
   gccttcaatc ggatccgctc caacctggac atccgggccc tagacagccc ccgaggcctt 1140
   cggacagagg tcacctccaa gatgttccag aagacgagga ctgggtcctt tcacatccgg 1200
   cggggggaag tgggtatata ccaggtgcag ctgcgggccc ttgagcacgt ggatgggacg 1260
   cacgtgtgcc agctgccgga ggaccagaag ggcaacatcc atctgaaacc ttccttctcc 1320
   gacggcctca agatggacgc gggcatcatc tgtgatgtgt gcacctgcga gctgcaaaaa 1380
   gaggtgcggt cagctcgctg cagcttcaac ggagacttcg tgtgcggaca gtgtgtgtgc 1440
   agcgagggct ggagtggcca gacctgcaac tgctccaccg gctctctgag tgacattcag 1500
   ccctgcctgc gggagggcga ggacaagccg tgctccggcc gtggggagtg ccagtgcggg 1560
   cactgtgtgt gctacggcga aggccgctac gagggtcagt tctgcgagta tgacaacttc 1620
   cagtgtcccc gcacttccgg gttcctctgc aatgaccgag gacgctgctc catgggccag 1680
   tgtgtgtgtg agcctggttg gacaggccca agctgtgact gtcccctcag caatgccacc 1740
   tgcatcgaca gcaatggggg catctgtaat ggacgtggcc actgtgagtg tggccgctgc 1800
   cactgccacc agcagtcgct ctacacggac accatctgcg agatcaacta ctcggcgtcc 1860
   acccgggcct ctgcgaggac ctacgctcct gcgtgcagtg ccaggcgtgg ggcaccggcg 1920
   agaagaaggg gcgcacgtgt gaggaatgca acttcaaggt caagatggtg gacgagctta 1980
   agagaggcga ggaggtggtg gtgcgctgct ccttccggga cgaggatgac gactgcacct 2040
   acagctacac catggaaggt gacggcgccc ctgggcccaa cagcactgtc ctggtgcaca 2100
   agaagaaggg actgccctcc gggctccttc tggtggctca tccccctgct cctcctcctc 2160
   ctgccgctcc tggccctgct actgctgcta tgctggaagt actgtgcctg ctgcaaggcc 2220
   tgcctggcac ttctcccgtg ctgcaaccga ggtcacatgg tgggctttaa ggaagaccac 2280
   tacatgctgc gggagaacct gatggcctct gaccacttgg acacgcccat gctgcgcagc 2340
   gggaacctca agggccgtga cgtggtccgc tggaaggtca ccaacaacat gcagcggcct 2400
   ggctttgcca ctcatgccgc cagcatcaac cccacagagc tggtgcccta cgggctgtcc 2460
   ttgcgcctgg cccgcctttg caccgagaac ctgctgaagc ctgacactcg ggagtgcgcc 2520
   cagctgcgcc aggaggtgga ggagaacctg aacgaggtct acaggcagat ctccggtgta 2580
   cacaagctcc agcagaccaa gttccggcag cagcccaatg ccgggaaaaa gcaagaccac 2640
   accattgtgg acacagtgct gatggcgccc cgctcggcca agccggccct gctgaagctt 2700
   acagagaagc aggtggaaca gagggccttc cacgacctca aggtggcccc cggctactac 2760
   accctcactg cagaccagga cgcccggggc atggtggagt tccaggaggg cgtggagctg 2820
   gtggacgtac gggtgcccct ctttatccgg cctgaggatg acgacgagaa gcagctgctg 2880
   gtggaggcca tcgacgtgcc cgcaggcact gccaccctcg gccgccgcct ggtaaacatc 2940
   accatcatca aggagcaagc cagagacgtg gtgtcctttg agcagcctga gttctcggtc 3000
   agccgcgggg accaggtggc ccgcatccct gtcatccggc gtgtcctgga cggcgggaag 3060
   tcccaggtct cctaccgcac acaggatggc accgcgcagg gcaaccggga ctacatcccc 3120
   gtggagggtg agctgctgtt ccagcctggg gaggcctgga aagagctgca ggtgaagctc 3180
   ctggagctgc aagaagttga ctccctcctg cggggccgcc aggtccgccg tttccacgtc 3240
   cagctcagca accctaagtt tggggcccac ctgggccagc cccactccac caccatcatc 3300
   atcagggacc cagatgaact ggaccggagc ttcacgagtc agatgttgtc atcacagcca 3360
   ccccctcacg gcgacctggg cgccccgcag aaccccaatg ctaaggccgc tgggtccagg 3420
   aagatccatt tcaactggct gcccccttct ggcaagccaa tggggtacag ggtaaagtac 3480
   tggattcagg gtgactccga atccgaagcc cacctgctcg acagcaaggt gccctcagtg 3540
   gagctcacca acctgtaccc gtattgcgac tatgagatga aggtgtgcgc ctacggggct 3600
   cagggcgagg gaccctacag ctccctggtg tcctgccgca cccaccagga agtgcccagc 3660
   gagccagggc gtctggcctt caatgtcgtc tcctccacgg tgacccagct gagctgggct 3720
   gagccggctg agaccaacgg tgagatcaca gcctacgagg tctgctatgg cctggtcaac 3780
   gatgacaacc gacctattgg gcccatgaag aaagtgctgg ttgacaaccc taagaaccgg 3840
   atgctgctta ttgagaacct tcgggagtcc cagccctacc gctacacggt gaaggcgcgc 3900
   aacggggccg gctgggggcc tgagcgggag gccatcatca acctggccac ccagcccaag 3960
   aggcccatgt ccatccccat catccctgac atccctatcg tggacgccca gagcggggag 4020
   gactacgaca gcttccttat gtacagcgat gacgttctac gctctccatc gggcagccag 4080
   aggcccagcg tctccgatga cactggctgc ggctggaagt tcgagcccct gctgggggag 4140
   gagctggacc tgcggcgcgt cacgtggcgg ctgcccccgg agctcatccc gcgcctgtcg 4200
   gccagcagcg ggcgctcctc cgacgccgag gcgccccacg ggcccccgga cgacggcggc 4260
   gcgggcggga agggcggcag cctgccccgc agtgcgacac ccgggccccc cggagagcac 4320
   ctggtgaatg gccggatgga ctttgccttc ccgggcagca ccaactccct gcacaggatg 4380
   accacgacca gtgctgctgc ctatggcacc cacctgagcc cacacgtgcc ccaccgcgtg 4440
   ctaagcacat cctccaccct cacacgggac tacaactcac tgacccgctc agaacactca 4500
   cactcgacca cactgccgag ggactactcc accctcacct ccgtctcctc ccacggcctc 4560
   cctcccatct gggaacacgg gaggagcagg cttccgctgt cctgggccct ggggtcccgg 4620
   agtcgggctc agatgaaagg gttcccccct tccaggggcc cacgagactc tataatcctg 4680
   gctgggaggc cagcagcgcc ctcctggggc ccagactctc gcctgactgc tggtgtgccc 4740
   gacacgccca cccgcctggt gttctctgcc ctggggccca catctctcag agtgagctgg 4800
   caggagccgc ggtgcgagcg gccgctgcag ggctacagtg tggagtacca gctgctgaac 4860
   ggcggtgagc tgcatcggct caacatcccc aaccctgccc agacctcggt ggtggtggaa 4920
   gacctcctgc ccaaccactc ctacgtgttc cgcgtgcggg cccagagcca ggaaggctgg 4980
   ggccgagagc gtgagggtgt catcaccatt gaatcccagg tgcacccgca gagcccactg 5040
   tgtcccctgc caggctccgc cttcactttg agcactccca gtgccccagg cccgctggtg 5100
   ttcactgccc tgagcccaga ctcgctgcag ctgagctggg agcggccacg gaggcccaat 5160
   ggggatatcg tcggctacct ggtgacctgt gagatggccc aaggaggagg tccagccacc 5220
   gcattccggg tggatggaga cagccccgag agccggctga ccgtgccggg cctcagcgag 5280
   aacgtgccct acaagttcaa ggtgcaggcc aggaccactg agggcttcgg gccagagcgc 5340
   gagggcatca tcaccataga gtcccaggat ggaggtccct tcccgcagct gggcagccgt 5400
   gccgggctct tccagcaccc gctgcaaagc gagtacagca gcatctccac cacccacacc 5460
   agcgccaccg agcccttcct agtgggtccg accctggggg cccagcacct ggaggcaggc 5520
   ggctccctca cccggcatgt gacccaggag tttgtgagcc ggacactgac caccagcgga 5580
   acccttagca cccacatgga ccaacagttc ttccaaactt ga 5622

<210> 19
<211> 5622
<212> DNA
<213> Artificial Sequence

<220>
<223> Description of Artificial Sequence; Note =
      synthetic construct

<400> 19
atggcagggc cacggcccag cccatgggcc aggctgctcc tggcagcctt gatcagcgtc 60
agcctctctg ggaccttgaa ccgctgcaag aaggccccaa taaagagctg cacggagtgt 120
gtccgtgtgg ataaggactg cgcctactgc acagacgaga tgttcaggga ccggcgctgc 180
aacacccagg cggagctgct ggccgcgggc tgccagcggg agagcatcgt ggtcatggag 240
agcagcttcc aaatcacaga ggagacccag attgacacca ccctgcggcg cagccagatg 300
tccccccaag gcctgcgggt ccgtctgcgg cccggtgagg agcggcattt tgagctggag 360
gtgtttgagc cactggagag ccccgtggac ctgtacatcc tcatggactt ctccaactcc 420
atgtccgatg atctggacaa cctcaagaag atggggcaga acctggctcg ggtcctgagc 480
cagctcacca gcgactacac tattggattt ggcaagtttg tggacaaagt cagcgtcccg 540
cagacggaca tgaggcctga gaagctgaag gagccttggc ccaacagtga cccccccttc 600
tccttcaaga acgtcatcag cctgacagaa gatgtggatg agttccggaa taaactgcag 660
ggagagcgga tctcaggcaa cctggatgct cctgagggcg gcttcgatgc catcctgcag 720
acagctgtgt gcacgaggga cattggctgg cgcccggaca gcacccacct gctggtcttc 780
tccaccgagt cagccttcca ctatgaggct gatggcgcca acgtgctggc tggcatcatg 840
agccgcaacg atgaacggtg ccacctggac accacgggca cctacaccca gtacaggaca 900
caggactacc cgtcggtgcc caccctggtg cgcctgctcg ccaagcacaa catcatcccc 960
atctttgctg tcaccaacta ctcctatagc tactacgaga agcttcacac ctatttccct 1020
gtctcctcac tgggggtgct gcaggaggac tcgtccaaca tcgtggagct gctggaggag 1080
gccttcaatc ggatccgctc caacctggac atccgggccc tagacagccc ccgaggcctt 1140
cggacagagg tcacctccaa gatgttccag aagacgagga ctgggtcctt tcacatccgg 1200
cggggggaag tgggtatata ccaggtgcag ctgcgggccc ttgagcacgt ggatgggacg 1260
cacgtgtgcc agctgccgga ggaccagaag ggcaacatcc atctgaaacc ttccttctcc 1320
gacggcctca agatggacgc gggcatcatc tgtgatgtgt gcacctgcga gctgcaaaaa 1380
gaggtgcggt cagctcgctg cagcttcaac ggagacttcg tgtgcggaca gtgtgtgtgc 1440
agcgagggct ggagtggcca gacctgcaac tgctccaccg gctctctgag tgacattcag 1500
ccctgcctgc gggagggcga ggacaagccg tgctccggcc gtggggagtg ccagtgcggg 1560
cactgtgtgt gctacggcga aggccgctac gagggtcagt tctgcgagta tgacaacttc 1620
cagtgtcccc gcacttccgg gttcctctgc aatgaccgag gacgctgctc catgggccag 1680
tgtgtgtgtg agcctggttg gacaggccca agctgtgact gtcccctcag caatgccacc 1740
tgcatcgaca gcaatggggg catctgtaat ggacgtggcc actgtgagtg tggccgctgc 1800
cactgccacc agcagtcgct ctacacggac accatctgcg agatcaacta ctcggcgtcc 1860
acccgggcct ctgcgaggac ctacgctcct gcgtgcagtg ccaggcgtgg ggcaccggcg 1920
agaagaaggg gcgcacgtgt gaggaatgca acttcaaggt caagatggtg gacgagctta 1980
agagaggcga ggaggtggtg gtgcgctgct ccttccggga cgaggatgac gactgcacct 2040
acagctacac catggaaggt gacggcgccc ctgggcccaa cagcactgtc ctggtgcaca 2100
agaagaaggg actgccctcc gggctccttc tggtggctca tccccctgct cctcctcctc 2160
ctgccgctcc tggccctgct actgctgcta tgctggaagt actgtgcctg ctgcaaggcc 2220

tgcctggcac ttctcccgtg ctgcaaccga ggtcacatgg tgggctttaa ggaagaccac 2280
tacatgctgc gggagaacct gatggcctct gaccacttgg acacgcccat gctgcgcagc 2340
gggaacctca agggccgtga cgtggtccgc tggaaggtca ccaacaacat gcagcggcct 2400
ggctttgcca ctcatgccgc cagcatcaac cccacagagc tggtgcccta cgggctgtcc 2460
ttgcgcctgg cccgcctttg caccgagaac ctgctgaagc ctgacactcg ggagtgcgcc 2520
cagctgcgcc aggaggtgga ggagaacctg aacgaggtct acaggcagat ctccggtgta 2580
cacaagctcc agcagaccaa gttccggcag cagcccaatg ccgggaaaaa gcaagaccac 2640
accattgtgg acacagtgct gatggcgccc cgctcggcca agccggccct gctgaagctt 2700
acagagaagc aggtggaaca gagggccttc cacgacctca aggtggcccc cggctactac 2760
accctcactg cagaccagga cgcccggggc atggtggagt tccaggaggg cgtggagctg 2820
gtggacgtac gggtgcccct ctttatccgg cctgaggatg acgacgagaa gcagctgctg 2880
gtggaggcca tcgacgtgcc cgcaggcact gccaccctcg gccgccgcct ggtaaacatc 2940
accatcatca aggagcaagc cagagacgtg gtgtcctttg agcagcctga gttctcggtc 3000
agccgcgggg accaggtggc ccgcatccct gtcatccggc gtgtcctgga cggcgggaag 3060
tcccaggtct cctaccgcac acaggatggc accgcgcagg gcaaccggga ctacatcccc 3120
gtggagggtg agctgctgtt ccagcctggg gaggcctgga aagagctgca ggtgaagctc 3180
ctggagctgc aagaagttga ctccctcctg cggggccgcc aggtccgccg tttccacgtc 3240
cagctcagca accctaagtt tggggcccac ctgggccagc cccactccac caccatcatc 3300
atcagggacc cagatgaact ggaccggagc ttcacgagtc agatgttgtc atcacagcca 3360
ccccctcacg gcgacctggg cgccccgcag aaccccaatg ctaaggccgc tgggtccagg 3420
aagatccatt tcaactggct gcccccttct ggcaagccaa tggggtacag ggtaaagtac 3480
tggattcagg gtgactccga atccgaagcc cacctgctcg acagcaaggt gccctcagtg 3540
gagctcacca acctgtaccc gtattgcgac tatgagatga aggtgtgcgc ctacggggct 3600
cagggcgagg gaccctacag ctccctggtg tcctgccgca cccaccagga agtgcccagc 3660
gagccagggc gtctggcctt caatgtcgtc tcctccacgg tgacccagct gagctgggct 3720
gagccggctg agaccaacgg tgagatcaca gcctacgagg tctgctatgg cctggtcaac 3780
gatgacaacc gacctattgg gcccatgaag aaagtgctgg ttgacaaccc taagaaccgg 3840
atgctgctta ttgagaacct tcgggagtcc cagccctacc gctacacggt gaaggcgcgc 3900
aacggggccg gctgggggcc tgagcgggag gccatcatca acctggccac ccagcccaag 3960
aggcccatgt ccatccccat catccctgac atccctatcg tggacgccca gagcggggag 4020
gactacgaca gcttccttat gtacagcgat gacgttctac gctctccatc gggcagccag 4080
aggcccagcg tctccgatga cactggctgc ggctggaagt tcgagcccct gctgggggag 4140
gagctggacc tgcggcgcgt cacgtggcgg ctgcccccgg agctcatccc gcgcctgtcg 4200
gccagcagcg ggcgctcctc cgacgccgag gcgccccacg ggcccccgga cgacggcggc 4260
gcgggcggga agggcggcag cctgccccgc agtgcgacac ccgggccccc cggagagcac 4320
ctggtgaatg gccggatgga ctttgccttc ccgggcagca ccaactccct gcacaggatg 4380
accacgacca gtgctgctgc ctatggcacc cacctgagcc cacacgtgcc ccaccgcgtg 4440
ctaagcacat cctccaccct cacacgggac tacaactcac tgacccgctc agaacactca 4500
cactcgacca cactgccgag ggactactcc accctcacct ccgtctcctc ccacggcctc 4560
cctcccatct gggaacacgg gaggagcagg cttccgctgt cctgggccct ggggtcccgg 4620
agtcgggctc agatgaaagg gttccccct tccaggggcc cacgagactc tataatcctg 4680
gctgggaggc cagcagcgcc ctcctggggc ccagactctc gcctgactgc tggtgtgccc 4740
gacacgccca cccgcctggt gttctctgcc ctggggccca catctctcag agtgagctgg 4800
caggagccgc ggtgcgagcg gccgctgcag ggctacagtg tggagtacca gctgctgaac 4860
ggcggtgagc tgcatcggct caacatcccc aaccctgccc agacctcggt ggtggtggaa 4920
gacctcctgc ccaaccactc ctacgtgttc cgcgtgcggg cccagagcca ggaaggctgg 4980
ggccgagagc gtgagggtgt catcaccatt gaatcccagg tgcacccgca gagcccactg 5040
tgtcccctgc caggctccgc cttcactttg agcactccca gtgccccagg cccgctggtg 5100
ttcactgccc tgagcccaga ctcgctgcag ctgagctggg agcggccacg gaggcccaat 5160
ggggatatcg tcggctacct ggtgacctgt gagatggccc aaggaggagg tccagccacc 5220
gcattccggg tggatggaga cagccccgag agccggctga ccgtgccggg cctcagcgag 5280
aacgtgccct acaagttcaa ggtgcaggcc aggaccactg agggcttcgg gccagagcgc 5340
gagggcatca tcaccataga gtcccaggat ggaggtccct tcccgcagct gggcagccgt 5400
gccgggctct tccagcaccc gctgcaaagc gagtacagca gcatctccac cacccacacc 5460
agcgccaccg agcccttcct agtgggtccg accctggggg cccagcacct ggaggcaggc 5520
ggctccctca cccggcatgt gacccaggag tttgtgagcc ggacactgac caccagcgga 5580
acccttagca cccacatgga ccaacagttc ttccaaactt ga 5622